(19) Europäisches Patentamt
European Patent Office
Office européen des brevets

(11) Publication number: **0 653 438 A2**

# (12) EUROPEAN PATENT APPLICATION

(21) Application number: **94112253.3**

(51) Int. Cl.[6]: **C07H 21/00**, A61K 31/70

(22) Date of filing: **05.08.94**

The applicant has subsequently filed a sequence listing and declared, that it includes no new matter.

(30) Priority: **06.08.93 JP 196327/93**

(43) Date of publication of application: **17.05.95 Bulletin 95/20**

(84) Designated Contracting States: **AT BE CH DE DK ES FR GB GR IE IT LI LU NL PT SE**

(71) Applicant: **TAKEDA CHEMICAL INDUSTRIES, LTD.**
**1-1, Doshomachi 4-chome**
**Chuo-ku,**
**Osaka 541 (JP)**

(72) Inventor: **Hiroshi, Tanimura**
**14-13-303, Takezono 2-chome**
**Tsukuba,**
**Ibaraki 305 (JP)**
Inventor: **Yoji, Hayase**
**12-1-403, Matsushiro 3-chome**
**Tsukuba,**
**Ibaraki 305 (JP)**
Inventor: **Takehiko, Naka**
**4-15-711, Kamokogahara 1-chome,**
**Higashinada-ku**
**Kobe,**
**Hyogo 658 (JP)**
Inventor: **Susumu, Iwasa**
**21-2, Ohsumigaoka 1-chome,**
**Tanabe-cho**
**Tsuzuki-gun,**
**Kyoto 610-03 (JP)**

(74) Representative: **von Kreisler, Alek, Dipl.-Chem. et al**
**Patentanwälte**
**von Kreisler-Selting-Werner**
**Bahnhofsvorplatz 1 (Deichmannhaus)**
**D-50667 Köln (DE)**

## (54) Oligonucleotide compounds, their production and use.

(57) An oligonucleotide compound or a salt thereof, which has a nucleotide sequence represented by formula (I):

EP 0 653 438 A2

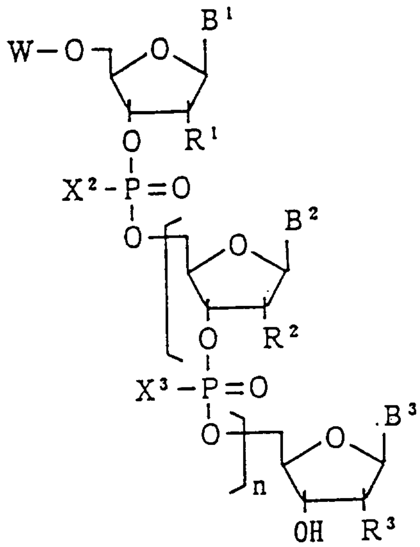

(I)

wherein W represents hydrogen or a protective group; $B^1$, $B^2$ and $B^3$ each represent nucleic acid residues, wherein each $B^2$ may be the same or different when n is 2 or more; $X^1$, $X^2$ and $X^3$ each represent OH, SH, $C_{1-5}$ alkyl, $C_{1-5}$ alkoxy, $C_{1-5}$ monoalkylamino or an aromatic ring group which may be substituted or unsubstituted, wherein each $X^3$ may be the same or different when n is 2 or more; $R^1$, $R^2$ and $R^3$ each represent hydrogen, OH, halogen, $C_{1-5}$ alkoxy or $C_{1-5}$ alkoxyalkyloxy, wherein each $R^2$ may be the same or different when n is 2 or more; and n represents an integer from 1 to 98 is disclosed.

The oligonucleotides (I) of the present invention or the salts thereof can (1) inhibit expression of genes associated with the development of malignant tumors in malignant tumor cells, (2) inhibit expression of viral genes derived from viruses, (3) inhibit expression of genes producing proteins which contribute to the development of inflammations, (4) control expression of drug-resistant genes which cause complications in chemotherapy of malignant tumors, and (5) inhibit production of cell growth factors related to reangiostenosis after PCTA (percutaneous transluminal coronary angioplasty) by complementation to DNAs or mRNAs of genes inducing various diseases. They can be therefore used as antitumor agents, antiviral agents, antiinflammatory agents and drugs for controlling expression of specific genes such as resistance genes. These oligonucleotides can also be used as probes. Further, these oligonucleotides can be used in novel drug screening assays.

## FIELD OF THE INVENTION

The present invention relates to oligonucleotide compounds or salts thereof which have the capability to control activation or exression of specified genes; these compounds and salts thereof can (a) inhibit expression of genes associated with the development of malignant tumors in malignant tumor cells, (b) inhibit expression of viral genes derived from viruses in mammalian, (c) inhibit expression of genes encoding proteins that contribute to the development of inflammations, (d) inhibit expression of drug-resistant genes, thereby enhancing the chemotherapy treatment by reducing one of the problems often associated with chemotherapy, or (e) inhibit production of cell growth factors related to reangiostenosis after PTCA (percutaneous transluminal coronary angioplasty); the production of the oligonucleotide compounds or salts thereof. These compounds and salts thereof can be used in in vitro drug screening assays to screen for a variety of compounds; they also are useful in compositions containing the oligonucleotide compounds or the salt thereof which inhibit expression of specified genes such as genes contributing to the development of malignant tumors, viral diseases or inflammations. These compounds can also be used as probes in screening assays.

## PRIOR ART

Many diseases are known to be associated with gene products produced as a result of expression of specific genes. Examples thereof include the followings:
Viral genes or tumor associated genes such as oncogenes. These pathogenic genes can be roughly divided into two kinds, genes existing in cells (e.g. conversion of a protooncogene to an oncogene) and genes called extraneous genes resulting from infection with extracellular viruses. Recent investigation has revealed that expression of these genes is one factor associated with the development of viral diseases or in the development of abnormal growth of normal cells. Further, adhesive proteins produced by certain genes such as ICAM-1 contribute to the development of inflammations by expression of the genes such as ICAM-1. Furthermore, although expression of drug-resistant genes is not directly the cause of disease, their expression can be considered to be the indirect cause thereof or can be considered an agent in incresing the severity of such diseases, because organisms that acquire drug resistance against antitumor agents on chemotherapy (e.g. for malignant tumors), cause difficulty in obtaining a complete cure. Accordingly, drugs which control activation or expression of the genes contributing or otherwise associated with the development of these diseases are expected to be useful as antiviral compositions, antitumor compositions and antiinflammatory compositions. These compounds can also be used to screen for drugs affecting other factors associated with these diseases. These compounds also can be used as probes in e.g. hybridization screens.

Techniques for controlling expression of genes by the use of oligonucleotide compounds having sequences in regions characteristic of the genes or sequences complementary to mRNAs transcribed from the genes, namely antisense methods have been discussed [G. Zon, Pharmaceutical Res., vol. 5, No. 9, 539 (1988); C. A. Stein et al., Cancer Res., vol. 48, 2659 (1988); E. Uhlman et al., Chemical Reviews, vol. 90, No. 4, 543 (1990); J. Goodchild, Bioconjugate Chem., vol. 1, No. 3, 165 (1990)]. In particular, attempts to control expression of viral genes and tumor genes using these antisense oligonucleotides have been studied. However, the use of natural type antisense oligonucleotides has generally proven unsatisfactory because they are easily decomposed with hydrolases such as nuclease. In attempts to increase stability against these hydrolases, various oligonucleotide compounds chemically modified have been proposed. For example, nucleotides in which phosphoric acid groups are modified include phosphorothioates [F. Eckstein, Angew. Chem., vol. 6, 431 (1983); F. Eckstein et al., Biochemistry, vol. 23, 3443 (1984); J. W. Stec et al., J. Am. Chem. Soc., vol. 106, 6077 (1984); and F. Eckstein et al., Annu. Rev. Biochem., vol. 54, 367 (1985)], methylphosphonates [P. S. Millar et al., Biochemistry, vol. 18, 5134 (1979); P. S. Millar et al., Biochimie, vol. 67, 769 (1985); and P. O. P. Ts'O et al., Ann. N. Y. Acad. Sci., vol. 507, 220 (1988)] and phosphorodithioates [M. H. Caruthers et al., Tetrahedron Lett., vol. 29, 2911 (1988); and M. H. Caruthers et al., J. Am. Chem. Soc., vol 111, 2321 (1989)]. Alternatively, in an attempt to enhance the stability by modifying the 2'-position of a ribose ring which is a constituent unit, the 2'-saccharide hydroxyl group has been methylated [Y. Furukawa et al., Chem. Pharm. Bull., vol. 13, 1273 (1965); and E. Ohtsuka et al., Nucleic Acids Res., vol. 15, 6131 (1987)]. Further, in an attempt to enhance the cell membrane permeability, derivatives have also been synthesized in which cholesterol is added to terminus of such modified nucleotides [R. L. Letsinger et al., Proc. Natl. Acad. Sci. U.S.A., vol. 86, 6553 (1989)] or poly(L-lysine) [M. Lemaitre et al., Proc. Natl. Acad. Sci. U.S.A., vol. 84, 648 (1987)]. Furthermore, an attempt has also been made to introduce antisense molecules into cells by encapsulation with liposomes [P. L. Felgner et al.,

Proc. Natl. Acad. Sci. U.S.A., vol. 84, 7143 (1987)].

Although methylphosphonates might be expected to enhance the stability against hydrolases, chemical stability and membrane permeability, because they become neutral molecules. They have the disadvantage that the solubility to aqueous solutions is extremely deteriorated. As to the phosphorodithioates, the chirality of the phosphoric acid groups disappears, but considerable distortion is applied, compared with the natural type oligonucleotides, because each of the phosphoric acid groups has two sulfur atoms, resulting in deterioration of double strand forming ability as antisnese. Further, the synthesis thereof is difficult compared with other compounds, so that they present a problem when synthesis in large amounts is desired. For the attempt to introduce poly(L-lysine), considerably strong cytotoxicity is generated [J. P. Leonetti et al, Gene, vol. 72, 323 (1988)], resulting in no practical modification reaction to antisense.

At present, taking into account the importance of an antisense effect, stability being shown against hydrolases and low cytotoxicity, the phosphorothioates are preferably used. However, the phosphorothioates are poor in double strand forming ability, because of the chirality developed in the phosphoric acid groups, and they further have the problem that the desired sulfurized materials must be isolated from oxides contained in slight amounts for purification in synthesis. Furthermore, the phosphorothioates suffer from the problems of low cell membrane permeability and low nuclear transition. They are also to raise believed problems with regard to drug effects when developed as antitumor compositions, antiviral compositions, antiinflammatory compositions, etc. and the persistency thereof. Thus, it would be useful to find new compounds that can be used in antisense methodology but which do not have all the problems of these other compounds. It would also be useful to have better compounds to use as probes for nucleic acids such as mRNA or DNA that have increase stability or better binding characteristics.

## SUMMARY OF THE INVENTION

An object of the present invention is to provide a novel oligonucleotide compound exhibiting resistance against hydrolases, having a chemically stable structure and having excellent cell membrane permeability, whereby a superior antisense effect can be exhibited.

Another object of the present invention is to provide a method for producing said oligonucleotide compound or salt thereof.

A further object of the present invention is to provide a phosphorylated saccharide compound, a branched saccharide compound and a nucleotide compound useful as an intermediate for producing said oligonucleotide compound.

A still further object of the present invention is to provide an antitumor composition, an antiviral composition, an antiinflammatory composition, etc. containing said oligonucleotide compound.

Yet another object of the present invention is to be able to use these compounds in screening for other useful compounds. Another object is a compound that can be used as a probe for target nucleic acid sequences.

As a result of intensive investigation for solving the above-mentioned problems, the present inventors have succeeded in synthesizing a oligonucleotide compounds and a salt thereof which has a nucleotide sequence represented by formula (I):

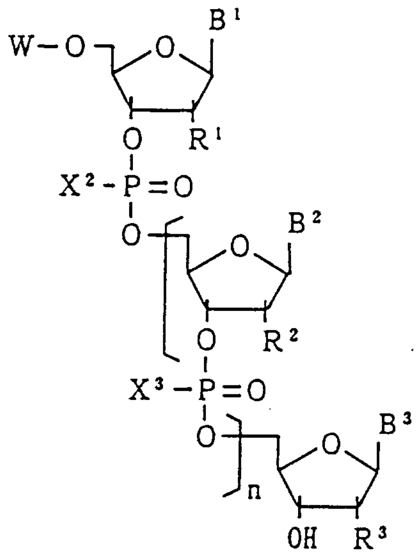

( I )

wherein W represents hydrogen or a protective group; $B^1$, $B^2$ and $B^3$ each represent nucleic acid residues, wherein each $B^2$ may be the same or different when n is 2 or more; $X^2$ and $X^3$ each represent OH, SH, $C_{1-5}$ alkyl, $C_{1-5}$ alkoxy, $C_{1-5}$ monoalkylamino or an aromatic ring group which may be substituted or unsubstituted, wherein each $X^3$ may be the same or different when n is 2 or more; $R^1$, $R^2$ and $R^3$ each represent hydrogen, OH, halogen, $C_{1-5}$ alkoxy or $C_{1-5}$ alkoxyalkyloxy, wherein each $R^2$ may be the same or different when n is 2 or more; and n represents an integer from 1 to 98;
with the proviso that (1) W represents hydrogen or a protective group when $X^2$ and $X^3$ each represent OH, SH, $C_{1-5}$ alkyl, $C_{1-5}$ alkoxy, $C_{1-5}$ monoalkylamino or an aromatic ring group which may be substituted or unsubstituted, and at least one of $X^2$ and $X^3$ is an aromatic ring group which may be substituted or unsubstituted; or
(2) W is represented by formula

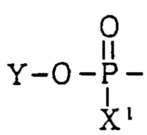

or

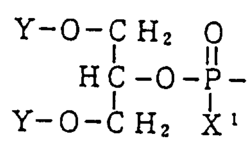

in which Y represents a saccharide residue; and $X^1$ represents OH, SH, $C_{1-5}$ alkyl, $C_{1-5}$ alkoxy, $C_{1-5}$ monoalkylamino or an aromatic ring group which may be substituted or unsubstituted when $X^2$ and $X^3$ each represent OH, SH, $C_{1-5}$ alkyl, $C_{1-5}$ alkoxy or $C_{1-5}$ monoalkylamino.

More specifically, the present inventors have succeeded in synthesizing an oligonucleotide compound and a salt thereof which has a nucleotide sequence represented by formula (Ia):

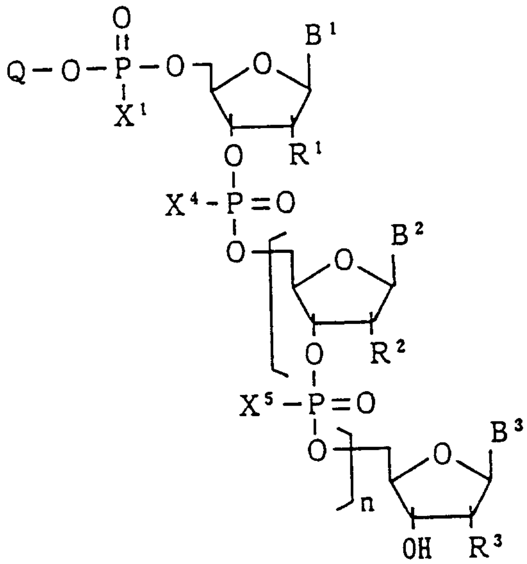

(I a)

in which Q represents Y or the formula

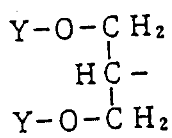

wherein, Y represents a saccharide residue; $X^1$ represents OH, SH, $C_{1-5}$ alkyl, $C_{1-5}$ alkoxy, $C_{1-5}$ monoalkylamino or an aromatic ring group which may be substituted or unsubstituted, $B^1$, $B^2$ and $B^3$ each represent nucleic acid residues, wherein each $B^2$ may be the same or different when n is 2 or more; $X^4$ and $X^5$ each represent OH, SH, $C_{1-5}$ alkyl, $C_{1-5}$ alkoxy or $C_{1-5}$ monoalkylamino, wherein each $X^5$ may be the same or different when n is 2 or more; $R^1$, $R^2$ and $R^3$ each represent hydrogen, OH, halogen, $C_{1-5}$ alkoxy or $C_{1-5}$ alkoxyalkyloxy, wherein each $R^2$ may be the same or different when n is 2 or more; and n represents an integer from 1 to 98;
and an oligonucleotide compound and a salt thereof which has a nucleotide sequence represented by formula (Ib):

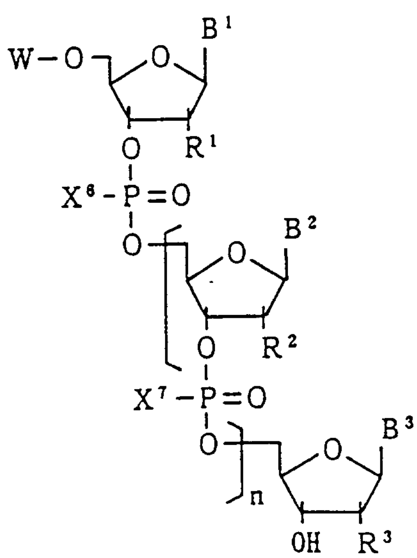

(I b)

wherein W represents hydrogen or a protective group; $B^1$, $B^2$ and $B^3$ each represent nucleic acid residues, wherein each $B^2$ may be the same or different when n is 2 or more; $X^6$ and $X^7$ each represent OH, SH, $C_{1-5}$ alkyl, $C_{1-5}$ alkoxy, $C_{1-5}$ monoalkylamino or an aromatic ring group which may be substituted or unsubstituted, wherein each $X^7$ may be the same or different when n is 2 or more, at least one of $X^6$ and $X^7$ represents an aromatic ring group which may be substituted or unsubstituted; $R^1$, $R^2$ and $R^3$ each represent hydrogen, OH, halogen, $C_{1-5}$ alkoxy or $C_{1-5}$ alkoxyalkyloxy, wherein each $R^2$ may be the same or different when n is 2 or more; and n represents an integer from 1 to 98.

Further, the present inventors have discovered that the oligonucleotide compound of formula (Ia) unexpectedly exhibits the resistance against hydrolases, and is also chemically stabilized by substituting the 2'-saccharide hydroxyl group by a halogen atom or alkylating or alkoxyalkylating the 2'-saccharide hydroxyl group.

Furthermore, the present inventors have discovered that said oligonucleotide compound has an enhanced complementary strand forming ability to a target DNA or RNA because the ribose ring thereof can take a 3'-end conformation.

Moreover, the present inventors have discovered that the following various advantages can be obtained by introducing various saccharide compounds through the 5'-terminal phosphoric acid groups of antisense molecules:

(1) The antisense molecules can be introduced into cells, utilizing receptors recognizing saccharide compounds such as glucose receptors, galactose receptors and mannose receptors occurring in vivo, by the use of chemically synthesized branched oligosaccharides to take into account the cluster effect which is a characteristic of the saccharide receptors;

(2) The affinity of antisense nucleotides for cell membranes can be enhanced by introducing substituent groups that increase hydrophobicity into the hydroxyl groups of the saccharide compounds; and

(3) For neutral antisense nucleotides such as methylphosphonates having the saccharide compounds in polyhydroxyl form bring about the effect of enhancing solubility to aqueous solutions, which results in enhancement of the effectiveness of these antisense molecules.

Meanwhile, the oligonucleotide compounds (Ib) are charaterized in that they have at least one phenylphosphonate type nucleotide in the molecule, preferabley at the 5'-terminus, more preferably two phenylphosphonate type nucleotides in the molecule, preferabley at the 5'- and 3'-terminus. The present inventors have discovered that this type of oligonucleotide compounds has superior gene-expression inhibiting activity regardless of the presence of a saccharide derivative as a phosphoric acid at the 5'-terminus. Namely, by introducing a phenyl group into a phosphoric acid group to make a phosphonate bond, the following advantages will be obtained:

(1) resistance against hydrolases can be increased, (2) the affinity of antisense nucleotides for cell membranes can be enhanced by increase of lipophilicity and as a result the transmembrane permeability may be increased, and

(3) the phenyl group enhances the stacking effect between the bases and may stabilize the tertiary structure of the oligomer.

In addition, the present inventors have succeeded in synthesizing novel saccharide deriatives and novel nucleotide compounds which are useful as intermediates for the above-mentioned oligonucleotide compounds (I) or salts thereof.

Namely, the present invention provides an oligonucleotide compound or a salt thereof; a method for producing the same; a saccharide derivative and a nucleotide derivative useful as an intermediate for producing said oligonucleotide compound; and an antitumor comosition, an antiviral composition, an antiinflammatory composition, etc. containing said oligonucleotide compound.

Specifically, the present invention provides

[A] oligonucleotide compounds (I) or a salt thereof, a method of preparing the same and use thereof;

[B] oligonucleotide compounds (Ia) or a salt thereof, a method of preparing the same and use thereof; [C] oligonucleotide compounds (Ib) or a salt thereof, a method of preparing the same and use thereof; [D-1] saccharide compounds (A); [D-2] saccharide compounds (B); [D-3] saccharide compounds (C); and [E] nucleotide compounds described as follows.

[A] Oligonucleotide Compounds of Formula (I) or a salt thereof as shown in (1) to (8) below, a method of preparing the same and use thereof

(1) An oligonucleotide compound or a salt thereof, which has a nucleotide sequence represented by formula (I):

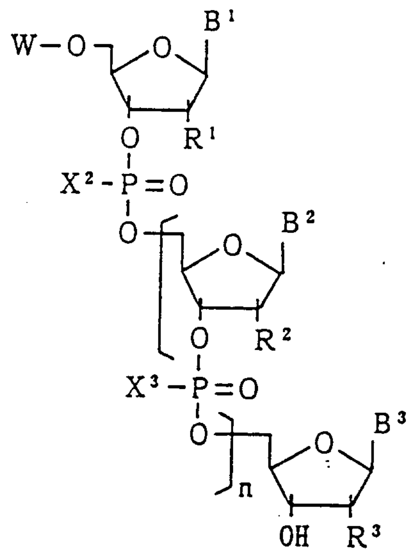

(I)

wherein W represents hydrogen or a protective group; $B^1$, $B^2$ and $B^3$ each represent nucleic acid residues, wherein each $B^2$ may be the same or different when n is 2 or more; $X^2$ and $X^3$ each represent OH, SH, $C_{1-5}$ alkyl, $C_{1-5}$ alkoxy, $C_{1-5}$ monoalkylamino or an aromatic ring group which may be substituted or unsubstituted, wherein each $X^3$ may be the same or different when n is 2 or more; $R^1$, $R^2$ and $R^3$ each represent hydrogen, OH, halogen, $C_{1-5}$ alkoxy or $C_{1-5}$ alkoxyalkyloxy, wherein each $R^2$ may be the same or different when n is 2 or more; and n represents an integer from 1 to 98;
with the proviso that (i) W represents hydrogen or a protective group when $X^2$ and $X^3$ each represent OH, SH, $C_{1-5}$ alkyl, $C_{1-5}$ alkoxy, $C_{1-5}$ monoalkylamino or an aromatic ring group which may be substituted or unsubstituted, and at least one of $X^2$ and $X^3$ is an aromatic ring group which may be substituted or unsubstituted when $X^2$ and $X^3$ each represent OH, SH, $C_{1-5}$ alkyl, $C_{1-5}$ alkoxy or $C_{1-5}$ monoalkylamino; or
(ii) W is represented by formula

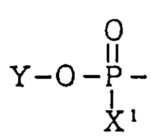

or

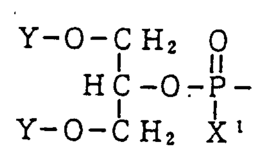

in which Y represents a saccharide residue; and $X^1$ represents OH, SH, $C_{1-5}$ alkyl, $C_{1-5}$ alkoxy, $C_{1-5}$ monoalkylamino or an aromatic ring group which may be substituted or unsubstituted.
(2) A method for producing the oligonucleotide compound described in [A] (1) above (hereinafter in part [A] the sections ( ) used will omit the [A] but do refer to senctions in Part [A]), which comprises removing a protective group of either (a) a compound represented by formula:

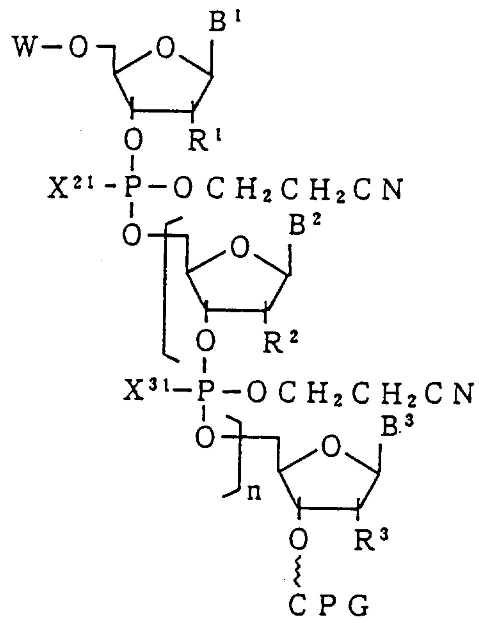

wherein W, $B^1$, $B^2$, $B^3$, $R^1$, $R^2$, $R^3$ and n have the same meanings as given in (1) above; $X^{21}$ and $X^{31}$ each represent O or S, wherein each $X^{31}$ may be the same or different when n is 2 or more; and CPG represents a solid phase carrier; or (b) a compound represented by the formula:

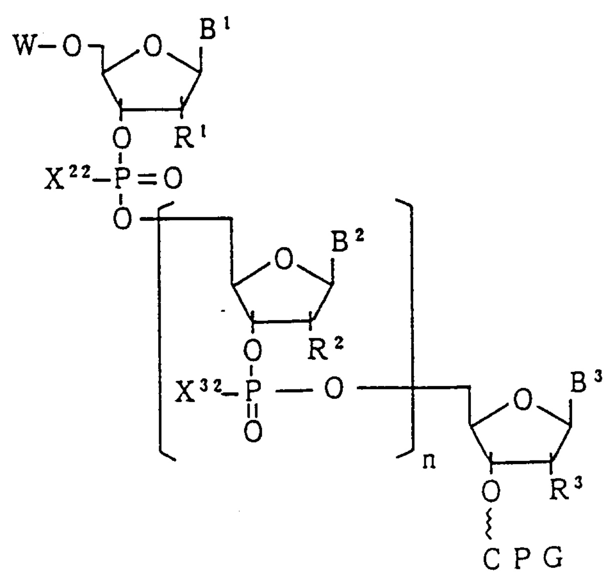

wherein W, $B^1$, $B^2$, $B^3$, $R^1$, $R^2$, $R^3$ and n have the same meanings as given above; $X^{22}$ and $X^{32}$ each represent $C_{1-5}$ alkyl, $C_{1-5}$ alkoxy, $C_{1-5}$ monoalkylamino or an aromatic ring group which may be substituted or unsubstituted, wherein each $X^{32}$ may be the same or different when n is 2 or more; and CPG represents a solid phase carrier.

(3) A composition for inhibiting a gene expression comprising the oligonucleotide compound of Formula (I) or the salt thereof of (1) above.

(4) The composition for inhibiting a gene expression described in (3) above, which is an inhibitor for expression of a gene contributing to the development of a malignant tumor, a viral disease or an inflammation.

(5) The composition for inhibiting a gene expression described in (3) or (4), in which inhibition of the gene expression is (i) inhibition of transcription from DNA to pre mRNA, (ii) inhibition of splicing from pre mRNA to mature mRNA, or (iii) inhibition of translation from mature mRNA to a protein.

(6) The composition for inhibiting a gene expression described in (4), in which the gene contributing to the development of the malignant tumor is a gene selected from the group of genes consisting of src, fps, yes, ros, myb, myc, erb, rel, mos, abl, ras, fos, fes, fms, sis, raf, neu and p53, the gene contributing to the development of the viral disease is a gene selected from the genome of the group of viruses consisting of human immune deficiency virus (HIV), an influenza virus, a herpes virus, a papilloma virus, a poliovirus, a picornavirus and an adenovirus, and the gene contributing to the development of the inflammation is a gene selected from the group consisting of ICAM-1, ELAM-1 and VLAM.

(7) The composition for inhibiting gene expression described in (3), in which the gene is ICAM-1.

(8) A composition for inhibiting reangiogenesis after PTCA which comprises a nucleotide compound (I) or a salt thereof as described in Section (1).

[B] oligonucleotide compounds (Ia) or a salt thereof as shown in (1) to (47) described below, a method of preparing the same and use thereof;

(1) An oligonucleotide compound or a salt thereof in which a phosphoric acid group bound to a saccharide residue is bound to the 5'-terminal hydroxyl group by a diester phosphate bond;

(2) The oligonucleotide compound described in [B] (1) above (hereinafter in Part [B] the sections ( ) used will omit the [B] but refer to sections in Part [B]), in which the oligonucleotide compound has a nucleotide sequence represented by formula:

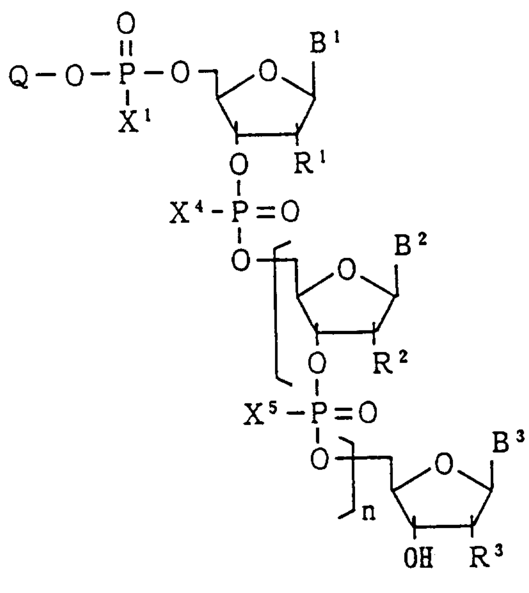

(I a)

in which Q represents Y or a formula

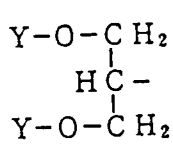

Y represents a saccharide residue; $X^1$ represents OH, SH, $C_{1-5}$ alkyl, $C_{1-5}$ alkoxy, $C_{1-5}$ monoalkylamino or an aromatic ring group which may be substituted or unsubstituted, $B^1$, $B^2$ and $B^3$ each represent nucleic acid residues, wherein each $B^2$ may be the same or different when n is 2 or more; $X^4$ and $X^5$ each represent OH, SH, $C_{1-5}$ alkyl, $C_{1-5}$ alkoxy or $C_{1-5}$ monoalkylamino, wherein each $X^5$ may be the same or different when n is 2 or more; $R^1$, $R^2$ and $R^3$ each represent hydrogen, OH, halogen, $C_{1-5}$ alkoxy or $C_{1-5}$ alkoxyalkyloxy, wherein each $R^2$ may be the same or different when n is 2 or more; and n represents an integer from 1 to 98.

(3) The oligonucleotide compound described in (2), in which a saccharide of the saccharide residue represented by Y is selected from the group consisting of (i) monosaccharides which may be substituted or unsubstituted, (ii) oligosaccharides which may be substituted or unsubstituted, and (iii) glycoside

compounds of the above-mentioned monosaccharides or the above-mentioned oligosaccharides.

(4) The oligonucleotide compound described in (2), in which a saccharide of the saccharide residue represented by Y is:

(i) a monosaccharide of which a hydroxyl group (preferably a hydroxyl group other than a hemiacetal hydroxyl group) may be substituted by about 1 to about 4 substituent groups selected from the group consisting of (a) acyl groups which may be substituted by about 1 to about 5 substituent groups selected from the group consisting of halogen, $C_{1-10}$ alkylcarbonyl, nitro, $C_{1-10}$ alkyl, $C_{1-10}$ alkoxy, phenyl, and naphthyl, (b) alkyl groups which may be substituted by about 1 to about 5 substituent groups selected from the group consisting of halogen, $C_{1-10}$ alkylcarbonyl, nitro, $C_{1-10}$ alkyl, $C_{1-10}$ alkoxy, phenyl and naphthyl, and (c) amino groups which may be substituted or unsubstituted,

(ii) an oligosaccharide of which a hydroxyl group (preferably a hydroxyl group other than a hemiacetal hydroxyl group) may be substituted by about 1 to about 4 substituent groups selected from the group consisting of (a) acyl groups which may be substituted by about 1 to about 5 substituent groups selected from the group consisting of halogen, $C_{1-10}$ alkylcarbonyl, nitro, $C_{1-10}$ alkyl, $C_{1-10}$ alkoxy, phenyl and naphthyl, (b) alkyl groups which may be substituted by about 1 to about 5 substituent groups selected from the group consisting of halogen, $C_{1-10}$ alkylcarbonyl, nitro, $C_{1-10}$ alkyl, $C_{1-10}$ alkoxy, phenyl and naphthyl, and (c) amino groups which may be substituted or unsubstituted, or

(iii) a glycoside compound in which the hydroxyl group of the above-mentioned monosaccharide or oligosaccharide is substituted by -O-$R^7$, -S-$R^8$ or -N-$R^9$ (wherein $R^7$, $R^8$ and $R^9$ each represent hydrocarbon groups of about 1 to carbons which may be substituted or unsubstituted) which is a non-saccharide component.

(5) The oligonucleotide compound described in (4), in which an acyl group is $C_{1-10}$ alkylcarbonyl, phenylcarbonyl or benzylcarbonyl.

(6) The oligonucleotide compound described in (4), in which an alkyl group is $C_{1-20}$ alkyl.

(7) The oligonucleotide compound described in (4), in which an amino group is represented by formula -$NR^4R^5$ wherein $R^4$ and $R^5$ each represent (i) hydrogen, (ii) acyl groups which may be substituted by about 1 to about 5 substituent groups selected from the group consisting of halogen, $C_{1-10}$ alkylcarbonyl, nitro, $C_{1-10}$ alkyl, $C_{1-10}$ alkoxy, phenyl and naphthyl, or (iii) alkyl groups which may be substituted by about 1 to about 5 substituent groups selected from the group consisting of halogen, $C_{1-10}$ alkylcarbonyl, nitro, $C_{1-10}$ alkyl, $C_{1-10}$ alkoxy, phenyl and naphthyl.

(8) The oligonucleotide compound described in (3) or (4), in which the monosaccharide is an aldopentose, a ketopentose, an aldohexose or a ketohexose.

(9) The oligonucleotide compound described in (8) in which the aldopentose is ribose, arabinose, xylose or lyxose, the ketopentose is ribulose or xylulose, the aldohexose is allose, altrose, glucose, mannose, gulose, idose, galactose or talose, and the ketohexose is psicose, fructose, sorbose or tagatose.

(10) The oligonucleotide compound described in (3) or (4), in which the oligosaccharide is selected from the group consisting of lactose, $\alpha$,$\alpha$-trehalose, N-acetylneuraminyllactose, difucosyllactose, fucosyllactose, lactulose, gentianose, isolychnoses, lychnoses, planteose, sucrose, bifrucose, 1,6-diglucosylmannitol, galactosylsucrose, 6-$\beta$-glucosylmannitol, laminaribiose, inulobioses, lycopose, maltose, isomaltotriose, melibiose, neobifucose, neokestose, eruloses, 6-$\alpha$-galactosylgalactose, kojibiose, maltlose, melezitose, turanose, sophorose, gentiooligosaccharides, vicianose, primeverose, sambubiose, lycobiose, lycotriose, solabiose, strophantobiose, digilanidobiose, odorotriose, fungitetraose and $\beta$-1,3-xylooligo.

(11) The oligonucleotide compound described in (3) or (4), in which the glycoside compound is one in which the hydroxyl group of the monosaccharide or the oligosaccharide is substituted by -O-$R^7$, -S-$R^8$ or -N-$R^9$ (wherein $R^7$, $R^8$ and $R^9$ each represent hydrocarbon groups which may be substituted or unsubstituted) which is a non-saccharide component.

(12) The oligonucleotide compound described in (11), in which each of $R^7$, $R^8$ and $R^9$ is:

(i) a $C_{1-30}$ alkyl group which may be substituted by about 1 to about 5 substituent groups selected from the group consisting of halogen, $C_{1-10}$ alkylcarbonyl, nitro and $C_{1-10}$ alkoxy,

(ii) a $C_{2-4}$ alkenyl group which may be substituted by about 1 to about 5 substituent groups selected from the group consisting of halogen, $C_{1-10}$ alkylcarbonyl, nitro and a $C_{1-10}$ alkoxy,

(iii) a $C_{2-4}$ alkynyl group which may be substituted by about 1 to about 5 substituent groups selected from the group consisting of halogen, $C_{1-10}$ alkylcarbonyl, nitro and $C_{1-10}$ alkoxy,

(iv) a $C_{6-12}$ aryl group which may be substituted by about 1 to about 5 substituent groups selected from the group consisting of halogen, $C_{1-10}$ alkylcarbonyl, nitro, $C_{1-10}$ alkyl and $C_{1-10}$ alkoxy, or

(v) an $C_{7-14}$ aralkyl group which may be substituted by about 1 to about 5 substituent groups selected from the group consisting of halogen, $C_{1-10}$ alkylcarbonyl, nitro, $C_{1-10}$alkyl and a $C_{1-10}$ alkoxy.

(13) The oligonucleotide compound described in (3) or (4), in which the glycoside compound is selected from the group consisting of n-octylglucoside, n-octyl-galactoside, n-octylmannoside, n-octylthioglucoside, n-octylthiogalactoside, n-octylthiomannoside, stearylglycoside, stearylgalactoside, stearylmannoside, stearylthioglycoside, stearylthiogalactoside, stearylthiomannoside, phenylglucoside, phenylgalactoside and phenylmannoside.

(14) The oligonucleotide compound described in (2), in which Q is Y, and a saccharide of the saccharide residue represented by Y is a monosaccharide, oligosaccharide or a glycoside compound thereof of which a hydroxyl group may be substituted by $C_{1-10}$ alkylcarbonyl, phenylcarbonyl or $C_{1-20}$ alkyl group.

(15) The oligonucleotide compound described in (14), in which the monosaccharide is galactose, mannose, galactosamine or glucosamine.

(16) The oligonucleotide compound described in (14), in which the oligosaccharide is melibiose, gentiobiose or isomaltotriose.

(17) The oligonucleotide compound described in (14), in which the glycoside compound is n-octylglucoside, n-octylthioglucoside or phenylglucoside.

(18) The oligonucleotide compound described in (4), in which the monosaccharide has a hydroxyl group substituted by amino groups which may be substituted or unsubstituted, wherein the monosaccharide is glucosamine or galactosamine in which the 2-amino groups may be substituted by $C_{1-6}$ alkylcarbonyl groups or phenylcarbonyl groups.

(19) The oligonucleotide compound described in (2), in which Q is Y, and a saccharide of the saccharide residue represented by Y is (i) galactose or mannose of which an alcoholic hydroxyl group may be substituted by $C_{1-6}$ alkylcarbonyl groups, (ii) melibiose, gentiobiose or isomaltotriose of which a hydroxyl group may be substituted by phenylcarbonyl groups, (iii) n-octylglucoside, n-octylthioglucoside or phenylglucoside, or (iv) glucosamine or galactosamine in which the 2-amino groups may be substituted by $C_{1-10}$ alkylcarbonyl groups or phenylcarbonyl groups.

(20) The oligonucleotide compound described in (2), in which Q is Y, and a saccharide of the saccharide residue represented by Y is galactose, mannose, melibiose, gentiobiose, isomaltotriose, n-octylglucoside, n-octylthioglucoside, phenylglucoside, galactosamine, N-benzoylgalactosamine, N-acetylgalactosamine, glucosamine, N-benzoylglucosamine, N-acetylglucosamine.

(21) The oligonucleotide compound described in (2), in which Q is represented by the formula,

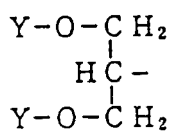

and a saccharide of the saccharide residue represented by Y is a monosaccharide.

(22) The oligonucleotide compound described in (21), in which the monosaccharide is galactose, mannose or glucose.

(23) The oligonucleotide compound described in any one of (2) to (22), in which the nucleic acid residue is selected from the group consisting of hypoxanthine-9-yl, guanine-9-yl, adenine-9-yl, uracil-1-yl, thymine-1-yl and cytosine-1-yl.

(24) The oligonucleotide compound described in any one of (2) to (23), in which $X^1$ is (i) OH, (ii) SH, (iii) $C_{1-5}$ alkyl, (iv) $C_{1-5}$ alkoxy, (v) $C_{1-5}$ monoalkylamino, (vi) $C_{6-11}$ aryl which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl, or (vii) a 5 to 13-membered aromatic heterocyclic group which comprises 1 to 12 carbon atoms and 1 to about 4 hetero atomos which are selected from the group consisting of nitrogen, oxygen and sulfur atoms which may be substituted by about 1 to about 5 substituents which are selected from the group consisting of $C_{1-6}$ alkyl, $C_{2-6}$ alkenyl, $C_{2-6}$ alkynyl, $C_{3-6}$ cycloalkyl, $C_{5-7}$ cycloalkenyl, $C_{7-11}$ aralkyl, $C_{6-14}$ aryl, $C_{1-6}$ alkoxy, $C_{6-14}$ aryloxy, $C_{1-6}$ alkylcarbonyl, $C_{6-14}$ arylcarbonyl, $C_{1-6}$ alkanoyloxy, $C_{6-14}$ arylcarbonyloxy, carboxyl, $C_{1-6}$ alkoxycarbonyl, carbamoyl, N-mono-$C_{1-4}$ alkylcarbamoyl, N,N-di-$C_{1-4}$alkylcarbamoyl, halogen, mono-, di- or tri-halogeno-$C_{1-4}$ alkyl, oxo, amidino, imino, amino, mono- or di-$C_{1-4}$ alkylamino, OH and nitro; $X^4$ and $X^5$ are each OH, SH, $C_{1-5}$ alkyl, $C_{1-5}$ alkoxy or $C_{1-5}$ monoalkylamino.

(25) The oligonucleotide compound described in (24), in which $C_{6-11}$ aryl group is phenyl or naphthyl, a 5 to 13-membered aromatic heterocyclic group which comprises carbon atoms and about 1 to about 4 hetero atomos which are selected from the group consisting of nitrogen, oxygen and sulfur atoms and is

preferably selected from the group consisting of 2- or 3-thienyl, 2- or 3-furyl, 2- or 3-pyrolyl, 2-, 3- or 4-pyridyl, 2-, 4- or 5-oxazolyl, 2-, 4-or 5-thiazolyl, 3-, 4- or 5-pyrazolyl, 2-, 4- or 5-imidazolyl, 3-, 4- or 5-isoxazolyl, 3-, 4- or 5-isothiazolyl, 3- or 5-(1,2,4-oxadiazolyl), 1,3,4-oxadiazolyl, 3- or 5-(1,2,4-thiadiazolyl), 1,3,4-thiadiazolyl, 4- or 5-(1,2,3-thiadiazolyl), 1,2,5-thiadiazolyl, 1,2,3-triazolyl, 1,2,4-triazolyl, 1H- or 2H-tetrazolyl, N-oxide-2-, 3- or 4-pyridyl, 2-, 4- or 5-pyrimidinyl, N-oxide-2-, 4- or 5-pyrimidinyl, thiomorpholinyl, morpholinyl, oxoimidadinyl, dioxotriazinyl, pyrrolidinyl, piperidinyl, pyranyl, thiopyranyl, 1,4-oxazinyl, 1,4-thiazinyl, 1,3-thiazinyl, piperadinyl, triazinyl, oxotriazinyl, 3- or 4-pyridazinyl, pyrazinyl, N-oxide-3- or 4-pyridazinyl, benzofuryl, benzothiazolyl, benzooxazolyl, tetrazolo[1,5-b]pyridazinyl, triazolo-[4,5-b]pyridazinyl, benzoimidazolyl, quinolyl, isoquinolyl, cynnorynyl, phthalazinyl, quinazolinyl, quinoxalinyl, indolizinyl, quinolizinyl, 1,8-naphthylizinyl, purinyl, pteridinyl, dibenzofuranyl, carbazolyl, acridinyl, phenanthridinyl, chromanyl, benzoxazinyl, phenazinyl, phenothiazinyl or phenoxazinyl.

(26) The oligonucleotide compound described in any one of (2) to (23), in which $X^1$ is OH; SH; $C_{1-5}$ alkyl; $C_{1-5}$ alkoxy; $C_{1-5}$ monoalkylamino; or phenyl which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl; and $X^4$ and $X^5$ are each OH, SH, $C_{1-5}$ alkyl, $C_{1-5}$ alkoxy or $C_{1-5}$ monoalkylamino.

(27) The oligonucleotide compound described in any one of (2) to (23), in which $X^1$ is selected from the group consisting of OH, SH, methyl, ethyl, propyl, phenyl, (o-, m-, p-)alkoxyphenyl, (o-, m-, p-)alkylphenyl, methoxy and ethoxy; and $X^4$ and $X^5$ are each selected from the group consisting of OH, SH, methyl, ethyl, propyl, methoxy and ethoxy.

(28) The oligonucleotide compound described in any one of (2) to (23), in which $X^1$ is selected from the group consisting of OH, SH, methyl, ethyl, propyl, phenyl, (o-, m-, p-) $C_{1-10}$ alkoxyphenyl, (o-, m-, p-) $C_{1-10}$ alkylphenyl, methoxy and ethoxy; and $X^4$ and $X^5$ are each selected from the group consisting of OH, SH, methyl, ethyl, propyl, methoxy and ethoxy.

(29) The oligonucleotide compound described in any one of (2) to (23), in which $X^1$ is OH, SH, methyl or phenyl, and $X^4$ and $X^5$ are each OH, SH or methyl.

(30) The oligonucleotide compound described in any one of (2) to (29), in which $R^1$, $R^2$ and $R^3$ are each selected from the group consisting of hydrogen, OH, fluoro, chloro, bromo, methoxy, ethoxy and methoxymethyloxy.

(31) The oligonucleotide compound described in any one of (2) to (29), in which $R^1$, $R^2$ and $R^3$ are each hydrogen.

(32) The oligonucleotide compound described in any one of (2) to (31), in which n is an integer of 1 to 38.

(33) The oligonucleotide compound described in any one of (2) to (32), which contains a nucleotide sequence represented by CAT.

(34) The oligonucleotide compound described in (2), which is represented by a formula:

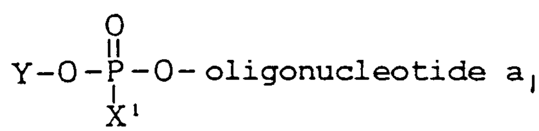

wherein Y represents a saccharide residue, and $X^1$ represents OH, SH, $C_{1-5}$ alkyl, $C_{1-5}$ alkoxy, $C_{1-5}$ monoalkylamino or an aromatic ring group which may be substituted or unsubstituted; "oligonucleotide $a_1$" represents a nucleotide sequence selected from the group consisting of SEQ ID NOs.: 1 to 31 and SEQ ID NO:34.

(35) The oligonucleotide compound described in (34), in which a saccharide of the saccharide residue represented by Y is galactose, mannose, melibiose, gentiobiose, isomaltotriose, n-octylglucoside, n-octylthioglucoside, phenylglucoside, galactosamine, N-benzoylgalactosamine, N-acetylgalactosamine, glucosamine, N-benzoylglucosamine, N-acetylglucosamine; $X^1$ is OH, SH, methyl or phenyl.

(36) The oligonucleotide compound described in (2), which is represented by a formula:

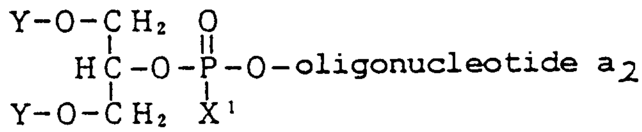

wherein Y represents a saccharide residue, and $X^1$ represents OH, SH, $C_{1-5}$ alkyl, $C_{1-5}$ alkoxy, $C_{1-5}$ monoalkylamino or an aromatic ring which may be substituted or unsubstituted; "oligonucleotide $a_2$" represents a nucleotide sequence selected from the group consisting of SEQ ID NOs.: 1 to 31 and SEQ ID NO:34.

(37) The oligonucleotide compound described in (36), in which a saccharide of the saccharide residue represented by Y is galactose, mannose or glucose; $X^1$ is OH, SH, methyl or phenyl.

(38) The oligonucleotide compound described in any one of (34) to (37), in which each of "oligonucleotide $a_1$" and "oligonucleotide $a_2$" is represented by SEQ ID NO: 1, SEQ ID NO:31 or SEQ ID NO:34.

(39) The oligonucleotide compound described in any one of (1) to (38), which is an antisence oligonucleotide compound.

(40) The oligonucleotide compound described in any one of (1) to (39), which is encapsulated in a liposome.

(41) A method for producing the oligonucleotide compound described in (2), which comprises removing a protective group of (a) a compound represented by formula:

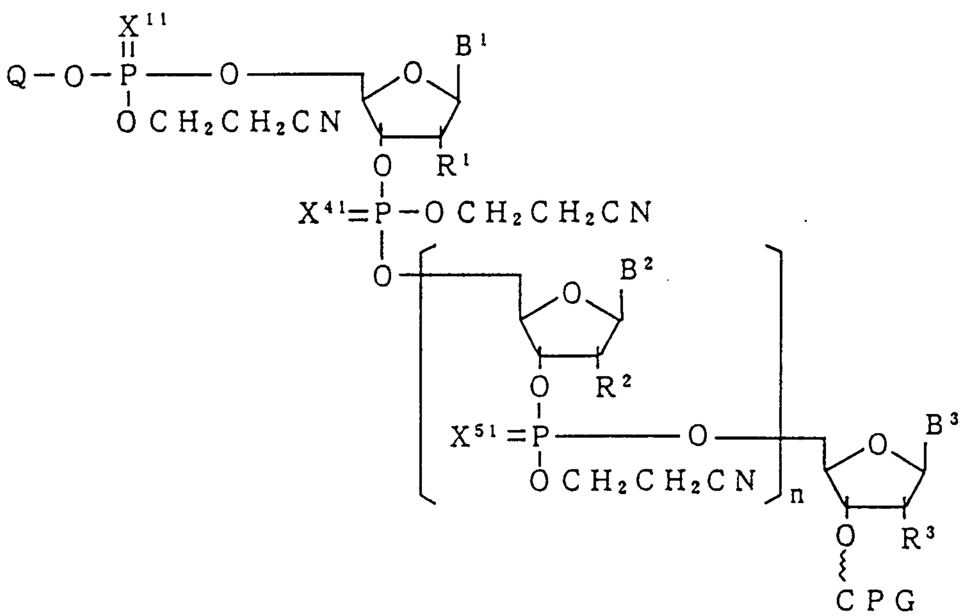

wherein Q, $B^1$, $B^2$, $B^3$, $R^1$, $R^2$, $R^3$ and n have the same meanings as given in [B] (2) above; $X^{11}$, $X^{41}$ and $X^{51}$ each represent O or S, wherein each $X^{51}$ may be the same or different when n is 2 or more; and CPG represents a solid phase carrier; or

(b) a compound represented by formula:

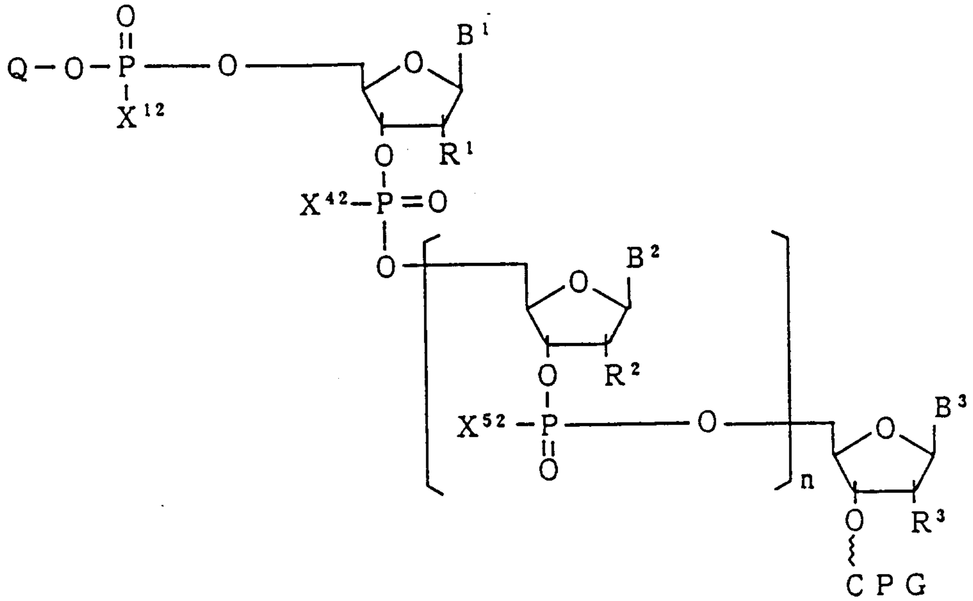

wherein Q, $B^1$, $B^2$, $B^3$, $R^1$, $R^2$, $R^3$ and n have the same meanings as given in (2) above; $X^{12}$, $X^{42}$ and $X^{52}$ each represent $C_{1-5}$ alkyl, $C_{1-5}$ alkoxy, $C_{1-5}$ monoalkylamino or an aromatic ring group which may be substituted or unsubstituted, wherein each $X^{52}$ may be the same or different when n is 2 or more; and CPG represents a solid phase carrier.

(42) A composition for inhibiting a gene expression comprising the oligonucleotide compound or the salt thereof described in any one of (1) to (40).

(43) The composition for inhibiting a gene expression described in (42), which is an inhibitor for expression of a gene contributing to the development of a malignant tumor, a viral disease or an inflammation.

(44) The composition for inhibiting a gene expression described in (42) or (43), in which inhibition of the gene expression is (i) inhibition of transcription from DNA to pre mRNA, (ii) inhibition of splicing from pre mRNA to mature mRNA, or (iii) inhibition of translation from mature mRNA to a protein.

(45) The composition for inhibiting a gene expression described in (43), in which the gene contributing to the development of the malignant tumor is a gene selected from the group of genes consisting of src, fps, yes, ros, myb, myc, erb, rel, mos, abl, ras, fos, fes, fms, sis, raf, neu and p53, the gene contributing to the development of the virus disease is a gene selected from the genome of the group of viruses consisting of HIV, an influenza virus, a herpes virus, a papilloma virus, a poliovirus, a picornavirus and an adenovirus, and the gene contributing to the development of the inflammation is a gene selected from the group consisting of ICAM-1, ELAM-1 and VLAM.

(46) The composition for inhibiting a gene expression described in (42), in which the gene is ICAM-1.

(47) A composition for inhibiting of reangiogenesis after PTCA which comprises a nucleotide compound or a salt thereof of any one of (1) to (40).

[C] oligonucleotide compounds of Formula (Ib) or a salt thereof as shown in (1) to (57) below, a method of preparing the same and use thereof;

(1) The oligonucleotide compound (Ib) or a salt thereof, which has a nucleotide sequence represented by formula:

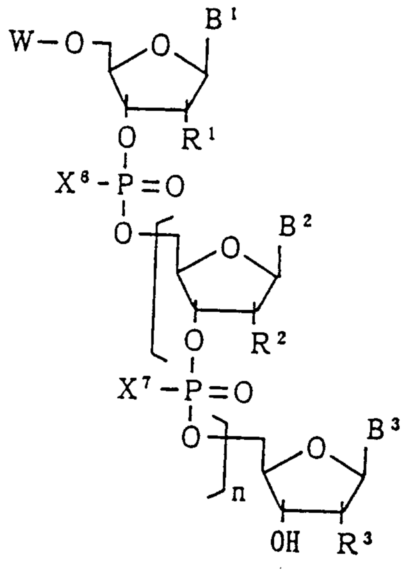

(I b)

wherein W represents hydrogen or a protective group; $B^1$, $B^2$ and $B^3$ each represent nucleic acid residues, wherein each $B^2$ may be the same or different when n is 2 or more; $X^6$ and $X^7$ each represent OH, SH, $C_{1-5}$ alkyl, $C_{1-5}$ alkoxy, $C_{1-5}$ monoalkylamino or an aromatic ring group which may be substituted or unsubstituted, wherein each $X^7$ may be the same or different when n is 2 or more, at least one of $X^6$ and $X^7$ represents an aromatic ring group which may be substituted or unsubstituted; $R^1$, $R^2$ and $R^3$ each represent hydrogen, OH, halogen, $C_{1-5}$ alkoxy or $C_{1-5}$ alkoxyalkyloxy, wherein each $R^2$ may be the same or different when n is 2 or more; and n represents an integer from 1 to 98.

(2) The oligonucleotide compound or a salt thereof described in [C] (1) above (hereinafter Part [C] the sections ( ) used will omit the [C] but refer to sections in part [C]), wherein a protective group represented by W is represented by

(i) formula

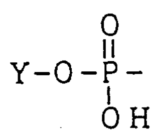

wherein Y is a saccharide residue;

(ii) formula

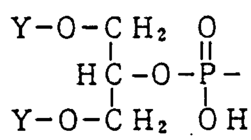

wherein Y is a saccharide residue;

(iii) $C_{1-6}$ alkyl which may be substituted with halogen atom(s);

(iv) $C_{6-11}$ aryl which may be substituted with halogen atom(s) or $C_{1-6}$ alkyl;

(v) $C_{7-12}$ aralkyl which may be substituted with halogen atom(s) or $C_{1-6}$ alkyl;

(vi) $C_{1-6}$ alkylcarbonyl which may be substituted with halogen atom(s);

(vii) phenyloxycarbonyl;

(viii) $C_{7-12}$ aralkyl-carbonyl;

(ix) pyranyl;

(x) furanyl;

(xi) silyl;

(xii) trityl which may be substituted with 1 to 3 methoxy groups; or

(xiii) phenylxanthenyl which may be substituted with methoxy groups.

(3) The oligonucleotide compound or a salt thereof described in (1), wherein W is hydrogen or represented by formula

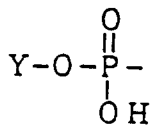

or formula

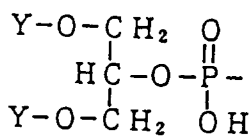

wherein Y is a saccharide residue.

(4) The oligonucleotide compound or a salt thereof described in any one of (2) or (3), in which a saccharide of the saccharide residue represented by Y is selected from the group consisting of (i) monosaccharides which may be substituted or unsubstituted, (ii) oligosaccharides which may be substituted or unsubstituted, and (iii) glycoside compounds of the above-mentioned monosaccharides or the above-mentioned oligosaccharides.

(5) The oligonucleotide compound or a salt thereof described in any one of (2) or (3), in which a saccharide of the saccharide residue represented by Y is:

(i) a monosaccharide of which a hydroxyl group (preferably a hydroxyl group other than a hemiacetal hydroxyl group) may be substituted by about 1 to about 4 substituent groups selected from the group consisting of (a) acyl groups which may be substituted by about 1 to about 5 substituent groups selected from the group consisting of halogen, $C_{1-10}$ alkylcarbonyl, nitro, $C_{1-10}$ alkyl, $C_{1-10}$ alkoxy, phenyl and naphthyl, (b) alkyl groups which may be substituted by about 1 to about 5 substituent groups selected from the group consisting of halogen, $C_{1-10}$ alkylcarbonyl, nitro, $C_{1-10}$ alkyl, $C_{1-10}$ alkoxy, phenyl and naphthyl, and (c) amino groups which may be substituted or unsubstituted,

(ii) an oligosaccharide of which a hydroxyl group (preferably a hydroxyl group other than a hemiacetal hydroxyl group) may be substituted by about 1 to about 4 substituent groups selected from the group consisting of (a) acyl groups which may be substituted by about 1 to about 5 substituent groups selected from the group consisting of halogen, $C_{1-10}$ alkylcarbonyl, nitro, $C_{1-10}$ alkyl, $C_{1-10}$ alkoxy, phenyl and naphthyl, (b) alkyl groups which may be substituted by about 1 to about 5 substituent groups selected from the group consisting of halogen, $C_{1-10}$ alkylcarbonyl, nitro, $C_{1-10}$ alkyl, $C_{1-10}$ alkoxy, phenyl and naphthyl, and (c) amino groups which may be substituted or unsubstituted, or

(iii) a glycoside compound in which the hydroxyl group of the above-mentioned monosaccharide or oligosaccharide is substituted by -O-$R^7$, -S-$R^8$ or -N-$R^9$ (wherein $R^7$, $R^8$ and $R^9$ each represent hydrocarbon groups which may be substituted or unsubstituted) which is a non-saccharide component.

(6) The oligonucleotide compound described in any one of (4) or (5), in which the monosaccharide is an aldopentose, a ketopentose, an aldohexose or a ketohexose.

(7) The oligonucleotide compound described in (6), in which the aldopentose is ribose, arabinose, xylose or lyxose, the ketopentose is ribulose or xylulose, the aldohexose is allose, altrose, glucose, mannose, gulose, idose, galactose or talose, and the ketohexose is psicose, fructose, sorbose or tagatose.

(8) The oligonucleotide compound described in any one of (4) or (5), in which the oligosaccharide is selected from the group consisting of lactose, α,α-trehalose, N-acetylneuraminyllactose, difucosyllactose, fucosyllactose, lactulose, gentianose, isolychnoses, lychnoses, planteose, sucrose, bifrucose, 1,6-diglucosylmannitol, galactosylsucrose, 6-β-glucosylmannitol, laminaribiose, inulobioses, lycopose, maltose, isomaltotriose, melibiose, neobifucose, neokestose, eruloses, 6-α-galactosylgalactose, kojibiose, maltlose, melezitose, turanose, sophorose, gentiooligosaccharides, vicianose, primeverose, sambubiose, lycobiose, lycotriose, solabiose, strophantobiose, digilanidobiose, odorotriose, fungitetraose and β-1,3-xylooligo.

(9) The oligonucleotide compound described in any one of (4) or (5), in which the glycoside compound is one in which the hydroxyl group of the monosaccharide or the oligosaccharide is substituted by $-O-R^7$, $-S-R^8$ or $-N-R^9$ (wherein $R^7$, $R^8$ and $R^9$ each represent hydrocarbon groups which may be substituted or unsubstituted) which is a non-saccharide component.

(10) The oligonucleotide compound described in (9), in which each of $R^7$, $R^8$ and $R^9$ is:

(i) a $C_{1-30}$ alkyl group which may be substituted by about 1 to about 5 substituent groups selected from the group consisting of halogen, $C_{1-10}$ alkylcarbonyl, nitro and $C_{1-10}$ alkoxy,

(ii) a $C_{2-4}$ alkenyl group which may be substituted by about 1 to about 5 substituent groups selected from the group consisting of halogen, $C_{1-10}$ alkylcarbonyl, nitro and a $C_{1-10}$ alkoxy,

(iii) a $C_{2-4}$ alkynyl group which may be substituted by about 1 to about 5 substituent groups selected from the group consisting of halogen, $C_{1-10}$ alkylcarbonyl, nitro and $C_{1-10}$ alkoxy,

(iv) a $C_{6-12}$ aryl group which may be substituted by about 1 to about 5 substituent groups selected from the group consisting of halogen, $C_{1-10}$ alkylcarbonyl, nitro, $C_{1-10}$ alkyl and $C_{1-10}$ alkoxy, or

(v) a $C_{7-14}$ aralkyl group which may be substituted by about 1 to about 5 substituent groups selected from the group consisting of halogen, $C_{1-10}$ alkylcarbonyl, nitro, $C_{1-10}$ alkyl and a $C_{1-10}$ alkoxy.

(11) The oligonucleotide compound described in (4) or (5), in which the glycoside compound is selected from the group consisting of n-octylglucoside, n-octyl-galactoside, n-octylmannoside, n-octylthioglucoside, n-octylthiogalactoside, n-octylthiomannoside, stearylglycoside, stearylgalactoside, stearylmannoside, stearylthioglycoside, stearylthiogalactoside, stearylthiomannoside, phenylglucoside, phenylgalactoside and phenylmannoside.

(12) The oligonucleotide compound described in (5), in which the monosaccharide has a hydroxyl group substituted by amino groups which may be substituted or unsubstituted, wherein the monosaccharide preferably is glucosamine or galactosamine in which the 2-amino groups may be substituted by $C_{1-6}$ alkylcarbonyl groups or phenylcarbonyl groups.

(13) The oligonucleotide compound described in (1), in which W is hydrogen or represented by the formula

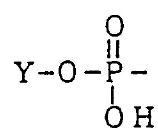

and a saccharide of the saccharide residue represented by Y is a monosaccharide, oligosaccharide or a glycoside compound thereof of which a hydroxyl group may be substituted by $C_{1-10}$ alkylcarbonyl, phenylcarbonyl or $C_{1-20}$ alkyl group.

(14) The oligonucleotide compound described in (13), in which the monosaccharide is galactose, mannose, galactosamine or glucosamine.

(15) The oligonucleotide compound described in (13), in which the oligosaccharide is melibiose, gentiobiose or isomaltotriose.

(16) The oligonucleotide compound described in (13), in which the glycoside compound is n-octylglucoside, n-octylthioglucoside or phenylglucoside.

(17) The oligonucleotide compound described in (1), in which W is hydrogen or represented by the formula

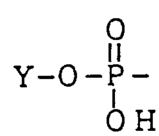

and a saccharide of the saccharide residue represented by Y is (i) galactose or mannose of which a hydroxyl group may be substituted by $C_{1-6}$ alkylcarbonyl groups, (ii) melibiose, gentiobiose or isomaltotriose of which an alcoholic hydroxyl group may be substituted by phenylcarbonyl groups, (iii) n-octylglucoside, n-octylthioglucoside or phenylglucoside, or (iv) glucosamine or galactosamine in which the 2-amino groups may be substituted by $C_{1-10}$ alkylcarbonyl groups or phenylcarbonyl groups.

(18) The oligonucleotide compound described in (1), in which W is hydrogen or represented by the formula

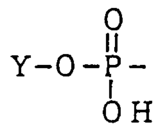

and a saccharide of the saccharide residue represented by Y is galactose, mannose, melibiose, gentiobiose, isomaltotriose, n-octylglucoside, n-octylthioglucoside, phenylglucoside, galactosamine, N-benzoylgalactosamine, N-acetylgalactosamine, glucosamine, N-benzoylglucosamine, N-acetylglucosamine.

(19) The oligonucleotide compound described in (1), in which W is hydrogen or represented by the formula,

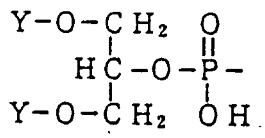

and a saccharide of the saccharide residue represented by Y is a monosaccharide.

(20) The oligonucleotide compound described in (19), in which the monosaccharide is galactose, mannose or glucose.

(21) The oligonucleotide compound described in any one of (1) to (20), in which the nucleic acid residue is selected from the group consisting of hypoxanthine-9-yl, guanine-9-yl, adenine-9-yl, uracil-1-yl, thymine-1-yl and cytosine-1-yl.

(22) The oligonucleotide compound described in any one of (1) to (21), in which $X^6$ and $X^7$ are each (i) OH, (ii) SH, (iii) $C_{1-5}$ alkyl, (iv) $C_{1-5}$ alkoxy, (v) $C_{1-5}$ monoalkylamino, (vi) $C_{6-11}$ aryl which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl, or (vii) a 5 to 13-membered aromatic heterocyclic ring which comprises 1 to 12 carbon atoms and 1 to about 4 hetero atomos which are selected from the group consisting of nitrogen, oxygen and sulfur atoms which may be substituted by about 1 to about 5 substituents which are selected from the group consisting of $C_{1-6}$ alkyl, $C_{2-6}$ alkenyl, $C_{2-6}$ alkynyl, $C_{3-6}$ cycloalkyl, $C_{5-7}$ cycloalkenyl, $C_{7-11}$ aralkyl, $C_{6-14}$ aryl, $C_{1-6}$ alkoxy, $C_{6-14}$ aryloxy, $C_{1-6}$ alkylcarbonyl, $C_{6-14}$ arylcarbonyl, $C_{1-6}$ alkanoyloxy, $C_{6-14}$ arylcarbonyloxy, carboxyl, $C_{1-6}$ alkoxycarbonyl, carbamoyl, N-mono-$C_{1-4}$ alkylcarbamoyl, N,N-di-$C_{1-4}$ alkylcarbamoyl, halogen, mono-, di- or tri-halogeno-$C_{1-4}$ alkyl, oxo, amidino, imino, amino, mono- or di-$C_{1-4}$ alkylamino, OH and nitro.

(23) The oligonucleotide compound described in (22), in which the $C_{6-11}$ aryl group is phenyl or naphthyl, a 5 to 13-membered aromatic heterocyclic ring which comprises 1 to 12 carbon atoms and about 1 to about 4 hetero atomos which are selected from the group consisting of nitrogen, oxygen and sulfur atoms and preferably is selected from the group consisting of 2- or 3-thienyl, 2- or 3-furyl, 2- or 3-pyrrolyl, 2-, 3- or 4-pyridyl, 2-, 4- or 5-oxazolyl, 2-, 4-or 5-thiazolyl, 3-, 4- or 5-pyrazolyl, 2-, 4- or 5-imidazolyl, 3-, 4- or 5-isoxazolyl, 3-, 4- or 5-isothiazolyl, 3- or 5-(1,2,4-oxadiazolyl), 1,3,4-oxadiazolyl, 3- or 5-(1,2,4-thiadiazolyl), 1,3,4-thiadiazolyl, 4- or 5-(1,2,3-thiadiazolyl), 1,2,5-thiadiazolyl, 1,2,3-triazolyl, 1,2,4-triazolyl, 1H- or 2H-tetrazolyl, N-oxide-2-, 3- or 4-pyridyl, 2-, 4- or 5-pyrimidinyl, N-oxide-2-, 4- or 5-pyrimidinyl, thiomorpholinyl, morpholinyl, oxoimidadinyl, dioxotriazinyl, pyrrolidinyl, piperidinyl, pyranyl, thiopyranyl, 1,4-oxazinyl, 1,4-thiazinyl, 1,3-thiazinyl, piperadinyl, triazinyl, oxotriazinyl, 3- or 4-pyridazinyl, pyrazinyl, N-oxide-3- or 4-pyridazinyl, benzofuryl, benzothiazolyl, benzooxazolyl, tetrazolo[1,5-b]-pyridazinyl, triazolo[4,5-b]pyridazinyl, benzoimidazolyl, quinolyl, isoquinolyl, cynnorynyl, phthalazinyl, quinazolinyl, quinoxalinyl, indolizinyl, quinolizinyl, 1,8-naphthylizinyl, purinyl, pteridinyl, dibenzofuranyl, carbazolyl, acridinyl, phenanthridinyl, chromanyl, benzoxazinyl, phenazinyl, phenothiazinyl or phenoxazinyl.

(24) The oligonucleotide compound described in any one of (1) to (23), in which $X^6$ and $X^7$ are each OH; SH; $C_{1-5}$ alkyl; $C_{1-5}$ alkoxy; $C_{1-5}$ monoalkylamino; or phenyl which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl.

(25) The oligonucleotide compound described in any one of (1) to (23), in which $X^6$ and $X^7$ are each selected from the group consisting of OH, SH, methyl, ethyl, propyl, phenyl, (o-, m-, p-)alkoxyphenyl, (o-, m-, p-)alkylphenyl, methoxy and ethoxy.

(26) The oligonucleotide compound described in any one of (1) to (23), in which $X^6$ and $X^7$ are each selected from the group consisting of OH, SH, methyl, ethyl, propyl, phenyl, (o-, m-, p-) $C_{1-10}$ alkoxyphenyl, (o-, m-, p-) $C_{1-10}$ alkylphenyl, methoxy and ethoxy.

(27) The oligonucleotide compound described in any one of (1) to (23), in which $X^6$ and $X^7$ are each OH, SH, methyl or phenyl.

(28) The oligonucleotide compound described in any one of (1) to (27), in which $R^1$, $R^2$ and $R^3$ are each selected from the group consisting of hydrogen, OH, fluoro, chloro, bromo, methoxy, ethoxy and methoxymethyloxy.

(29) The oligonucleotide compound described in any one of (1) to (27), in which $R^1$, $R^2$ and $R^3$ are each hydrogen.

(30) The oligonucleotide compound described in any one of (1) to (29), in which n is an integer from 1 to 38.

(31) The oligonucleotide compound described in (1), which has a nucleotide sequence represented by the formula:

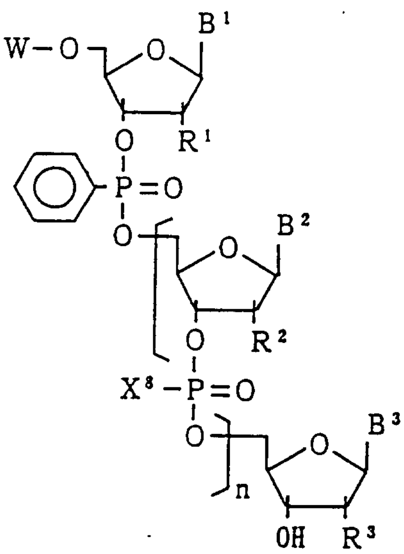

wherein W represents hydrogen or a protective group; $B^1$, $B^2$ and $B^3$ each represent nucleic acid residues, wherein each $B^2$ may be the same or different when n is 2 or more; $X^6$ represents OH, SH, $C_{1-5}$ alkyl, $C_{1-5}$ alkoxy, $C_{1-5}$ monoalkylamino or an aromatic ring group which may be substituted or unsubstituted; $R^1$, $R^2$ and $R^3$ each represent hydrogen, OH, halogen, $C_{1-5}$ alkoxy or $C_{1-5}$ alkoxyalkyloxy, wherein each $R^2$ may be the same or different when n is 2 or more; and n represents an integer from 1 to 98.

(32) The oligonucleotide compound or a salt thereof described in (1), which has a nucleotide sequence represented by formula:

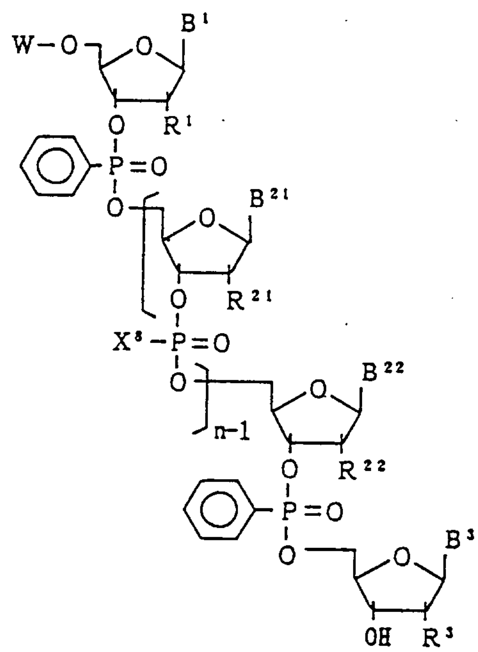

wherein W represents hydrogen or a protective group; $B^1$, $B^{21}$, $B^{22}$ and $B^3$ each represent nucleic acid residues, wherein each $B^{21}$ may be the same or different when n is 2 or more; $X^8$ represents OH, SH, $C_{1-5}$ alkyl, $C_{1-5}$ alkoxy, $C_{1-5}$ monoalkylamino or an aromatic ring group which may be substituted or unsubstituted; $R^1$, $R^2$ and $R^3$ each represent hydrogen, OH, halogen, $C_{1-5}$ alkoxy or $C_{1-5}$ alkoxyalkyloxy, wherein each $R^{21}$ may be the same or different when n is 2 or more; and n represents an integer from 1 to 98.

(33) The oligonucleotide compound described in (31) or (32), in which the nucleic acid residue is selected from the group consisting of hypoxanthine-9-yl, guanine-9-yl, adenine-9-yl, uracil-1-yl, thymine-1-yl and cytosine-1-yl.

(34) The oligonucleotide compound described in (31) or (32), in which $X^8$ is (i) OH, (ii) SH, (iii) $C_{1-5}$ alkyl, (iv) $C_{1-5}$ alkoxy, (v) $C_{1-5}$ monoalkylamino, (vi) $C_{6-11}$ aryl which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl, or (vii) a 5 to 13-membered aromatic heterocyclic ring which comprises 1 to 12 carbon atoms and 1 to about 4 hetero atomos which are selected from the group consisting of nitrogen, oxygen and sulfur atoms which may be substituted by about 1 to about 5 substituents which are selected from the group consisting of $C_{1-6}$ alkyl, $C_{2-6}$ alkenyl, $C_{2-6}$ alkynyl, $C_{3-6}$ cycloalkyl, $C_{5-7}$ cycloalkenyl, $C_{7-11}$ aralkyl, $C_{6-14}$ aryl, $C_{1-6}$ alkoxy, $C_{6-14}$ aryloxy, $C_{1-6}$ alkylcarbonyl, $C_{6-14}$ arylcarbonyl, $C_{1-6}$ alkanoyloxy, $C_{6-14}$ arylcarbonyloxy, carboxyl, $C_{1-6}$ alkoxycarbonyl, carbamoyl, N-mono-$C_{1-4}$ alkylcarbamoyl, N,N-di-$C_{1-4}$ alkylcarbamoyl, halogen, mono-, di- or tri-halogeno-$C_{1-4}$ alkyl, oxo, amidino, imino, amino, mono- or di-$C_{1-4}$ alkylamino, OH and nitro.

(35) The oligonucleotide compound described in (34), in which $C_{6-11}$ aryl group is phenyl or naphthyl, or a 5 to 13-membered aromatic heterocyclic ring which comprises carbon atoms and about 1 to about 4 hetero atomos which are selected from the group consisting of nitrogen, oxygen and sulfur atoms and preferably is selected from the group consisting of 2- or 3-thienyl, 2- or 3-furyl, 2- or 3-pyrrolyl, 2-, 3- or 4-pyridyl, 2-, 4- or 5-oxazolyl, 2-, 4-or 5-thiazolyl, 3-, 4- or 5-pyrazolyl, 2-, 4- or 5-imidazolyl, 3-, 4- or 5-isoxazolyl, 3-, 4- or 5-isothiazolyl, 3- or 5-(1,2,4-oxadiazolyl), 1,3,4-oxadiazolyl, 3- or 5-(1,2,4-thiadiazolyl), 1,3,4-thiadiazolyl, 4- or 5-(1,2,3-thiadiazolyl), 1,2,5-thiadiazolyl, 1,2,3-triazolyl, 1,2,4-triazolyl, 1H- or 2H-tetrazolyl, N-oxide-2-, 3- or 4-pyridyl, 2-, 4- or 5-pyrimidinyl, N-oxide-2-, 4- or 5-pyrimidinyl, thiomorpholinyl, morpholinyl, oxoimidadinyl, dioxotriazinyl, pyrrolidinyl, piperidinyl, pyranyl, thiopyranyl, 1,4-oxazinyl, 1,4-thiazinyl, 1,3-thiazinyl, piperadinyl, triazinyl, oxotriazinyl, 3- or 4-pyridazinyl, pyrazinyl, N-oxide-3- or 4-pyridazinyl, benzofuryl, benzothiazolyl, benzooxazolyl, tetrazolo[1,5-b]pyridazinyl, triazolo-[4,5-b]pyridazinyl, benzoimidazolyl, quinolyl, isoquinolyl, cynnorynyl, phthalazinyl, quinazolinyl, quinoxalinyl, indolizinyl, quinolizinyl, 1,8-naphthylizinyl, purinyl, pteridinyl, dibenzofuranyl, carbazolyl, acridinyl, phenanthridinyl, chromanyl, benzoxazinyl, phenazinyl, phenothiazinyl or phenoxazinyl.

(36) The oligonucleotide compound described in any one of (31) to (35), in which $X^8$ is OH; SH; $C_{1-5}$ alkyl; $C_{1-5}$ alkoxy; $C_{1-5}$ monoalkylamino; or phenyl which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl.

(37) The oligonucleotide compound described in any one of (31) to (35), in which $X^8$ is selected from the group consisting of OH, SH, methyl, ethyl, propyl, phenyl, (o-, m-, p-)alkoxyphenyl, (o-, m-, p-)alkylphenyl, methoxy and ethoxy.

(38) The oligonucleotide compound described in any one of (31) to (35), in which $X^8$ is selected from the group consisting of OH, SH, methyl, ethyl, propyl, phenyl, (o-, m-, p-) $C_{1-10}$ alkoxyphenyl, (o-, m-, p-) $C_{1-10}$ alkylphenyl, methoxy and ethoxy.

(39) The oligonucleotide compound described in any one of (31) to (35), in which $X^8$ is OH, SH, methyl or phenyl.

(40) The oligonucleotide compound described in any one of (31) to (39), in which $R^1$, $R^2$ and $R^3$ are each selected from the group consisting of hydrogen, OH, fluoro, chloro, bromo, methoxy, ethoxy and methoxymethyloxy.

(41) The oligonucleotide compound described in any one of (31) to (39), in which $R^1$, $R^2$ and $R^3$ are each hydrogen.

(42) The oligonucleotide compound described in any one of (31) to (41), in which n is an integer from 1 to 38.

(43) The oligonucleotide compound described in any one of (31) to (41), in which n is an integer from 1 to 28.

(44) The oligonucleotide compound described in (32), in which n is an integer from 4 to 38; in the polymer unit, the first $X^8$ is phenyl and the (n-1)th $X^8$ is phenyl.

(45) The oligonucleotide compound or a salt thereof described in (1), which has a nucleotide sequence represented by formula:

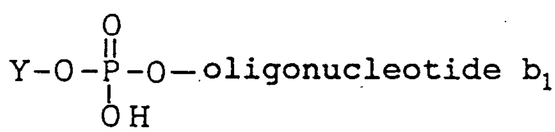

wherein Y represents a saccharide residue, and "oligonucleotide $b_1$" represents a nucleotide sequence selected from the group consisting of SEQ ID NO: 32, SEQ ID NO:33, SEQ ID NO:35, SEQ ID NO:37, SEQ ID NO:39, SEQ ID NO: 40, SEQ ID NO:41, SEQ ID NO:42, SEQ ID NO:43, SEQ ID NO:44, SEQ ID NO:45, SEQ ID NO:46, SEQ ID NO:47 and SEQ ID NO:48.

(46) The oligonucleotide compound described in (45), in which a saccharide of the saccharide residue represented by Y is galactose, mannose, melibiose, gentiobiose, isomaltotriose, n-octylglucoside, n-octylthioglucoside, phenylglucoside, galactosamine, N-benzoylgalactosamine, N-acetylgalactosamine, glucosamine, N-benzoylglucosamine, N-acetylglucosamine.

(47) The oligonucleotide compound described in (45), in which a saccharide of the saccharide residue represented by Y is melibiose, gentiobiose or N-benzoylgalactosamine.

(48) The oligonucleotide compound described in (1), which has a nucleotide sequence represented by formula:

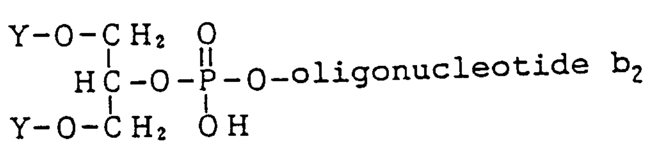

wherein Y represents a saccharide residue, and "oligonucleotide $b_2$" represents a nucleotide sequence selected from the group consisting of SEQ ID NO: 32, SEQ ID NO:33, SEQ ID NO:35, SEQ ID NO:37, SEQ ID NO:39, SEQ ID NO: 40, SEQ ID NO:41, SEQ ID NO:42, SEQ ID NO:43, SEQ ID NO:44, SEQ ID NO:45, SEQ ID NO:46, SEQ ID NO:47 and SEQ ID NO:48.

(49) The oligonucleotide compound described in (48), in which a saccharide of the saccharide residue represented by Y is galactose, mannose or glucose.

(50) The oligonucleotide compound described in (48), in which a saccharide of the saccharide residue represented by Y is galactose.

(51) A method for producing the oligonucleotide compound of (1), which comprises removing a protective group of either (a) a compound represented by formula:

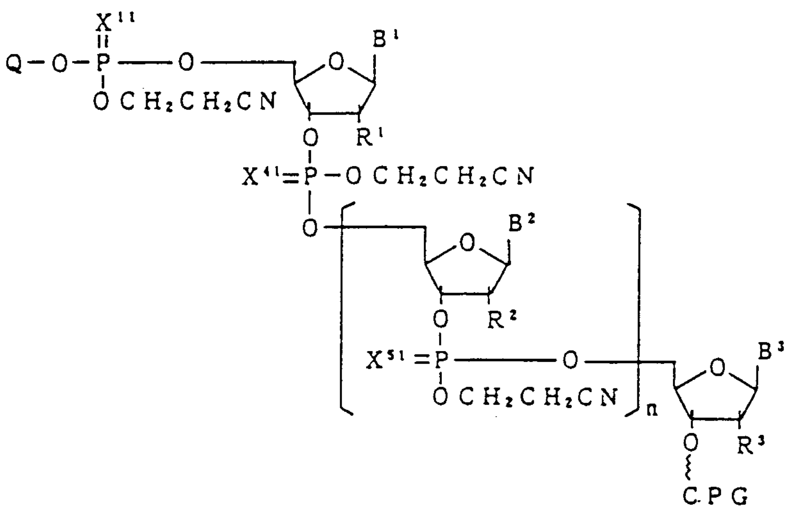

wherein Q, $B^1$, $B^2$, $B^3$, $R^1$, $R^2$, $R^3$ and n have the same meanings as given in (1) above; $X^{11}$, $X^{41}$ and $X^{51}$ each represent O or S, wherein each $X^{51}$ may be the same or different when n is 2 or more; and CPG represents a solid phase carrier; or

(b) a compound represented by formula:

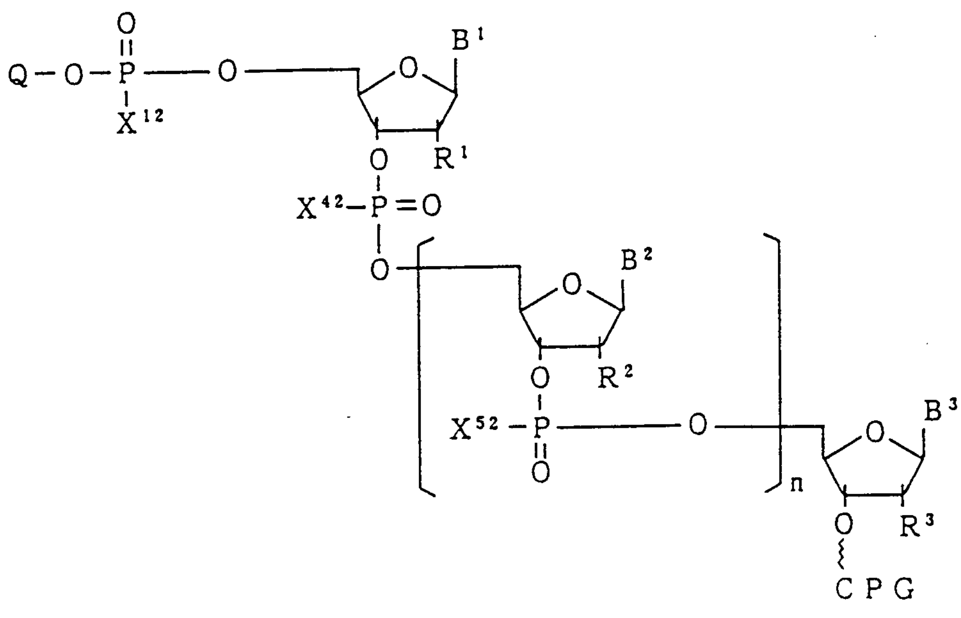

wherein Q, $B^1$, $B^2$, $B^3$, $R^1$, $R^2$, $R^3$ and n have the same meanings as given in (1); $X^{12}$, $X^{42}$ and $X^{52}$ each represent $C_{1-5}$ alkyl, $C_{1-5}$ alkoxy, $C_{1-5}$ monoalkylamino or aromatic ring group which may be substituted or unsubstituted, wherein each $X^{52}$ may be the same or different when n is 2 or more; and CPG represents a solid phase carrier.

(52) A composition for inhibiting a gene expression comprising the oligonucleotide compound or the salt thereof described in any one of (1) to (50).

(53) The composition for inhibiting a gene expression described in (52), which is an inhibitor for expression of a gene contributing to the development of a malignant tumor, a viral disease or an inflammation.

(54) The composition for inhibiting a gene expression described in (52) or (53), in which inhibition of the gene expression is (i) inhibition of transcription from DNA to pre mRNA, (ii) inhibition of splicing from pre mRNA to mature mRNA, or (iii) inhibition of translation from mature mRNA to a protein.

(55) The composition for inhibiting a gene expression described in (53), in which the gene contributing to the development of the malignant tumor is a gene selected from the group of genes consisting of src, fps, yes, ros, myb, myc, erb, rel, mos, abl, ras, fos, fes, fms, sis, raf, neu and p53, the gene contributing to the development of the virus disease is a gene selected from the genome of the group of viruses consisting of HIV, an influenza virus, a herpes virus, a papilloma virus, a poliovirus, a picornavirus and an adenovirus, and the gene contributing to the development of the inflammation is a gene selected from the group consisting of ICAM-1, ELAM-1 and VLAM.

(56) The composition for inhibiting a gene expression described in (52), in which the gene is ICAM-1.

(57) A composition for inhibiting reangiogenesis after PTCA which comprises a nucleotide compound or a salt thereof of any one of (1) to (50).

[D-1] A saccharide derivative as shown in (1) to (5) below:

(1) A saccharide derivative represented by the following formula:

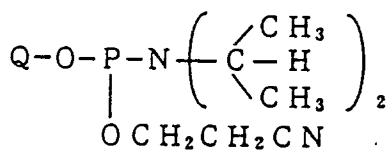

wherein Q represents Y or formula:

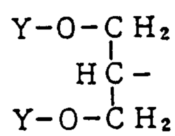

wherein Y represents a saccharide residue.

(2) The saccharide derivative described in [D-1] (1) (hereinafter in part [D-1] the [D-1] will be omitted, but the section refers to Part [D-1]), in which Q is Y, and a saccharide of the saccharide residue represented by Y is (i) galactose, mannose, melibiose, gentiobiose or isomaltotriose; or a hydroxyl group thereof is substituted by $C_{1-5}$ alkylcarbonyl or benzoyl group, (ii) glucoside or thioglucoside, or a hydroxyl group other than a hemiacetal hydroxyl group thereof is substituted by $C_{1-5}$ alkylcarbonyl, benzoyl or $C_{1-20}$ alkyl, or (iii) glucosamine or galactosamine, or a hydroxyl group thereof is substituted by $C_{1-5}$ alkylcarbonyl or benzoyl which may be substituted by halogens.

(3) The saccharide compound described in (1), in which Q is Y, and a saccharide of the saccharide residue represented by Y is 1,2,3,4-O-tetraacetylgalactose, 1,2,3,4-O-tetraacetylmannose, 1-O-n-octyl-2,3,4-O-tribenzoylglucoside, 1-O-n-octyl-2,3,4-O-trithiobenzoylglucoside, 1-O-phenyl-2,3,4-O-tribenzoylglucoside, 1,3,4-O-tribenzoyl,2-N-benzoylgalactosamine, 1,3,4-O-triacetyl,2-N-acetylgalactosamine, 1,3,4-O-triacetyl,2-N-acetylglucosamine, 1,3,4-O-tribenzoyl,2-N-trifluoroacetylgalactosamine, 1,3,4-O-tribenzoyl,2-N-trifluoroacetylglucosamine, heptabenzoylmelibiose, heptabenzoylgentiobiose or octabenzoylmaltotriose.

(4) The saccharide compound described in (1), in which Q is represented by formula:

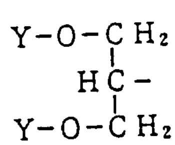

wherein Y represents a saccharide residue, and a saccharide of the saccharide residue represented by Y is a monosaccharide.

(5) The saccharide derivative described in (1), in which Q is represented by formula:

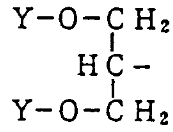

wherein Y represents a saccharide residue, and a saccharide of the saccharide residue represented by Y is galactose, mannose or glucose of which a hydroxyl group may be substituted by $C_{1-5}$ alkylcarbonyl.

[D-2] A saccharide derivative as shown in (1) to (6) below:

(1) A saccharide derivative which is represented by formula:

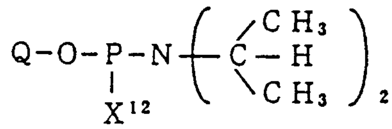

wherein Q is Y or represented by formula

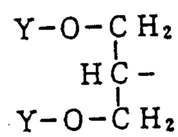

in which Y represents a saccharide residue; and $X^{12}$ represents $C_{1-5}$ alkyl, $C_{1-5}$ alkoxy, $C_{1-5}$ monoalkylamino or an aromatic group which may be substituted or unsubstituted.

(2) The saccharide derivative described in [D-2] (1) (hereinafter in Part [D-2] the reference to [D-2] will be omitted, but the section refers to Part [D-1]), in-which Q is Y, and a saccharide of the saccharide residue represented by Y is (i) galactose, mannose, melibiose, gentiobiose or isomaltotriose; or a hydroxyl group thereof is substituted by a $C_{1-5}$ alkylcarbonyl or a benzoyl group, (ii) glucoside or thioglucoside, or a hydroxyl group thereof is substituted by a $C_{1-5}$ alkylcarbonyl, a benzoyl group or a $C_{1-20}$ alkyl, or (iii) glucosamine or galactosamine, or a hydroxyl group thereof is substituted by $C_{1-5}$ alkylcarbonyl or benzoyl which may be substituted by halogens.

(3) The saccharide derivative described in (1), in which Q is Y, and a saccharide of the saccharide residue represented by Y is 1,2,3,4-O-tetraacetylgalactose, 1,2,3,4-O-tetraacetylmannose, 1-O-n-octyl-2,3,4-O-tribenzoylglucoside, 1-O-n-octyl-2,3,4-O-trithiobenzoylglucoside, 1-O-phenyl-2,3,4-O-tribenzoylglucoside, 1,3,4-O-tribenzoyl,2-N-benzoylgalactosamine, 1,3,4-O-triacetyl,2-N-acetylgalactosamine, 1,3,4-O-triacetyl,2-N-acetylglucosamine, 1,3,4-O-tribenzoyl,2-N-trifluoroacetylgalactosamine, 1,3,4-O-tribenzoyl,2-N-trifluoroacetylglucosamine, heptabenzoylmelibiose, heptabenzoylgentiobiose or octabenzoylmaltotriose.

(4) The saccharide derivative described in (1), in which Q is Y, and a saccharide of the saccharide residue represented by Y is 1-O-n-octyl-2,3,4-O-tribenzoylglucoside or 1-O-n-octyl-2,3,4-O-trithiobenzoylglucoside.

(5) The saccharide derivative described in (1), in which Q is represented by formula:

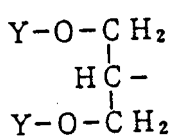

wherein Y represents a saccharide residue, and a saccharide of the saccharide residue represented by Y is a monosaccharide of which a hydroxyl group may be substituted by $C_{1-5}$ alkylcarbonyl.

(6) The saccharide derivative described in (1), in which Q is represented by formula:

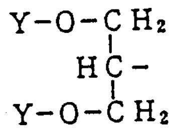

wherein Y represents a saccharide residue, and a saccharide of the saccharide residue represented by Y is galactose, mannose or glucose of which a hydroxyl group may be substituted by $C_{1-5}$ alkylcarbonyl.

(7) The saccharide derivative described in any one of (1) to (6), in which $X^{12}$ is $C_{1-5}$ alkyl; $C_{1-5}$ alkoxy; $C_{1-5}$ monoalkylamino; or phenyl which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl.

[D-3] A saccharide derivative as shown in (1) to (4) below:

(1) A saccharide derivative which is represented by formula:

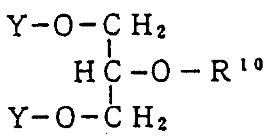

wherein Y represents a saccharide residue; and $R^{10}$ represents hydrogen or benzyl group.

(2) The saccharide derivative described in [D-3] (1) (hereinafter in Part [D-3] the reference to [D-3] will be omitted, but the section refers to Part [D-3]), in which the saccharide of the saccharide residue represented by Y is a monosaccharide of which a hydroxyl group may be substituted by $C_{1-5}$ alkylcarbonyl.

(3) The saccharide derivative described in (1), in which the saccharide of the saccharide residue represented by Y is galactose, mannose or glucose of which an alcoholic hydroxyl group may be substituted by $C_{1-5}$ alkylcarbonyl.

(4) The saccharide derivative described in (1), in which the saccharide of the saccharide residue represented by Y is galactose of which a hydroxyl group may be substituted by $C_{1-5}$ alkylcarbonyl.

[E] A nucleotide compound as shown in (1) to (8) below:

(1) A nucleotide compound which is represented by formula:

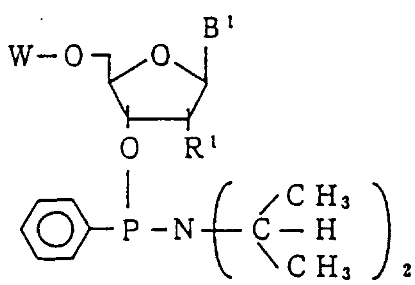

wherein W represents hydrogen or a protective group; $B^1$ represents a nucleic acid residue, $R^1$ represents hydrogen, OH, halogen, $C_{1-5}$ alkoxy or $C_{1-5}$ alkoxyalkyloxy.

(2) The nucleotide compound described in [E] (1) (hereinafter in Part [E] before the Section ( ) will be omitted), in which W is hydrogen or represented by formula

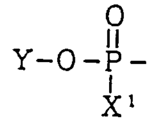

or

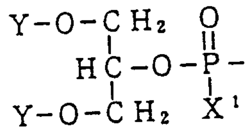

in which Y represents a saccharide residue; $X^1$ represents OH, SH, $C_{1-5}$ alkyl, $C_{1-5}$ alkoxy, $C_{1-5}$ monoalkylamino or an aromatic ring group which may be substituted or unsubstituted.

(3) The nucleotide compound described in (1), in which W is hydrogen or represented by formula

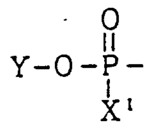

and a saccharide of the saccharide residue represented by Y is (i) galactose, mannose, melibiose, gentiobiose or isomaltotriose; or a hydroxyl group thereof is substituted by a $C_{1-5}$ alkylcarbonyl or benzoyl group, (ii) glucoside or thioglucoside, or a hydroxyl group thereof is substituted by a $C_{1-5}$ alkylcarbonyl, a benzoyl or a $C_{1-20}$ alkyl, or (iii) glucosamine or galactosamine, or a hydroxyl group thereof is substituted by a $C_{1-5}$ alkylcarbonyl or a benzoyl group which may be substituted by halogens.

(4) The nucleotide compound described in (1), in which W is hydrogen or represented by formula

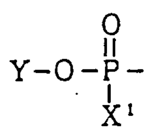

and a saccharide of the saccharide residue represented by Y is 1,2,3,4-O-tetraacetylgalactose, 1,2,3,4-O-tetraacetylmannose, 1-O-n-octyl-2,3,4-O-tribenzoylglucoside, 1-O-n-octyl-2,3,4-O-trithiobenzoylglucoside, 1-O-phenyl-2,3,4-O-tribenzoylglucoside, 1,3,4-O-tribenzoyl,2-N-benzoylgalactosamine, 1,3,4-O-triacetyl,2-N-acetylgalactosamine, 1,3,4-O-triacetyl,2-N-acetylglucosamine, 1,3,4-O-tribenzoyl,2-N-trifluoroacetylgalactosamine, 1,3,4-O-tribenzoyl,2-N-trifluoroacetylglucosamine, heptabenzoylmelibiose, heptabenzoylgentiobiose or octabenzoylmaltotriose.

(5) The nucleotide compound described in (1), in which W is hydrogen or represented by formula:

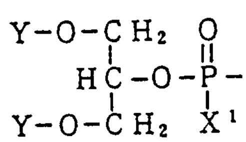

wherein a saccharide of the saccharide residue represented by Y is a monosaccharide of which a hydroxyl group may be substituted by $C_{1-5}$ alkylcarbonyl.

(6) The nucleotide compound as claimed in (1), in which W is hydrogen or represented by formula:

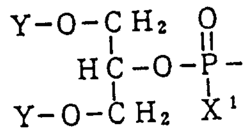

wherein a saccharide of the saccharide residue represented by Y is galactose, mannose or glucose of which a hydroxyl group may be substituted by $C_{1-5}$ alkylcarbonyl.

(7) The nucleotide compound described in (1) to (6), in which $X^1$ is (i) OH, (ii) SH, (iii) $C_{1-5}$ alkyl, (iv) $C_{1-5}$ alkoxy, (v) $C_{1-5}$ monoalkylamino, or (vi) phenyl which may be substituted by about 1 to about 5 substituents which are selected from the group consisting of halogen, $C_{1-10}$ alkylcarbonyl, OH, nitro, $C_{1-10}$ alkyl, $C_{1-10}$ alkoxy, phenyl and naphthyl.

(8) The nucleotide compound described in (1) to (6), in which $X^1$ is OH, SH, methyl or phenyl.

In the present specification, W represents hydrogen or a protective group.

The protective group represented by W may be any protective group as long as it can protect an OH group. For example, the protective groups include

(i) a group represented by formula

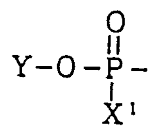

in which Y represents a saccharide residue; and $X^1$ represents OH, SH, $C_{1-5}$ alkyl, $C_{1-5}$ alkoxy, $C_{1-5}$ monoalkylamino or an aromatic ring group which may be substituted or unsubstituted,

(ii) a group represented by formula

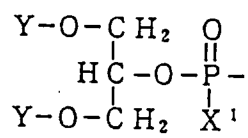

in which Y represents a saccharide residue; and $X^1$ represents OH, SH, $C_{1-5}$ alkyl, $C_{1-5}$ alkoxy, $C_{1-5}$ monoalkylamino or an aromatic ring group which may be substituted or unsubstituted,

(iii) a $C_{1-6}$ alkyl group (for example, methyl, ethyl, n-propyl, i-propyl, n-butyl and tert-butyl) which may be substituted by a halogen (for example, fluoro, chloro, bromo, iodo) or nitro,

(iv) a $C_{6-11}$ aryl group (for example, phenyl and naphthyl) which may be substituted by a halogen (for example, fluoro, chloro, bromo, iodo), $C_{1-6}$ alkyl group (for example, methyl, ethyl, n-propyl, i-propyl, n-butyl and tert-butyl), phenyl or nitro,

(v) a $C_{7-12}$ aralkyl group (for example, benzyl) which may be substituted by a halogen (for example, fluoro, chloro, bromo, iodo), $C_{1-6}$ alkyl group (for example, methyl, ethyl, n-propyl, i-propyl, n-butyl and tert-butyl), phenyl or nitro,

(vi) a $C_{1-6}$ alkylcarbonyl group (for example, formyl, methylcarbonyl, ethylcarbonyl) which may be substituted by halogen (for example, fluoro, chloro, bromo, iode),

(vii) phenyloxycarbonyl (for example, benzoxycarbonyl);

(viii) $C_{7-12}$ aralkyl-carbonyl (for example, benzyloxycarbonyl);

(ix) pyranyl;

(x) furanyl;

(xi) silyl;

(xii) trityl which may be substituted with 1 to 3 methoxy groups (for example, trityl, methoxy trityl, dimethoxy trityl, trimethoxy trityl); or

(xiii) phenylxanthenyl which may be substituted with methoxy groups (for example, phenylxanthenyl and methoxyphenylxanthenyl).

In the present specification, Y represents a saccharide residue.

As the saccharides of the saccharide residues, any saccharides per se known in the art can be used, whether they are chemically synthesized or naturally occurring. Specifically, monosaccharides which may be substituted or condensation products thereof can be used.

Examples of the monosaccharides used include aldopentoses which may be substituted or unsubstituted (for example, ribose, arabinose, xylose and lyxose), ketopentoses (for example, ribulose and xylulose), aldohexoses (for example, allose, altrose, glucose, mannose, gulose, idose, galactose and talose), and ketohexoses (for example, psicose, fructose, sorbose and tagatose).

In the monosaccharides, for example, the hydroxyl groups may be substituted by about 1 to about 4 substituent groups. Examples of the substituent groups include acyl groups which may be substituted or unsubstituted, alkyl groups which may be substituted or unsubstituted, and amino groups which may be substituted or unsubstituted.

The acyl groups which may be substituted include $C_{1-10}$ alkylcarbonyl (for example, $C_{1-6}$ alkylcarbonyl such as formyl, acetyl and ethylcarbonyl), phenylcarbonyl, $C_{7-10}$ aralkylcarbonyl (for example, benzylcarbonyl), $C_{1-10}$ alkoxycarbonyl (for example, methoxycarbonyl, ethoxycarbonyl and n-propoxycarbonyl), phenyloxycarbonyl (for example, benzoxycarbonyl), naphthyloxycarbonyl, $C_{7-10}$ aralkyloxycarbonyl (for example, benzyloxycarbonyl) and arylsulfenyl (for example, phenylsulfenyl and naphthylsulfenyl). As the acyl groups, for example, $C_{1-10}$ acyl groups are preferred. For example, the $C_{1-10}$ alkylcarbonyl groups (for example, $C_{1-6}$ alkylcarbonyl such as formyl, acetyl and ethylcarbonyl), the phenylcarbonyl group and the benzylcarbonyl group are preferred. The substituent groups for the acyl groups include, for example, halogen atoms (for example, fluoro, chloro, bromo and iodo), $C_{1-10}$ alkylcarbonyl (for example, formyl, methylcarbonyl and ethylcarbonyl), nitro, $C_{1-10}$ alkyl (for example, methyl and ethyl), $C_{1-10}$ alkoxy (for example, methoxy and ethoxy), phenyl and naphthyl, and the number of the substituent groups is usually about 1 to about 5, and preferably about 1 to about 3.

The alkyl groups which may be substituted include, for example, $C_{1-20}$ alkyl (for example, methyl, ethyl, n-propyl, i-propyl, n-butyl, n-pentyl, n-hexyl, n-heptyl and n-octyl), and $C_{1-6}$ alkyl (for example, methyl, ethyl, n-propyl, i-propyl, n-butyl, n-pentyl and n-hexyl) can be preferably used. The substituent groups for these alkyl groups include, for example, halogen (for example, fluoro, chloro, bromo and iodo), $C_{1-10}$ alkylcarbonyl (for example, $C_{1-6}$ alkylcarbonyl such as formyl, acetyl and ethylcarbonyl), nitro, $C_{1-10}$ alkyl (for example, methyl and ethyl), $C_{1-10}$ alkoxy (for example, methoxy and ethoxy), phenyl and naphthyl, and the number of the substituent groups is usually about 1 to about 5, and preferably about 1 to about 3.

The amino groups which may be substituted include, for example, groups represented by formula $-NR^4R^5$, wherein $R^4$ and $R^5$ each represent hydrogen atoms, acyl groups which may be substituted or unsubstituted or alkyl groups which may be substituted or unsubstituted. The substituent groups described above can be used for the acyl groups which may be substituted or unsubstituted and the alkyl groups which may be substituted. These amino groups are preferably substituted at the 2-positions of monosaccharides. Thus, the monosaccharides of which the hydroxyl groups are substituted by the amino groups are so-called amino saccharide. Preferred examples of such amino saccharide include glucosamine, galactosamine, N-methylglucosamine, 3-amino-3-deoxyglucose, glosamine, neosamine B, neosamine C, fucosamine, rhamnosamine, aminomannuronic acid, quinovosamine, in which the amino groups may be substituted by $R^4$ and/or $R^5$ as described above. Particularly preferred examples thereof include glucosamine and galactosamine in which the 2-amino groups may be substituted by $C_{1-6}$ alkylcarbonyl groups or phenylcarbonyl groups, etc. (for example, glucosamine, galactosamine, N-acetylglucosamine, N-acetylgalactosamine, N-benzoylglucosamine and N-benzoylgalactosamine), especially glucosamine, N-acetylglucosamine, N-benzoylglucosamine and N-benzoylgalactosamine.

Among the above-mentioned monosaccharides, for example, aldopentoses (for example, ribose and arabinose) and aldohexoses (for example, glucose, mannose and galactose) are more preferred. Galactose, mannose and glucose are even more preferable.

As the substituent groups for the hydroxyl groups of the monosaccharides, for example, $C_{1-10}$ alkylcarbonyl (for example, $C_{1-6}$ alkylcarbonyl such as formyl, acetyl and ethylcarbonyl), acyl such as phenylcarbonyl, and $C_{1-20}$ alkyl (for example, methyl, ethyl, n-propyl, i-propyl, n-butyl, n-pentyl, n-hexyl, n-heptyl and n-octyl) are preferred.

As the monosaccharides represented by Y in formula (I), the aldohexoses in which the hydroxyl groups may be substituted by the $C_{1-6}$ alkylcarbonyl groups are particularly preferred. More specifically, for example, galactose, mannose, 1,2,3,4-O-tetraacetylgalactose and 1,2,3,4-O-tetraacetylmannose, glucosamine, N-acetylglucosamine, N-benzoylglucosamine and N-benzoylgalactosamine are preferred.

As the condensation products of the monosaccharides, for example, oligosaccharides and glycoside compounds which may be substituted are employed.

As the oligosaccharides, for example, condensation products of 2 to 15 monosaccharides described above, and preferably of 2 to 8 monosaccharides are used. Condensation products of 2 to 5 monosaccharides described above, and preferably of 2 and 3 monosaccharides are also employed. Specifically, the condensation products which naturally occur in a free state or as constituents of glycosides are used. Examples thereof include (1) oligosaccharides occurring in the animal kingdom such as lactose, $\alpha,\alpha$-trehalose, N-acetylneuraminyllactose, difucosyllactose, fucosyllactose and lactulose, (2) oligosaccharides occurring in the vegetable kingdom such as gentianose, isolychnose, lychnose, planteose, sucrose, bifrucose, 1,6-diglucosylmannitol, galactosylsucrose 6-$\beta$-glucosylmannitol, laminaribiose, inulobioses, lycopose, maltose, isomaltotriose, melibiose, neobifucose, neokestose, eruloses, 6-$\alpha$-galactosylgalactose, kojibiose, maruzulose, melezitose and turanose, and (3) oligosaccharides existing as constituents of glycosides such as sophorose, gentiooligosaccharides, vicianose, primeverose, sanbubiose, lycobiose, lycotriose, solabiose, strophantobiose, digilanidobiose, odorotriose, fungitetraose and $\beta$-1,3-xylooligosaccharides. Of these, for example, gentiobiose, melibiose and isomaltotriose are preferred.

In the above-mentioned oligosaccharides, for example, the hydroxyl groups may be substituted. As the substituent groups therefor, substituent groups similar to those for the above-mentioned monosaccharides, for example, acyl groups which may be substituted, alkyl groups which may be substituted, and amino groups which may be substituted are employed.

As the glycoside compounds, glycoside compounds in which the hydroxyl groups of the above-mentioned monosaccharides or oligosaccharides are substituted by aglycons, non-saccharide components, (for example, O-glycoside compounds, S-glycoside compounds and N-glycoside compounds) are used. Examples of the aglycons used herein include -O-$R^7$, -S-$R^8$ and -N-$R^9$, wherein $R^7$, $R^8$ and $R^9$ each represent hydrocarbon groups which may be substituted. Examples of the hydrocarbon groups which may be substituted are, represented by $R^7$, $R^8$ and $R^9$, and include alkyl, alkenyl, alkynyl, aryl and aralkyl. Examples of the alkyl groups include $C_{1-30}$ alkyl (for example, methyl, ethyl, n-propyl, i-propyl, n-butyl, n-pentyl, n-hexyl, n-heptyl, n-octyl and stearyl), and particularly $C_{1-20}$ alkyl (for example, methyl, ethyl, n-propyl, i-propyl, n-butyl, n-pentyl, n-hexyl, n-heptyl, n-octyl and stearyl) are preferred. As the alkenyl groups, for example, $C_{2-4}$ alkenyl (for example, allyl and propenyl) are used. As the alkynyl groups, for example, $C_{2-4}$ alkynyl (for example, ethynyl and propynyl) are used. As the aryl groups, for example, $C_{6-12}$ aryl such as phenyl and naphthyl are used. As the aralkyl groups, for example, $C_{7-14}$ aralkyl (for example, benzyl, phenylethyl, naphthylmethyl and naphthylethyl) are used.

Examples of the substituent groups for the above-mentioned alkyl, alkenyl and alkynyl groups include halogen (for example, fluoro, chloro, bromo and iodo), $C_{1-10}$ alkylcarbonyl (for example, formyl, acetyl and ethylcarbonyl), nitro and $C_{1-10}$ alkoxy (for example, methoxy and ethoxy). The number of the substituent groups is usually about 1 to about 5, and preferably about 1 to about 3.

Examples of the substituent groups of the above-mentioned aryl and aralkyl groups include halogen (for example, fluoro, chloro, bromo and iodo), $C_{1-10}$ alkylcarbonyl (for example, formyl, acetyl and ethylcarbonyl), nitro, $C_{1-10}$ alkyl (for example, methyl and ethyl) and $C_{1-10}$ alkoxy (for example, methoxy and ethoxy). The number of the substituent groups is usually about 1 to about 5, and preferably about 1 to about 3.

$R^7$, $R^8$ and $R^9$ are each preferably $C_{1-30}$ alkyl (for example, methyl, ethyl, n-propyl, i-propyl, n-butyl, n-pentyl, n-hexyl, n-heptyl, n-octyl and stearyl) or $C_{6-12}$ aryl (for example, phenyl and naphthyl).

Preferred examples of the glycoside compounds include n-octylglucoside, n-octylgalactoside, n-octylmannoside, n-octylthioglucoside, n-octylthiogalactoside, n-octylthiomannoside, stearylglycoside, stearylgalactoside, stearylmannoside, stearylthioglycoside, stearylthiogalactoside, stearylthiomannoside, phenylglucoside, phenylgalactoside and phenylmannoside.

Preferable saccharides of a saccharide residue represented by Y include Gal(galactose), Man(mannose), Glu(glucose), n-oct-Glc(n-octylglucoside), n-oct-SGlc(n-octyl-thioglucoside), PhGlc(phenylglucoside), PhGal(phenylgalactoside), GalN(galactosamine), N-AcGal(N-acetylgalactosamine), N-BzGal(N-benzoylgalactosamine), GulN(glucosamine), N-AcGul(N-acetylglucosamine), Mel(melibiose), Gen(gentiobiose) and Iso(isomaltotriose).

For the oligonucleotide compounds represented by (Ia) of the present invention, Gal(galactose), Man(mannose), n-oct-Glc(n-octylglucoside), n-oct-SGlc(n-octylthioglucoside), PhGlc(phenylglucoside), PhGal(phenylgalactoside), GalN(galactosamine), N-AcGal(N-acetylgalactosamine), N-BzGal(N-benzoylgalactosamine), GulN(glucosamine), N-AcGul(N-acetylglucosamine), Mel(melibiose), Gen(gentiobiose) and Iso(isomaltotriose) are preferable. For the oligonucleotide compounds of Foumula (Ib) of the present invention, Mel(melibiose), Gen(gentiobiose) and N-BzGal(N-benzoylgalactosamine) are preferable.

In the present specification, $X^1$ represents OH, SH, $C_{1-5}$ alkyl, $C_{1-5}$ alkoxy, $C_{1-5}$ monoalkylamino or an aromatic ring group which may be substituted or unsubstituted.

As the $C_{1-5}$ alkyl groups, for example, methyl, ethyl, n-propyl, i-propyl, n-butyl, i-butyl and tert-butyl may be used. As the $C_{1-5}$ alkoxy groups, for example, methoxy, ethoxy and propoxy may be used. As the $C_{1-5}$ monoalkylamino groups, methylamino, ethylamino and n-propylamino may be used. The substituent groups for the phenyl groups which may be substituted include, for example, halogen (for example, fluoro, chloro, bromo and iodo), $C_{1-10}$ alkylcarbonyl (for example, $C_{1-6}$ alkylcarbonyl such as formyl, acetyl and ethylcarbonyl), OH, nitro, $C_{1-10}$ alkyl (for example, methyl and ethyl), $C_{1-10}$ alkoxy (for example, methoxy, ethoxy, n-propyloxy, i-propyloxy, n-butyloxy, i-butyloxy and tert-butyloxy), phenyl and naphthyl. The number of the substituent is usually about 1 to about 5, and preferably about 1 to about 3.

The aromatic ring groups include aryl group and aromatic heterocyclic group.

The aryl group includes $C_{6-11}$ aryl such as phenyl and naphthyl, and phenyl is preferable.

The substituents for the aryl group include halogen(for example, fluoro, chloro, bromo and iodo), $C_{1-10}$ alkyl-carbonyl (for example, formyl, acetyl, propionyl, n-butiryl and isobutiryl), OH, nitro, $C_{1-10}$ alkyl (for example, methyl, ethyl, propyl, isopropyl, butyl, isobutyl, sec-butyl, tert-butyl), $C_{1-10}$ alkoxy (for example, methoxy, ethoxy, propoxy, iso-propoxy, n-butoxy, iso-butoxy, sec-butoxy and tert-butoxy), phenyl and naphthyl. The number of the substituent groups is usually about 1 to about 5, and preferably about 1 to about 3.

The aromatic heterocyclic groups include a 5 to 13-membered aromatic heterocyclic ring which comprises 1 to 12 carbon atoms and 1 to about 4 hetero atomos which are selected from the group consisting of nitrogen, oxygen and sulfur atoms which may be substituted by about 1 to about 5 substituents, especially 5 to 9-membered aromatic heterocyclic groups are preferable.

The aromatic heterocyclic groups include (1) 5-membered aromatic heterocyclic groups which contain 1 to 8 carbon atoms and 1 to 4 hetero atoms selected from the group consisting of oxygen, sulfur and nitrogen such as 2-or 3-thienyl, 2- or 3-furyl, 2- or 3-pyrrolyl, 2-, 3- or 4-pyridyl, 2-, 4- or 5-oxazolyl, 2-, 4- or 5-thiazolyl, 3-, 4- or 5-pyrazolyl, 2-, 4- or 5-imidazolyl, 3-, 4- or 5-isoxazolyl, 3-, 4- or 5-isothiazolyl, 3- or 5-(1,2,4-oxadiazolyl), 1,3,4-oxadiazolyl, 3- or 5-(1,2,4-thiadiazolyl), 1,3,4-thiadiazolyl, 4- or 5-(1,2,3-thiadiazolyl), 1,2,5-thiadiazolyl, 1,2,3-triazolyl, 1,2,4-triazolyl, 1H- or 2H-tetrazolyl; (2) 6-membered aromatic heterocyclic groups which contain 2 to 5 carbon atoms and 1 to 4 hetero atoms selected from the group consisting of oxygen, sulfur and nitrogen such as N-oxide-2-, 3- or 4-pyridyl, 2-, 4- or 5-pyrimidinyl, N-oxide-2-, 4- or 5-pyrimidinyl, thiomorpholinyl, morpholinyl, oxoimidadinyl, dioxotriazinyl, pyrrolidinyl, piperidinyl, pyranyl, thiopyranyl, 1,4-oxazinyl, 1,4-thiazinyl, 1,3-thiazinyl, piperadinyl, triazinyl, oxotriazinyl, 3- or 4-pyridazinyl, pyrazinyl, N-oxide-3- or 4-pyridazinyl;

(3) 5 to 9-membered aromatic heterocyclic groups which contain 1 to 8 carbon atoms and 1 to 4 hetero atoms selected from the group consisting of oxygen, sulfur and nitrogen such as benzofuryl, benzothiazolyl, benzooxazolyl, tetrazolo[1,5-b]pyridazinyl, triazolo[4,5-b]pyridazinyl, benzoimidazolyl, quinolyl, isoquinolyl, cynnorynyl, phthalazinyl, quinazolinyl, quinoxalinyl, indolizinyl, quinolizinyl, 1,8-naphthylizinyl, purinyl, pteridinyl, dibenzofuranyl, carbazolyl, acridinyl, phenanthridinyl, chromanyl, benzoxazinyl, phenazinyl, phenothiazinyl or phenoxazinyl.

The substituents for the aromatic heterocyclic groups include $C_{1-6}$ alkyl (for example, methyl, ethyl, propyl, isopropyl, butyl, isobutyl, sec-butyl, tert-butyl), $C_{2-6}$ alkenyl (for example, vinyl, 1-methylvinyl, 1-propenyl, allyl), $C_{2-6}$ alkynyl (for example, ethynyl, 1-propynyl, propargyl), $C_{3-6}$ cycloalkyl (for example, cyclopropyl, cyclobutyl, cyclopentyl, cyclohexyl), $C_{5-7}$ cycloalkenyl (for example, cyclopentenyl, cyclohex-enyl), $C_{7-11}$ aralkyl (for example, benzyl, $\alpha$-methylbenzyl, phenetyl), $C_{6-14}$ aryl (for example, phenyl, naphthyl), $C_{1-6}$ alkoxy (for example, methoxy, ethoxy, propoxy, iso-propoxy, n-butoxy, iso-butoxy, sec-butoxy and tert-butoxy), $C_{6-14}$ aryloxy (for example, phenoxy), $C_{1-6}$ alkylcarbonyl (for example, formyl, acetyl, propionyl, n-butyryl, iso-butyryl), $C_{6-14}$ arylcarbonyl (for example, benzoyl), $C_{1-6}$ alkanoyloxy (for example, formyloxy, acetyloxy, propionyloxy, n-butyryloxy, iso-butyryloxy), $C_{6-14}$ arylcarbonyloxy (for example, benzoyloxy), carboxyl, $C_{1-6}$ alkoxycarbonyl (for example, methoxycarbonyl, ethoxycarbonyl, n-propoxycarbonyl, iso-propoxycarbonyl, n-butoxycarbonyl, iso-butoxycarbonyl, tert-butoxycarbonyl), carbamoyl, N-mono-$C_{1-4}$ alkylcarbamoyl (for example, N-methylcarbamoyl, N-ethylcarbamoyl, N-propylcarbamoyl, N-isopropylcarbamoyl, N-butylcarbamoyl), N,N-di-$C_{1-4}$ alkylcarbamoyl (for example, N,N-dimethylcarbamoyl, N,N-diethylcarbamoyl, N,N-dipropylcarbamoyl, N,N-dibutylcarbamoyl), halogen (for example, fluoro, chloro, bromo, iodo), mono-, di- or trihalogeno-$C_{1-4}$ alkyl (for example, chloromethyl, dichloromethyl, trifluoromethyl, trifluoroethyl), oxo, amidino, imino, amino, mono- or di-$C_{1-4}$ alkylamino (for example, methylamino, ethylamino, propylamino, isopropylamino, butylamino, dimethylamino, diethylamino, dipropylamino, diisopropylamino, dibutylamino), OH and nitro. The number of the substituent groups is usually about 1 to about 5, and preferably about 1 to about 3.

$X^1$ is preferably OH, SH, methyl, ethyl, n-propyl, phenyl, (o-, m-, p-)$C_{1-10}$ alkoxyphenyl, (o-, m-, p-)$C_{1-10}$ alkylphenyl, methoxy or ethoxy. In particular, OH, SH, methyl and phenyl are preferred.

In the present specification, Q represents Y (Y is a saccharide residue) or formula

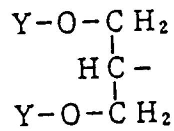

wherein Y represents a saccharide residue.

The saccharide residue represented by Y is the same as the above-mentioned residues.

In the present specification, $B^1$, $B^2$, $B^{21}$, $B^{22}$ and $B^3$ each represent a nucleic acid residue. The nucleic acid residues include hypoxanthine-9-yl, guanine-9-yl, adenine-9-yl, uracil-1-yl, thymine-1-yl and cytosine-1-yl. Each $B^2$ may be the same or different when n is 2 or more. $B^1$, $B^2$, $B^{21}$, $B^{22}$ and $B^3$ may be selected according to the nucleotide sequence to be synthesized.

$X^2$, $X^3$, $X^6$, $X^7$ and $X^6$ are each OH, SH, $C_{1-5}$ alkyl, $C_{1-5}$ alkoxy, $C_{1-5}$ monoalkylamino or an aromatic ring group which may be substituted or unsubstituted. $X^3$, $X^7$ and $X^6$ each may be the same or different when n is 2 or more.

$X^4$ and $X^6$ are each OH, SH, $C_{1-5}$ alkyl, $C_{1-5}$ alkoxy or $C_{1-5}$ monoalkylamino. Each $X^6$ may be the same or different when n is 2 or more.

$C_{1-5}$ alkyl represented by $X^2$, $X^3$, $X^4$, $X^6$, $X^6$, $X^7$ and $X^6$ include methyl, ethyl, n-propyl, i-propyl, n-butyl, i-butyl, tert-butyl.

$C_{1-5}$ alkoxy represented by $X^2$, $X^3$, $X^4$, $X^6$, $X^6$, $X^7$ and $X^6$ include methoxy, ethoxy, propoxy.

$C_{1-5}$ monoalkylamino represented by $X^2$, $X^3$, $X^4$, $X^6$, $X^6$, $X^7$ and $X^6$ include methylamino, ethylamino, n-propylamino.

Substituents for the phenyl which may be substituted include halogen (for example, fluoro, chloro, bromo and iodo), $C_{1-10}$ alkyl-carbonyl (for example $C_{1-6}$ alkyl-carbonyl such as formyl, acetyl and ethylcarbonyl), OH, nitro, $C_{1-10}$ alkyl (for example, methyl, ethyl, propytl, iso-propyl, n-butyl, iso-butyl, sec-butyl and tert-butyl), $C_{1-10}$ alkoxy (for example, methoxy, ethoxy, propoxy, iso-propoxy, n-butoxy, iso-butoxy, sec-butoxy and tert-butoxy), phenyl and naphthyl. The number of the substituent groups is usually about 1 to about 5, and preferably about 1 to about 3.

An aromatic ring group which may be substituted or unsubstituted and represented by $X^2$, $X^3$, $X^6$, $X^7$ and $X^6$ include an aryl group, and an aromatic heterocyclic group.

The aryl groups include $C_{6-11}$ aryl (for example, phenyl, naphthyl), and phenyl is preferable.

Substituents for the aryl which may be substituted include halogen (for example, fluoro, chloro, bromo and iodo), $C_{1-10}$ alkyl-carbonyl (for example, formyl, acetyl, propionyl, n-butiryl and isobutiryl), OH, nitro, $C_{1-10}$ alkyl (for example, methyl, ethyl, propytl, iso-propyl, n-butyl, iso-butyl, sec-butyl and tert-butyl), $C_{1-10}$ alkoxy (for example, methoxy, ethoxy, propoxy, iso-propoxy, n-butoxy, iso-butoxy, sec-butoxy and tert-butoxy), phenyl and naphthyl. The number of the substituent groups is usually about 1 to about 5, and preferably about 1 to about 3.

The aromatic heterocyclic groups include a 5 to 13-membered aromatic heterocyclic group which comprises 1 to 12 carbon atoms and 1 to about 4 hetero atomos which are selected from the group consisting of nitrogen, oxygen and sulfur atoms which may be substituted by about 1 to about 5 substituents, 5 to 9-membered aromatic heterocyclic groups are preferable.

The aromatic heterocyclic groups include (1) 5-membered aromatic heterocyclic groups which contain 1 to 4 carbon atoms and 1 to 4 hetero atoms selected from the group consisting of oxygen, sulfur and nitrogen such as 2-or 3-thienyl, 2- or 3-furyl, 2- or 3-pyrrolyl, 2-, 3- or 4-pyridyl, 2-, 4- or 5-oxazolyl, 2-, 4- or 5-thiazolyl, 3-, 4- or 5-pyrazolyl, 2-, 4- or 5-imidazolyl, 3-, 4- or 5-isoxazolyl, 3-, 4- or 5-isothiazolyl, 3- or 5-(1,2,4-oxadiazolyl), 1,3,4-oxadiazolyl, 3- or 5-(1,2,4-thiadiazolyl), 1,3,4-thiadiazolyl, 4- or 5-(1,2,3-thiadiazolyl), 1,2,5-thiadiazolyl, 1,2,3-triazolyl, 1,2,4-triazolyl, 1H- or 2H-tetrazolyl; (2) 6-membered aromatic heterocyclic groups which contain 2 to 5 carbon atoms and 1 to 4 hetero atoms selected from the group consisting of oxygen, sulfur and nitrogen such as N-oxide-2-, 3- or 4-pyridyl, 2-, 4- or 5-pyrimidinyl, N-oxide-2-, 4- or 5-pyrimidinyl, thiomorpholinyl, morpholinyl, oxoimidadinyl, dioxotriazinyl, pyrolydinyl, piperidinyl, pyranyl, thiopyranyl, 1,4-oxazinyl, 1,4-thiazinyl, 1,3-thiazinyl, piperadinyl, triazinyl, oxotriazinyl, 3- or 4-pyridazinyl, pyrazinyl, N-oxide-3- or 4-pyridazinyl;

(3) 5 to 9-membered aromatic heterocyclic groups which contain 1 to 8 carbon atoms and 1 to 4 hetero atoms selected from the group consisting of oxygen, sulfur and nitrogen such as benzofuryl, benzothiazolyl, benzooxazolyl, tetrazolo[1,5-b]pyridazinyl, triazolo[4,5-b]pyridazinyl, benzoimidazolyl, quinolyl, isoquinolyl,

cynnorynyl, phthalazinyl, quinazolinyl, quinoxalinyl, indolizinyl, quinolizinyl, 1,8-naphthylizinyl, purinyl, pteridinyl, dibenzofuranyl, carbazolyl, acridinyl, phenanthridinyl, chromanyl, benzoxazinyl, phenazinyl, phenothiazinyl or phenoxazinyl.

The substituents for the aromatic heterocyclic groups include $C_{1-6}$ alkyl (for example, methyl, ethyl, propyl, isopropyl, butyl, isobutyl, sec-butyl, tert-butyl), $C_{2-6}$ alkenyl (for example, vinyl, 1-methylvinyl, 1-propenyl, allyl), $C_{2-6}$ alkynyl (for example, ethynyl, 1-propynyl, propargyl), $C_{3-6}$ cycloalkyl (for example, cyclopropyl, cyclobutyl, cyclopentyl, cyclohexyl), $C_{5-7}$ cycloalkenyl (for example, cyclopentenyl, cyclohexenyl), $C_{7-11}$ aralkyl (for example, benzyl, $\alpha$-methylbenzyl, phenetyl), $C_{6-14}$ aryl (for example, phenyl, naphthyl), $C_{1-6}$ alkoxy (for example, methoxy, ethoxy, propoxy, iso-propoxy, n-butoxy, iso-butoxy, sec-butoxy and tert-butoxy), $C_{6-14}$ aryloxy (for example, phenoxy), $C_{1-6}$ alkylcarbonyl (for example, formyl, acetyl, propionyl, n-butyryl, iso-butyryl), $C_{6-14}$ arylcarbonyl (for example, benzoyl), $C_{1-6}$ alkanoyloxy (for example, formyloxy, acetyloxy, propionyloxy, n-butyryloxy, iso-butyryloxy), $C_{6-14}$ arylcarbonyloxy (for example, benzoyloxy), carboxyl, $C_{1-6}$ alkoxycarbonyl (for example, methoxycarbonyl, ethoxycarbonyl, n-propoxycarbonyl, iso-propoxycarbonyl, n-butoxycarbonyl, iso-butoxycarbonyl, tert-butoxycarbonyl), carbamoyl, N-mono-$C_{1-4}$ alkylcarbamoyl (for example, N-methylcarbamoyl, N-ethylcarbamoyl, N-propylcarbamoyl, N-isopropylcarbamoyl, N-butylcarbamoyl), N,N-di-$C_{1-4}$ alkylcarbamoyl (for example, N,N-dimethylcarbamoyl, N,N-diethylcarbamoyl, N,N-dipropylcarbamoyl, N,N-dibutylcarbamoyl), halogen (for example, fluoro, chloro, bromo, iodo), mono-, di- or trihalogeno-$C_{1-4}$ alkyl (for example, chloromethyl, dichloromethyl, trifluoromethyl, trifluoroethyl), oxo, amidino, imino, amino, mono- or di-$C_{1-4}$ alkylamino (for example, methylamino, ethylamino, propylamino, isopropylamino, butylamino, dimethylamino, diethylamino, dipropylamino, diisopropylamino, dibutylamino), OH and nitro. The number of the substituent groups is usually about 1 to about 6, and preferably about 1 to about 3.

$X^2$, $X^3$, $X^6$, $X^7$ and $X^6$ are each OH, SH, methyl, ethyl, n-propyl, phenyl, (o-, m-, p-)$C_{1-10}$ alkoxyphenyl, (o-, m-, p-)$C_{1-10}$ alkylphenyl, methoxy or ethoxy. In particular, OH, SH, methyl and phenyl are preferred.

$X^4$ and $X^6$ are preferably each OH, SH, methyl, ethyl, n-propyl, methoxy or ethoxy. In particular, OH, SH, methyl are preferred.

In the present specification, $R^1$, $R^2$, $R^{21}$, $R^{22}$ and $R^3$ are each hydrogen, OH, halogen, $C_{1-5}$ alkoxy, $C_{1-5}$ alkoxyalkyloxy, $R^2$ and $R^{21}$ may be the same or different when n is 2 or more. The halogen includes fluoro, chloro, bromo and iodo. $C_{1-5}$ alkoxy includes methoxy, ethoxy, n-propoxy, i-propoxy, n-butoxy. $C_{1-5}$ alkoxyalkyloxy includes $C_{1-5}$ alkoxy-$C_{1-6}$ alkyloxy such as methoxymethyloxy, methoxyethyloxy, ethoxymethyloxy, ethoxyethyloxy, ethoxypropyloxy.

$R^1$, $R^2$, $R^{21}$, $R^{22}$ and $R^3$ are each preferably hydrogen, OH, halogen, methoxy, ethoxy, methoxymethyloxy, and more preferably are hydrogens.

n represents an integer from 1 to 98. n is preferably an integer from 1 to 38, more preferably 1 to 28, and most preferably 5 to 18. Namely, the oligonucleotide compounds of the present invention are usually comprised of about 3 to about 100 nucletides, preferably about 3 to about 40 nucleotides, more preferably about 3 to about 30 nucleotides, and most preferably about 7 to about 20 nucleotides.

When an oligonucleotide chain is described below, for example in the nucleotide compound represented by Formula (Ia), oligonucleotide TTT ($B^1$, $B^2$ and $B^3$ are each thymine-1-yl; $R^1$, $R^2$ and $R^3$ are each hydrogen; n = 1) is disclosed as TTT when $X^1$, $X^2$ and $X^3$ are each OH, as $T_sT_sT$ when $X^1$ is OH and $X^2$ and $X^3$ are each SH, as $T_{Me}T_{Me}T$ when $X^1$ is OH and $X^2$ and $X^3$ are each methyl, as $T_{Ph}T_{Ph}T$ when $X^1$ is OH and $X^2$ and $X^3$ are each phenyl, and as $T_sT_{Ph}T$ when $X^1$ is OH and $X^2$ is SH and $X^3$ is phenyl.

In the oligonucleotide compounds of Formula (Ia) of the present invention, Q preferably represents Y or formula

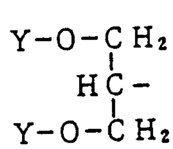

When Q is Y, a preferred saccharide of a saccharide residue represented by Y includes monosaccharides, oligosaccharides or glycoside compounds thereof which may be substituted by for example, $C_{1-10}$ alkylcarbonyl (for example, $C_{1-6}$ alkylcarbonyl such as formyl, acetyl and ethylcarbonyl), acyl such as phenylcarbonyl, and $C_{1-20}$ alkyl (for example, methyl, ethyl, n-propyl, i-propyl, n-butyl, n-pentyl, n-hexyl, n-heptyl and n-octyl).

Examples of the preferred monosaccharides used include aldopentoses (for example, ribose, arabinose) and aldohexoses (for example, glucose, mannose, galactose). Galactose, mannose and galactosamine are more preferable.

The preferred oligosaccharides include melibiose, gentiobiose and isomaltotriose. The preferred glycoside compounds include n-octylglucoside, n-octylthioglucoside and phenylglucoside. Glucosamine or galactosamine in which the 2-amino may be substituted by $C_{1-6}$ alkylcarbonyl or phenylcarbonyl is also preferable. Among them, (1) galactose or mannose in which a hydroxyl group may be substituted by $C_{1-6}$ alkylcarbonyl groups (for example, formyl, acetyl, ethylcarbonyl), (2) melibiose, gentiobiose or isomaltotriose in which an alcoholic hydroxyl group may be substituted by phenylcarbonyl, (3) n-octylglucoside, n-octylthioglucoside or phenylglucoside, and (4) glucosamine or galactosamine which may be substituted by $C_{1-10}$ alkylcarbonyl groups (for example, $C_{1-6}$ alkylcarbonyl such as formyl, acetyl, ethylcarbonyl) are preferable. Specifically, Gal(galactose), Man(mannose), n-oct-Glc(n-octylglucoside), n-oct-SGlc(n-octylthioglucoside), PhGlc(phenylglucoside), PhGal(phenylgalactoside), GalN(galactosamine), N-AcGal(N-acetylgalactosamine), N-BzGal(N-benzoylgalactosamine), GulN(glucosamine), N-AcGul(N-acetylglucosamine), Mel(melibiose), Gen(gentiobiose) and Iso(isomaltotriose) are more preferable.

Further, aldohexoses in which a hydroxyl group may be substituted by $C_{1-6}$ alkylcarbonyl groups (for example, formyl, acetyl, ethylcarbonyl) are also preferable. Specifically galactose, mannose, 1,2,3,4-O-tetraacetylgalactose, 1,2,3,4-O-tetraacetylmannose, glucosamine, N-acetylglucosamine, N-benzoylglucosamine and N-benzoylgalactosamine are more preferable.

When Q is represented by formula

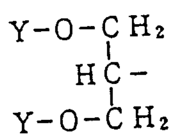

a preferred saccharide of a saccharide residue represented by Y includes monosaccharides. Galactose, mannose and glucose are more preferable, and galactose is more preferable.

$X^1$ is preferably (1) OH, (2) SH, (3) $C_{1-5}$ alkyl, (4) $C_{1-5}$ alkoxy, (5) $C_{1-5}$ monoalkylamino, (6) $C_{6-11}$ aryl (for example, phenyl, naphthyl) which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl, or (7) a 5 to 13-membered aromatic heterocyclic group which comprises 1 to 12 carbon atoms and 1 to about 4 hetero atomos which are selected from the group consisting of nitrogen, oxygen and sulfur atoms which may be substituted by about 1 to about 5 substituents wherein the susbstituents are selected from the group consisting of $C_{1-6}$ alkyl, $C_{2-6}$ alkenyl, $C_{2-6}$ alkynyl, $C_{3-6}$ cycloalkyl, $C_{5-7}$ cycloalkenyl, $C_{7-11}$ aralkyl, $C_{6-14}$ aryl, $C_{1-6}$ alkoxy, $C_{6-14}$ aryloxy, $C_{1-6}$ alkylcarbonyl, $C_{6-14}$ arylcarbonyl, $C_{1-6}$ alkanoyloxy, $C_{6-14}$ arylcarbonyloxy, carboxyl, $C_{1-6}$ alkoxycarbonyl, carbamoyl, N-mono-$C_{1-4}$ alkylcarbamoyl, N,N-di-$C_{1-4}$ alkylcarbamoyl, halogen, mono-, di- or tri-halogeno-$C_{1-4}$ alkyl, oxo, amidino, imino, amino, mono- or di-$C_{1-4}$ alkylamino, OH and nitro.

Preferred 5 to 13-membered aromatic heterocyclic group which comprises 1 to 12 carbon atoms and about 1 to about 4 hetero atomos which are selected from the group consisting of nitrogen, oxygen and sulfur atoms are 2- or 3-thienyl, 2- or 3-furyl, 2- or 3-pyrrolyl, 2-, 3- or 4-pyridyl, 2-, 4- or 5-oxazolyl, 2-, 4- or 5-thiazolyl, 3-, 4- or 5-pyrazolyl, 2-, 4- or 5-imidazolyl, 3-, 4- or 5-isoxazolyl, 3-, 4- or 5-isothiazolyl, 3- or 5-(1,2,4-oxadiazolyl), 1,3,4-oxadiazolyl, 3- or 5-(1,2,4-thiadiazolyl), 1,3,4-thiadiazolyl, 4- or 5-(1,2,3-thiadiazolyl), 1,2,5-thiadiazolyl, 1,2,3-triazolyl, 1,2,4-triazolyl, 1H- or 2H-tetrazolyl, N-oxide-2-, 3- or 4-pyridyl, 2-, 4- or 5-pyrimidinyl, N-oxide-2-, 4- or 5-pyrimidinyl, thiomorpholinyl, morpholinyl, oxoimidadinyl, dioxotriazinyl, pyrrolidinyl, piperidinyl, pyranyl, thiopyranyl, 1,4-oxazinyl, 1,4-thiazinyl, 1,3-thiazinyl, piperadinyl, triazinyl, oxotriazinyl, 3- or 4-pyridazinyl, pyrazinyl, N-oxide-3- or 4-pyridazinyl, benzofuryl, benzothiazolyl, benzooxazolyl, tetrazolo[1,5-b]pyridazinyl, triazolo[4,5-b]pyridazinyl, benzoimidazolyl, quinolyl, isoquinolyl, cynnorynyl, phthalazinyl, quinazolinyl, quinoxalinyl, indolizinyl, quinolizinyl, 1,8-naphthylizinyl, purinyl, pteridinyl, dibenzofuranyl, carbazolyl, acridinyl, phenanthridinyl, chromanyl, benzoxazinyl, phenazinyl, phenothiazinyl or phenoxazinyl.

$X^1$ is preferabley OH, SH, methyl, ethyl, n-propyl, phenyl, (o-, m-, p-)$C_{1-10}$ alkoxyphenyl, (o-, m-, p-)$C_{1-10}$ alkylphenyl, methoxy or ethoxy. In particular, OH, SH, methyl and phenyl are preferred.

$X^4$ and $X^5$ are preferably each (1) OH, (2) SH, (3) $C_{1-5}$ alkyl, (4) $C_{1-5}$ alkoxy, (5) $C_{1-5}$ monoalkylamino. In particular, OH, SH, methyl, ethyl, n-propyl, methoxy or ethoxy is more preferable. In particular,

OH, SH, methyl are even more preferred.

Preferable combinations of $X^1$, $X^4$ and $X^5$ include

(i) $X^1$ is (1) OH, (2) SH, (3) $C_{1-5}$ alkyl, (4) $C_{1-5}$ alkoxy, (5) $C_{1-5}$ monoalkylamino or (6) phenyl which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl; $X^4$ and $X^5$ are each OH, SH, $C_{1-5}$ alkyl, $C_{1-5}$ alkoxy or $C_{1-5}$ monoalkylamino;

(ii) $X^1$ is OH, SH, methyl, ethyl, propyl, phenyl, (o-, m-, p-) alkoxyphenyl, (o-, m-, p-) alkylphenyl, methoxy or ethoxy; $X^4$ and $X^5$ are each OH, SH, methyl, ethyl, n-propyl, methoxy or ethoxy;

(iii) $X^1$ is OH, SH, methyl, ethyl, propyl, phenyl, (o-, m-, p-)$C_{1-10}$ alkoxyphenyl, (o-, m-, p-)$C_{1-10}$ alkylphenyl, methoxy or ethoxy; $X^4$ and $X^5$ are each OH, SH, methyl, ethyl, n-propyl, methoxy or ethoxy; and

(iv) $X^1$ is OH, SH, methyl or phenyl; $X^4$ and $X^5$ are each OH, SH or methyl.

The preferred nucleic acid residues represented by $B^1$, $B^2$ and $B^3$ include hypoxanthine-9-yl, guanine-9-yl, adenine-9-yl, uracil-1-yl, thymine-1-yl and cytosine-1-yl. The nucleic acid residues can be selected according to the desired nucleotide sequences.

$R^1$, $R^2$ and $R^3$ are each preferabley hydrogen, OH, halogen, methoxy, ethoxy, methoxymethyl, and especially hydrogen is preferable.

n represents an integer from 1 to 98. Above all, n is preferably an integer from 1 to 38, more preferably 1 to 28, and most preferably 5 to 18.

The oligonucleotide compounds of Formula (Ia) of the present invention may preferably be obtained by combination of any of the above mentioned preferrws compounds. Especially, an oligonucleotide compound which comprise a nucleotide sequence, CAT, is preferred.

Further, so-called antisense-oligonucletide compounds are more preferable as the oligonucleotide compounds of Formula (Ia) of the present invention. In addition, they are more preferable when encapsulated within a liposome such as lipofectin.

In the oligonucleotide compounds of Formula (Ib) of the present invention, W preferably represents hydrogen or formula

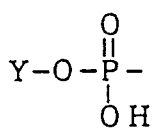

or formula

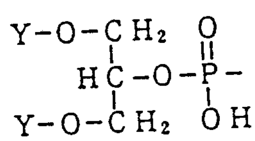

When W is represented by formula

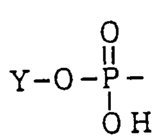

a preferred saccharide of a saccharide residue represented by Y includes monosaccharides, oligosaccharides or glycoside compounds thereof which may be substituted by for example, $C_{1-10}$ alkylcarbonyl (for example, $C_{1-6}$ alkylcarbonyl such as formyl, acetyl and ethylcarbonyl),
acyl such as phenylcarbonyl, and $C_{1-20}$ alkyl (for example, methyl, ethyl, n-propyl, i-propyl, n-butyl, n-pentyl, n-hexyl, n-heptyl and n-octyl).

Examples of the preferred monosaccharides used include aldopentoses (for example, ribose, arabinose) and aldohexoses (for example, glucose, mannose, galactose). Galactose, mannose and galactosamine are preferable.

The preferred oligosaccharides include melibiose, gentiobiose and isomaltotriose. The preferable glycoside compounds include n-octylglucoside, n-octylthioglucoside and phenylglucoside. Glucosamine or galactosamine in which the 2-amino may be substituted by $C_{1-6}$ alkylcarbonyl or phenylcarbonyl is also preferable. Among them, (1) galactose or mannose in which a hydroxyl group may be substituted by $C_{1-6}$ alkylcarbonyl groups (for example, formyl, acetyl, ethylcarbonyl), (2) melibiose, gentiobiose or isomaltotriose in which a hydroxyl group may be substituted by phenylcarbonyl, (3) n-octylglucoside, n-octylthioglucoside or phenylglucoside, and (4) glucosamine or galactosamine which may be substituted by $C_{1-10}$ alkylcarbonyl groups (for example, $C_{1-6}$ alkylcarbonyl such as formyl, acetyl, ethylcarbonyl) are preferable. Specifically, Gal(galactose), Man(mannose), n-oct-Glc(n-octylglucoside), n-oct-SGlc(n-octylthioglucoside), PhGlc(phenylglucoside), PhGal(phenylgalactoside), GalN(galactosamine), N-AcGal(N-acetylgalactosamine), N-BzGal(N-benzoylgalactosamine), GulN(glucosamine), N-AcGul(N-acetylglucosamine), Mel(melibiose), Gen(gentiobiose) and Iso(isomaltotriose) are preferable. Especially, Mel(melibiose), Gen(gentiobiose) and N-BzGal(N-benzoylgalactosamine) are more preferable.

Further, aldohexoses in which a hydroxyl group may be substituted by $C_{1-6}$ alkylcarbonyl groups (for example, formyl, acetyl, ethylcarbonyl) are also preferable. Specifically galactose, mannose, 1,2,3,4-O-tetraacetylgalactose, 1,2,3,4-O-tetraacetylmannose, glucosamine, N-acetylglucosamine, N-benzoylglucosamine and N-benzoylgalactosamine are more preferable.

When W is represented by formula,

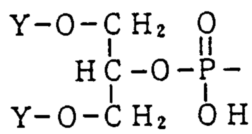

a preferred saccharide of a saccharide residue represented by Y includes monosaccharides. As the monosaccharide, galactose, mannose and glucose are preferable, and galactose is more preferable.

$X^6$ and $X^7$ are each preferably (1) OH, (2) SH, (3) $C_{1-5}$ alkyl, (4) $C_{1-5}$ alkoxy, (5) $C_{1-5}$ monoalkylamino, (6) $C_{6-11}$ aryl (for example, phenyl, naphthyl) which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl, or (7) a 5 to 13-membered aromatic heterocyclic group which comprises 1 to 12 carbon atoms and 1 to about 4 hetero atomos which are selected from the group consisting of nitrogen, oxygen and sulfur atoms which may be substituted by about 1 to about 5 substituents which are selected from the group consisting of $C_{1-6}$ alkyl, $C_{2-6}$ alkenyl, $C_{2-6}$ alkynyl, $C_{3-6}$ cycloalkyl, $C_{5-7}$ cycloalkenyl, $C_{7-11}$ aralkyl, $C_{6-14}$ aryl, $C_{1-6}$ alkoxy, $C_{6-14}$ aryloxy, $C_{1-6}$ alkylcarbonyl, $C_{6-14}$ arylcarbonyl, $C_{1-6}$ alkanoyloxy, $C_{6-14}$ arylcarbonyloxy, carboxyl, $C_{1-6}$ alkoxycarbonyl, carbamoyl, N-mono-$C_{1-4}$ alkylcarbamoyl, N,N-di-$C_{1-4}$ alkylcarbamoyl, halogen, mono-, di- or tri-halogeno-$C_{1-4}$ alkyl, oxo, amidino, imino, amino, mono- or di-$C_{1-4}$ alkylamino, OH and nitro.

Preferred 5 to 13-membered aromatic heterocyclic group which comprises 1 to 12 carbon atoms and about 1 to about 4 hetero atomos which are selected from the group consisting of nitrogen, oxygen and sulfur atoms are 2- or 3-thienyl, 2- or 3-furyl, 2- or 3-pyrrolyl, 2-, 3- or 4-pyridyl, 2-, 4- or 5-oxazolyl, 2-, 4- or 5-thiazolyl, 3-, 4- or 5-pyrazolyl, 2-, 4- or 5-imidazolyl, 3-, 4- or 5-isoxazolyl, 3-, 4- or 5-isothiazolyl, 3- or 5-(1,2,4-oxadiazolyl), 1,3,4-oxadiazolyl, 3- or 5-(1,2,4-thiadiazolyl), 1,3,4-thiadiazolyl, 4- or 5-(1,2,3-thiadiazolyl), 1,2,5-thiadiazolyl, 1,2,3-triazolyl, 1,2,4-triazolyl, 1H- or 2H-tetrazolyl, N-oxide-2-, 3- or 4-pyridyl, 2-, 4- or 5-pyrimidinyl, N-oxide-2-, 4- or 5-pyrimidinyl, thiomorpholinyl, morpholinyl, oxoimidadinyl, dioxotriazinyl, pyrrolidinyl, piperidinyl, pyranyl, thiopyranyl, 1,4-oxazinyl, 1,4-thiazinyl, 1,3-thiazinyl, piperadinyl, triazinyl, oxotriazinyl, 3- or 4-pyridazinyl, pyrazinyl, N-oxide-3- or 4-pyridazinyl, benzofuryl, benzothiazolyl, benzooxazolyl, tetrazolo[1,5-b]pyridazinyl, triazolo[4,5-b]pyridazinyl, benzoimidazolyl, quinolyl, isoquinolyl, cynnorynyl, phthalazinyl, quinazolinyl, quinoxalinyl, indolizinyl, quinolizinyl, 1,8-naphthylizinyl, purinyl, pteridinyl, dibenzofuranyl, carbazolyl, acridinyl, phenanthridinyl, chromanyl, benzoxazinyl, phenazinyl, phenothiazinyl or phenoxazinyl.

$X^6$ and $X^7$ are each preferably (1) OH, (2) SH, (3) $C_{1-5}$ alkyl, (4) $C_{1-5}$ alkoxy, (5) $C_{1-5}$ monoalkylamino, or (6) phenyl which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl. $X^6$ and $X^7$ are each more preferabley OH, SH, methyl, ethyl, n-propyl, phenyl, (o-, m-, p-)$C_{1-10}$ alkoxyphenyl, (o-, m-, p-)$C_{1-10}$ alkylphenyl, methoxy or ethoxy. In particular, OH, SH, methyl and phenyl are more preferred.

The preferred nucleic acid residues represented by $B^1$, $B^2$ and $B^3$ include hypoxanthine-9-yl, guanine-9-yl, adenine-9-yl, uracil-1-yl, thymine-1-yl and cytosine-1-yl. The nucleic acid residues can be selected according to the desired nucleotide sequences.

$R^1$, $R^2$, $R^{21}$, $R^{22}$ and $R^3$ are each preferably hydrogen, OH, halogen, methoxy, ethoxy, methoxymethyloxy, and more preferably are hydrogens.

n represents an integer from 1 to 98. Above all, n is preferably an integer from 1 to 38, more preferably 1 to 28, and most preferably 5 to 18.

In the oligonucleotide compounds of Formula (Ib) of the present invention, those which have a phenylphosphonate type nucleotide at 5'-terminus are preferable. Thus, an oligonucleotide compound (Ib-1) which comprises a nucleotide sequence represented by formula

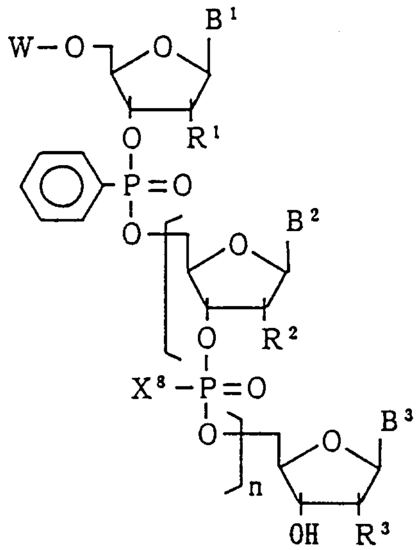

is preferable. The preferred examples of W, $B^1$, $B^2$, $B^3$, $R^1$, $R^2$, $R^3$ and n are the same as the preferable ones in the oligonucletide compound of Formula (Ib).

$X^8$ is preferably (1) OH, (2) SH, (3) $C_{1-5}$ alkyl, (4) $C_{1-5}$ alkoxy, (5) $C_{1-5}$ monoalkylamino, (6) $C_{6-11}$ aryl (for example, phenyl, naphthyl) which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl, or (7) a 5 to 13-membered aromatic heterocyclic group which comprises 1 to 12 carbon atoms and 1 to about 4 hetero atomos which are selected from the group consisting of nitrogen, oxygen and sulfur atoms which may be substituted by about 1 to about 5 substituents which are selected from the group consisting of $C_{1-6}$ alkyl, $C_{2-6}$ alkenyl, $C_{2-6}$ alkynyl, $C_{3-6}$ cycloalkyl, $C_{5-7}$ cycloalkenyl, $C_{7-11}$ aralkyl, $C_{6-14}$ aryl, $C_{1-6}$ alkoxy, $C_{6-14}$ aryloxy, $C_{1-6}$ alkylcarbonyl, $C_{6-14}$ arylcarbonyl, $C_{1-6}$ alkanoyloxy, $C_{6-14}$ arylcarbonyloxy, carboxyl, $C_{1-6}$ alkoxycarbonyl, carbamoyl, N-mono-$C_{1-4}$ alkylcarbamoyl, N,N-di-$C_{1-4}$ alkylcarbamoyl, halogen, mono-, di- or tri-halogeno-$C_{1-4}$ alkyl, oxo, amidino, imino, amino, mono- or di-$C_{1-4}$ alkylamino, OH and nitro.

Preferred 5 to 13-membered aromatic heterocyclic group which comprises 1 to 12 carbon atoms and about 1 to about 4 hetero atomos which are selected from the group consisting of nitrogen, oxygen and sulfur atoms are 2- or 3-thienyl, 2- or 3-furyl, 2- or 3-pyrrolyl, 2-, 3- or 4-pyridyl, 2-, 4- or 5-oxazolyl, 2-, 4- or 5-thiazolyl, 3-, 4- or 5-pyrazolyl, 2-, 4- or 5-imidazolyl, 3-, 4- or 5-isoxazolyl, 3-, 4- or 5-isothiazolyl, 3- or 5-(1,2,4-oxadiazolyl), 1,3,4-oxadiazolyl, 3- or 5-(1,2,4-thiadiazo1yl), 1,3,4-thiadiazolyl, 4- or 5-(1,2,3-thiadiazolyl), 1,2,5-thiadiazolyl, 1,2,3-triazolyl, 1,2,4-triazolyl, 1H- or 2H-tetrazolyl, N-oxide-2-, 3- or 4-pyridyl, 2-, 4- or 5-pyrimidinyl, N-oxide-2-, 4- or 5-pyrimidinyl, thiomorpholinyl, morpholinyl, oxoimidadinyl, dioxotriazinyl, pyrrolidinyl, piperidinyl, pyranyl, thiopyranyl, 1,4-oxazinyl, 1,4-thiazinyl, 1,3-thiazinyl, piperadinyl, triazinyl, oxotriazinyl, 3- or 4-pyridazinyl, pyrazinyl, N-oxide-3- or 4-pyridazinyl, benzofuryl, benzothiazolyl, benzooxazolyl, tetrazolo[1,5-b]pyridazinyl, triazolo[4,5-b]pyridazinyl, benzoimidazolyl, quinolyl, isoquinolyl, cynnorynyl, phthalazinyl, quinazolinyl, quinoxalinyl, indolizinyl, quinolizinyl, 1,8-naphthylizinyl, purinyl, pteridinyl, dibenzofuranyl, carbazolyl, acridinyl, phenanthridinyl, chromanyl, benzoxazinyl, phenazinyl, phenothiazinyl or phenoxazinyl.

$X^8$ is more preferabley OH, SH, methyl, ethyl, n-propyl, phenyl, (o-, m-, p-)$C_{1-10}$ alkoxyphenyl, (o-, m-, p-)$C_{1-10}$ alkylphenyl, methoxy or ethoxy. In particular, OH, SH, methyl and phenyl are more preferred.

In the oligonucleotide compounds of Formula (Ib) of the present invention, those which have phenylphosphonate type nucleotides at the second position from the 5'-terminus and the second position from the 3'-terminus are preferable. Thus, an oligonucleotide compound of Formula (Ib-2) which comprises a nucleotide sequence represented by formula

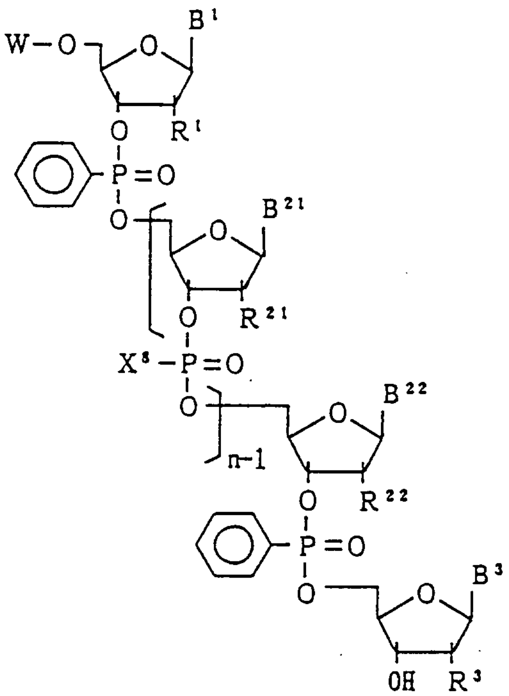

is preferable. The preferred examples of W, $B^1$, $B^3$, $R^1$, $R^3$ and n are the same as the preferable ones in the oligonucletide compound of Formula (Ib).

More preferable oligonucleotide compounds of Formula (Ib) include those which have two phenylphosphonate type nucleotides at 5'-terminus and two phenylphosphonate type nucleotides at the second and the third positions from the 3'-terminus. Thus, the oligonucleotide compounds which have a repetition of "n-1" (n is an integer from 4 to 38; preferably 4 to 28; more preferably 4 to 18) in the above-metioned oligonucleotide compound (Ib-2), in which the first $X^8$ is phenyl and the n-1th $X^8$ is phenyl are especially preferable.

The preferred examples of $B^{21}$ and $B^{22}$ are the same as the preferable scope of $B^2$ in the oligonucleotide compounds of Formula (Ib).

The preferred examples of $R^{21}$ and $R^{22}$ are the same as the preferable scope of $R^2$ in the oligonucleotide compounds of Formula (Ib).

The preferred examples of $X^8$ are the same as the preferable scope of $X^8$ in the oligonucleotide compounds of Formula (Ib-1).

The oligonucleotide compounds of Formula (Ib) may be so-called antisense-oligonucleotide compounds and may be encapsulated within a liposome such as lipofectin.

The oligonucleotide compounds of the present invention may be any, as long as they inhibit expression of a target DNA, namely (1) they are complementary to a sequence of the objective DNA and inhibit transcription from the DNA to pre mRNA, (2) they are complementary to a sequence of pre mRNA and inhibit splicing from pre mRNA to mature mRNA, or (3) they inhibit translation from mature mRNA to a protein. The oligonucleotide compounds may be complementary to any of an exon site, an intron site and a site containing both exon and intron, as long as the target is DNA. Specifically, they inhibit expression of genes producing proteins which contribute to the development of various diseases (for example, malignant tumors, viral diseases, reangiostenosis after PTCA and inflammations). Especially, the oligonucleotide compounds of Formula (Ia) of the present invention or a salt thereof can be used as a so-called antisense-oligonucleotide compounds.

Examples of the genes contributing to the development of malignant tumors include oncogenes, for example src, fps, yes, ros, myb, myc, erb, rel, mos, abl, ras, fos, fes, fms, sis, raf, neu and p53 genes. Examples of genes contributing to the development of the viral diseases include genes from genomes of viruses such as HIV (e.g. env, gag, pol, nef, tat, and rev), influenza viruses, herpes viruses, papilloma viruses, polioviruses, picornaviruses and adenoviruses. Examples of the genes producing proteins (particularly, adhesive proteins) contributing to the development of the inflammations include ICAM-1, ELAM-1 and VLAM. Examples of the genes producing proteins contributing to the development of the reangiostenosis after PTCA include PDGF and FGF.

The oligonucleotide compounds of the present invention may be partially complementary to the DNA or mRNA sequences of these genes. For example, the DNA or mRNA sequence contains 5'-ATG-3' or 5'-AUG-3' which is an expression initiation codon. Accordingly, if they are complementary to a sequence containing ATG, AUG or a sequence before and behind them, expression of the DNA or the mRNA can be inhibited. It is therefore preferred that the oligonucleotide compounds of the present invention are, for example, oligonucleotide compounds having nucleotide sequences each represented by 5'-CAT-3' which are complementary to 5'-ATG-3' or 5'-AUG-3'.

Preferred embodiments of the oligonucleotide compounds of the present invention are described below. However, it is understood that they are not intended to limit the scope of the invention.

The oligonucleotide compounds of Formula (Ia) complementary to ICAM-1, a gene producing an adhesive protein contributing to the development of the inflammations, include oligonucleotide compounds of Formula (Ia-1) represented by formula

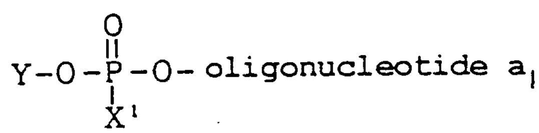

wherein Y and $X^1$ have the same meanings as the above-mentioned ones, and oligonucleotide compounds of Formula (Ia-2) represented by formula

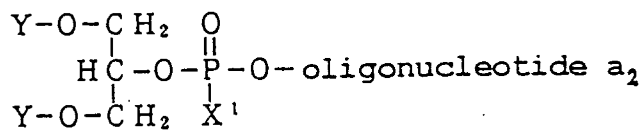

wherein Y and $X^1$ have the same meanings as the above-mentioned ones.

Examples of Oligonucleotide $a_1$ and Oligonucleotide $a_2$ in the oligonucleotide compounds (Ia-1) and (Ia-2) include:

| | |
|---|---|
| TGGGAGCCATAGCGAGGC | (SEQ ID NO: 1) |
| TGGGAGCCATAGCGAGGCT | (SEQ ID NO: 2) |
| GGCTGCTGGGAGCCATAGCGAGGCTGAGGT | (SEQ ID NO: 3) |
| GGCTGCTGGGAGCCATAGCGAGGCTGAGG | (SEQ ID NO: 4) |
| GGCTGCTGGGAGCCATAGCGAGGCTGAG | (SEQ ID NO: 5) |
| GGCTGCTGGGAGCCATAGCGAGGCTGA | (SEQ ID NO: 6) |

| | |
|---|---|
| GGCTGCTGGGAGCCATAGCGAGGCTG | (SEQ ID NO: 7) |
| GGCTGCTGGGAGCCATAGCGAGGCT | (SEQ ID NO: 8) |
| GGCTGCTGGGAGCCATAGCGAGGC | (SEQ ID NO: 9) |
| GGCTGCTGGGAGCCATAGCGAGG | (SEQ ID NO: 10) |
| GGCTGCTGGGAGCCATAGCGAG | (SEQ ID NO: 11) |
| GGCTGCTGGGAGCCATAGCGA | (SEQ ID NO: 12) |
| GGCTGCTGGGAGCCATAGCG | (SEQ ID NO: 13) |
| GGCTGCTGGGAGCCATAGC | (SEQ ID NO: 14) |
| GGCTGCTGGGAGCCATAG | (SEQ ID NO: 15) |
| GGCTGCTGGGAGCCATA | (SEQ ID NO: 16) |
| GGCTGCTGGGAGCCAT | (SEQ ID NO: 17) |
| GCTGCTGGGAGCCATAGCGAGGCTGAGGT | (SEQ ID NO: 18) |
| CTGCTGGGAGCCATAGCGAGGCTGAGGT | (SEQ ID NO: 19) |
| TGCTGGGAGCCATAGCGAGGCTGAGGT | (SEQ ID NO: 20) |
| GCTGGGAGCCATAGCGAGGCTGAGGT | (SEQ ID NO: 21) |
| CTGGGAGCCATAGCGAGGCTGAGGT | (SEQ ID NO: 22) |
| TGGGAGCCATAGCGAGGCTGAGGT | (SEQ ID NO: 23) |
| GGGAGCCATAGCGAGGCTGAGGT | (SEQ ID NO: 24) |
| GGAGCCATAGCGAGGCTGAGGT | (SEQ ID NO: 25) |
| GAGCCATAGCGAGGCTGAGGT | (SEQ ID NO: 26) |
| AGCCATAGCGAGGCTGAGGT | (SEQ ID NO: 27) |
| GCCATAGCGAGGCTGAGGT | (SEQ ID NO: 28) |
| CCATAGCGAGGCTGAGGT | (SEQ ID NO: 29) |
| CATAGCGAGGCTGAGGT | (SEQ ID NO: 30) |

$X^1$, $X^4$ and $X^5$ are each OH in the above oligonulceotides. $X^4$ and/or $X^5$ may also be SH or methyl, and examples of the oligonucleotide $a_1$ and oligonucleotide $a_2$ include

| | |
|---|---|
| $T_sG_sG_sG_sA_sG_sC_sC_sA_sT_sA_sG_sC_sG_sA_sG_sG_sC$ | (SEQ ID NO: 31) |
| $T_{Me}G_{Me}GGAGCCATAGCGAG_{Me}G_{Me}C$ | (SEQ ID NO: 34) |

The "oligonucleotide $a_1$" and "oligonucleotide $a_2$" represented by SEQ ID NO:1, SEQ ID NO:31 or SEQ ID NO:34 are preferable.

Y in the oligonucleotide compounds of Formula (Ia-1) is preferabley Gal(galactose), Man(mannose), n-oct-Glc(n-octylglucoside), n-oct-SGlc(n-octyl-thioglucoside), PhGlc(phenylglucoside), PhGal-(phenylgalactoside), GalN(galactosamine), N-AcGal(N-acetylgalactosamine), N-BzGal(N-benzoylgalactosamine), GulN(glucosamine), N-AcGul(N-acetylglucosamine), Mel(melibiose), Gen(gentiobiose) or Iso-(isomaltotriose).

Y in the oligonucleotide compounds of Formula (Ia-2) is preferabley Gal(galactose), Man(mannose) or Glu(glucose), and more preferably Gal(galactose).

The oligonucleotide compounds of Formula (Ib) complementary to ICAM-1, a gene producing an adhesive protein contributing to the development of the inflammations, include oligonucleotide compounds (Ib-3) represented by formula

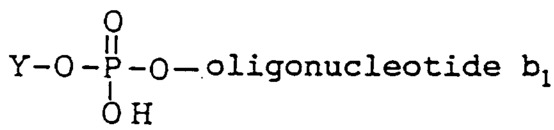

wherein Y has the same meanings as the above-mentioned ones, and oligonucleotide compounds (Ib-4) represented by formula

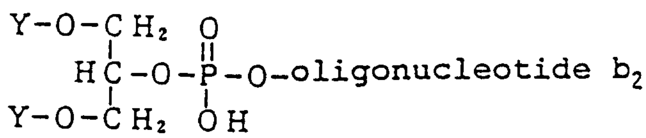

wherein Y has the same meanings as the above-mentioned ones.

Examples of "Oligonucleotide $b_1$" and "Oligonucleotide $b_2$" in the oligonucleotide compounds (Ib-3) and (Ib-4) are as follows:

| | |
|---|---|
| TGGGAGCCATAGCGAGGC | (SEQ ID NO: 1) |
| TGGGAGCCATAGCGAGGCT | (SEQ ID NO: 2) |
| GGCTGCTGGGAGCCATAGCGAGGCTGAGGT | (SEQ ID NO: 3) |
| GGCTGCTGGGAGCCATAGCGAGGCTGAGG | (SEQ ID NO: 4) |
| GGCTGCTGGGAGCCATAGCGAGGCTGAG | (SEQ ID NO: 5) |
| GGCTGCTGGGAGCCATAGCGAGGCTGA | (SEQ ID NO: 6) |
| GGCTGCTGGGAGCCATAGCGAGGCTG | (SEQ ID NO: 7) |
| GGCTGCTGGGAGCCATAGCGAGGCT | (SEQ ID NO: 8) |
| GGCTGCTGGGAGCCATAGCGAGGC | (SEQ ID NO: 9) |
| GGCTGCTGGGAGCCATAGCGAGG | (SEQ ID NO: 10) |
| GGCTGCTGGGAGCCATAGCGAG | (SEQ ID NO: 11) |
| GGCTGCTGGGAGCCATAGCGA | (SEQ ID NO: 12) |
| GGCTGCTGGGAGCCATAGCG | (SEQ ID NO: 13) |
| GGCTGCTGGGAGCCATAGC | (SEQ ID NO: 14) |
| GGCTGCTGGGAGCCATAG | (SEQ ID NO: 15) |
| GGCTGCTGGGAGCCATA | (SEQ ID NO: 16) |
| GGCTGCTGGGAGCCAT | (SEQ ID NO: 17) |
| GCTGCTGGGAGCCATAGCGAGGCTGAGGT | (SEQ ID NO: 18) |
| CTGCTGGGAGCCATAGCGAGGCTGAGGT | (SEQ ID NO: 19) |
| TGCTGGGAGCCATAGCGAGGCTGAGGT | (SEQ ID NO: 20) |

GCTGGGAGCCATAGCGAGGCTGAGGT (SEQ ID NO: 21)

CTGGGAGCCATAGCGAGGCTGAGGT (SEQ ID NO: 22)

TGGGAGCCATAGCGAGGCTGAGGT (SEQ ID NO: 23)

GGGAGCCATAGCGAGGCTGAGGT (SEQ ID NO: 24)

GGAGCCATAGCGAGGCTGAGGT (SEQ ID NO: 25)

GAGCCATAGCGAGGCTGAGGT (SEQ ID NO: 26)

AGCCATAGCGAGGCTGAGGT (SEQ ID NO: 27)

GCCATAGCGAGGCTGAGGT (SEQ ID NO: 28)

CCATAGCGAGGCTGAGGT (SEQ ID NO: 29)

CATAGCGAGGCTGAGGT (SEQ ID NO: 30)

CCCCCACCACTTCCCCT (SEQ ID NO: 49)

CCCTGTCCCGGATAGGT (SEQ ID NO: 50)

GGAGCCATAGCGAGGC (SEQ ID NO: 51)

AGCCATAGCGAG (SEQ ID NO: 52)

AGCCATAGC (SEQ ID NO: 53)

GGTTTTTTTTTTTTTGGG (SEQ ID NO: 54)

AATTTTTTTTTTTTTAAA (SEQ ID NO: 55)

$X^6$ and $X^7$ are each OH in the above oligonulceotides. At least one of $X^6$ and $X^7$ may be phenyl (at least one of the nucleotides are phenylphosphonate type), and the preferable examples of the "oligonucleotide $b_1$" and "oligonucleotide $b_2$" include

$T_{Ph}G_{Ph}GGAGCCATAGCGAG_{Ph}G_{Ph}C$ (SEQ ID NO: 32)

$T_{Ph}GGGAGC_{Ph}C_{Ph}AT_{Ph}AGC_{Ph}GAGGC_{Ph}T$ (SEQ ID NO: 33)

$T_{Ph}GGGAGCCATAGCGAGG_{Ph}C$ (SEQ ID NO: 35)

$C_{Ph}G_{Ph}GAGCGATACCGAGG_{Ph}G_{Ph}T$ (SEQ ID NO: 37)

$T_{Ph}G_{Ph}GGAGC_{Ph}C_{Ph}AT_{Ph}AGC_{Ph}GAG_{Ph}G_{Ph}C$ (SEQ ID NO: 39)

$C_{Ph}G_{Ph}GAGC_{Ph}GAT_{Ph}AC_{Ph}C_{Ph}GAGG_{Ph}G_{Ph}T$ (SEQ ID NO: 40)

$C_{Ph}C_{Ph}CCCACCACTTCCC_{Ph}C_{Ph}T$ (SEQ ID NO: 41)

$C_{Ph}C_{Ph}CTGTCCCGGGATAG_{Ph}G_{Ph}T$ (SEQ ID NO: 42)

$T_{Ph}G_{Ph}G_{Ph}G_{Ph}A_{Ph}G_{Ph}C_{Ph}C_{Ph}A_{Ph}T_{Ph}A_{Ph}G_{Ph}C_{Ph}G_{Ph}A_{Ph}G_{Ph}G_{Ph}C$ (SEQ ID NO: 43)

$G_{Ph}G_{Ph}AGCCATAGCGAG_{Ph}G_{Ph}C$ (SEQ ID NO: 44)

$A_{Ph}G_{Ph}CCATAGCG_{Ph}A_{Ph}G$ (SEQ ID NO: 45)

$A_{Ph}G_{Ph}CCATA_{Ph}G_{Ph}C$ (SEQ ID NO: 46)

$G_{Ph}G_{Ph}TTTTTTTTTTTTG_{Ph}G_{Ph}G$ (SEQ ID NO: 47)

$A_{Ph}A_{Ph}TTTTTTTTTTTTA_{Ph}A_{Ph}A$ (SEQ ID NO: 48)

Y in the oligonucleotide compounds of Formula (Ib-3) is preferabley Gal(galactose), Man(mannose), n-oct-Glc(n-octylglucoside), n-oct-SGlc(n-octyl-thioglucoside), PhGlc(phenylglucoside), PhGal-(phenylgalactoside), GalN(galactosamine), N-AcGal(N-acetylgalactosamine), N-BzGal(N-benzoylgalactosamine), GulN(glucosamine), N-AcGul(N-acetylglucosamine), Mel(melibiose), Gen(gentiobiose) or Iso-(isomaltotriose), and more preferably Mel(melibiose), Gen(gentiobiose) or Iso(isomaltotriose).

Y in the oligonucleotide compounds of Formula (Ib-4) is preferabley Gal(galactose), Man(mannose) or Glu(glucose).

The oligonucleotide compounds of the present invention are more speicifically those which are obtained in Examples 3, 4, 6, 8, 10, 12, 14, 16, 18, 20, 22, 24, 26, 28, 30, 32 to 60 and 64, especially preferably those obtained in Examples 10, 12, 14, 16, 18, 20, 22, 24, 26, 32, 33, 34, 35, 44, 45, 46, and more preferably those obtained in Examples 18, 44, 45 and 46.

The oligonucleotide compounds of Formula (I) of the present invention are synthesized in the following manner. First, for the oligonucleotides, starting nucleosides are selectively protected at base sites such as purine and pyrimidine and at saccharide hydroxyl groups, for example, according to known methods such as represented by [Oligonucleotide Synthesis; A Practical Approach edited by M. J. Gait, IRL PRESS (1984) which is incorporated herein by reference]. Then, the 3'-hydroxyl groups are made into phosphinic acid compounds or H-phosphonate compounds [M. D. Matteuci et al., Tetrahedron Lett., vol. 21, 719 (1980); S. L. Beaucage et al., Tetrahedron Lett., vol. 22, 1859 (1981); L. J. McBride et al., Tetrahedron Lett., vol. 24, 245 (1983); B. C. Froehler et al., Nucleic Acids Res., vol. 14, 5399 (1986); B. C. Froehler et al., Tetrahedron Lett., vol. 27, 469 (1986); P. J. Garegg et al., Tetrahedron Lett., vol. 27, 4051 (1986); and E. Uhlman et al., Chemical Reviews, vol. 90, No. 4, 543 (1990) which are incorporated herein by reference]. Using these as units, the oligonucleotides can be synthesized according to the known phosphoramidite method or H-phosphonate method as described in the above-mentioned references.

Preparing methods of the oligonucleotide compounds of Formula (Ia) and (Ib) will be explained in detail below:

[Preparing method of the oligonucleotide compounds (Ia)]

With the saccharide residues to be introduced into the oligonucleotide compounds, the starting saccharide compounds are first protected at secondary hydroxyl groups thereof as needed, for example, according to known methods [Reagents for Organic Synthesis, vol. 1, 453, edited by L. Fieser, John Wiley and Sons, INC. (1967); D. D. Reynolds et al., Org. Syn., Coll., vol. 3, 432 (1955); and R. R. Schmidt, Angew. Chem. Int. Ed. Engl., vol. 25, 212 (1986) which are incorporated herein by reference]. Then, the primary hydroxyl groups thereof are made into the phosphinic acid compounds or H-phosphonate compounds according to known methods as described in the above-mentioned references. Using these as units, the saccharide residues can be synthesized according to the known phosphoramidite method or H-phosphonate method as described in the above-mentioned references.

A method of preparing the oligonucleotide compounds of Formula (Ia) of the present invention in which Q is Y, and a saccharide of the saccharide residue represented by Y is a galactose compound is illustrated

by means of examples below. However, the oligonucleotide compounds of Formula (Ia) in which Y is another group and the oligonucleotide compounds (Ia) in which Q is represented by formula

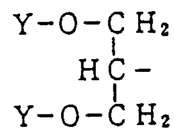

can also be prepared similarly.

The oligonucleotide compounds of Formula (Ia) of the present invention (the oligonucleotide compounds into which the galactose compound is introduced) can be produced according to the methods represented by the following reaction formulas (a) to (c). Phosphorylation of the hydroxyl groups at the 6-positions of the saccharide compounds protected by appropriate protective groups (for example, tetra-O-benzoylgalactose) is conducted according to methods described in known literatures [M. D. Matteuci et al., Tetrahedron Lett., vol. 21, 719 (1980); S. L. Beaucage et al., Tetrahedron Lett., vol. 22, 1859 (1981); L. J. McBride et al., Tetrahedron Lett., vol. 24, 245 (1983); B. C. Froehler et al., Nucleic Acids Res., vol. 14, 5399 (1986); B. C. Froehler et al., Tetrahedron Lett., vol. 27, 469 (1986); P. J. Garegg et al., Tetrahedron Lett., vol. 27, 4051 (1986); and E. Uhlman et al., Chemical Reviews, vol. 90, No. 4, 543 (1990) which are incorporated herein by reference].

In the following manufacturing method, saccharide derivatives (A) is represented by the following formula:

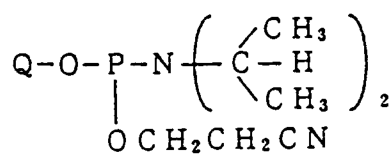

wherein Q has the same meanings as above and saccharide derivatives (B) is represented by the following formula:

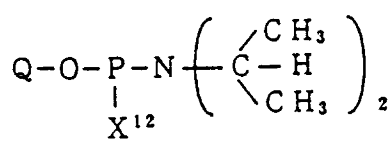

wherein Q has the same meanings as above, $X^{12}$ represents $C_{1-5}$ alkyl, $C_{1-5}$ alkoxy, $C_{1-5}$ monoalkylamino or an aromatic ring group which may be substituted, are useful as intermediate compounds of the oligonucleotide compounds of Formula (I).

In the saccharide compounds of Formula (A) and (B), when Q is Y, a saccharide of a saccharide residue represented by Y is similar to those cited in the above-mentioned oligonucleotide compounds (I). Of these, (1) galactose, mannose, melibiose, gentiobiose or isomaltotriose; or a hydroxyl group thereof is substituted by $C_{1-5}$ alkylcarbonyl (for example, acetyl) or benzoyl group, (2) glucoside or thioglucoside, or a hydroxyl group thereof is substituted by $C_{1-5}$ alkylcarbonyl (for example, acetyl), benzoyl or $C_{1-20}$ alkyl (for example, octyl), or (3) glucosamine or galactosamine, or a hydroxyl group thereof is substituted by $C_{1-5}$ alkylcarbonyl (for example, acetyl) which may be substituted by halogens (for example, fluoro) or benzoyl. Specifically, 1,2,3,4-O-tetraacetylgalactose, 1,2,3,4-O-tetraacetylmannose, 1-O-n-octyl-2,3,4-O-tribenzoylglucoside, 1-O-n-octyl-2,3,4-O-tribenzoylthioglucoside, 1-O-phenyl-2,3,4-O-tribenzoylglucoside, 1,3,4-O-tribenzoyl,2-N-benzoylgalactosamine, 1,3,4-O-triacetyl,2-N-acetylgalactosamine, 1,3,4-O-triacetyl,2-N-acetylglucosamine, 1,3,4-O-tribenzoyl,2-N-trifluoroacetylgalactosamine, 1,3,4-O-tribenzoyl,2-N-trifluoroacetylglucosamine, heptabenzoylmelibiose, heptabenzoylgentiobiose and octabenzoylmaltotriose are preferable examples. In the saccharide compounds (B), 1-O-n-octyl-2,3,4-O-tribenzoylglucoside and 1-O-n-octyl-2,3,4-O-tribenzoylthioglucoside are more preferable.

In the saccharide derivatives (B), $C_{1-5}$ alkyl, $C_{1-5}$ alkoxy, $C_{1-5}$ monoalkylamino or an aromatic ring group which may be substituted or unsubstituted and the substituents may be the same as for $X^1$, $X^2$ and $X^3$ above-mentioned, a phenyl group is for example preferable.

As the saccharide derivatives of Formula (A) and (B) in which Q is Y, those which bind to a 5'-terminal side of an oligonucleotide resulting in Gal(galactose), Man(mannose), n-oct-Glc(n-octylglucoside), n-oct-SGlc(n-octyl-thioglucoside), PhGlc(phenylglucoside), PhGal(phenylgalactoside), GalN(galactosamine), N-AcGal(N-acetylgalactosamine), N-BzGal(N-benzoylgalactosamine), GulN(glucosamine), N-AcGul(N-acetyl-glucosamine), Mel(melibiose), Gen(gentiobiose) and Iso(isomaltotriose) are preferable. As the saccharide derivatives (B) in which Q is Y, those which bind to a 5'-terminal side of an oligonucleotide resulting in n-oct-Glc(n-octylglucoside) and n-oct-SGlc(n-octyl-thioglucoside) are more preferable. Accordingly, those saccharides in which a hydroxyl group are substituted are preferable, for example, the products in Examples 1, 2, 5, 7, 9, 11, 13, 15, 17, 19, 21, 23, 25, 27, 29 and 31 are preferable.

As the saccharide compounds (A) and (B) in which Q is represented by formula

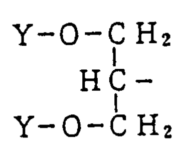

those which bind to a 5'-terminal side of an oligonucleotide resulting in Gal(galactose), Man(mannose) and Glu(glucose) are preferred, especially Gal(galactose) is preferred. For example, the saccharide derivatives (A) and (B) wherein the saccharide of saccharide residue represented by Y is monosaccharide (ex. galactose, mannose or glucose, especially galactose) of which a hydroxyl group may be substituted by $C_{1-5}$ alkylcarbonyl (ex. acetyl) are preferable. For example, the products in Example 63 is preferable.

(1) Method for Producing Oligonucleotide Compound (Ia) Wherein $X^1$, $X^4$ and $X^7$ Are Each OH or SH:

Reaction formula (a):

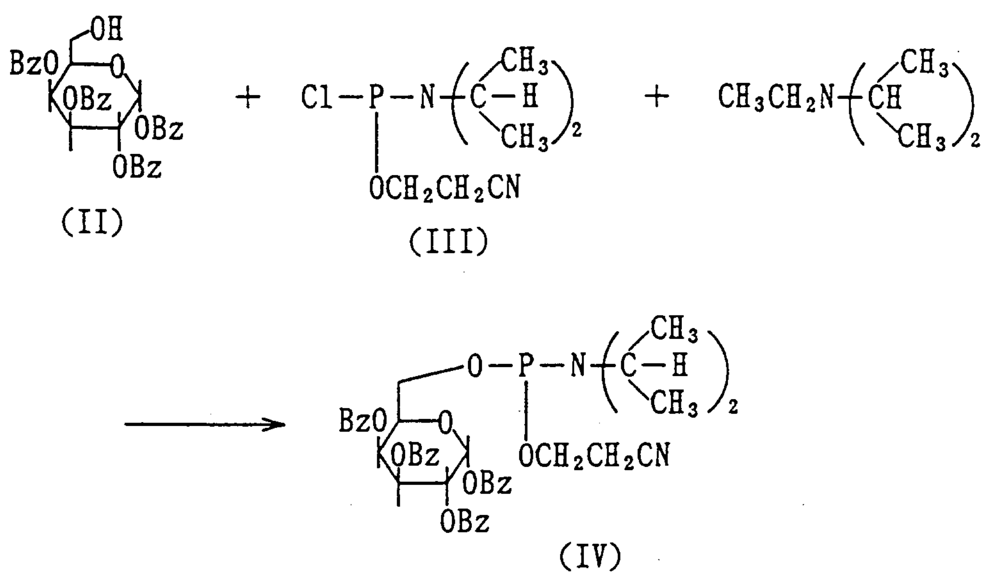

(II) (III) (IV)

wherein Bz represents benzoyl.

In reaction formula (a), the hydroxyl group of compound (II) is reacted with phosphorylating agent (III) in which O adjacent to P is protected by a cyanoethyl group to obtain compound (IV). About 1 to about 2 mmols of phosphorylating agent (III) and about 2 to about 5 mmols of ethyldiisopropylamine per mmol of compound (II) are used, and halogenated hydrocarbons (for example, methylene chloride and chloroform), ethers (for example, diethyl ether and tetrahydrofuran), aromatic hydrocarbons (for example,

benzene and toluene), acetonitrile, pyridine, dimethyl-formamide, dimethylsulfoxide and alcohols (for example, methanol and ethanol) can be used as solvents. Among these solvents, methylene chloride is preferably used. The reaction is usually conducted at a reaction temperature of about -20° to about 70°C, preferably about 15° to about 35°C, for about 0.5 to about 5 hours, preferably about 1 to about 2 hours, thereby obtaining compound (IV).

Starting material compounds (II) and (III) can be produced according to methods well known in the art or methods in accordance therewith. For example, starting material compound (II) can be prepared according to methods described in known literature references such as [Reagents for Organic Synthesis, vol. 1, 453, edited by L. Fieser, John Wiley and Sons, INC. (1967); D. D. Reynolds et al., Org. Syn., Coll., vol. 3, 432 (1955); and R. R. Schmidt, Angew. Chem. Int. Ed. Engl., vol. 25, 212 (1986) which are incorporated herein by reference].

Starting material compound (III) can be prepared according to methods described in known literatures [M. D. Matteuci et al., Tetrahedron Lett., vol. 21, 719 (1980); S. L. Beaucage et al., Tetrahedron Lett., vol. 22, 1859 (1981); L. J. McBride et al., Tetrahedron Lett., vol. 24, 245 (1983); B. C. Froehler et al., Nucleic Acids Res., vol. 14, 5399 (1986); B. C. Froehler et al., Tetrahedron Lett., vol. 27, 469 (1986); P. J. Garegg et al., Tetrahedron Lett., vol. 27, 4051 (1986); and E. Uhlman et al., Chemical Reviews, vol. 90, No. 4, 543 (1990) which are incorporated herein by reference].

Reaction formula (b):

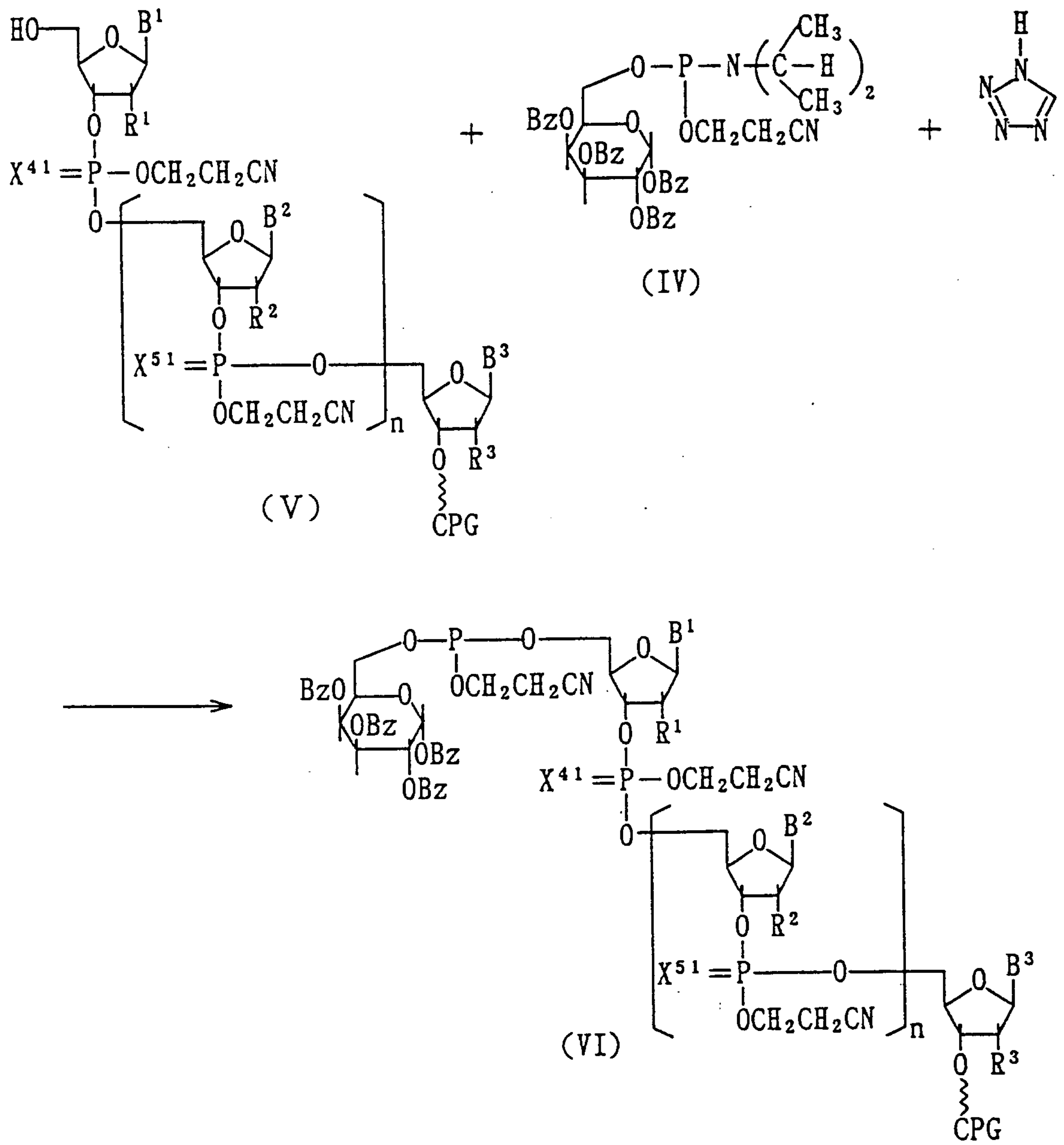

(V) (IV) (VI)

wherein Bz represents benzoyl; $X^{41}$ and $X^{51}$ each represent O or S; each $X^{41}$ may be the same or different when n is 2 or more; CPG represents a solid phase carrier (Controlled Pore Glass); and the other symbols have the same meanings as given above.

In reaction formula (b), compound (IV) obtained according to reaction formula (a) is reacted with a nucleotide compound appropriately protected (for example, O adjacent to P is protected by cyanoethyl) or oligonucleotide compound (V). About 20 to about 100 micromols of phosphited saccharide compound compound (V) and about 20 to about 100 micromols of 1H-tetrazole per micromol of compound (IV) are used, and halogenated hydrocarbons (for example, methylene chloride and chloroform), ethers (for example, diethyl ether and tetrahydrofuran), aromatic hydrocarbons (for example, benzene and toluene), acetonitrile, pyridine, dimethylformamide, dimethyl-sulfoxide and alcohols (for example, methanol and ethanol) can be used as solvents. Among these solvents, acetonitrile is preferably used. The reaction is usually conducted at a reaction temperature of about -20° to about 70°C, preferably about 15° to about 35°C, for about 3 to about 30 minutes, preferably about 5 to about 20 minutes to obtain compound (VI).

The nucleotide derivative or oligonucleotide compound (V), a starting material compound which may be appropriately protected, can be produced according to methods well known in the art or methods in accordance therewith. For example, 1H-Tetrazole, can be a starting material compound, can be produced according to methods well known in the art or methods in accordance therewith. That is, the production thereof can be conducted according to the method of synthesizing oligonucleotides using general nucleotide compounds [Oligonucleotide Synthesis; A Practical Approach, edited by M. J. Gait, IRL PRESS, (1984)]. A sequence of nucleotide chain elongation reaction can be carried out either by a manual method or by a method using an automatic synthesizer. In view of simple operation and accurate synthesis, a method using an automatic synthesizer is preferably employed.

Reaction formula (c):

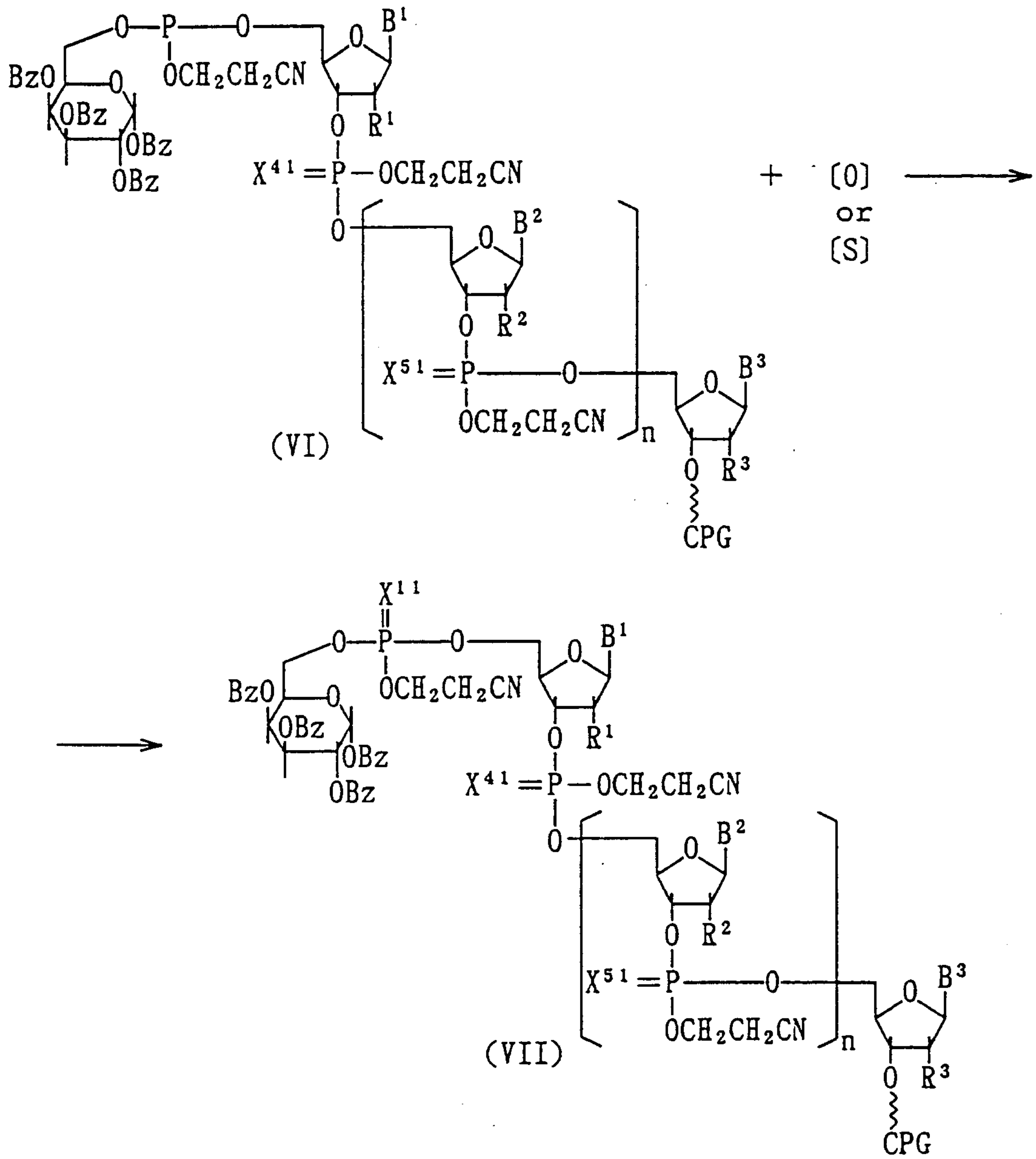


wherein Bz represents benzoyl; $X^{11}$, $X^{41}$ and $X^{51}$ each represent O or S. Each $X^{41}$ may be the same or different when n is 2 or more; CPG represents a solid phase carrier (Controlled Pore Glass); and the other symbols have the same meanings as given above.

In reaction formula (c), compound (VI) obtained according to reaction formula (b) is oxidized or sulfurized to obtain compound (VII). Usually, an oxidizing agent (for example, mCPBA, NBA, NBS, $I_2$ or DMSO-DCC, preferably $I_2$) or a sulfurizing agent (for example, di(phenylacetyl) disulfide, 3H-1,2-benzodithiol-3-on, tetraethylthiuram disulfide, dibenzoyl tetrasulfide, dibenzoyl trisulfide or dibenzoyl disulfide, preferably tetraethylthiuram disulfide) is added in an amount of about 20 to about 100 micromols per micromol of compound (VI), followed by reaction at a reaction temperature of about -20° to about 70°C, preferably about 15° to about 35°C. In the case of an oxidation reaction, the reaction time is usually about 30 seconds to about 100 minutes, and preferably about 30 seconds to about 5 minutes, and in the case of a sulfurizing reaction, the reaction time is usually about 30 seconds to about 100 minutes, and preferably about 30 seconds to about 20 minutes.

Then, the protective groups of compound (VII) thus obtained are removed, whereby the oligonucleotide compound (I) in which $X^1$, $X^4$ and $X^5$ are each OH or SH can be produced.

For removing the protective groups, methods well known in the art or methods in accordance therewith are used. For example, an ammonia/pyridine (5:1) solution is reacted at 60°C for 6 hours, thereby excising the nucleotide chains from the solid phase carrier, and removing the protective groups from the phosphoric acid groups, the base moieties and the saccharide moieties. After removal of all the protective groups, the oligonucleotide compounds (I) can be purified by reverse phase HPLC, etc. [Oligonucleotide Synthesis; A Practical Approach, edited by M. J. Gait, IRL PRESS, (1984)].

(2) Method for Producing Oligonucleotide Compound of Formula (Ia) in Which $X^1$, $X^4$ and $X^5$ Are Each $C_{1-5}$ Alkyl, $C_{1-5}$ Alkoxy or $C_{1-5}$ Monoalkylamino Which May Be Substituted:

Reaction formula (d):

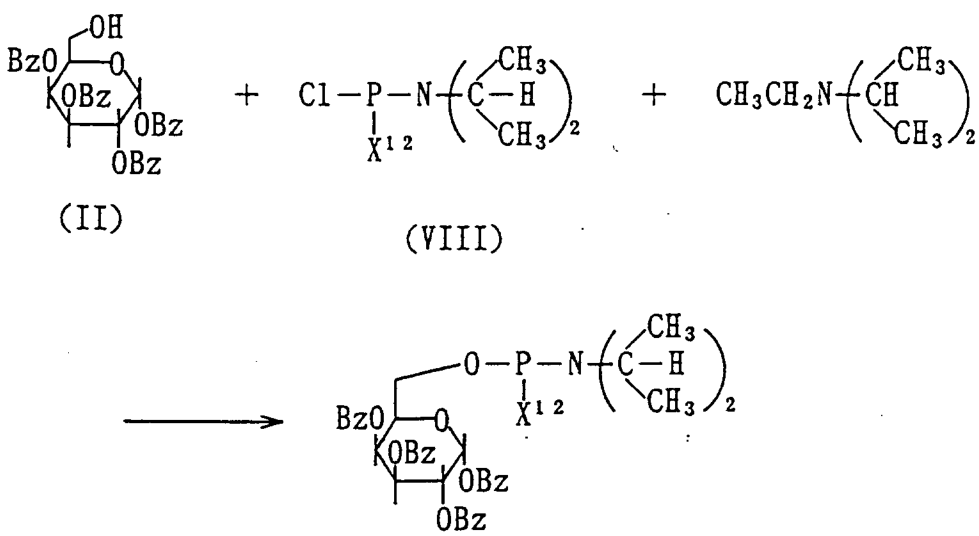

(II)

(VIII)

(IX)

Reaction formula (e):

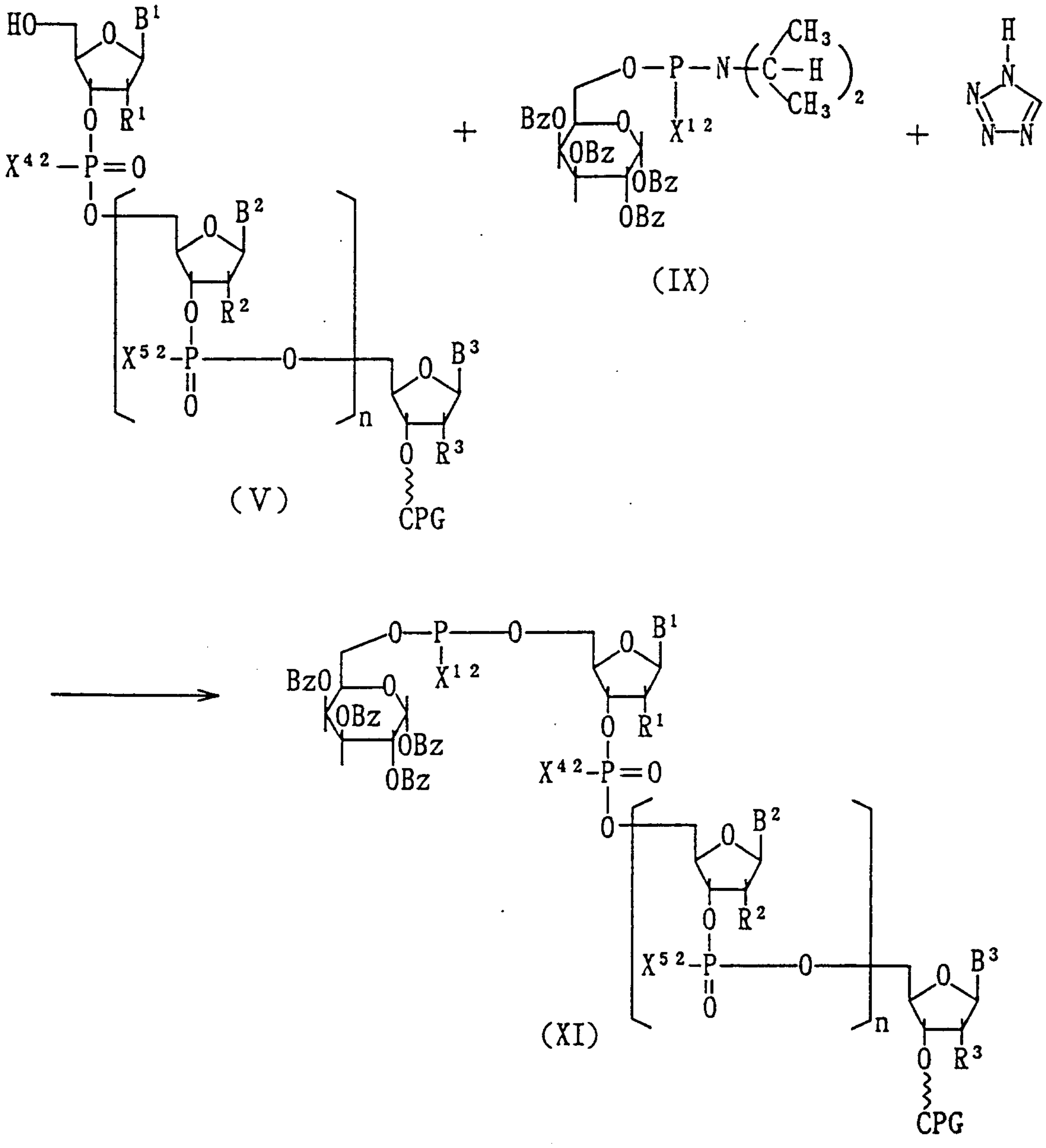

(V)

(IX)

(XI)

Reaction formula (f):

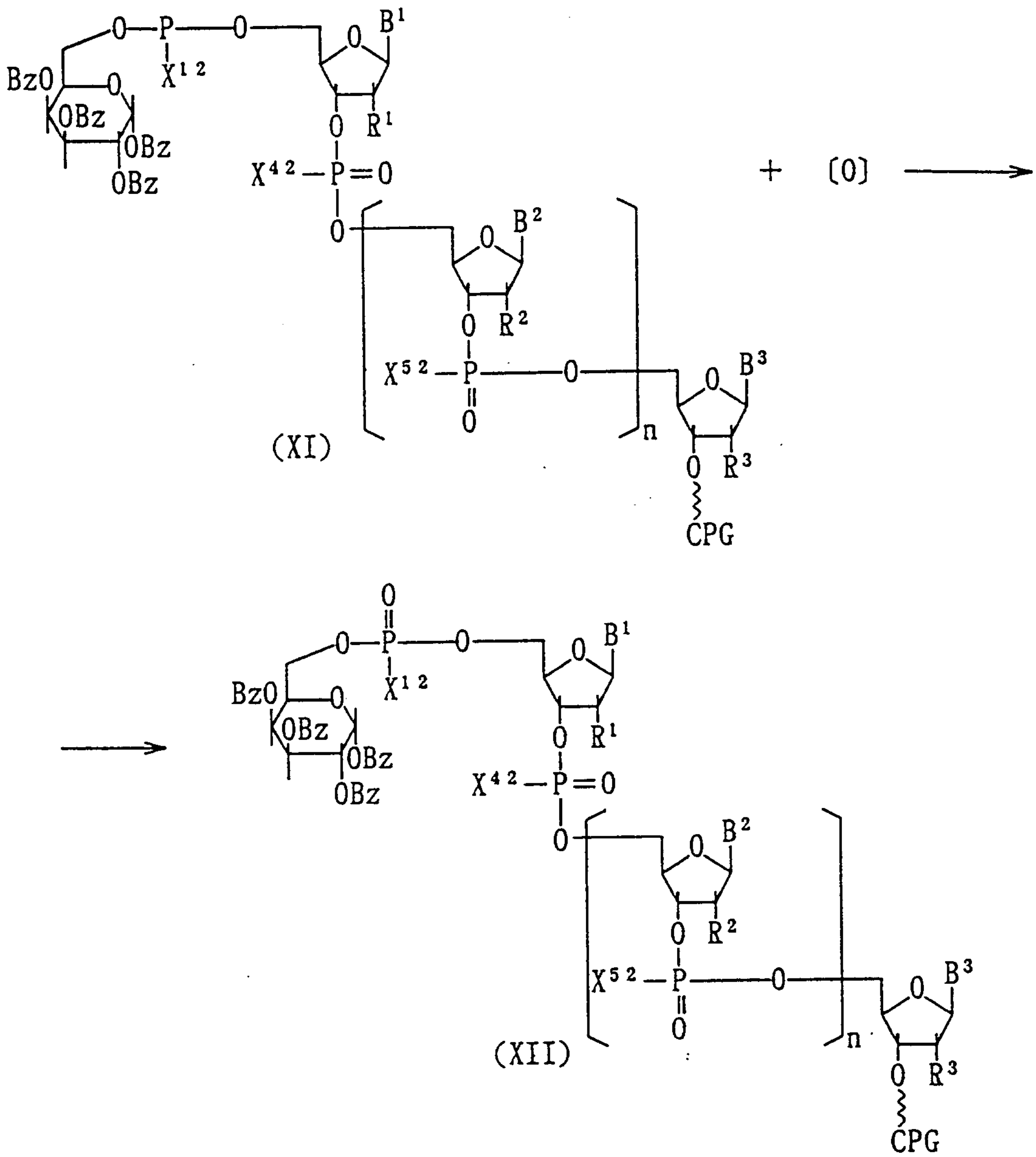

(XI)

(XII)

Phosphorylating agent (VIII), a starting compound, can be produced according to methods per se known in the art or methods in accordance therewith. For example, it can be produced according to methods described in known literature references [M. D. Matteuci et al., Tetrahedron Lett., vol. 21, 719 (1980); S. L. Beaucage et al., Tetrahedron Lett., vol. 22, 1859 (1981); L. J. McBride et al., Tetrahedron Lett., vol. 24, 245 (1983); B. C. Froehler et al., Nucleic Acids Res., vol. 14, 5399 (1986); B. C. Froehler et al., Tetrahedron Lett., vol. 27, 469 (1986); P. J. Garegg et al., Tetrahedron Lett., vol. 27, 4051 (1986); and E. Uhlman et al., Chemical Reviews, vol. 90, No. 4, 543 (1990) which are incorporated herein by reference].

Reaction formula (d) can be allowed to proceed similarly to the above-mentioned reaction formula (a), reaction formula (e) similarly to the above-mentioned reaction formula (b), and reaction formula (f) similarly to the above-mentioned reaction formula (c).

Then, the protective groups of compounds (XII) thus obtained are removed, whereby the oligonucleotide compound (Ia) can be produced in which $X^1$, $X^4$ and $X^5$ are each $C_{1-5}$ alkyl, $C_{1-5}$ alkoxy or $C_{1-5}$ monoalkylamino.

[Preparing method of oligonucleotide compounds of Formula (Ib)]

In the following manufacturing method, nucleotide derivatives represented by the following formula:

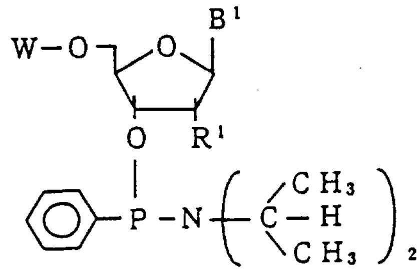

wherein W, $B^1$ and $R^1$ have the same meanings as above are useful as intermediate compounds for the oligonucleotide compounds of Formula (Ib).

W is preferably hydrogen or represented by formula

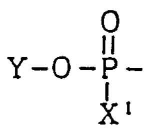

or

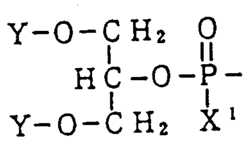

wherein Y and $X^1$ are the same as above.

When W is represented by formula

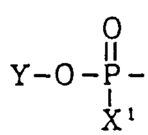

a saccharide of a saccharide residue represented by Y is preferably (1) galactose, mannose, melibiose, gentiobiose or isomaltotriose; or a hydroxyl group thereof is substituted by a $C_{1-5}$ alkylcarbonyl or a benzoyl group, (2) glucoside or thioglucoside, or a hydroxyl group thereof is substituted by a $C_{1-5}$ alkylcarbonyl, a benzoyl or a $C_{1-20}$ alkyl, or (3) glucosamine or galactosamine wherein a hydroxyl group thereof may be substituted by a $C_{1-5}$ alkylcarbonyl or a benzoyl which may be substituted by halogens. Specifically, 1,2,3,4-O-tetraacetylgalactose, 1,2,3,4-O-tetraacetylmannose, 1-O-n-octyl-2,3,4-O-tribenzoylglucoside, 1-O-n-octyl-2,3,4-O-trithiobenzoylglucoside, 1-O-phenyl-2,3,4-O-tribenzoylglucoside, 1,3,4-O-tribenzoyl,2-N-benzoylgalactosamine, 1,3,4-O-triacetyl,2-N-acetylgalactosamine, 1,3,4-O-triacetyl,2-N-acetylglucosamine, 1,3,4-O-tribenzoyl,2-N-trifluoroacetylgalactosamine, 1,3,4-O-tribenzoyl,2-N-trifluoroacetylglucosamine, heptabenzoylmelibiose, heptabenzoylgentiobiose and octabenzoylmaltotriose are preferred examples.

$X^1$ is preferably (1) OH, (2) SH, (3) $C_{1-5}$ alkyl, (4) $C_{1-5}$ alkoxy, (5) $C_{1-5}$ monoalkylamino or (6) phenyl which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl; OH, SH, methyl and phenyl are more preferred.

When W is represented by formula

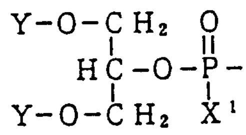

wherein Y and $X^1$ have the same meaning as above, a saccharide of a saccharide residue represented by Y is preferably a monosaccharide, for example, galactose, mannose or glucose; and galactose of which a hydroxyl group may be substituted by $C_{1-5}$ alkylcarbonyl (ex. acetyl) is more preferred.

$X^1$ is preferably (1) OH, (2) SH, (3) $C_{1-5}$ alkyl, (4) $C_{1-5}$ alkoxy, (5) $C_{1-5}$ monoalkylamino or (6) phenyl which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl; OH, SH, methyl and phenyl are preferably.

A method of preparing the oligonucleotide compounds of Formula (Ib) of the present invention, the oligonucleotide compounds of (Ib-1) which have a phosphonate type nucleotide at 5'-terminus in which W is a protective group;PiX (phenylxanthenyl) is illustrated by means of the examples below. However, the oligonucleotide compounds of Formula (Ib) in which W is represented by formula

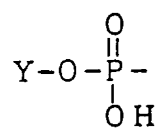

or

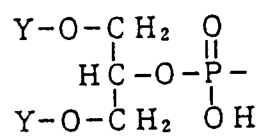

can also be obtained similarly.

(1) Method for Producing Oligonucleotide Compound of (Ib-1) Wherein $X^8$ is OH or SH (Oligonucleotide Compound (Ib) wherein $X^8$ is phenyl, $X^7$ is OH or SH):

Reaction formula (a):

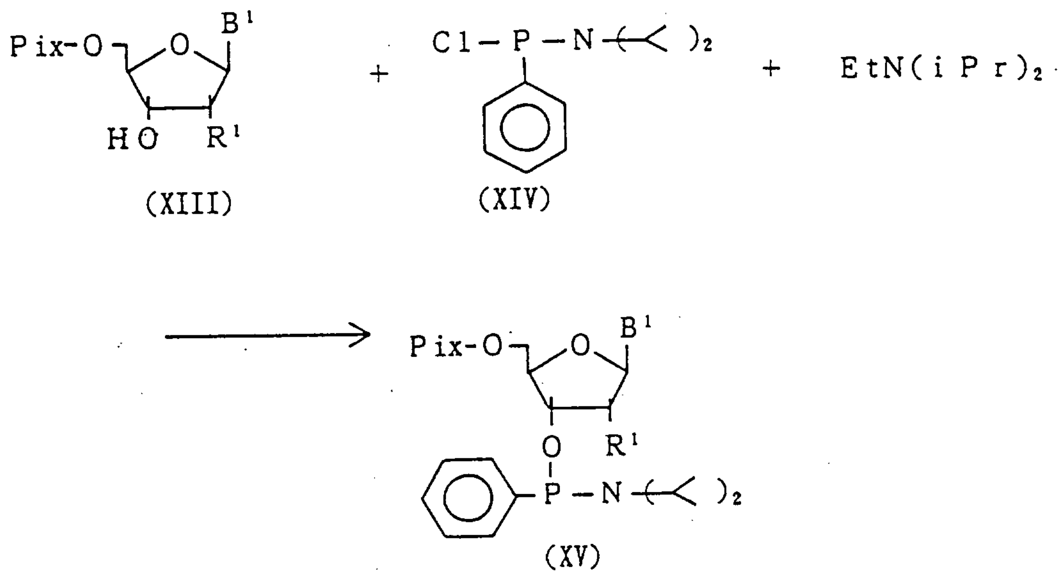


(XIII) (XIV) (XV)

wherein PiX represents phenylxanthenyl, and EtN(iPr)$_2$ represents ethyldiisopropylamine.

In reaction formula (a), the hydroxyl group of compound (XIII) is reacted with phosphorylating agent (XIV) in which a phenyl group is directly bound to P to obtain compound (XV). About 1 to about 2 mmols of phosphorylating agent (XIV) and about 2 to about 5 mmols of ethyldiisopropylamine per mmol of compound (XIII) are used, and halogenated hydrocarbons (for example, methylene chloride and chloroform), ethers (for example, diethyl ether and tetrahydrofuran), aromatic hydrocarbons (for example, benzene and toluene), acetonitrile, pyridine, dimethyl-formamide, dimethylsulfoxide and alcohols (for example, methanol and ethanol) can be used as solvents. Among these solvents, methylene chloride is preferably used. The reaction is usually conducted at a reaction temperature of about -20° to about 70°C, preferably about 15° to about 35°C, for about 0.5 to about 5 hours, preferably about 1 to about 2 hours, thereby obtaining compound (IXV).

Starting material compounds (XIII) and (XIV) can be produced according to methods well known in the art or methods in accordance therewith, for example, according to methods described in known literature references [M. D. Matteuci et al., Tetrahedron Lett., vol. 21, 719 (1980); S. L. Beaucage et al., Tetrahedron Lett., vol. 22, 1859 (1981); L. J. McBride et al., Tetrahedron Lett., vol. 24, 245 (1983); B. C. Froehler et al., Nucleic Acids Res., vol. 14, 5399 (1986); B. C. Froehler et al., Tetrahedron Lett., vol. 27, 469 (1986); P. J. Garegg et al., Tetrahedron Lett., vol. 27, 4051 (1986); and E. Uhlman et al., Chemical Reviews, vol. 90, No. 4, 543 (1990) which are incorporated by reference].

Reaction formula (b):

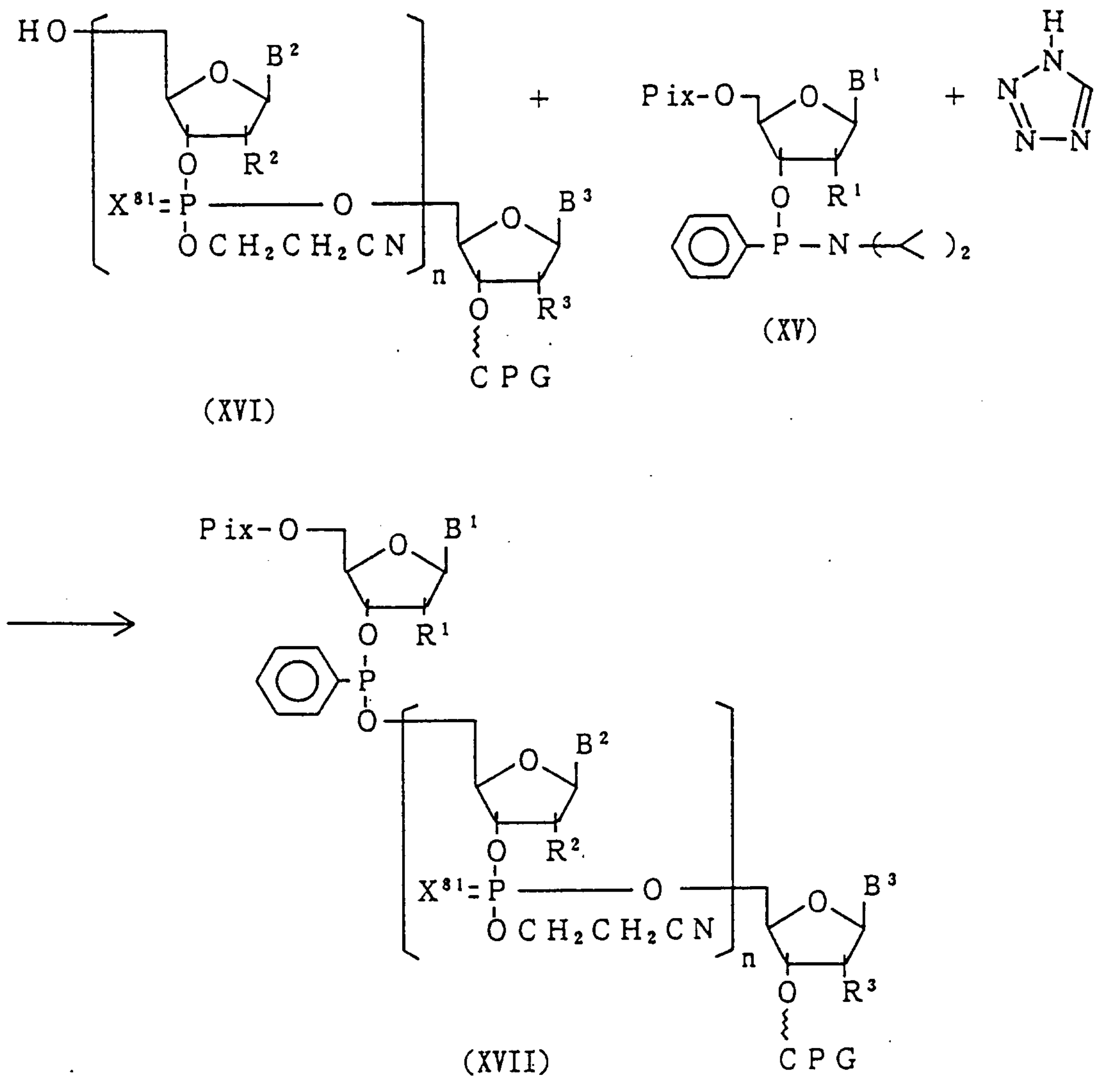

wherein $X^{81}$ represents O or S; each $X^{81}$ may be the same or different when n is 2 or more.

In reaction formula (b), compound (XV) obtained according to reaction formula (a) is reacted with a nucleotide compound appropriately protected (for example, O adjacent to P is protected by cyanoethyl) or oligonucleotide compound (XVI). About 20 to about 100 micromols of phenylphosphonite type nucleotide compound (XV) and about 20 to about 100 micromols of 1H-tetrazole per micromol of compound (XVI) are used, and halogenated hydrocarbons (for example, methylene chloride and chloroform), ethers (for example, diethyl ether and tetrahydrofuran), aromatic hydrocarbons (for example, benzene and toluene), acetonitrile, pyridine, dimethylformamide, dimethyl-sulfoxide and alcohols (for example, methanol and ethanol) can be used as solvents. Among these solvents, acetonitrile is preferably used. The reaction is usually conducted at a reaction temperature of about -20° to about 70°C, preferably about 15° to about 35°C, for about 3 to about 30 minutes, preferably about 5 to about 20 minutes to obtain compound (XVII).

The nucleotide compound or oligonucleotide compound of (XVI), a starting material compound, which may be appropriately protected can be produced according to methods per se known in the art or methods in accordance therewith. For example, 1H-Tetrazole, can be a starting material compound, can be produced according to methods well known in the art or methods in accordance therewith. That is, the production thereof can be conducted according to the method of synthesizing oligonucleotides using

general nucleotide compounds [Oligonucleotide Synthesis; A Practical Approach, edited by M. J. Gait, IRL PRESS, (1984)]. A sequence of nucleotide chain elongation reaction can be carried out either by a manual method or by a method using an automatic synthesizer. In view of simple operation and accurate synthesis, a method using the automatic synthesizer is preferably employed.

Reaction formula (c):

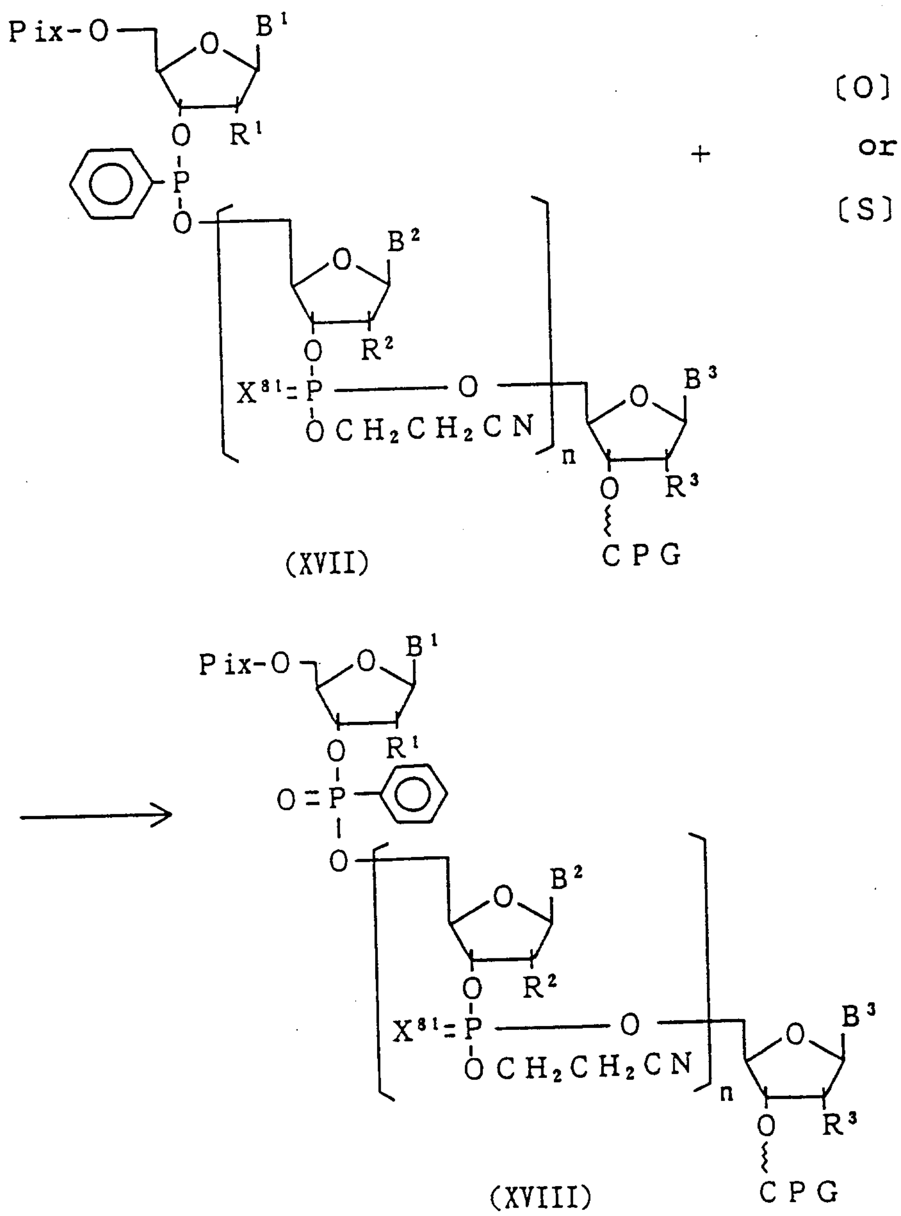


(XVII)

(XVIII)

In reaction formula (c), compound (XVII) obtained according to reaction formula (b) is oxidized or sulfurized to obtain compound (XVIII). Usually, an oxidizing agent (for example, mCPBA, NBA, NBS, $I_2$ or DMSO-DCC, preferably $I_2$) or a sulfurizing agent [for example, di(phenylacetyl) disulfide, 3H-1,2-benzodithiol-3-on, tetraethylthiuram disulfide, dibenzoyl tetrasulfide, dibenzoyl trisulfide or dibenzoyl disulfide, preferably tetraethylthiuram disulfide] is added in an amount of about 20 to about 100 micromols per micromol of compound (XVIII), followed by reaction at a reaction temperature of about -20° to about 70°C, preferably about 15° to about 35°C. In the case of oxidation reaction, the reaction time is usually about 30 seconds to about 100 minutes, and preferably about 30 seconds to about 5 minutes, and in the case of sulfurizing reaction, the reaction time is usually about 30 seconds to about 100 minutes, and preferably about 30 seconds to about 20 minutes.

Then, the protective groups of compound (XVIII) thus obtained are removed, whereby the oligonucleotide compound of Formula (Ib) can be produced.

For removing the protective groups, methods well known in the art or methods in accordance therewith are used. For example, an ammonia/pyridine (5:1) solution is reacted at 60 °C for 6 hours, thereby excising the nucleotide chains from the solid phase carrier, and removing the protective groups from the phosphoric acid groups, the base moieties and the saccharide moieties. After removal of all the protective groups, the oligonucleotide compounds (I) can be purified by reverse phase HPLC, etc. [Oligonucleotide Synthesis; A Practical Approach, edited by M. J. Gait, IRL PRESS, (1984)].

Further, in the above-mentioned preparing method, after removing Pix, other protective groups, for example, represented by formula

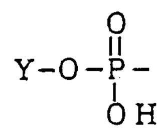

or

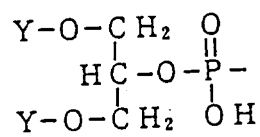

wherein Y and $X^1$ have the same meanings as above, can be introduced by the methodology well known in the art or similar methods.

(2) Method for Producing Oligonucleotide Compound of Formula (Ib-1) in Which $X^8$ is $C_{1-5}$ Alkyl, $C_{1-5}$ Alkoxy, $C_{1-5}$ Monoalkylamino or Aromatic Ring Group Which May Be Substituted:

Reaction formula (d):

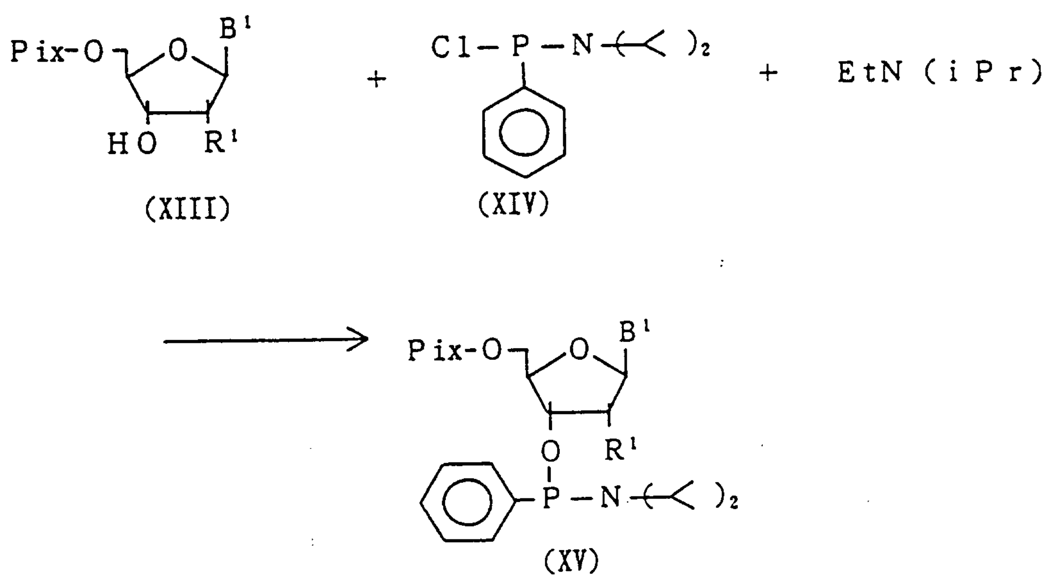


(XIII) (XIV) (XV)

wherein PiX represents phenylxanthenyl, and $EtN(iPr)_2$ represents ethyldiisopropylamine.

Reaction formula (e):

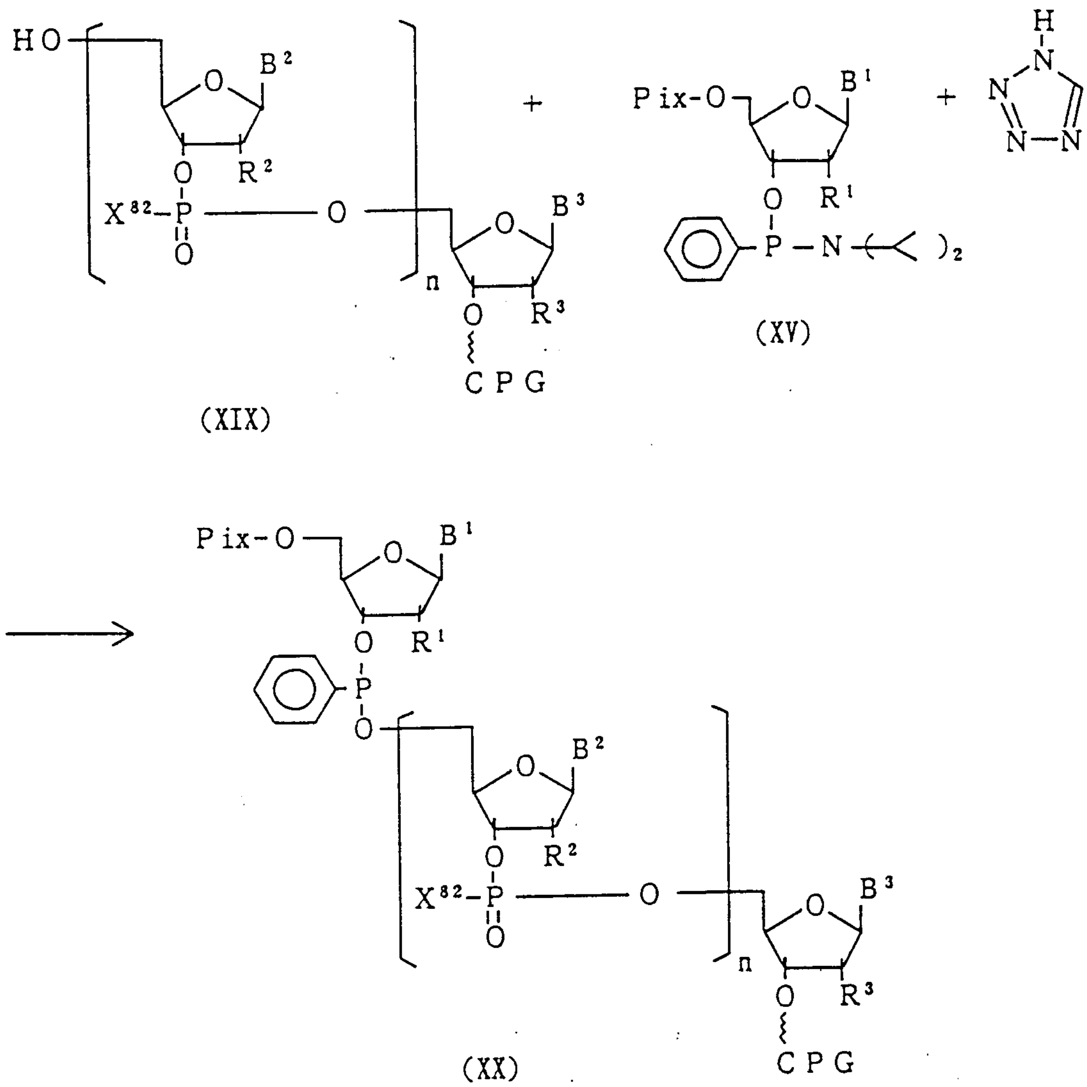

(XIX)

(XV)

(XX)

wherein $X^{82}$ represents $C_{1-5}$ alkyl, $C_{1-5}$ alkoxy, $C_{1-5}$ monoalkylamino or aromatic ring which may be substituted; each $X^{82}$ may be the same or different when n is 2 or more.

Reaction formula (f):

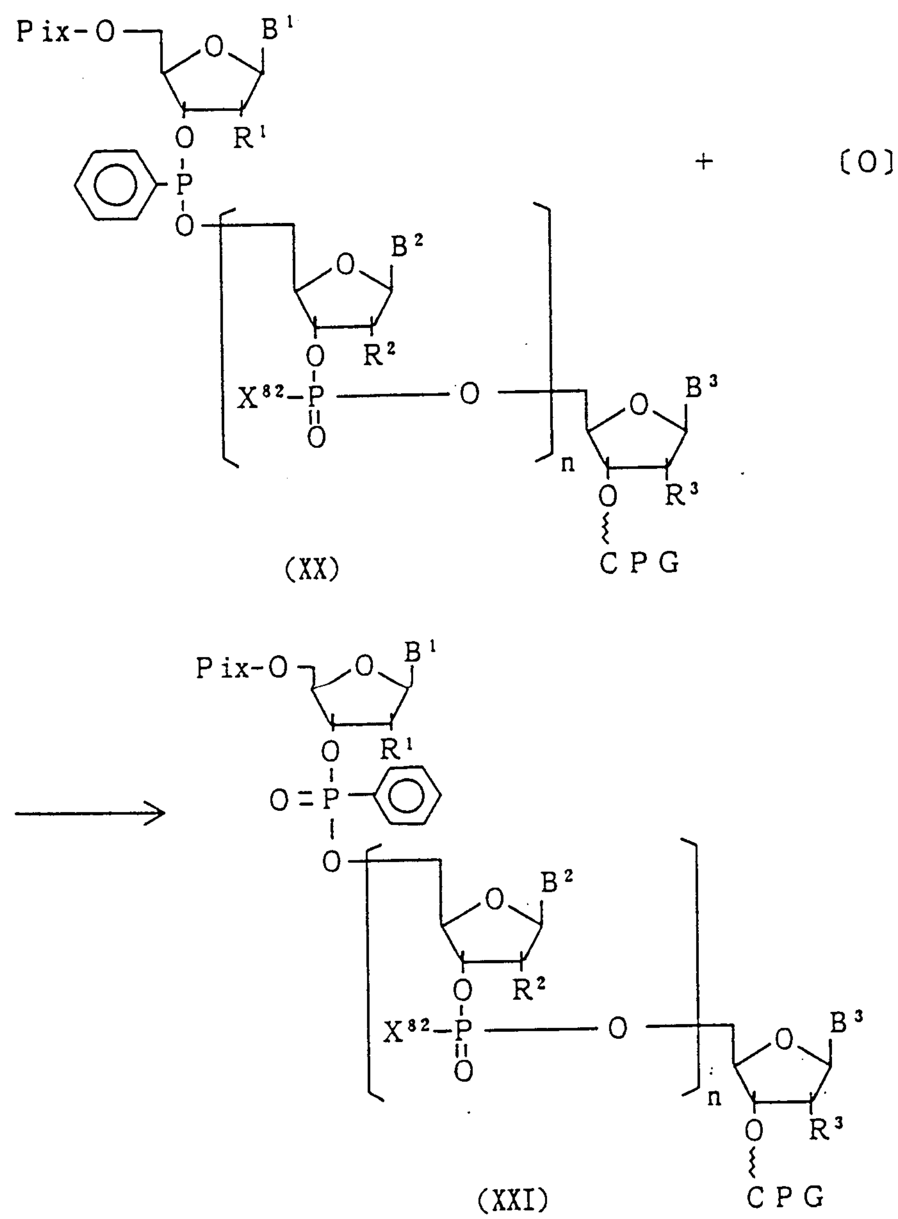


(XX)

(XXI)

Nucleotide compounds or oligonucleotide compounds (XIX), a starting compound, can be produced according to methods well known in the art or methods in accordance therewith. That is, the production thereof can be conducted according to the method of synthesizing oligonucleotides using general nucleotide compounds [Oligonucleotide Synthesis; A Practical Approach, edited by M. J. Gait, IRL PRESS, (1984)]. A sequence of nucleotide chain elongation reaction can be carried out either by a manual method or by a method using an automatic synthesizer. In view of simple operation and accurate synthesis, a method using the automatic synthesizer is preferably employed.

Reaction formula (d) can be allowed to proceed similarly to the above-mentioned reaction formula (a), reaction formula (e) similarly to the above-mentioned reaction formula (b), and reaction formula (f) similarly to the above-mentioned reaction formula (c).

Then, the protective groups of compounds (XIII) thus obtained are removed, whereby the oligonucleotide compound (Ib) can be produced in which $X^6$ is phenyl, $X^7$ is $C_{1-5}$ alkyl, $C_{1-5}$ alkoxy, $C_{1-5}$ monoalkylamino or an aromatic ring group which may be substituted.

For removing protective groups, similar methods are used. Further, in the above-mentioned preparing method, after removing Pix, other protective groups can be introduced by methods well known in the art of

similar methodology.

As described above, the saccharide compounds (A) and (B) of the present invention can be produced by methods well known in the art of similar methodology. For saccharide derivatives (A) of the present invention, methods of preparation the saccharide derivatives which have a branched oligosaccharide in which Q is represented by formula

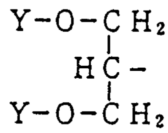

are illustrated as follows:

Reaction formula (a):

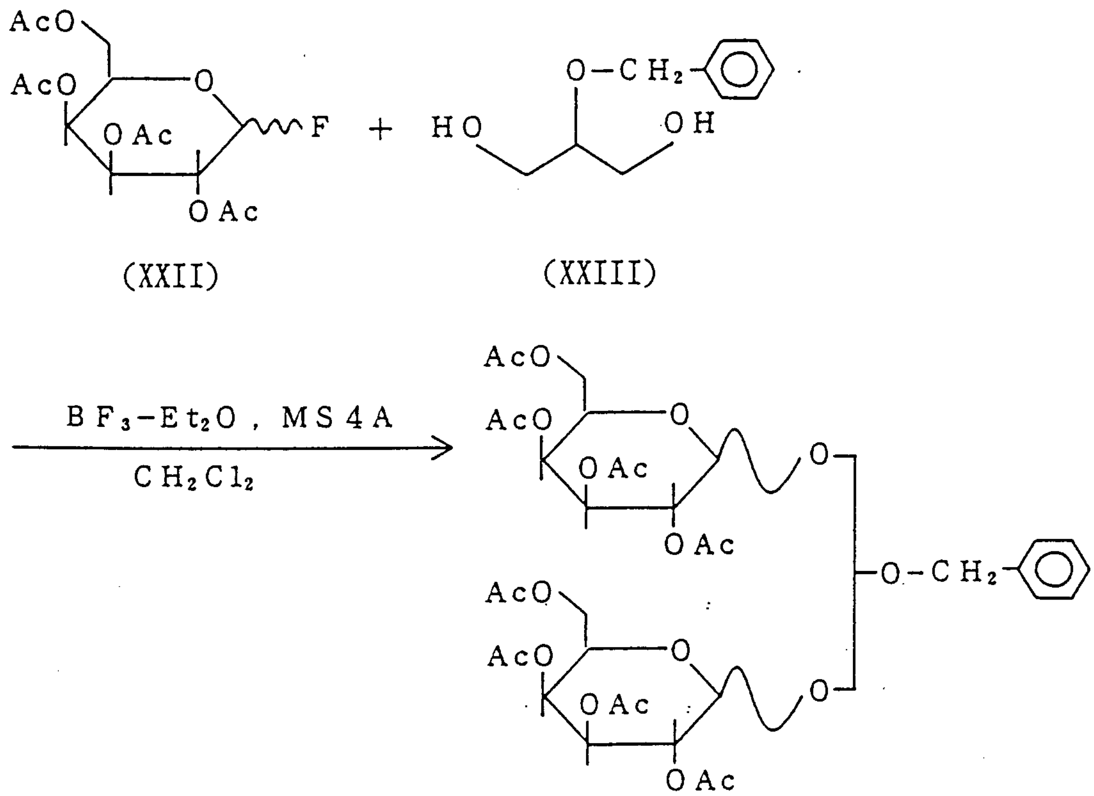


(XXII) (XXIII)

(XXIV)

In reaction formula (a), the hydroxyl group of the glycerol compound protected by benzyl (XXIII) is reacted with a fluorinated galactose compound (XXII) to obtain compound (XXIV). About 2 to 5 mmols of fluorinated galactose compound and about 3 to about 12 mmols of Lewis acid ($BF_3$-$Et_2O$, $SnCl_4$, $Cp_2HfCl_2$-$AgClO_4$, $Cp_2ZrCl_2$-$AgClO_4$, preferably $BF_3$-$Et_2$; Cp means cyclopentadienyl.) and molecular shieve are used per 1 mmole of compound (XXIII), and halogenated hydrocarbons (for example, methylene chloride and chloroform), ethers (for example, diethyl ether and tetrahydrofuran), aromatic hydrocarbons (for example, benzene and toluene), acetonitrile, pyridine, dimethyl-formamide, dimethylsulfoxide and alcohols (for example, methanol and ethanol) can be used as solvents. Among these solvents, methylene chloride is preferably used. The reaction is usually conducted at a reaction temperature of about -78° to about 80°C, preferably about 15° to about 35°C, for about 0.5 to about 5 hours, preferably about 1 to about 2 hours, thereby obtaining compound (XXIV).

Starting material compounds (XXII) and (XXIII) can be produced according to methods well known in the art or methods in accordance therewith. For example, starting material compound (XXII) can be prepared according to methods described in known literature references [K. Suzuki, et al., Tetrahedron Lett., vol. 29, 3571 (1988); K. Suzuki, et al., Tetrahedron Lett., vol. 29, 3575 (1988); K. Suzuki, et al., Tetrahedron Lett., vol. 30, 6879 (1989); K. Suzuki, et al., Tetrahedron Lett., vol. 30, 4853 (1989); T. Ogawa, et al., Tetrahedron Lett., vol. 31, 1597 (1990); T. Ogawa, et al., Tetrahedron Lett., vol. 33, 6343 (1992); T. Ogawa, et al., Tetrahedron Lett., vol. 33, 7907 (1992); T. Ogawa, et al., Tetrahedron Lett., vol, 34, 1061 (1993); K. C. Nicolaou et al., J. Am. Chem. Soc., vol. 112, 3693 (1990); K. C. Nicolaou et al., J. Am. Chem. Soc., vol. 114, 8701 (1992) which are incorporated herein by reference].

**Reaction formula (b):**

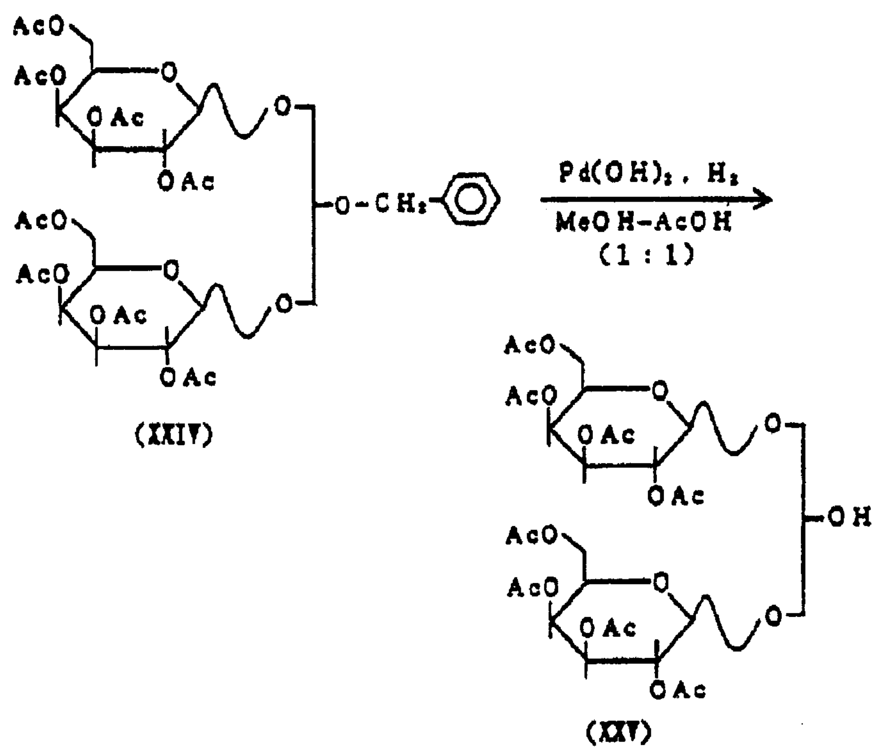


(XXIV)

(XXV)

In reaction formula (b), a benzyl group of the compound (XXIV) is removed by catalytic reduction to obtain compound (XXV). 30 to 60 mg of 10% $Pd(OH)_2$ is added to 1 micromol of compound (XXIV), and halogenated hydrocarbons (for example, methylene chloride and chloroform), ethers (for example, diethyl ether and tetrahydrofuran), aromatic hydrocarbons (for example, benzene and toluene), acetonitrile, pyridine, dimethylformamide, dimethylsulfoxide and alcohols (for example, methanol and ethanol) can be used is solvents. Among those solvents, methanolethylacetate (1:1) is preferably used, under middle pressure (3.5 kg/cm$^2$), hydrogen is added usually at a reaction temperature of about -78° to about 80°C, preferably about 15° to about 35°C, usually for about 1 to about 24 hours, preferably about 3 to about 15 hours to obtain compound (XXV).

Reaction formula (c):

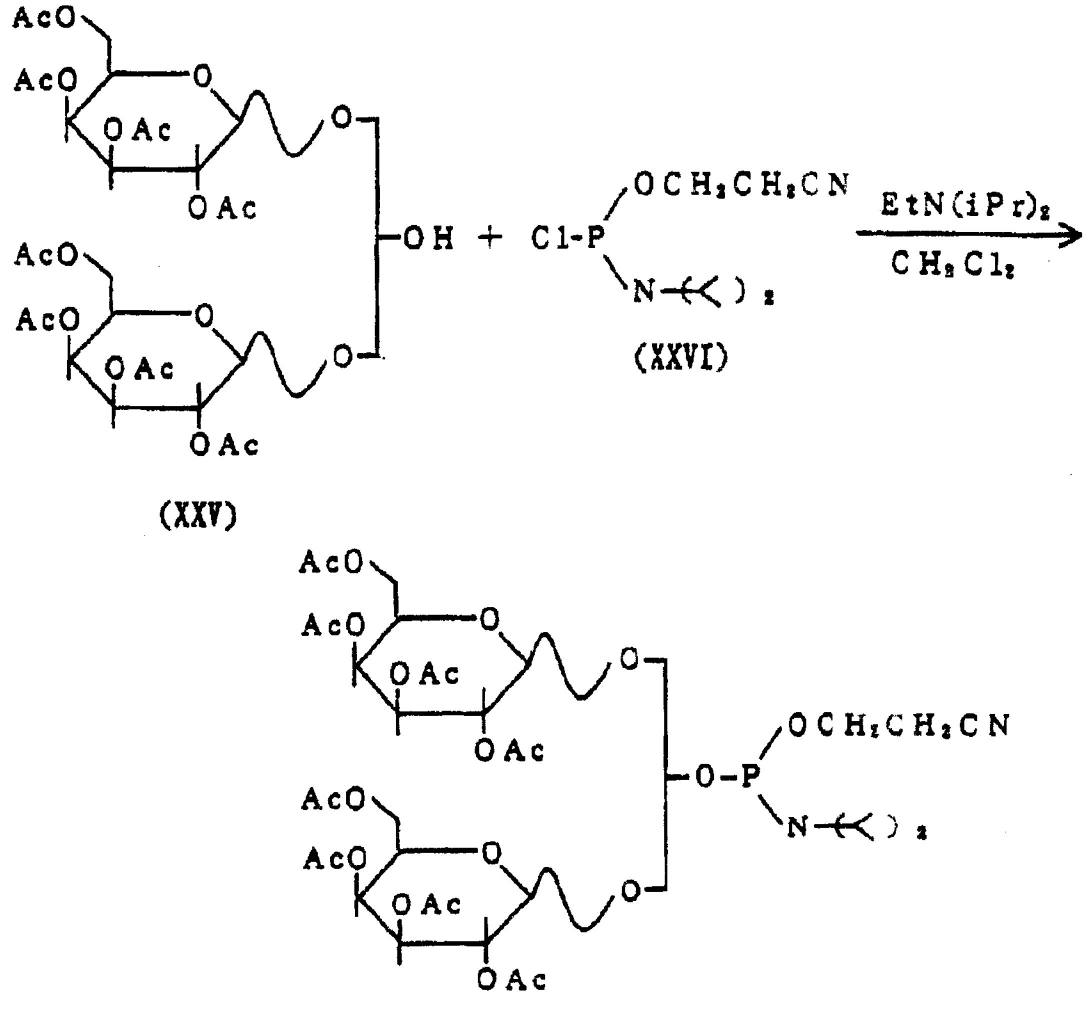


(XXV)

(XXVI)

(XXVII)

In reaction formula (c), the OH group of compound (XXV) is reacted with a phosphorylating agent (XXVI) in which O adjacent to P is protected by cyanoethyl to obtain compound (XXVII). About 1 to about 2 mmole of the phosphorylating agent (XXVI) and about 2 to about 5 mmole of ethyldiisopropylamine are used, and halogenated hydrocarbons (for example, methylene chloride and chloroform), ethers (for example, diethyl ether and tetrahydrofuran), aromatic hydrocarbons (for example, benzene and toluene), acetonitrile, pyridine, dimethylformamide, dimethyl-sulfoxide and alcohols (for example, methanol and ethanol) can be used as solvents. Among these solvents, methylene chloride is preferably used. The reaction is usually conducted at a reaction temperature of about -20° to about 70°C, preferably about 15° to about 35°C, for about 0.5 to about 5 hours, preferably about 1 to about 2 hours to obtain compound (XXVII).

A starting material (XXVI) can be prepared according to methods described in known literature references [M. D. Matteuci et al., Tetrahedron Lett., vol. 21, 719 (1980); S. L. Beaucage et al., Tetrahedron Lett., vol. 22, 1859 (1981); L. J. McBride et al., Tetrahedron Lett., vol, 24, 245 (1983); B. C. Froehler at al., Nucleic Acids Res., vol. 14, 5399 (1986); B. C. Froehler et al., Tetrahedron Lett., vol. 27, 469 (1986); P. J. Garegg et al., Tetrahedron Lett., vol. 27, 4051 (1986); and E. Uhlman et al., Chemical Reviews, vol. 90, No. 4, 543 (1990) which are incorporated herein by reference].

The saccharide derivative represented by the formula

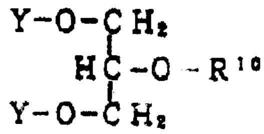

wherein Y represents a saccharide residue and $R^{10}$ represents hydrogen or a benzyl group, which involves the starting material (XXV) used in the above preparing method, is a novel compound and is useful as an intermediate compound of the saccharide derivitives (A) and (B) wherein Q represents a formula

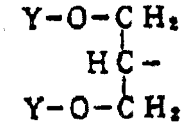
,

As the saccharide of saccharide residue represented by Y, monosaccharide (ex. galactose, mannose or glucose; and galactose is more preferable) of which a hydroxyl group may be substituted by $C_{1-5}$ alkylcarbonyl (ex. acetyl) is preferable. For example, those obtained in Examples 61 and 62 are preferable.

When the resulting oligonucleotide compounds (I), (Ia) and (Ib) are obtained in the free state, they can be converted to their salts according to methods per se known in the art or methods in accordance therewith. Conversely, when obtained in their salt state, they can be converted to the free forms or other salts according to methods per se known in the art or methods in accordance therewith.

The salts of the oligonucleotide compounds (I), (Ia) and (Ib) of the present invention are preferably physiologically acceptable salts with acids. Examples of such salts include salts with inorganic acids (for example, hydrochloric acid, phosphoric acid, hydrobromic acid and sulfuric acid) and salts with organic acids (for example, acetic acid, formic acid, propionic acid, fumaric acid, maleic acid, succinic acid, tartaric acid, citric acid, malic acid, oxalic acid, benzoic acid, methanesulfonic acid and benzenesulfonic acid).

The oligonucleotide compounds of Formulas (I), (Ia) and (Ib) of the present invention [hereinafter, oligonucleotide compounds (I) includes oligonucleotide compounds (Ia) and (Ib)] or the salts thereof can inhibit expression of genes producing proteins harmful in vivo, or expression of genes producing proteins (for example, proteins which bind to promoters to initiate transcription) responsible for expression of genes producing harmful proteins, by their complementality to DNAs or mRNAs of genes contributing to the development of various diseases (for example, malignant tumors, viral diseases, inflammations and reangiostenosis after PTCA). For example, they can (1) inhibit expression of genes associated with the development of malignant tumors in malignant tumor cells, (2) inhibit expression of viral genes derived from viruses, (3) inhibit expression of genes producing proteins which contribute to the development of inflammations, (4) control expression of drug-resistant genes which expression can reduce the efficacy of chemotherapy of malignant tumors, and (5) inhibit production of cell growth factors related to reangiostenosis after PTCA. They can therefore be used as antitumor agents, antiviral agents, antiinflammatory agents and drugs for controlling expression of specific genes such as resistance genes. These compounds can also be used in drug screening assays to look for other compounds that will affect the condition being examined. For example, when looking at a tumor cell where the tumor is associated with the expression of N-ras, one can use an oligopeptide compound that targets the N-ras (i.e. is antisense to a critical part of the N-ras such as the mRNA initiation site) and screen for other compounds that also affect the tumor. Thus, eocktails of agents can be selected and developed. The oligonucleotide compounds of Formula (Ia) can preferably be used as a so-called antisense-oligonucleotide compounds.

Many diseases are known which are associated with or caused by gene products produced by expression of specific genes. Examples thereof include the following. For example, the conversion of a proto-one oncogene to an oncogene (e.g. ras), excess expression of as oncogene (e.g. neu/erb B2). Viral genes or tumor genes having pathogenicity can roughly be divided into two kinds, those genes already existing in cells (e.g. a constituent gene such as ras) and genes called extraneous genes resulting from infection with extracellular viruses. Recent investigation has revealed that expression of these genes is one factor in the development of viral diseases or in the development of abnormal growth of normal cells. Further, adhesive proteins produced by expression of genes coding therefore such as ICAM-1 can contribute to the development of inflammations. Furthermore, although the expression of these genes is not directly the cause of disease, the expression of drug-resistant genes is undesirable and can contribute to

the severity of a condition, because organisms acquire drug resistance against antitumor agents, which trouble in chemotherapy of malignant tumors, resulting in the inability to obtain a complete cure.

The oligonucleotide compounds (I) of the present invention or salts thereof are therefore effective as stable, low-toxic therapeutic compositions (for example, antitumor compositions, antiviral compositions and antiinflammatory compositions) to warm-blooded mammals (for example, rats, mice, rabbits, pigs, sheep, monkeys, dogs, cats and humans, by controlling activation or expression of the genes contributing to the development of these diseases.

The oligonucleotide compounds (I) of the present invention or salts thereof can be used for example encapsulated within a liposome such as lipofectin in order to increase a cell membrane permeability and lipophilicity. The oligonucleotide compounds of Formula (Ia) of the present invention or salts thereof are preferably used being encapsulated within a liposome such as lipofectin.

When the oligonucleotide compounds (I) of the present invention or the salts thereof are used as the above-mentioned compositions, they can be given orally as tablets, capsules, elixirs and microcapsules coated with saccharide as needed, or parenterally in the form of injections such as sterile solutions or suspension with water or with pharmaceutically acceptable solutions other than water. For example, the oligonucleotide compounds (I) of the present invention or the salts thereof can be mixed with carriers, flavoring agents, excipients, vehicles, preservatives, stabilizing agents, binders, etc. in the form of unit dosage as required for generally acceptable pharmaceutical practice to prepare preparations. The amount of active ingredients in these preparations is adjusted so as to obtain appropriate doses within specified ranges.

Additives which can be mixed with tablets, capsules, etc. include, for example, binders such as gelatin, corn starch, tragacanth and gum arabic; excipients such as crystalline cellulose; swelling agents such as corn starch, gelatin and alginic acid; lubricants such as magnesium stearate; sweeteners such as sucrose, lactose and saccharine; and flavoring agents such as peppermint, acamono oil and cherry. When the preparation unit is in capsule form, liquid carriers such as fat and oil may further be added to materials of the above-mentioned types. Sterile compositions for injection can be formulated according to usual pharmaceutical practice such as dissolution or suspension of active substances and naturally occurring vegetable oils such as sesame oil and coconut oil in vehicles such as water for injection. Aqueous solutions for injection include physiological saline and isotonic solutions containing glucose or other adjuvants (for example, D-sorbitol, D-mannitol and sodium chloride), and may be used in combination with appropriate solubilizing adjuvants such as alcohols (for example, ethanol), palyalcohols (for example, polypropylene glycol and polyethylene glycol) and nonionic detergents (for example, Polysolvate 80 and HCO-50). Oily solutions include sesame oil and soybean oil, and may be used in combination with solubilizing adjuvants such as benzyl benzoate, benzyl alcohol, etc. The preparations may further contain buffers (for example, phosphate buffer and sodium acetate buffer), soothing agents (for example, benzalkonium chloride and procaine hydrochloride), stabilizing agents (for example, human serum albumin and polyethylene glycol), preservatives (for example, benzyl alcohol and phenol), antioxidants, etc. The injections thus prepared are usually filled into appropriate ampuls.

Although the dosage varies depending upon the kind of the subject disease, the symptom disease stage, etc., the oral dosage is generally about 0.1 to about 100 mg per day, preferably 1.0 to 50 mg, and more preferably 1.0 to 20 mg, for adults (taken as 60 kg). The parenteral dosage varies depending upon the object to which the preparations are given, the organ to which they are given, the symptom, the route of administration, etc. For example, when the preparations are given in the injection form, it is advantageous to be intravenously injected in a dosage of about 0.01 to about 30 mg per day, preferably 0.1 to 20 mg, and more preferably 0.1 to 10 mg, for adults (taken as 60 kg).

In the case of other mammals, they are also given in a similar dosage in terms of a weight per kg of object.

Further, the oligonucleotide compounds (I) or salts thereof of the present invention have excellent complementality to DNA. They can therefore also be used as probes for screening DNA. In general, for screening DNA, a method is used in which an appropriate oligonucleotide is synthesized and the desired DNA is screened using it as a probe. However, the low complementation frequently shown for the synthesized oligonucleotide to the DNA disadvantageously results in a reduction in screening efficiency. The oligonucleotide compounds (I) of the present invention or the salts thereof have excellent complementation to DNA, so that they are also effective as materials to conduct high-efficient screening. The compounds of the present invention can simply be substituted in probes and assays in place of other oligonucleotides. Thus, known techniques can be used when the present invention is used as a probe.

Moreover, the oligonucleotide compounds (I) of the present invention or the salts thereof have the saccharide compounds, etc. at the 5'-termini. Accordingly, saccharide analysis is conducted after complementary binding to the subject DNA, whereby the subject DNA can also be detected.

EXAMPLES

The present invention will be described in more detail through the following Reference Examples, Examples and Experimental Examples. It is understood of course that they are not intended to limit the scope of the invention.

[Reference Example 1]

Synthesis of ICAM-1 Antisense Phosphorothioate: $T_sG_sG_sG_sA_sG_sC_sC_sA_sT_sA_sG_sC_sG_sA_sG_sG_sC$ (SEQ ID NO: 31)

500 mg (10 micromols) of CPG into which 20 micromols/g of a cytidine compound was introduced, the N-4 amino group and the 5'-hydroxyl group of said cytidine compound being protected by a benzoyl group and a DMTr group,
respectively, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% $TCA/CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M guanosinephosphonamidite unit and 0.4 M tetrazole;
(5) sulfurizing reaction was conducted for 15 minutes using 0.5 M tetraethylthiuram disulfide/acetonitrile;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected guanosine compound could be condensed with the cytidine compound introduced into CPG, by a phosphorothioate bond. A subsequent nucleotide sequence of a guanosine compound was bound thereto by repetition of operations similar to those described above. The nucleotide chain was successively extended by repetition of the above-mentioned operations. DMTr released by the operation (1) was colorimetrically determined, and the average yield of each condensation reaction calculated from the results was 99% or more. Deprotecting operations after completion of synthesis reaction were conducted according to methods known in the art [Oligonucleotide Synthesis; A Practical Approach, edited by M. J. Gait, IRL PRESS, (1984) incorporated herein by reference]. CPG was taken out of the reaction column, and reacted with an ammonia/pyridine (5:1) solution at 60°C for 6 hours, thereby excising the nucleotide chain from the solid phase carrier and removing the protective group. Then, the product was purified by the use of reverse phase HPLC (μBondasphere $C_{18}$). By the above-mentioned operations, a desired product, ICAM-1 antisense phosphorothioate: $T_sG_sG_sG_sA_sG_sC_sC_sA_sT_sA_sG_sC_sG_sA_sG_sG_sC$, was synthesized.

Yield: 893.3 OD
λmax: 257.4 nm
λmin: 230.2 nm

[Reference Example 2]

Synthesis of Phosphorothioate Oligonucleotide Having the Reverse Sequence to That of ICAM-1 Antisense Phosphorothioate: $C_sG_sG_sA_sG_sC_sG_sA_sT_sA_sC_sC_sG_sA_sG_sG_sG_sT$ (SEQ ID NO:36)

500 mg (10 micromols) of CPG into which 20 micromols/g of a thymidine compound was introduced, the 5'-hydroxyl group of said thymidine compound being protected by a DMTr group, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic

synthesizer. Then, the following operations were carried out in the reaction column by the use of the automatic synthesizer:

(1) Treatment with 3.0% $TCA/CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M guanosinephosphonamidite unit and 0.4 M tetrazole;
(5) sulfurizing reaction was conducted for 15 minutes using 0.5 M tetraethylthiuram disulfide/acetonitrile;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected guanosine compound could be condensed with the thymidine compound introduced into CPG, by a phosphorothioate bond. A subsequent nucleotide sequence of a guanosine compound was bound thereto by repetition of operations similar to those described above. The nucleotide chain was successively extended by repetition of the above-mentioned operations. DMTr released by the operation (1) was colorimetrically determined, and the average yield of each condensation reaction calculated from the results was 99% or more. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphorothioate oligonucleotide having the reverse sequence to that of ICAM-1 antisense phosphorothioate: $C_sG_sG_sA_sG_sC_sG_sA_sT_sA_sC_sC_sG_sA_sG_sG_sG_sT$ was synthesized.

Yield: 719.1 OD
λmax: 257.1 nm
λmin: 230.9 nm

[Reference Example 3]

Synthesis of Phosphorothioate Reverse Sequence Oligonucleotide to Which N-Benzoylgalactosaminephosphate Was Bound: (N-BzGalPO)-$C_sG_sG_sA_sG_sC_sG_sA_sT_sA_sC_sC_sG_sA_sG_sG_sG_sT$ (SEQ ID NO: 36)

50 mg (1 micromol) of CPG into which 20 micromols/g of a thymidine compound was introduced, the 5'-hydroxyl group of said thymidine compound being protected by a DMTr group, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% $TCA/CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M guanosinephosphonamidite unit and 0.4 M tetrazole;
(5) sulfurizing reaction was conducted for 15 minutes using 0.5 M tetraethylthiuram disulfide/acetonitrile;
(6) A 0.25 M acetic anhydride/1-methylimidazole/lutidine/tetrahydrofuran solution was reacted for 30 seconds to block the unreacted 5'-hydroxyl group; and
(7) washing with acetonitrile and drying with an argon gas were carried out.

According to the above-mentioned operations, the protected guanosine compound could be condensed with the thymidine compound introduced into CPG, by a phosphorothioate bond. A subsequent nucleotide sequence of a guanosine compound was bound thereto by repetition of operations similar to those described above. The nucleotide chain was successively extended by repetition of the above-mentioned operations. DMTr released by the operation (1) was colorimetrically determined, and the average yield of each condensation reaction calculated from the results was 99% or more. After condensation of cytidylic acid as the 18th nucleotide, the N-benzoylgalactosamine compound obtained in Example 17 was bound to

the 5'-terminus. In this case, the N-benzoylgalactosamine compound was dissolved to give a concentration of 0.2 M, and the solution was loaded on the automatic synthesizer, followed by repetition of the above-mentioned operations (1) to (7) with the exception that the operations (1), (4) and (5) were conducted as follows:

(1) Treatment with 3.0% $TCA/CH_2Cl_2$ was conducted for 60 seconds to remove the 5'-DMTr group of the cytidine compound;

(4) Condensation reaction was conducted for 5 minutes using the 0.2 M N-benzoylgalactosamineamidite unit synthesized in Example 17 and 0.4 M tetrazole; and

(5) An oxidation reaction was conducted for 30 seconds using 0.1 M iodine/water/pyridine/THF.

By the above-mentioned operations, the N-benzoylgalactosamine compound could be introduced into the 5'-terminus of the reverse sequence oligonucleotide. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphorothioate reverse sequence oligonucleotide to which N-benzoylgalactosaminephosphate was bound: (N-BzGalPO)-$C_sG_sG_sA_sG_sC_sG_sA_sT_sA_sC_sC_sG_sA_sG_sG_sG_sT$ was synthesized.

Yield: 70.5 OD

λmax: 257.2 nm

λmin: 229.6 nm

[Reference Example 4]

Synthesis of Phosphate Reverse Sequence Oligonucleotide into Which Methylphosphonate Bonds Were Partially Lntroduced: $C_{Me}G_{Me}GAGCGATACCGAGG_{Me}G_{Me}T$ (SEQ ID NO: 38) ($C_{Me}$, $G_{Me}$: Methylphosphonate; A, G, C, T: Phosphate)

50 mg (1 micromol) of CPG into which 20 micromols/g of a thymidine compound was introduced, the 5'-hydroxyl group of said thymidine compound being protected by a DMTr group, was packed into a reaction column mounted on an automatic synthesizer (DNA Synthesizer Model 381 A, ABI). Then, a guanosinephenylphosphoramidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% $TCA/CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephenylphosphonamidite unit and 0.4 M tetrazole;

(5) Oxidation reaction was conducted for 30 seconds using 0.1 M iodine/water/pyridine/THF;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine phenylphosphonate compound could be condensed with the thymidine compound introduced into CPG. A subsequent nucleotide sequence of a guanosine phenylphosphonate compound was bound thereto by repetition of operations similar to those described above. A further subsequent nucleotide sequence of guanosine phosphate was bound thereto. In this case, a guanosinephosphonamidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% $TCA/CH_2Cl_2$ was conducted for 60 seconds to remove the 5'-Pix group of the protected guanosine phenylphosphonate compound. This solution was stored to calculate the condensation yield; and

(4) Condensation reaction was conducted for 5 minutes using the 0.2 M guanosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate reverse sequence oligonucleotide in which phenylphosphonate bonds were introduced into the 5'- and 3'-terminal dinucleotides: $C_{Me}G_{Me}$GAGCGATACCGAGG$_{Me}G_{Me}$T was synthesized.

Yield: 71.0 OD
λmax: 256.4 nm
λmin: 234.0 nm

[Reference Example 5]

Synthesis of Phosphate Antisense Oligonucleotide into Which Methylphosphonate Bonds Were Partially Introduced: $T_{Me}G_{Me}$GGAGCCATAGCGAG$_{Me}G_{Me}$C (SEQ ID NO: 34)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinemethylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M guanosinemethylphosphonamidite unit and 0.4 M tetrazole;
(5) An oxidation reaction was conducted for 30 seconds using 0.1 M iodine/water/pyridine/THF;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected guanosine methylphosphonate compound could be condensed with the cytidine compound introduced into CPG. A subsequent nucleotide sequence of a guanosine methylphosphonate compound was bound thereto by repetition of operations similar to those described above. A further subsequent nucleotide sequence of adenosine phosphate was bound thereto. In this case, a adenosinephosphonamidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-DMTr group of the protected guanosine methylphosphonate compound. This solution was stored to calculate the condensation yield; and
(4) Condensation reaction was conducted for 5 minutes using the 0.2 M adenosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. Deprotecting operations after completion of synthesis reaction were conducted according to methods known in the art [Oligonucleotide Synthesis: A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which methylphosphonate bonds were introduced into the each 5'-and 3'-terminal dinucleotides : $T_{Me}G_{Me}$GGAGCCATAGCGAG$_{Me}G_{Me}$C was synthesized.

Yield: 17.0 OD
λmax: 255.6 nm
λmin: 229.8 nm

[Example 1]

Synthesis of 1,2,3,4-O-Tetraacetylgalactose-6-O-N,N-diisopropylamino-O-cyanoethylphosphoramidite

1,2,3,4-O-Tetraacetylgalactose (0.87 g, 2.49 mmols) was dehydrated by azeotropic distillation with three 2 ml portions of anhydrous pyridine, followed by azeotropic distillation with three 2 ml portions of anhydrous toluene and three 2 ml portions of anhydrous methylene chloride. Then, the resulting product was dissolved in 5 ml of methylene chloride, and diisopropylethylamine (2.17 ml, 12.45 mmols) and N,N-diisopropylamino-O-cyanoethylphosphorchloride (1.11 ml, 4.98 mmols) were added to this solution, followed by reaction at room temperature for 1 hour. Thereafter, 50 ml of methylene chloride was added for dilution, and the resulting solution was washed with three 50 ml portions of a saturated aqueous solution of sodium hydrogencarbonate, followed by drying with anhydrous sodium sulfate. After removal of the solvent by distillation under reduced pressure, the resulting product was purified by silica gel column chromatography (hexane/methylene chloride = 80:20, 2% triethylamine) to obtain a desired product, 1,2,3,4-O-tetraacetyl-galactose-6-O-N,N-diisopropylamino-O-cyanoethylphosphoramidite (1.17 g, 2.14 mmols, 86%).
$^1$H-NMR ($CDCl_3$) δ6.34(m,1H,H-1), 5.67-5.30(m,3H,H-2,H-3,H-4), 4.34-4.18(m,3H,H-5,H-6), 3.90-3.56(m,2H, methine of isopropyl), 2.65(m,4H, methylene of cyanoethyl), 2.14-1.99(m,12H, methyl of acetyl), 1.31-1.19-(m,12H, methyl of isopropyl) ppm
$^{31}$P-NMR ($CDCl_3$) δ152.2, 152.3, 153.6, 153.7 ppm

[Example 2]

Synthesis of 1,2,3,4-O-Tetraacetylmannose-6-O-N,N-diisopropylamino-O-cyanoethylphosphoramidite

1,2,3,4-O-Tetraacetylmannose (0.97 g, 2.78 mmols) was dehydrated by azeotropic distillation with three 2 ml portions of anhydrous pyridine, followed by azeotropic distillation with three 2 ml portions of anhydrous toluene and three 2 ml portions of anhydrous methylene chloride. Then, the resulting product was dissolved in 5 ml of methylene chloride, and diisopropylethylamine (2.42 ml, 13.90 mmols) and N,N-diisopropylamino-O-cyanoethylphosphorchloride (1.24 ml, 5.56 mmols) were added to this solution, followed by reaction at room temperature for 1 hour. Thereafter, 50 ml of methylene chloride was added for dilution, and the resulting solution was washed with three 50 ml portions of a saturated aqueous solution of sodium hydrogencarbonate, followed by drying with anhydrous sodium sulfate. After removal of the solvent by distillation under reduced pressure, the resulting product was purified by silica gel column chromatography (hexane/methylene chloride = 80:20, 2% triethylamine) to obtain a desired product, 1,2,3,4-O-tetraacetyl-mannose-6-O-N,N-diisopropylamino-O-cyanoethylphosphoramidite (1.25 g, 2.28 mmols, 82%).
$^1$H-NMR ($CDCl_3$) δ6.08(d,1H,H-1), 5.36-5.25(m,3H,H-2,H-3,H-4), 3.87-3.77(m,3H,H-5,H-6), 3.64(m,2H, methine of isopropyl), 2.66(m,4H, methylene of cyanoethyl), 2.19-2.01(m,12H, methyl of acetyl), 1.30-1.16-(m,12H, methyl of isopropyl) ppm
$^{31}$P-NMR ($CDCl_3$) δ149.6, 149.7, 150.2, 150.3 ppm

[Example 3]

Synthesis of Phosphorothioate Oligonucleotide to Which Galactose Was Bound: (GAL)-$T_sG_sG_sG_sA_sG_sC_sC_sA_sT_sA_sG_sC_sG_sA_sG_sG_sC$ (SEQ ID NO: 31)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephosphonamidite unit and 0.4 M tetrazole;

(5) sulfurizing reaction was conducted for 15 minutes using 0.5 M tetraethylthiuram disulfide/acetonitrile;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine compound could be condensed with the cytidine compound introduced into CPG, by a phosphorothioate bond. A subsequent nucleotide sequence of guanosine compound was bound thereto. In this case, a guanosinephosphonamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-DMTr group of the protected guanosine compound. This solution was stored to calculate the condensation yield; and

(4) Condensation reaction was conducted for 5 minutes using the 0.2 M guanosinephosphonamidite unit and 0.4 M tetrazole.

The nucleotide chain was successively extended by repetition of the above-mentioned operations. DMTr released by the operation (1) was colorimetrically determined, and the average yield of each condensation reaction calculated from the results was 99% or more. After condensation of thymidylic acid as the 18th nucleotide, the galactose compound synthesized in Example 1 described above was bound to the 5'-terminus. In this case, the galactose compound was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-DMTr group of the protected thymidine compound; and

(4) Condensation reaction was conducted for 5 minutes using the 0.2 M galactosephosphonamidite unit synthesized in Example 1 and 0.4 M tetrazole.

By the above-mentioned operations, the galactose compound could be introduced into the 5'-terminus of the oligonucleotide. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphorothioate oligonucleotide to which galactose was bound: (GAL)-$T_sG_sG_sG_sA_sG_sC_sC_sA_sT_sA_sG_sC_sG_sA_sG_sG_sC$ was synthesized.

Yield: 43.65 OD

λmax: 256.3 nm

λmin: 229.3nm

[Example 4]

Synthesis of Phosphorothioate Oligonucleotide to Which Mannose Was Bound: (MAN)-$T_sG_sG_sG_sA_sG_sC_sC_sA_sT_sA_sG_sC_sG_sA_sG_sG_sC$ (SEQ ID NO: 31)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephosphonamidite unit and 0.4 M tetrazole;

(5) A sulfurizing reaction was conducted for 15 minutes using 0.5 M tetraethylthiuram disulfide/acetonitrile;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine compound could be condensed with the cytidine compound introduced into CPG, by a phosphorothioate bond. A subsequent nucleotide sequence of guanosine compound was bound thereto. In this case, a guanosinephosphonamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-DMTr group of the protected guanosine compound. This solution was stored to calculate the condensation yield; and

(4) Condensation reaction was conducted for 5 minutes using the 0.2 M guanosinephosphonamidite unit and 0.4 M tetrazole.

The nucleotide chain was successively extended by repetition of the above-mentioned operations. DMTr released by the operation (1) was colorimetrically determined, and the average yield of each condensation reaction calculated from the results was 99% or more. After condensation of thymidylic acid as the 18th nucleotide, the mannose compound synthesized in Example 2 described above was bound to the 5'-terminus. In this case, the mannose compound was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-DMTr group of the protected thymidine compound; and

(4) Condensation reaction was conducted for 5 minutes using the 0.2 M mannosephosphoramidite unit synthesized in Example 2 and 0.4 M tetrazole.

By the above-mentioned operations, the mannose compound could be introduced into the 5'-terminus of the oligonucleotide. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60°C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphorothioate oligonucleotide to which mannose was bound: (MAN)-$T_sG_sG_sG_sA_sG_sC_sC_sA_sT_sA_sG_sC_sG_sA_sG_sG_sC$ was synthesized.

Yield: 33.47 OD

λmax: 256.3 nm

λmin: 229.3 nm

[Example 5]

Synthesis of 1-O-n-Octyl-2,3,4-O-tribenzoylglucoside-6-O-N,N-diisopropylamino-O-cyanoethylphosphoramidite

1-O-n-Octyl-2,3,4-O-tribenzoylglucoside (1.22 g, 2.02 mmols) was dehydrated by azeotropic distillation with three 2 ml portions of anhydrous pyridine, followed by azeotropic distillation with three 2 ml portions of anhydrous toluene and three 2 ml portions of anhydrous methylene chloride. Then, the resulting product was dissolved in 5 ml of methylene chloride, and diisopropylethylamine (1.76 ml, 10.10 mmols) and N,N-diisopropylamino-O-cyanoethylphosphorchloride (0.90 ml, 4.04 mmols) were added to this solution, followed by reaction at room temperature for 1 hour. Thereafter, 50 ml of methylene chloride was added for dilution, and the resulting solution was washed with three 50 ml portions of a saturated aqueous solution of sodium hydrogencarbonate, followed by drying with anhydrous sodium sulfate. After removal of the solvent by distillation under reduced pressure, the resulting product was purified by silica gel column chromatography (hexane/methylene chloride = 80:20, 2% triethylamine) to obtain a desired product, 1-O-n-octyl-2,3,4-O-tribenzoylglucoside-6-O-N,N-diisopropylamino-O-cyanoethylphosphoramidite (1.42 g, 1.78 mmols, 88%).

$^1$H-NMR ($CDCl_3$) δ8.02-7.80(m,6H,o-H of benzoyl), 7.55-7.26(m,9H,m,p-H of benzoyl), 5.82(d,1H,H-1), 5.60-5.29, 5.60-5.30(m,3H,H-2,3,4), 3.95-3.77(m,3H,H-5,H-6), 3.59-3.50(m,2H,CH of isopropyl), 2.58-2.55-

(m,2H,$CH_2$ of cyanoethyl), 1.17-1.09(m,20H,$CH_3$ of isopropyl,$CH_2$ of octyl), 0.83(t,3H,$CH_3$ of octyl) ppm
$^{31}$P-NMR ($CDCl_3$) δ149.57, 149.65, 149.68, 149.74 ppm

[Example 6]

Synthesis of Phosphorothioate Antisense Oligonucleotide to Which n-Octylglucoside Was Bound: (n-oct-Glc)-$T_sG_sG_sG_sA_sG_sC_sC_sA_sT_sA_sG_sC_sG_sA_sG_sG_sC$ (SEQ ID NO: 31)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M guanosinephosphonamidite unit and 0.4 M tetrazole;
(5) A sulfurizing reaction was conducted for 15 minutes using 0.5 M tetraethylthiuram disulfide/acetonitrile;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected guanosine compound could be condensed with the cytidine compound introduced into CPG, by a phosphorothioate bond. A subsequent nucleotide sequence of guanosine compound was bound thereto. In this case, a guanosinephosphonamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-DMTr group of the protected guanosine compound. This solution was stored to calculate the condensation yield; and
(4) Condensation reaction was conducted for 5 minutes using the 0.2 M guanosinephosphonamidite unit and 0.4 M tetrazole.

The nucleotide chain was successively extended by repetition of the above-mentioned operations. DMTr released by the operation (1) was colorimetrically determined, and the average yield of each condensation reaction calculated from the results was 99% or more. After condensation of thymidylic acid as the 18th nucleotide, the n-octylglucoside compound synthesized in Example 5 was bound to the 5'-terminus. In this case, the n-octylglucoside compound was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-DMTr group of the protected thymidine compound; and
(4) Condensation reaction was conducted for 5 minutes using the 0.2 M n-octylglucosidephosphoramidite unit synthesized in Example 5 and 0.4 M tetrazole.

By the above-mentioned operations, the n-octylglucoside compound could be introduced into the 5'-terminus of the antisense oligonucleotide. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60°C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphorothioate oligonucleotide to which n-octylglucoside was bound: (n-oct-Glc)-$T_sG_sG_sG_sA_sG_sC_sC_sA_sT_sA_sG_sC_sG_sA_sG_sG_sC$ was synthesized.

Yield: 38.7 OD
λmax: 256.5 nm

λmin: 229.1 nm

[Example 7]

Synthesis of 1-O-n-Octyl-2,3,4-O-tribenzoylthioglucoside-6-O-N,N-diisopropylamino-O-cyanoethyl-phosphoramidite

1-O-n-Octyl-2,3,4-O-tribenzoylthioglucoside (0.76 g, 1.22 mmols) was dehydrated by azeotropic distillation with three 2 ml portions of anhydrous pyridine, followed by azeotropic distillation with three 2 ml portions of anhydrous toluene and three 2 ml portions of anhydrous methylene chloride. Then, the resulting product was dissolved in 5 ml of methylene chloride, and diisopropylethylamine (1.06 ml, 6.10 mmols) and N,N-diisopropylamino-O-cyanoethylphosphorchloride (0.50 ml, 2.44 mmols) were added to this solution, followed by reaction at room temperature for 1 hour. Thereafter, 50 ml of methylene chloride was added for dilution, and the resulting solution was washed with three 50 ml portions of a saturated aqueous solution of sodium hydrogencarbonate, followed by drying with anhydrous sodium sulfate. After removal of the solvent by distillation under reduced pressure, the resulting product was purified by silica gel column chromatography (hexane/methylene chloride = 80:20, 2% triethylamine) to obtain a desired product, 1-O-n-octyl-2,3,4-O-tribenzoylthioglucoside-6-O-N,N-diisopropylamino-O-cyanoethylphosphoramidite (0.82 g, 1.00 mmol, 82%).

$^1$H-NMR ($CDCl_3$) δ7.97-7.93(m,6H,o-H of benzoyl), 7.52-7.26(m,9H,m,p-H of benzoyl), 5.82(d,1H,H-1), 5.61-5.45, 4.82-4.75(m,3H,H-2,3,4), 3.85-3.78(m,3H,H-5,H-6), 3.68-3.50(m,2H,CH of isopropyl), 2.61-2.54-(m,2H,$CH_2$ of cyanoethyl), 1.29-1.10(m,20H,$CH_3$ of isopropyl,$CH_2$ of octyl), 0.90-0.86(t,3H,$CH_3$ of octyl) ppm

$^{31}$P-NMR ($CDCl_3$) δ149.51, 149.91, 151.18 ppm

[Example 8]

Synthesis of Phosphorothioate Antisense Oligonucleotide to Which n-Octylthioglucoside Was Bound: (n-oct-SGlc)-$T_sG_sG_sG_sA_sG_sC_sC_sA_sT_sA_sG_sC_sG_sA_sG_sG_sC$ (SEQ ID NO: 31)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephosphonamidite unit and 0.4 M tetrazole;

(5) A sulfurizing reaction was conducted for 15 minutes using 0.5 M tetraethylthiuram disulfide/acetonitrile;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine compound could be condensed with the cytidine compound introduced into CPG, by a phosphorothioate bond. A subsequent nucleotide sequence of guanosine compound was bound thereto. In this case, a guanosinephosphonamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-DMTr group of the protected guanosine compound. This solution was stored to calculate the condensation yield; and

(4) Condensation reaction was conducted for 5 minutes using the 0.2 M guanosinephosphonamidite unit and 0.4 M tetrazole.

The nucleotide chain was successively extended by repetition of the above-mentioned operations. DMTr released by the operation (1) was colorimetrically determined, and the average yield of each condensation reaction calculated from the results was 99% or more. After condensation of thymidylic acid as the 18th nucleotide, the n-octylthioglucoside compound synthesized in Example 7 was bound to the 5'-terminus. In this case, the n-octylthioglucoside compound was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-DMTr group of the protected thymidine compound; and

(4) Condensation reaction was conducted for 5 minutes using the 0.2 M n-octylthioglucosidephosphoramidite unit synthesized in Example 7 and 0.4 M tetrazole.

By the above-mentioned operations, the n-octylthioglucoside compound could be introduced into the 5'-terminus of the antisense oligonucleotide. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphorothioate antisense oligonucleotide to which n-octylthioglucoside was bound: (n-oct-SGlc)-$T_sG_sG_sG_sA_sG_sC_sC_sA_sT_sA_sG_sC_sG_sA_sG_sG_sC$ was synthesized.

Yield: 14.7 OD

λmax: 256.9 nm

λmin: 229.6 nm

[Example 9]

Synthesis of 1-O-n-Octyl-2,3,4-O-tribenzoylglucoside-6-O-(N,N-diisopropylamino)phenylphosphonamidite

1-O-n-Octyl-2,3,4-O-tribenzoylglucoside (1.15 g, 1.91 mmols) was dehydrated by azeotropic distillation with three 2 ml portions of anhydrous pyridine, followed by azeotropic distillation with three 2 ml portions of anhydrous toluene and three 2 ml portions of anhydrous methylene chloride. Then, the resulting product was dissolved in 5 ml of methylene chloride, and diisopropylethylamine (1.66 ml, 9.54 mmols) and chloro-N,N-diisopropylaminophenylphosphonamidite (0.93 ml, 3.82 mmols) were added to this solution, followed by reaction at room temperature for 1 hour. Thereafter, 50 ml of methylene chloride was added for dilution, and the resulting solution was washed with three 50 ml portions of a saturated aqueous solution of sodium hydrogencarbonate, followed by drying with anhydrous sodium sulfate. After removal of the solvent by distillation under reduced pressure, the resulting product was purified by silica gel column chromatography (hexane/methylene chloride = 80:20, 2% triethylamine) to obtain a desired product, 1-O-n-octyl-2,3,4-O-tribenzoylglucoside-6-O-(N,N-diisopropylamino)phenylphosphonamidite (1.01 g, 1.24 mmols, 65%).

$^1$H-NMR ($CDCl_3$) δ7.98-7.82(m,11H,o-H of benzoyl, phenyl), 7.53-7.24(m,9H,m-,p-H of benzoyl), 5.93-5.83-(s,1H,H-1), 4.84-4.80, 5.54-5.45(m,3H,H-2,3,4), 3.92-3.77(m,3H,H-5,6), 3.56-3.47(m,2H,CH of isopropyl), 1.38-1.22(m,2OH,$CH_3$ of isopropyl,$CH_2$ of octyl), 0.86-0.80(t,3H,$CH_3$ of octyl) ppm

$^{31}$P-NMR ($CDCl_3$) δ120.86, 121.95 ppm

[Example 10]

Synthesis of Phosphorothioate Antisense Oligonucleotide to Which Octylglucoside Phenylphosphonate Was Bound: (n-oct-GlcPh)-$T_sG_sG_sG_sA_sG_sC_sC_sA_sT_sA_sG_sC_sG_sA_sG_sG_sC$ (SEQ ID NO: 31)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephosphonamidite unit and 0.4 M tetrazole;

(5) A sulfurizing reaction was conducted for 15 minutes using 0.5 M tetraethylthiuram disulfide/acetonitrile;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine compound could be condensed with the cytidine compound introduced into CPG, by a phosphorothioate bond. A subsequent nucleotide sequence of guanosine compound was bound thereto. In this case, a guanosinephosphonamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% $TCA/CH_2Cl_2$ was conducted for 60 seconds to remove the 5'-DMTr group of the protected guanosine compound. This solution was stored to calculate the condensation yield; and

(4) Condensation reaction was conducted for 5 minutes using the 0.2 M guanosinephosphonamidite unit and 0.4 M tetrazole.

The nucleotide chain was successively extended by repetition of the above-mentioned operations. DMTr released by the operation (1) was colorimetrically determined, and the average yield of each condensation reaction calculated from the results was 99% or more. After condensation of thymidylic acid as the 18th nucleotide, the n-octylglucoside phenylphosphonate compound synthesized in Example 9 was bound to the 5'-terminus. In this case, the n-octylglucoside phenyl-phosphonate compound was similarly dissolved to give a concentration of 0.2 M, and the solution was loaded on the automatic synthesizer, followed by repetition of the above-mentioned operations (1) to (7) with the exception that the operations (1), (4) and (5) were conducted as follows:

(1) Treatment with 3.0% $TCA/CH_2Cl_2$ was conducted for 60 seconds to remove the 5'-DMTr group of the protected thymidine compound;

(4) Condensation reaction was conducted for 5 minutes using the 0.2 M n-octylglucosidephenylphosphoramidite unit synthesized in Example 9 and 0.4 M tetrazole; and

(5) Oxidation reaction was conducted for 30 seconds using 0.1 M iodine/water/pyridine/THF.

By the above-mentioned operations, the n-octylglucoside phenylphosphonate compound could be introduced into the 5'-terminus of the antisense oligonucleotide. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphorothioate antisense oligonucleotide to which n-octylglucoside phenylphosphonate was bound: (n-oct-GlcPh)-$T_sG_sG_sG_sA_sG_sC_sC_sA_sT_sA_sG_sC_sG_sA_sG_sG_sC$ was synthesized.

Yield: 52.2 OD

λmax: 256.3 nm

λmin: 229.3 nm

[Example 11]

Synthesis of 1-O-n-Octyl-2,3,4-O-tribenzoylthioglucoside-6-O-(N,N-diisopropylamino)phenylphosphonamidite

1-O-n-Octyl-2,3,4-O-tribenzoylthioglucoside (0.84 g, 1.35 mmols) was dehydrated by azeotropic distillation with three 2 ml portions of anhydrous pyridine, followed by azeotropic distillation with three 2 ml portions of anhydrous toluene and three 2 ml portions of anhydrous methylene chloride. Then, the resulting product was dissolved in 5 ml of methylene chloride, and diisopropylethylamine (1.17 ml, 6.75 mmols) and chloro-N,N-diisopropylaminophenylphosphonamidite (0.66 ml, 2.69 mmols) were added to this solution, followed by reaction at room temperature for 1 hour. Thereafter, 50 ml of methylene chloride was added for dilution, and the resulting solution was washed with three 50 ml portions of a saturated aqueous solution of sodium hydrogencarbonate, followed by drying with anhydrous sodium sulfate. After removal of the solvent by distillation under reduced pressure, the resulting product was purified by silica gel column chromatography (hexane/methylene chloride = 80:20, 2% triethylamine) to obtain a desired product, 1-O-n-octyl-2,3,4-O-tribenzoylthioglucoside-6-O-(N,N-diisopropylamino)phenylphosphonamidite (0.51 g, 0.62 mmol, 46%).

$^1$H-NMR ($CDCl_3$) δ7.97-7.80(m,11H,o-H of benzoyl, phenyl), 7.79-7.19(m,9H,m-,p-H of benzoyl), 5.91(d,1H,H-1), 5.58-5.46, 4.85-4.79, 4.07-4.00(m,6H,H-2,3,4,5,6), 3.33-3.28(m,2H,CH of isopropyl), 1.57-1.00(m,2OH,$CH_3$ of isopropyl,$CH_2$ of octyl), 0.85(t,3H,$CH_3$ of octyl) ppm

$^{31}$P-NMR ($CDCl_3$) δ119.68, 122.60 ppm

[Example 12]

Synthesis of Phosphorothioate Antisense Oligonucleotide to Which n-Octylthioglucoside Phenylphosphonate Was Bound: (n-oct-SGlcPh)-$T_sG_sG_sG_sA_sG_sC_sC_sA_sT_sA_sG_sC_sG_sA_sG_sG_sC$ (SEQ ID NO: 31)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% $TCA/CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephosphonamidite unit and 0.4 M tetrazole;

(5) A sulfurizing reaction was conducted for 15 minutes using 0.5 M tetraethylthiuram disulfide/acetonitrile;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine compound could be condensed with the cytidine compound introduced into CPG, by a phosphorothioate bond. A subsequent nucleotide sequence of guanosine compound was bound thereto. In this case, a guanosinephosphonamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% $TCA/CH_2Cl_2$ was conducted for 60 seconds to remove the 5'-DMTr group of the protected guanosine compound. This solution was stored to calculate the condensation yield; and

(4) Condensation reaction was conducted for 5 minutes using the 0.2 M guanosinephosphonamidite unit and 0.4 M tetrazole.

The nucleotide chain was successively extended by repetition of the above-mentioned operations. DMTr released by the operation (1) was colorimetrically determined, and the average yield of each condensation reaction calculated from the results was 99% or more. After condensation of thymidylic acid as the 18th nucleotide, the n-octylthioglucoside phenylphosphonate compound synthesized in Example 11 was bound to the 5'-terminus. In this case, the octylglucoside phenylphosphonate compound was similarly dissolved to give a concentration of 0.2 M, and the solution was loaded on the automatic synthesizer, followed by repetition of the above-mentioned operations (1) to (7) with the exception that the operations (1), (4) and (5) were conducted as follows:

(1) Treatment with 3.0% $TCA/CH_2Cl_2$ was conducted for 60 seconds to remove the 5'-DMTr group of the protected thymidine compound;

(4) Condensation reaction was conducted for 5 minutes using the 0.2 M n-octylthioglucosidephenylphosphonamidite unit synthesized in Example 11 and 0.4 M tetrazole; and

(5) An oxidation reaction was conducted for 30 seconds using 0.1 M iodine/water/pyridine/THF.

By the above-mentioned operations, the n-octylthioglucoside phenylphosphonate compound could be introduced into the 5'-terminus of the antisense oligonucleotide. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column,

and reacted with an ammonia/pyridine (5:1) solution at 60°C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphorothioate antisense oligonucleotide to which octylthioglucoside phenylphosphonate was bound: (n-oct-SGlcPh)-$T_sG_sG_sG_sA_sG_sC_sC_sA_sT_sA_sG_sC_sG_sA_sG_sG_sC$ was synthesized.

Yield: 40.3 OD

λmax: 256.8 nm

λmin: 229.6 nm

[Example 13]

Synthesis of 1-O-Phenyl-2,3,4-O-tribenzoylglucoside-6-O-N,N-diisopropylamino-O-cyanoethylphosphoramidite

1-O-Phenyl-2,3,4-O-tribenzoylglucoside (1.14 g, 2.00 mmols) was dehydrated by azeotropic distillation with three 2 ml portions of anhydrous pyridine, followed by azeotropic distillation with three 2 ml portions of anhydrous toluene and three 2 ml portions of anhydrous methylene chloride. Then, the resulting product was dissolved in 5 ml of methylene chloride, and diisopropylethylamine (1.74 ml, 10.00 mmols) and N,N-diisopropylamino-O-cyanoethylphosphorchloride (0.89 ml, 4.00 mmols) were added to this solution, followed by reaction at room temperature for 1 hour. Thereafter, 50 ml of methylene chloride was added for dilution, and the resulting solution was washed with three 50 ml portions of a saturated aqueous solution of sodium hydrogencarbonate, followed by drying with anhydrous sodium sulfate. After removal of the solvent by distillation under reduced pressure, the resulting product was purified by silica gel column chromatography (hexane/methylene chloride = 80:20, 2% triethylamine) to obtain a desired product, 1-O-phenyl-2,3,4-O-tribenzoylglucoside-6-O-N,N-diisopropylamino-O-cyanoethylphosphoramidite (1.31 g, 1.71 mmols, 85%).

$^1$H-NMR ($CDCl_3$) δ7.97-7.80(m,6H,o-H of benzoyl), 7.51-7.25, 7.18-7.06(m,14H,m-,p-H of benzoyl, phenyl), 6.00-5.37(m,4H,H-1,2,3,4),4.18-4.12, 3.92-3.72(3H,H-5,6), 3.61-3.50(m,2H,CH of isopropyl), 2.57-2.42(4H,$CH_2$ of cyanoethyl), 1.16-1.04(12H,$CH_3$ of isopropyl) ppm

$^{31}$P-NMR ($CDCl_3$) δ149.67, 149.19 ppm

[Example 14]

Synthesis of Phosphorothioate Antisense Oligonucleotide to Which Phenylglucoside Was Bound: (PhGlc)-$T_sG_sG_sG_sA_sG_sC_sC_sA_sT_sA_sG_sC_sG_sA_sG_sG_sC$ (SEQ ID NO: 31)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephosphonamidite unit and 0.4 M tetrazole;

(5) A sulfurizing reaction was conducted for 15 minutes using 0.5 M tetraethylthiuram disulfide/acetonitrile;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine compound could be condensed with the cytidine compound introduced into CPG, by a phosphorothioate bond. A subsequent nucleotide sequence of guanosine compound was bound thereto. In this case, a guanosinephosphonamidite unit was similarly dissolved to give a concentration of 0.2 M, and the solution was loaded on the automatic synthesizer, followed by repetition of the above-mentioned operations (1) to (7) with the exception that the

operations (1) and (4) were conducted as follows:

(1) Treatment with 3.0% TCA/$CH_2Cl_2$ was conducted for 60 seconds to remove the 5'-DMTr group of the protected guanosine compound. This solution was stored to calculate the condensation yield; and

(4) Condensation reaction was conducted for 5 minutes using the 0.2 M guanosinephosphonamidite unit and 0.4 M tetrazole.

The nucleotide chain was successively extended by repetition of the above-mentioned operations. DMTr released by the operation (1) was colorimetrically determined, and the average yield of each condensation reaction calculated from the results was 99% or more. After condensation of thymidylic acid as the 18th nucleotide, the phenylglucoside compound synthesized in Example 13 was bound to the 5'-terminus. In this case, the phenylglucoside compound was similarly dissolved to give a concentration of 0.2 M, and the solution was loaded on the automatic synthesizer, followed by repetition of the above-mentioned operations (1) to (7) with the exception that the operations (1), (4) and (5) were conducted as follows:

(1) Treatment with 3.0% TCA/$CH_2Cl_2$ was conducted for 60 seconds to remove the 5'-DMTr group of the protected thymidine compound;

(4) Condensation reaction was conducted for 5 minutes using the 0.2 M phenylglucosidephosphoramidite unit synthesized in Example 13 and 0.4 M tetrazole; and

(5) An oxidation reaction was conducted for 30 seconds using 0.1 M iodine/water/pyridine/THF.

By the above-mentioned operations, the phenylglucoside compound could be introduced into the 5'-terminus of the antisense oligonucleotide. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC ($\mu$Bondasphere $C_{18}$). By the above-mentioned operations, a desired phosphorothioate antisense oligonucleotide to which phenylglucoside was bound: (PhGlc)-$T_sG_sG_sG_sA_sG_sC_sC_sA_sT_sA_sG_sC_sG_sA_sG_sG_sC$ was synthesized.

Yield: 56.6 OD

λmax: 256.7 nm

λmin: 228.9 nm

[Example 15]

Synthesis of 1-O-Phenyl-2,3,4-O-tribenzoylgalactoside-6-O-N,N-diisopropylamino-O-cyanoethylphosphoramidite

1-O-Phenyl-2,3,4-O-tribenzoylgalactoside (1.14 g, 2.00 mmols) was dehydrated by azeotropic distillation with three 2 ml portions of anhydrous pyridine, followed by azeotropic distillation with three 2 ml portions of anhydrous toluene and three 2 ml portions of anhydrous methylene chloride. Then, the resulting product was dissolved in 5 ml of methylene chloride, and diisopropylethylamine (1.74 ml, 10.00 mmols) and N,N-diisopropylamino-O-cyanoethylphosphorchloride (0.89 ml, 4.00 mmols) were added to this solution, followed by reaction at room temperature for 1 hour. Thereafter, 50 ml of methylene chloride was added for dilution, and the resulting solution was washed with three 50 ml portions of a saturated aqueous solution of sodium hydrogencarbonate, followed by drying with anhydrous sodium sulfate. After removal of the solvent by distillation under reduced pressure, the resulting product was purified by silica gel column chromatography (hexane/methylene chloride = 80:20, 2% triethylamine) to obtain a desired product, 1-O-phenyl-2,3,4-O-tribenzoylgalactoside-6-O-N,N-diisopropylamino-O-cyanoethylphosphoramidite (1.37 g, 1.79 mmols, 90%).

$^1$H-NMR ($CDCl_3$) $\delta$8.12-7.80(m,6H,o-H of benzoyl), 7.50-7.05(m,14H,m-,p-H of benzoyl, phenyl), 6.03-5.98, 5.79-5.60, 5.43-5.29(m,4H,H-1,2,3,4), 4.36-4.12(3H,H-5,6), 3.60-3.47(m,2H,CH of isopropyl), 2.75-2.52-(4H,$CH_2$ of cyanoethyl), 1.30-1.06(12H,$CH_3$ of isopropyl) ppm

$^{31}$P-NMR ($CDCl_3$) $\delta$149.98, 149.60 ppm

[Example 16]

Synthesis of Phosphorothioate Antisense Oligonucleotide to Which Phenylgalactoside Was Bound: (PhGal)-$T_sG_sG_sG_sA_sG_sC_sC_sA_sT_sA_sG_sC_sG_sA_sG_sG_sC$ (SEQ ID NO: 31)

50 mg (1 micromol) of CPG into which 20 micromols/g of a cytidine compound was introduced, the N-4 amino group and the 5'-hydroxyl group of said cytidine compound being protected by a benzoyl group and

a DMTr group, respectively, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephosphonamidite unit and 0.4 M tetrazole;

(5) A sulfurizing reaction was conducted for 15 minutes using 0.5 M tetraethylthiuram disulfide/acetonitrile;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine compound could be condensed with the cytidine compound introduced into CPG, by a phosphorothioate bond. A subsequent nucleotide sequence of a guanosine derivative was bound thereto. In this case, a guanosinephosphonamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-DMTr group of the protected guanosine compound. This solution was stored to calculate the condensation yield; and

(4) Condensation reaction was conducted for 5 minutes using the 0.2 M guanosinephosphonamidite unit and 0.4 M tetrazole.

The nucleotide chain was successively extended by repetition of the above-mentioned operations. DMTr released by the operation (1) was colorimetrically determined, and the average yield of each condensation reaction calculated from the results was 99% or more. After condensation of thymidylic acid as the 18th nucleotide, the phenylgalactoside compound synthesized in Example 15 was bound to the 5'-terminus. In this case, the phenylgalactoside compound was similarly dissolved to give a concentration of 0.2 M, and the solution was loaded on the automatic synthesizer, followed by repetition of the above-mentioned operations (1) to (7) with the exception that the operations (1), (4) and (5) were conducted as follows:

(1) Treatment with 3.0% TCA/$CH_2Cl_2$ was conducted for 60 seconds to remove the 5'-DMTr group of the protected thymidine compound;

(4) Condensation reaction was conducted for 5 minutes using the 0.2 M n-phenylgalactosidephosphoramidite unit synthesized in Example 15 and 0.4 M tetrazole; and

(5) An oxidation reaction was conducted for 30 seconds using 0.1 M iodine/water/pyridine/THF.

By the above-mentioned operations, the phenylgalactoside compound could be introduced into the 5'-terminus of the antisense oligonucleotide. Deprotecting operations after completion of synthesis reaction were conducted according to methods known in the art [Oligonucleotide Synthesis: A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphorothioate antisense oligonucleotide to which phenylgalactoside was bound: (PhGal)-$T_sG_sG_sG_sA_sG_sC_sC_sA_sT_sA_sG_sC_sG_sA_sG_sG_sC$ was synthesized.

Yield: 61.5 OD

λmax: 257.1 nm

λmin: 229.4 nm

[Example 17]

Synthesis of 1,3,4-O-Tribenzoyl,2-N-benzoylgalactosamine-6-O-N,N-diisopropylamino-O-cyanoethyl-phosphoramidite

1,3,4-O-Tribenzoyl,2-N-benzoylgalactosamine (1.05 g, 1.76 mmols) was dehydrated by azeotropic distillation with three 2 ml portions of anhydrous pyridine, followed by azeotropic distillation with three 2 ml portions of anhydrous toluene and three 2 ml portions of anhydrous methylene chloride. Then, the resulting product was dissolved in 5 ml of methylene chloride, and diisopropylethylamine (1.53 ml, 8.79 mmols) and N,N-diisopropylaminoO-cyanoethylphosphorchloride (0.78 ml, 3.52 mmols) were added to this solution, followed by reaction at room temperature for 1 hour. Thereafter, 50 ml of methylene chloride was added for dilution, and the resulting solution was washed with three 50 ml portions of a saturated aqueous solution of sodium hydrogencarbonate, followed by drying with anhydrous sodium sulfate. After removal of the solvent by distillation under reduced pressure, the resulting product was purified by silica gel column chromatography (hexane/methylene chloride = 80:20, 2% triethylamine) to obtain a desired product, 1,3,4-O-tribenzoyl,2-N-benzoylgalactosamine-6-O-N,N-diisopropyl amino-O-cyanoethylphosphoramidite (0.97 g, 1.22 mmols, 70%).

$^{1}$H-NMR ($CDCl_3$) $\delta$8.50(s,1H,-NH), 8.22-7.95(m,8H,o-H of benzoyl), 7.82-7.35(m,12H,m-,p-H of benzoyl), 6.61-6.56, 6.32-6.18(4H,H-1,2,3,4), 4.32-3.91, 3.87-3.64(m,3H,H-5,6), 3.45-3.22(m,2H,CH of isopropyl), 2.73-2.45(m,4H,$CH_2$ of cyanoethyl), 1.39-1.02(m,12H,$CH_3$ of isopropyl) ppm

$^{31}$P-NMR ($CDCl_3$) $\delta$149.84, 150.09, 150.43 ppm

[Example 18]

Synthesis of Phosphorothioate Antisense Oligonucleotide to Which N-Benzoylgalactosamine Phosphate Was Bound: (N-BzGalPO)-$T_sG_sG_sG_sA_sG_sC_sC_sA_sT_sA_sG_sC_sG_sA_sG_sG_sC$ (SEQ ID NO: 31)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephosphonamidite unit and 0.4 M tetrazole;

(5) A sulfurizing reaction was conducted for 15 minutes using 0.5 M tetraethylthiuram disulfide/acetonitrile;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine compound could be condensed with the cytidine compound introduced into CPG, by a phosphorothioate bond. A subsequent nucleotide sequence of guanosine compound was bound thereto. In this case, a guanosinephosphonamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-DMTr group of the protected guanosine compound. This solution was stored to calculate the condensation yield; and

(4) Condensation reaction was conducted for 5 minutes using the 0.2 M guanosinephosphonamidite unit and 0.4 M tetrazole.

The nucleotide chain was successively extended by repetition of the above-mentioned operations. DMTr released by the operation (1) was colorimetrically determined, and the average yield of each condensation reaction calculated from the results was 99% or more. After condensation of thymidylic acid

as the 18th nucleotide, the N-benzoylgalactosamine compound synthesized in Example 17 was bound to the 5'-terminus. In this case, the N-benzoylgalactosamine compound was similarly dissolved to give a concentration of 0.2 M, and the solution was loaded on the automatic synthesizer, followed by repetition of the above-mentioned operations (1) to (7) with the exception that the operations (1), (4) and (5) were conducted as follows:

(1) Treatment with 3.0% TCA/$CH_2Cl_2$ was conducted for 60 seconds to remove the 5'-DMTr group of the protected thymidine compound;

(4) Condensation reaction was conducted for 5 minutes using the 0.2 M N-benzoylgalactosamineamidite unit synthesized in Example 17 and 0.4 M tetrazole; and

(5) An oxidation reaction was conducted for 30 seconds using 0.1 M iodine/water/pyridine/THF.

By the above-mentioned operations, the N-benzoylgalactosamine compound could be introduced into the 5'-terminus of the antisense oligonucleotide. Deprotecting operations after completion of synthesis reaction were conducted according to methods known in the art [Oligonucleotide Synthesis: A Practical Approach, edited by M. J. Gait, IRL PRESS, (1984)]. CPG was taken out of the reaction column, and reacted with an ammonia/pyridine (5:1) solution at 60°C for 6 hours, thereby excising the nucleotide chain from the solid phase carrier and removing the protective group. Then, the product was purified by the use of reverse phase HPLC ($\mu$Bondasphere $C_{18}$). By the above-mentioned operations, a desired phosphorothioate antisense oligonucleotide to which N-benzoylgalactosamine phosphate was bound: (N-BzGalPO)-$T_sG_sG_sG_sA_sG_sC_sC_sA_sT_sA_sG_sC_sG_sA_sG_sG_sC$was synthesized.

Yield: 51.6 OD

$\lambda$max: 256.2 nm

$\lambda$min: 228.9 nm

[Example 19]

Synthesis of 1,3,4-O-Triacetyl,2-N-acetylgalactosamine-6-O-N,N-diisopropylamino-O-cyanoethyl-phosphoramidite

1,3,4-O-Triacetyl,2-N-acetylgalactosamine (0.38 g, 1.09 mmols) was dehydrated by azeotropic distillation with three 2 ml portions of anhydrous pyridine, followed by azeotropic distillation with three 2 ml portions of anhydrous toluene and three 2 ml portions of anhydrous methylene chloride. Then, the resulting product was dissolved in 5 ml of methylene chloride, and diisopropylethylamine (0.87 ml, 5.00 mmols) and N,N-diisopropylamino-O-cyanoethylphosphorchloride (0.45 ml, 2.00 mmols) were added to this solution, followed by reaction at room temperature for 1 hour. Thereafter, 50 ml of methylene chloride was added for dilution, and the resulting solution was washed with three 50 ml portions of a saturated aqueous solution of sodium hydrogencarbonate, followed by drying with anhydrous sodium sulfate. After removal of the solvent by distillation under reduced pressure, the resulting product was purified by silica gel column chromatography (hexane/methylene chloride = 80:20, 2% triethylamine) to obtain a desired product, 1,3,4-O-triacetyl,2-N-acetylgalactosamine-6-O-N,N-diisopropylamino-O-cyanoethylphosphoramidite (0.40 g, 0.75 mmol, 69%).

$^1$H-NMR ($CDCl_3$) $\delta$8.50(s,1H,-NH), 6.20, 5.70-5.56, 5.32(m,4H,H-1,2,3,4), 4.22-4.10(m,3H,H-5,6), 3.90-3.77, 3.61-3.47(m,12H,$CH_3$ of acetyl), 2.80-2.50(m,4H,$CH_2$ of cyanoethyl), 1.30-1.11(m,12H,$CH_3$ of isopropyl) ppm

$^{31}$P-NMR ($CDCl_3$) $\delta$149.50, 149.57, 150.81 ppm

[Example 20]

Synthesis of Phosphorothioate Antisense Oligonucleotide to Which N-Acetylgalactosamine Phosphate Was Bound: (N-AcGal)-$T_sG_sG_sG_sA_sG_sC_sC_sA_sT_sA_sG_sC_sG_sA_sG_sG_sC$ (SEQ ID NO: 31)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephosphonamidite unit and 0.4 M tetrazole;

(5) A sulfurizing reaction was conducted for 15 minutes using 0.5 M tetraethylthiuram disulfide/acetonitrile;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine compound could be condensed with the cytidine compound introduced into CPG, by a phosphorothioate bond. A subsequent nucleotide sequence of guanosine compound was bound thereto. In this case, a guanosinephosphonamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% $TCA/CH_2Cl_2$ was conducted for 60 seconds to remove the 5'-DMTr group of the protected guanosine compound. This solution was stored to calculate the condensation yield; and

(4) Condensation reaction was conducted for 5 minutes using the 0.2 M guanosinephosphonamidite unit and 0.4 M tetrazole.

The nucleotide chain was successively extended by repetition of the above-mentioned operations. DMTr released by the operation (1) was colorimetrically determined, and the average yield of each condensation reaction calculated from the results was 99% or more. After condensation of thymidylic acid as the 18th nucleotide, the N-acetylgalactosamine compound synthesized in Example 19 was bound to the 5'-terminus. In this case, the N-acetylgalactosamine compound was similarly dissolved to give a concentration of 0.2 M, and the solution was loaded on the automatic synthesizer, followed by repetition of the above-mentioned operations (1) to (7) with the exception that the operations (1), (4) and (5) were conducted as follows:

(1) Treatment with 3.0% $TCA/CH_2Cl_2$ was conducted for 60 seconds to remove the 5'-DMTr group of the protected thymidine compound;

(4) Condensation reaction was conducted for 5 minutes using the 0.2 M N-acetylgalactosamineamidite unit synthesized in Example 19 and 0.4 M tetrazole; and

(5) An oxidation reaction was conducted for 30 seconds using 0.1 M iodine/water/pyridine/THF.

By the above-mentioned operations, the N-acetylgalactosamine compound could be introduced into the 5'-terminus of the antisense oligonucleotide. Deprotecting operations after completion of synthesis reaction were conducted according to methods known in the art [Oligonucleotide Synthesis: A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphorothioate antisense oligonucleotide to which N-acetylgalactosamine phosphate was bound: (N-AcGal)-$T_sG_sG_sG_sA_sG_sC_sC_sA_sT_sA_sG_sC_sG_sA_sG_sG_sC$was synthesized.

Yield: 9.1 OD

λmax: 257.4 nm

λmin: 229.6 nm

[Example 21]

Synthesis of 1,3,4-O-Triacetyl,2-N-acetylglucosamine-6-O-N,N-diisopropylamino-O-cyanoethylphosphoramidite

1,3,4-O-Triacetyl,2-N-acetylglucosamine (0.82 g, 1.39 mmols) was dehydrated by azeotropic distillation with three 2 ml portions of anhydrous pyridine, followed by azeotropic distillation with three 2 ml portions of anhydrous toluene and three 2 ml portions of anhydrous methylene chloride. Then, the resulting product was dissolved in 5 ml of methylene chloride, and diisopropylethylamine (1.21 ml, 6.95 mmols) and N,N-diisopropylamino-O-cyanoethylphosphorchloride (0.62 ml, 2.78 mmols) were added to this solution, followed by reaction at room temperature for 1 hour. Thereafter, 50 ml of methylene chloride was added for dilution, and the resulting solution was washed with three 50 ml portions of a saturated aqueous solution of sodium hydrogencarbonate, followed by drying with anhydrous sodium sulfate. After removal of the solvent by

distillation under reduced pressure, the resulting product was purified by silica gel column chromatography (hexane/methylene chloride = 80:20, 2% triethylamine) to obtain a desired product, 1,3,4-O-triacetyl,2-N-acetylglucosamine-6-O-N,N-diisopropylamino-O-cyanoethylphosphoramidite (0.47 g, 0.59 mmol, 42%).
$^{1}$H-NMR ($CDCl_3$) $\delta$8.38(s,1H,-NH), 6.15-5.99, 5.80-5.52(m,4H,H-1,2,3,4), 4.28-4.08(m,3H,H-5,6), 3.96-3.57-(m,12H,$CH_3$ of acetyl), 2.78-2.53(m,4H,$CH_2$ of cyanoethyl), 1.41-1.08(m,12H,$CH_3$ of isopropyl) ppm
$^{31}$P-NMR ($CDCl_3$) $\delta$149.76, 150.72 ppm

[Example 22]

Synthesis of Phosphorothioate Antisense Oligonucleotide to Which N-Acetylglucosamine Phosphate Was Bound: (N-AcGul)-$T_sG_sG_sG_sA_sG_sC_sC_sA_sT_sA_sG_sC_sG_sA_sG_sG_sC$ (SEQ ID NO: 31)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephosphonamidite unit and 0.4 M tetrazole;

(5) A sulfurizing reaction was conducted for 15 minutes using 0.5 M tetraethylthiuram disulfide/acetonitrile;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine compound could be condensed with the cytidine compound introduced into CPG, by a phosphorothioate bond. A subsequent nucleotide sequence of guanosine compound was bound thereto. In this case, a guanosinephosphonamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-DMTr group of the protected guanosine compound. This solution was stored to calculate the condensation yield; and

(4) Condensation reaction was conducted for 5 minutes using the 0.2 M guanosinephosphonamidite unit and 0.4 M tetrazole.

The nucleotide chain was successively extended by repetition of the above-mentioned operations. DMTr released by the operation (1) was colorimetrically determined, and the average yield of each condensation reaction calculated from the results was 99% or more. After condensation of thymidylic acid as the 18th nucleotide, the N-acetylglucosamine compound synthesized in Example 21 was bound to the 5'-terminus. In this case, the N-acetylglucosamine compound was similarly dissolved to give a concentration of 0.2 M, and the solution was loaded on the automatic synthesizer, followed by repetition of the above-mentioned operations (1) to (7) with the exception that the operations (1), (4) and (5) were conducted as follows:

(1) Treatment with 3.0% TCA/$CH_2Cl_2$ was conducted for 60 seconds to remove the 5'-DMTr group of the protected thymidine compound;

(4) Condensation reaction was conducted for 5 minutes using the 0.2 M N-acetylglucosamineamidite unit synthesized in Example 21 and 0.4 M tetrazole; and

(5) An oxidation reaction was conducted for 30 seconds using 0.1 M iodine/water/pyridine/THF.

By the above-mentioned operations, the N-acetylglucosamine compound could be introduced into the 5'-terminus of the antisense oligonucleotide. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid

phase carrier and removing the protective group. Then, the product was purified by the use of reverse phase HPLC (μBondasphere $C_{18}$). By the above-mentioned operations, a desired phosphorothioate antisense oligonucleotide to which N-acetylglucosamine phosphate was bound: (N-AcGul)-$T_sG_sG_sG_sA_sG_sC_sC_sA_sT_sA_sG_sC_sG_sA_sG_sG_sC$ was synthesized.

Yield: 42.5 OD

λmax: 257.3 nm

λmin: 229.6 nm

[Example 23]

Synthesis of 1,3,4-O-Tribenzoyl,2-N-trifluoroacetylgalactosamine-6-O-N,N-diisopropylamino-O-cyanoethylphosphoramidite

1,3,4-O-Tribenzoyl,2-N-trifluoroacetylgalactosamine (0.58 g, 0.99 mmol) was dehydrated by azeotropic distillation with three 2 ml portions of anhydrous pyridine, followed by azeotropic distillation with three 2 ml portions of anhydrous toluene and three 2 ml portions of anhydrous methylene chloride. Then, the resulting product was dissolved in 5 ml of methylene chloride, and diisopropylethylamine (0.87 ml, 5.00 mmols) and N,N-dissopropylamino-O-cyanoethylphosphorchloride (0.45 ml, 2.00 mmols) were added to this solution, followed by reaction at room temperature for 1 hour. Thereafter, 50 ml of methylene chloride was added for dilution, and the resulting solution was washed with three 50 ml portions of a saturated aqueous solution of sodium hydrogencarbonate, followed by drying with anhydrous sodium sulfate. After removal of the solvent by distillation under reduced pressure, the resulting product was purified by silica gel column chromatography (hexane/methylene chloride = 80:20, 2% triethylamine) to obtain a desired product, 1,3,4-O-tribenzoyl,2-N-trifluoroacetylgalactosamine-6-O-N,N-diisopropylamino-O-cyanoethylphosphoramidite (0.42 g, 0.53 mmol, 53%).

$^1$H-NMR ($CDCl_3$) δ8.70(s,1H,-NH), 8.18-7.79(m,6H,o-H of benzoyl), 7.66-7.21(m,m-,p-H of benzoyl), 6.66-6.15(m,4H,H-1,2,3,4),4.04-3.70(m,3H,H-5,6), 3.61-3.42(m,2H,CH of cyanoethyl), 2.68-2.41(m,4H,$CH_2$ of cyanoethyl), 1.31-0.92(m,12H,$CH_3$ of cyanoethyl) ppm

$^{31}$P-NMR ($CDCl_3$) δ148.96, 149.38, 150.10 ppm

[Example 24]

Synthesis of Phosphorothioate Antisense Oligonucleotide to Which Galactosamine Phosphate Was Bound: (GalN)-$T_sG_sG_sG_sA_sG_sC_sC_sA_sT_sA_sG_sC_sG_sA_sG_sG_sC$ (SEQ ID NO: 31)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephosphonamidite unit and 0.4 M tetrazole;

(5) A sulfurizing reaction was conducted for 15 minutes using 0.5 M tetraethylthiuram disulfide/acetonitrile;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine compound could be condensed with the cytidine compound introduced into CPG, by a phosphorothioate bond. A subsequent nucleotide sequence of guanosine compound was bound thereto. In this case, a guanosinephosphonamidite unit was similarly dissolved to give a concentration of 0.2 M, and the solution was loaded on the automatic synthesizer, followed by repetition of the above-mentioned operations (1) to (7) with the exception that the

operations (1) and (4) were conducted as follows:

(1) Treatment with 3.0% TCA/$CH_2Cl_2$ was conducted for 60 seconds to remove the 5'-DMTr group of the protected guanosine compound. This solution was stored to calculate the condensation yield; and

(4) Condensation reaction was conducted for 5 minutes using the 0.2 M guanosinephosphonamidite unit and 0.4 M tetrazole.

The nucleotide chain was successively extended by repetition of the above-mentioned operations. DMTr released by the operation (1) was colorimetrically determined, and the average yield of each condensation reaction calculated from the results was 99% or more. After condensation of thymidylic acid as the 18th nucleotide, the galactosamine compound synthesized in Example 23 was bound to the 5'-terminus. In this case, the galactosamine compound was similarly dissolved to give a concentration of 0.2 M, and the solution was loaded on the automatic synthesizer, followed by repetition of the above-mentioned operations (1) to (7) with the exception that the operations (1), (4) and (5) were conducted as follows:

(1) Treatment with 3.0% TCA/$CH_2Cl_2$ was conducted for 60 seconds to remove the 5'-DMTr group of the protected thymidine compound;

(4) Condensation reaction was conducted for 5 minutes using the 0.2 M galactosamineamidite unit synthesized in Example 23 and 0.4 M tetrazole; and

(5) An oxidation reaction was conducted for 30 seconds using 0.1 M iodine/water/pyridine/THF.

By the above-mentioned operations, the galactosamine compound could be introduced into the 5'-terminus of the antisense oligonucleotide. Deprotecting operations after completion of synthesis reaction were conducted according to methods known in the art [Oligonucleotide Synthesis: A Practical Approach, edited by M. J. Gait, IRL PRESS, (1984)]. CPG was taken out of the reaction column, and reacted with an ammonia/pyridine (5:1) solution at 60°C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphorothioate antisense oligonucleotide to which galactosamine phosphate was bound: (GalN)-$T_sG_sG_sG_sA_sG_sC_sC_sA_sT_sA_sG_sC_sG_sA_sG_sG_sC$ was synthesized.

Yield: 38.7 OD

λmax: 256.4 nm

λmin: 229.3 nm

[Example 25]

Synthesis of 1,3,4-O-Tribenzoyl,2-N-trifluoroacetylglucosamine-6-O-N,N-diisopropylamino-O-cyanoethylphosphoramidite

1,3,4-O-Tribenzoyl,2-N-trifluoroacetylglucosamine (0.82 g, 1.39 mmols) was dehydrated by azeotropic distillation with three 2 ml portions of anhydrous pyridine, followed by azeotropic distillation with three 2 ml portions of anhydrous toluene and three 2 ml portions of anhydrous methylene chloride. Then, the resulting product was dissolved in 5 ml of methylene chloride, and diisopropylethylamine (1.21 ml, 6.95 mmols) and N,N-diisopropylamino-O-cyanoethylphosphorchloride (0.62 ml, 2.78 mmols) were added to this solution, followed by reaction at room temperature for 1 hour. Thereafter, 50 ml of methylene chloride was added for dilution, and the resulting solution was washed with three 50 ml portions of a saturated aqueous solution of sodium hydrogencarbonate, followed by drying with anhydrous sodium sulfate. After removal of the solvent by distillation under reduced pressure, the resulting product was purified by silica gel column chromatography (hexane/methylene chloride = 80:20, 2% triethylamine) to obtain a desired product, 1,3,4-O-tribenzoyl,2-N-trifluoroacetylglucosamine-6-O-N,N-diisopropylamino-O-cyanoethylphosphoramidite (0.47 g, 0.59 mmol, 42%).

$^1$H-NMR ($CDCl_3$) δ8.74(s,1H,-NH), 8.19-7.81(m,6H,o-H of benzoyl), 7.62-7.26(m,m-,p-H of benzoyl), 6.63-6.32(m,4H,H-1,2,3,4),4.22-3.84(m,3H,H-5,6), 3.58-3.42(m,2H,CH of cyanoethyl), 2.70-2.56(m,4H,$CH_2$ of cyanoethyl), 1.26-1.07(m,12H,$CH_3$ of cyanoethyl) ppm

$^{31}$P-NMR ($CDCl_3$) δ150.33, 150.77, 151.39 ppm

[Example 26]

Synthesis of Phosphorothioate Antisense Oligonucleotide to Which Glucosamine Phosphate Was Bound: (GulN)-$T_sG_sG_sG_sA_sG_sC_sC_sA_sT_sA_sG_sC_sG_sA_sG_sG_sC$ (SEQ ID NO: 31)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephosphonamidite unit and 0.4 M tetrazole;

(5) A sulfurizing reaction was conducted for 15 minutes using 0.5 M tetraethylthiuram disulfide/acetonitrile;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine compound could be condensed with the cytidine compound introduced into CPG, by a phosphorothioate bond. A subsequent nucleotide sequence of guanosine compound was bound thereto. In this case, a guanosinephosphonamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-DMTr group of the protected guanosine compound. This solution was stored to calculate the condensation yield; and

(4) Condensation reaction was conducted for 5 minutes using the 0.2 M guanosinephosphonamidite unit and 0.4 M tetrazole.

The nucleotide chain was successively extended by repetition of the above-mentioned operations. DMTr released by the operation (1) was colorimetrically determined, and the average yield of each condensation reaction calculated from the results was 99% or more. After condensation of thymidylic acid as the 18th nucleotide, the glucosamine compound synthesized in Example 25 was bound to the 5'-terminus. In this case, the glucosamine compound was similarly dissolved to give a concentration of 0.2 M, and the solution was loaded on the automatic synthesizer, followed by repetition of the above-mentioned operations (1) to (7) with the exception that the operations (1), (4) and (5) were conducted as follows:

(1) Treatment with 3.0% TCA/$CH_2Cl_2$ was conducted for 60 seconds to remove the 5'-DMTr group of the protected thymidine compound;

(4) Condensation reaction was conducted for 5 minutes using the 0.2 M glucosamineamidite unit synthesized in Example 25 and 0.4 M tetrazole; and

(5) An oxidation reaction was conducted for 30 seconds using 0.1 M iodine/water/pyridine/THF.

By the above-mentioned operations, the glucosamine compound could be introduced into the 5'-terminus of the antisense oligonucleotide. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60°C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphorothioate antisense oligonucleotide to which glucosamine phosphate was bound: (GulN)-$T_sG_sG_sG_sA_sG_sC_sC_sA_sT_sA_sG_sC_sG_sA_sG_sG_sC$ was synthesized.

Yield: 33.4 OD

λmax: 257.3 nm

λmin: 229.4 nm

[Example 27]

Synthesis of Heptabenzoylmelibiose-N,N-diisopropylamino-O-cyanoethylphosphoramidite

Heptabenzoylmelibiose (1.07 g, 1.00 mmol) was dehydrated by azeotropic distillation with three 2 ml portions of anhydrous pyridine, followed by azeotropic distillation with three 2 ml portions of anhydrous toluene and three 2 ml portions of anhydrous methylene chloride. Then, the resulting product was dissolved in 5 ml of methylene chloride, and diisopropylethylamine (0.67 ml, 5.00 mmols) and N,N-diisopropylamino-O-cyanoethylphosphorchloride (0.45 ml, 2.00 mmols) were added to this solution, followed by reaction at room temperature for 1 hour. Thereafter, 50 ml of methylene chloride was added for dilution, and the resulting solution was washed with three 50 ml portions of a saturated aqueous solution of sodium hydrogencarbonate, followed by drying with anhydrous sodium sulfate. After removal of the solvent by distillation under reduced pressure, the resulting product was purified by silica gel column chromatography (hexane/methylene chloride = 80:20, 2% triethylamine) to obtain a desired product, heptabenzoylmelibiose-N,N-diisopropylamino-O-cyanoethylphosphoramidite (1.91 g, 1.50 mmols, 75%).
$^{1}$H-NMR ($CDCl_3$) $\delta$8.13-8.09, 8.03-7.87, 7.84-7.80(m,14H,o-H of benzoyl), 7.56-7.24(m,21H,m-,p-H of benzoyl), 5.99-5.80, 5.58-5.41(m,8H,H-1,2,3,4,1',2',3',4'), 4.61-4.40, 3.83-3.41(m,6H,H-5,6), 2.58-2.40(m,4H,$CH_2$ of cyanoethyl), 1.12-0.96(m,12H,$CH_3$ of isopropyl) ppm
$^{31}$P-NMR ($CDCl_3$) $\delta$149.45, 149.72 ppm

[Example 28]

Synthesis of Phosphorothioate Antisense Oligonucleotide to Which Melibiose Phosphate Was Bound: (Mel)-$T_sG_sG_sG_sA_sG_sC_sC_sA_sT_sA_sG_sC_sG_sA_sG_sG_sC$ (SEC ID NO: 31)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M guanosinephosphonamidite unit and 0.4 M tetrazole;
(5) A sulfurizing reaction was conducted for 15 minutes using 0.5 M tetraethylthiuram disulfide/acetonitrile;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected guanosine compound could be condensed with the cytidine compound introduced into CPG, by a phosphorothioate bond. A subsequent nucleotide sequence of guanosine compound was bound thereto. In this case, a guanosinephosphonamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-DMTr group of the protected guanosine compound. This solution was stored to calculate the condensation yield; and
(4) Condensation reaction was conducted for 5 minutes using the 0.2 M guanosinephosphonamidite unit and 0.4 M tetrazole.

The nucleotide chain was successively extended by repetition of the above-mentioned operations. DMTr released by the operation (1) was colorimetrically determined, and the average yield of each condensation reaction calculated from the results was 99% or more. After condensation of thymidylic acid as the 18th nucleotide, the melibiose compound synthesized in Example 27 was bound to the 5'-terminus. In this case, the melibiose compound was similarly dissolved to give a concentration of 0.2 M, and the

solution was loaded on the automatic synthesizer, followed by repetition of the above-mentioned operations (1) to (7) with the exception that the operations (1), (4) and (5) were conducted as follows:

(1) Treatment with 3.0% TCA/$CH_2Cl_2$ was conducted for 60 seconds to remove the 5'-DMTr group of the protected thymidine compound;

(4) Condensation reaction was conducted for 5 minutes using the 0.2 M melibioseamidite unit synthesized in Example 27 and 0.4 M tetrazole; and

(5) An oxidation reaction was conducted for 30 seconds using 0.1 M iodine/water/pyridine/THF.

By the above-mentioned operations, the melibiose compound could be introduced into the 5'-terminus of the antisense oligonucleotide. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphorothioate antisense oligonucleotide to which melibiose phosphate was bound: (Mel)-$T_sG_sG_sG_sA_sG_sC_sC_sA_sT_sA_sG_sC_sG_sA_sG_sG_sC$ was synthesized.

Yield: 78.0 OD

λmax: 256.6 nm

λmin: 229.2 nm

[Example 29]

Synthesis of Heptabenzoylgentiobiose-N,N-diisopropylamino-O-cyanoethylphosphoramidite

Heptabenzoylgentiobiose (1.24 g, 1.16 mmols) was dehydrated by azeotropic distillation with three 2 ml portions of anhydrous pyridine, followed by azeotropic distillation with three 2 ml portions of anhydrous toluene and three 2 ml portions of anhydrous methylene chloride. Then, the resulting product was dissolved in 5 ml of methylene chloride, and diisopropylethylamine (1.01 ml, 5.80 mmols) and N,N-diisopropylamino-O-cyanoethylphosphorchloride (0.50 ml, 2.32 mmols) were added to this solution, followed by reaction at room temperature for 1 hour. Thereafter, 50 ml of methylene chloride was added for dilution, and the resulting solution was washed with three 50 ml portions of a saturated aqueous solution of sodium hydrogencarbonate, followed by drying with anhydrous sodium sulfate. After removal of the solvent by distillation under reduced pressure, the resulting product was purified by silica gel column chromatography (hexane/methylene chloride = 80:20, 2% triethylamine) to obtain a desired product, heptabenzoylgentiobiose-N,N-diisopropylamino-O-cyanoethylphosphoramidite (1.36 g, 1.97 mmols, 93%).

$^1$H-NMR ($CDCl_3$) δ8.10-7.92, 7.86-7.71(m,14H,o-H of benzoyl), 7.52-7.19(m,21H,m-,p-H of benzoyl), 6.01-5.83, 5.57-5.45(m,8H,H-1,2,3,4,1',2',3',4'), 4.63-4.46, 3.81-3.43(m,6H,H-5,6), 2.55-2.43(m,4H,$CH_2$ of cyanoethyl), 1.15-0.94(m,12H,$CH_3$ of isopropyl) ppm

$^{31}$P-NMR ($CDCl_3$) δ150.12, 150.43 ppm

[Example 30]

Synthesis of Phosphorothioate Antisense Oligonucleotide to Which Gentiobiose Phosphate Was Bound: (Gen)-$T_sG_sG_sG_sA_sG_sC_sC_sA_sT_sA_sG_sC_sG_sA_sG_sG_sC$ (SEQ ID NO: 31)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephosphonamidite unit and 0.4 M tetrazole;

(5) A sulfurizing reaction was conducted for 15 minutes using 0.5 M tetraethylthiuram disulfide/acetonitrile;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine compound could be condensed with the cytidine compound introduced into CPG, by a phosphorothioate bond. A subsequent nucleotide sequence of guanosine compound was bound thereto. In this case, a guanosinephosphonamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-DMTr group of the protected guanosine compound. This solution was stored to calculate the condensation yield; and

(4) Condensation reaction was conducted for 5 minutes using the 0.2 M guanosinephosphonamidite unit and 0.4 M tetrazole.

The nucleotide chain was successively extended by repetition of the above-mentioned operations. DMTr released by the operation (1) was colorimetrically determined, and the average yield of each condensation reaction calculated from the results was 99% or more. After condensation of thymidylic acid as the 18th nucleotide, the gentiobiose compound synthesized in Example 29 was bound to the 5'-terminus. In this case, the gentiobiose compound was similarly dissolved to give a concentration of 0.2 M, and the solution was loaded on the automatic synthesizer, followed by repetition of the above-mentioned operations (1) to (7) with the exception that the operations (1), (4) and (5) were conducted as follows:

(1) Treatment with 3.0% TCA/$CH_2Cl_2$ was conducted for 60 seconds to remove the 5'-DMTr group of the protected thymidine compound;

(4) Condensation reaction was conducted for 5 minutes using the 0.2 M gentiobioseamidite unit synthesized in Example 29 and 0.4 M tetrazole; and

(5) An oxidation reaction was conducted for 30 seconds using 0.1 M iodine/water/pyridine/THF.

By the above-mentioned operations, the gentiobiose compound could be introduced into the 5'-terminus of the antisense oligonucleotide. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60°C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphorothioate antisense oligonucleotide to which gentiobiose phosphate was bound: (Gen)-$T_sG_sG_sG_sA_sG_sC_sC_sA_sT_sA_sG_sC_sG_sA_sG_sG_sC$ was synthesized.

Yield: 37.9 OD

λmax: 256.6 nm

λmin: 229.0 nm

[Example 31]

Synthesis of Octabenzoylisomaltotriose-N,N-diisopropylamino-O-cyanoethylphosphoramidite

Octabenzoylisomaltotriose (1.56 g, 1.00 mmol) was dehydrated by azeotropic distillation with three 2 ml portions of anhydrous pyridine, followed by azeotropic distillation with three 2 ml portions of anhydrous toluene and three 2 ml portions of anhydrous methylene chloride. Then, the resulting product was dissolved in 5 ml of methylene chloride, and diisopropylethylamine (0.87 ml, 5.00 mmols) and N,N-diisopropylamino-O-cyanoethylphosphorchloride (0.45 ml, 2.00 mmols) were added to this solution, followed by reaction at room temperature for 1 hour. Thereafter, 50 ml of methylene chloride was added for dilution, and the resulting solution was washed with three 50 ml portions of a saturated aqueous solution of sodium hydrogencarbonate, followed by drying with anhydrous sodium sulfate. After removal of the solvent by distillation under reduced pressure, the resulting product was purified by silica gel column chromatography (hexane/methylene chloride = 80:20, 2% triethylamine) to obtain a desired product, octabenzoylisomaltotriose-N,N-diisopropylamino-O-cyanoethylphosphoramidite (1.21 g, 0.69 mmol, 69%).

$^{31}$P-NMR ($CDCl_3$) δ150.92, 151.23 ppm

[Example 32]

Synthesis of Phosphorothioate Antisense Oligonucleotide to Which Isomaltotriose Phosphate Was Bound: (Iso)-$T_sG_sG_sG_sA_sG_sC_sC_sA_sT_sA_sG_sC_sG_sA_sG_sG_sC$ (SEQ ID NO: 31)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M guanosinephosphonamidite unit and 0.4 M tetrazole;
(5) A sulfurizing reaction was conducted for 15 minutes using 0.5 M tetraethylthiuram disulfide/acetonitrile;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected guanosine compound could be condensed with the cytidine compound introduced into CPG, by a phosphorothioate bond. A subsequent nucleotide sequence of guanosine compound was bound thereto. In this case, a guanosinephosphonamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-DMTr group of the protected guanosine compound. This solution was stored to calculate the condensation yield; and
(4) Condensation reaction was conducted for 5 minutes using the 0.2 M guanosinephosphonamidite unit and 0.4 M tetrazole.

The nucleotide chain was successively extended by repetition of the above-mentioned operations. DMTr released by the operation (1) was colorimetrically determined, and the average yield of each condensation reaction calculated from the results was 99% or more. After condensation of thymidylic acid as the 18th nucleotide, the isomaltotriose compound synthesized in Example 31 was bound to the 5'-terminus. In this case, the isomaltotriose compound was similarly dissolved to give a concentration of 0.2 M, and the solution was loaded on the automatic synthesizer, followed by repetition of the above-mentioned operations (1) to (7) with the exception that the operations (1), (4) and (5) were conducted as follows:

(1) Treatment with 3.0% TCA/$CH_2Cl_2$ was conducted for 60 seconds to remove the 5'-DMTr group of the protected thymidine compound;
(4) Condensation reaction was conducted for 5 minutes using the 0.2 M isomaltotrioseamidite unit synthesized in Example 31 and 0.4 M tetrazole; and
(5) An oxidation reaction was conducted for 30 seconds using 0.1 M iodine/water/pyridine/THF.

By the above-mentioned operations, the isomaltotriose compound could be introduced into the 5'-terminus of the antisense oligonucleotide. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphorothioate antisense oligonucleotide to which isomaltotriose phosphate was bound: (Iso)-$T_sG_sG_sG_sA_sG_sC_sC_sA_sT_sA_sG_sC_sG_sA_sG_sG_sC$ was synthesized.

Yield: 51.8 OD
λmax: 256.4 nm
λmin: 228.9 nm

[Example 33]

Synthesis of Phosphate Antisense Oligonucleotide into Which Phenylphosphonate Bonds Were Partially Introduced: $T_{Ph}G_{Ph}GGAGCCATAGCGAG_{Ph}G_{Ph}C$ (SEQ ID NO: 32)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% $TCA/CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M guanosinephenylphosphonamidite unit and 0.4 M tetrazole;
(5) An oxidation reaction was conducted for 30 seconds using 0.1 M iodine/water/pyridine/THF;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected guanosine phenylphosphonate compound could be condensed with the cytidine compound introduced into CPG. A subsequent nucleotide sequence of a guanosine phenylphosphonate compound was bound thereto by repetition of operations similar to those described above. A further subsequent nucleotide sequence of adenosine phosphate was bound thereto. In this case, an adenosine-phosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% $TCA/CH_2Cl_2$ was conducted for 60 seconds to remove the 5'-Pix group of the protected guanosine phenylphosphonate compound. This solution was stored to calculate the condensation yield; and
(4) Condensation reaction was conducted for 5 minutes using the 0.2 M guanosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenylphosphonate bonds were introduced into the 5,- and 3'-terminal dinucleotides: $T_{Ph}G_{Ph}GGAGCCATAGCGAG_{Ph}G_{Ph}C$ was synthesized.

Yield: 35.4 OD
λmax: 256.5 nm
λmin: 229.1 nm

[Example 34]

Synthesis of Phosphate Antisense Oligonucleotide into Which Phenylphosphonate Bonds Were Partially Introduced and to the 5'-Terminus of Which Gentiobiose Was Bound: (Gen)-$T_{Ph}G_{Ph}GGAGCCATAGCGAG_{Ph}G_{Ph}C$ (SEQ ID NO: 32)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephenylphosphonamidite unit was dissolved in anhydrous

acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephenylphosphonamidite unit and 0.4 M tetrazole;

(5) An oxidation reaction was conducted for 30 seconds using 0.1 M iodine/water/pyridine/THF;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected adenosine phenylphosphonate compound could be condensed with the cytidine compound introduced into CPG. A subsequent nucleotide sequence of an adenosine phenylphosphonate compound was bound thereto by repetition of operations similar to those described above. A further subsequent nucleotide sequence of guanosine phosphate was bound thereto. In this case, a guanosine-phosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected guanosine phenylphosphonate compound. This solution was stored to calculate the condensation yield; and

(4) Condensation reaction was conducted for 5 minutes using the 0.2 M adenosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. After condensation of thymidylphosphonic acid as the 18th nucleotide, a gentiobiose compound was bound to the 5'-terminus. In this case, a gentiobioseamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected thymidylphosphonic acid compound; and

(4) Condensation reaction was conducted for 5 minutes using the 0.2 M gentiobiosephosphoramidite unit and 0.4 M tetrazole.

The gentiobiose compound could be introduced into the 5'-terminus of the oligonucleotide by the above-mentioned operations. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/ pyridine (5:1) solution at 60°C for 6 hours, thereby excising the nucleotide chain from the solid phase carrier and removing the protective group. Then, the product was purified by the use of reverse phase HPLC (µBondasphere $C_{18}$). By the above-mentioned operations, a desired phosphate antisense oligonucleotide in which phenylphosphonate bonds were introduced into the 5'- and 3'-terminal dinucleotides and gentiobiose was bound to the 5'-terminus: (Gen)-$T_{Ph}G_{Ph}$GGAGCCATAGCGAG$_{Ph}G_{Ph}$C was synthesized.

Yield: 17.0 OD

λmax: 255.6 nm

λmin: 229.8 nm

[Example 35]

Synthesis of Phosphate Antisense Oligonucleotide into Which Phenylphosphonate Bonds Were Partially Introduced and to the 5'-Terminus of Which Melibiose Was Bound: (Mel)-$T_{Ph}G_{Ph}$GGAGCCATAGCGAG$_{Ph}G_{Ph}$C (SEQ ID NO:32)

50 mg (1 micromol) of CPG into which 20 micromols/g of a cytidine compound was introduced, the N-4 amino group and the 5'-hydroxyl group of said cytidine compound being protected by a benzoyl group and

a DMTr group, respectively, was packed into a reaction column mounted on an automatic synthesizer (DNA Synthesizer Model 381 A, ABI). Then, a guanosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephenylphosphonamidite unit and 0.4 M tetrazole;

(5) An oxidation reaction was conducted for 30 seconds using 0.1 M iodine/water/pyridine/THF;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine phenylphosphonate compound could be condensed with the cytidine compound introduced into CPG. A subsequent nucleotide sequence of a guanosine phenylphosphonate compound was bound thereto by repetition of operations similar to those described above. A further subsequent nucleotide sequence of adenosine phosphate was bound thereto. In this case, an adenosine-phosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected guanosine phenylphosphonate compound. This solution was stored to calculate the condensation yield; and

(4) Condensation reaction was conducted for 5 minutes using the 0.2 M adenosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. After condensation of thymidylphosphonic acid as the 18th nucleotide, a melibiose compound was bound to the 5'-terminus. In this case, a melibioseamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected thymidylphosphonic acid compound; and

(4) Condensation reaction was conducted for 5 minutes using the 0.2 M melibiosephosphoramidite unit and 0.4 M tetrazole.

The melibiose compound could be introduced into the 5'-terminus of the oligonucleotide by the above-mentioned operations. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60°C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenylphosphonate bonds were introduced into the 5'- and 3'-terminal dinucleotides and melibiose was bound to the 5'-terminus: (Mel)-$T_{Ph}G_{Ph}$GGAGCCATAGCGA$G_{Ph}G_{Ph}$C was synthesized.

Yield: 22.5 OD

λmax: 255.2 nm

λmin: 229.6 nm

[Example 36]

Synthesis of Phosphate Antisense Oligonucleotide into Which Phenylphosphonate Bonds Were Partially Introduced: $T_{Ph}GGGAGC_{Ph}C_{Ph}AT_{Ph}AGC_{Ph}GAGGC_{Ph}T$ (SEQ ID NO: 33)

50 mg (1 micromol) of CPG into which 20 micromols/g of a thymidine compound was introduced, the 5'-hydroxyl group of said thymidine compound being protected by a DMTr group, was packed into a reaction column mounted on an automatic synthesizer (DNA Synthesizer Model 381 A, ABI). Then, a cytidinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M cytidinephenylphosphonamidite unit and 0.4 M tetrazole;
(5) An oxidation reaction was conducted for 30 seconds using 0.1 M iodine/water/pyridine/THF;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected cytidine phenylphosphonate compound could be condensed with the thymidine compound introduced into CPG. A subsequent nucleotide sequence of a guanosine phosphate compound was bound thereto. In this case, a guanosinephosphonamidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Mox group of the protected cytidine phenylphosphonate compound. This solution was stored to calculate the condensation yield; and
(4) Condensation reaction was conducted for 5 minutes using the 0.2 M guanosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60°C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenylphosphonate bonds were introduced into pyrimidine nucleotides: $T_{Ph}GGGAGC_{Ph}C_{Ph}AT_{Ph}AGC_{Ph}GAGGC_{Ph}T$ was synthesized.

Yield: 44.4 OD
λmax: 256.1 nm
λmin: 228.2 nm

[Example 37]

Synthesis of Phosphate Antisense Oligonucleotide into Which Phenylphosphonate Bonds Were Partially Introduced and to the 5'-Terminus of Which Gentiobiose Was Bound: (Gen)-$T_{Ph}GGGAGC_{Ph}C_{Ph}AT_{Ph}AGC_{Ph}GAGGC_{Ph}T$ (SEQ ID NO: 33)

50 mg (1 micromol) of CPG into which 20 micromols/g of a thymidine compound was introduced, the 5'-hydroxyl group of said thymidine compound being protected by a DMTr group, was packed into a reaction column mounted on an automatic synthesizer (DNA Synthesizer Model 381 A, ABI). Then, a cytidinephenylphosphoramidite unit was dissolved in anhydrous acetonitrile to give a concentration of 0.2 M, and the resulting solution was filled into a unit storage bottle, which was mounted on the automatic

synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M cytidinephenylphosphonamidite unit and 0.4 M tetrazole;

(5) An oxidation reaction was conducted for 30 seconds using 0.1 M iodine/water/pyridine/THF;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected cytidine phenylphosphonate compound could be condensed with the thymidine compound introduced into CPG. A subsequent nucleotide sequence of a guanosine phosphate was bound thereto. In this case, a guanosine-phosphonamidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Mox group of the protected cytidine phenylphosphonate compound. This solution was stored to calculate the condensation yield; and

(4) Condensation reaction was conducted for 5 minutes using the 0.2 M guanosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. After condensation of thymidylphosphonic acid as the 19th nucleotide, a gentiobiose compound was bound to the 5'-terminus. In this case, a gentiobioseamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected thymidylphosphonic acid compound; and

(4) Condensation reaction was conducted for 5 minutes using the 0.2 M gentiobiosephosphoramidite unit and 0.4 M tetrazole.

The gentiobiose compound could be introduced into the 5'-terminus of the oligonucleotide by the above-mentioned operations. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/ pyridine (5:1) solution at 60°C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenylphosphonate bonds were introduced into pyrimidine nucleotides and gentiobiose was bound to the 5'-terminus: (Gen)-$T_{Ph}$GGGAG$C_{Ph}C_{Ph}$A$T_{Ph}$AG$C_{Ph}$GAGG$C_{Ph}$T was synthesized.

Yield: 32.4 OD

λmax: 255.7 nm

λmin: 229.2 nm

[Example 38]

Synthesis of Phosphate Antisense Oligonucleotide into Which Methylphosphonate Bonds Were Partially Introduced and to the 5'-Terminus of Which Gentiobiose Was Bound: (Gen)-$T_{Me}G_{Me}$GGAGCCATAGCGA$G_{Me}G_{Me}$C (SEQ ID NO: 34)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinemethylphosphonamidite unit was dissolved in anhydrous acetonitrile to give a concentration of 0.2 M, and the resulting solution was filled into a unit storage bottle,

which was mounted on the automatic synthesizer. Then, the following operations were carried out in the reaction column by the use of the automatic synthesizer:

(1) Treatment with 3.0% $TCA/CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M guanosinemethylphosphonamidite unit and 0.4 M tetrazole;
(5) An oxidation reaction was conducted for 30 seconds using 0.1 M iodine/water/pyridine/THF;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected guanosine methylphosphonate compound could be condensed with the cytidine compound introduced into CPG. A subsequent nucleotide sequence of a guanosine methylphosphonate compound was bound thereto by repetition of operations similar to those described above. A further subsequent nucleotide sequence of adenosine phosphate was bound thereto. In this case, an adenosine-phosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% $TCA/CH_2Cl_2$ was conducted for 60 seconds to remove the 5'-DMTr group of the protected guanosine methylphosphonate compound. This solution was stored to calculate the condensation yield; and
(4) Condensation reaction was conducted for 5 minutes using the 0.2 M adenosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. After condensation of thymidylphosphonic acid as the 18th nucleotide, a gentiobiose compound was bound to the 5'-terminus. In this case, a gentiobioseamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% $TCA/CH_2Cl_2$ was conducted for 60 seconds to remove the 5'-DMTr group of the protected thymidylphosphonic acid compound; and
(4) Condensation reaction was conducted for 5 minutes using the 0.2 M gentiobiosephosphoramidite unit and 0.4 M tetrazole.

The gentiobiose compound could be introduced into the 5'-terminus of the oligonucleotide by the above-mentioned operations. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/ pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which methylphosphonate bonds were introduced into the 5'- and 3'-terminal dinucleotides and gentiobiose was bound to the 5'-terminus: (Gen)-$T_{Me}G_{Me}$GGAGCCATAGCGAG$_{Me}G_{Me}$C was synthesized.

Yield: 65.7 OD
λmax: 256.0 nm
λmin: 229.8 nm

[Example 39]

Synthesis of Phosphate Antisense Oligonucleotide into Which Methylphosphonate Bonds Were Partially Introduced and to the 5'-Terminus of Which Melibiose Was Bound: (Mel)-$T_{Me}G_{Me}$GGAGCCATAGCGAG$_{Me}G_{Me}$C (SEQ ID NO: 34)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA

Synthesizer Model 381 A, ABI). Then, a guanosinemethylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% $TCA/CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinemethylphosphonamidite unit and 0.4 M tetrazole;

(5) An oxidation reaction was conducted for 30 seconds using 0.1 M iodine/water/pyridine/THF;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine methylphosphonate compound could be condensed with the cytidine compound introduced into CPG. A subsequent nucleotide sequence of a guanosine methylphosphonate compound was bound thereto by repetition of operations similar to those described above. A further subsequent nucleotide sequence of adenosine phosphate was bound thereto. In this case, an adenosine-phosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% $TCA/CH_2Cl_2$ was conducted for 60 seconds to remove the 5'-DMTr group of the protected guanosine methylphosphonate compound. This solution was stored to calculate the condensation yield; and

(4) Condensation reaction was conducted for 5 minutes using the 0.2 M adenosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. After condensation of thymidylphosphonic acid as the 18th nucleotide, a melibiose compound was bound to the 5'-terminus. In this case, a melibioseamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% $TCA/CH_2Cl_2$ was conducted for 60 seconds to remove the 5'-DMTr group of the protected thymidylphosphonic acid compound; and

(4) Condensation reaction was conducted for 5 minutes using the 0.2 M melibiosephosphoramidite unit and 0.4 M tetrazole.

The melibiose compound could be introduced into the 5'-terminus of the oligonucleotide by the above-mentioned operations. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which methylphosphonate bonds were introduced into the 5'- and 3'-terminal dinucleotides and melibiose was bound to the 5'-terminus: (Mel)-$T_{Me}G_{Me}$GGAGCCATAGCGAG$_{Me}G_{Me}$C was synthesized.

Yield: 59.3 OD

λmax: 256.9 nm

λmin: 227.8 nm

[Example 40]

Synthesis of Phosphate Antisense Oligonucleotide into Which Phenylphosphonate Bonds Were Partially Introduced and to the 5'-Terminus of Which N-Benzoylgalactosamine Was Bound: (N-BzCal)-$T_{Ph}G_{Ph}$GGAGC-CATAGCGA$G_{Ph}G_{Ph}$C (SEQ ID NO:32)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-Pix group. This solution was stored to calculate the condensation yield;
(2) CPG was washed with acetonitrile;
(3) CPG was dried with an argon gas;
(4) Condensation reaction was conducted for 5 minutes using the 0.2 M guanosinephenylphosphonamidite unit and 0.4 M tetrazole;
(5) An oxidation reaction was conducted for 30 seconds using 0.1 M iodine/water/pyridine/THF;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected guanosine phenylphosphonate compound could be condensed with the cytidine compound introduced into CPG. A subsequent nucleotide sequence of a guanosine phenylphosphonate compound was bound thereto by repetition of operations similar to those described above. A further subsequent nucleotide sequence of adenosine phosphate was bound thereto. In this case, an adenosine-phosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected guanosine phenylphosphonate compound. This solution was stored to calculate the condensation yield; and
(4) Condensation reaction was conducted for 5 minutes using the 0.2 M adenosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. After condensation of thymidylphosphonic acid as the 18th nucleotide, an N-benzoylgalactosamine compound was bound to the 5'-terminus. In this case, an N-benzoylgalactosamineamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected thymidylphosphonic acid compound; and
(4) Condensation reaction was conducted for 5 minutes using the 0.2 M N-benzoylgalactosaminephosphoramidite unit and 0.4 M tetrazole.

The N-benzoylgalactosamine compound could be introduced into the 5'-terminus of the oligonucleotide by the above-mentioned operations. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/ pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenylphosphonate bonds were introduced into the 5'- and 3'-terminal dinucleotides and N-benzoylgalactosamine was bound to the 5'-terminus: (N-BzGal)-$T_{Ph}G_{Ph}$GGAGCCATAGCGA$G_{Ph}G_{Ph}$C was synthesized.

Yield: 21.2 OD

λmax: 256.5 nm
λmin: 228.9 nm

[Example 41]

Synthesis of Phosphate Antisense Oligonucleotide into Which Phenylphosphonate Bonds Were Partially Introduced: $T_{Ph}GGGAGCCATAGCGAGG_{Ph}C$ (SEQ ID NO: 35)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% $TCA/CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M guanosinephenylphosphonamidite unit and 0.4 M tetrazole;
(5) An oxidation reaction was conducted for 30 seconds using 0.1 M iodine/water/pyridine/THF;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected guanosine phenylphosphonate compound could be condensed with the cytidine compound introduced into CPG. A subsequent nucleotide sequence of guanosine phosphate was bound thereto. In this case, a guanosinephosphonamidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% $TCA/CH_2Cl_2$ was conducted for 60 seconds to remove the 5'-Pix group of the protected guanosine phenylphosphonate compound. This solution was stored to calculate the condensation yield; and
(4) Condensation reaction was conducted for 5 minutes using the 0.2 M guanosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60°C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenylphosphonate bonds were introduced into the 5'- and 3'-terminal mononucleotides: $T_{Ph}GGGAGCCATAGCGAGG_{Ph}C$ was synthesized.

Yield: 20.9 OD
λmax: 256.0 nm
λmin: 228.4 nm

[Example 42]

Synthesis of Phosphate Antisense Oligonucleotide into Which Phenylphosphonate Bonds Were Partially Introduced and to the 5'-Terminus of Which Gentiobiose Was Bound: (Gen)-$T_{Ph}GGGAGCCATAGC$-$GAGG_{Ph}C$ (SEQ ID NO: 35)

50 mg (1 micromol) of CPG into which 20 micromols/g of a cytidine compound was introduced, the N-4 amino group and the 5'-hydroxyl group of said cytidine compound being protected by a benzoyl group and

a DMTr group, respectively, was packed into a reaction column mounted on an automatic synthesizer (DNA Synthesizer Model 381 A, ABI). Then, a guanosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M guanosinephenylphosphonamidite unit and 0.4 M tetrazole;
(5) An oxidation reaction was conducted for 30 seconds using 0.1 M iodine/water/pyridine/THF;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected guanosine phenylphosphonate compound could be condensed with the cytidine compound introduced into CPG. A subsequent nucleotide sequence of a guanosine phosphate was bound thereto. In this case, a guanosinephosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected guanosine phenylphosphonate compound. This solution was stored to calculate the condensation yield; and
(4) Condensation reaction was conducted for 5 minutes using the 0.2 M guanosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. After condensation of thymidylphosphonic acid as the 18th nucleotide, a gentiobiose compound was bound to the 5'-terminus. In this case, a gentiobioseamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected thymidylphosphonic acid compound; and
(4) Condensation reaction was conducted for 5 minutes using the 0.2 M gentiobiosephosphoramidite unit and 0.4 M tetrazole.

The gentiobiose compound could be introduced into the 5'-terminus of the oligonucleotide by the above-mentioned operations. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/ pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenylphosphonate bonds were introduced into the 5'- and 3'-terminal mononucleotides and gentiobiose was bound to the 5'-terminus: (Gen)-$T_{Ph}$GGGAGCCATAGCGAGG$_{Ph}$C was synthesized.

Yield: 21.1 OD
λmax: 256.3 nm
λmin: 228.4 nm

[Example 43]

Synthesis of Phosphate Antisense Oligonucleotide into Which Phenylphosphonate Bonds Were Partially Introduced and to the 5'-Terminus of Which N-Benzoylgalactosamine Was Bound: (N-BzCal)-$T_{Ph}$GGGAGCCATAGCGAGG$_{Ph}$C (SEQ ID NO: 35)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M guanosinephenylphosphonamidite unit and 0.4 M tetrazole;
(5) An oxidation reaction was conducted for 30 seconds using 0.1 M iodine/water/pyridine/THF;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected guanosine phenylphosphonate compound could be condensed with the cytidine compound introduced into CPG. A subsequent nucleotide sequence of guanosine phosphate was bound thereto. In this case, a guanosinephosphonamidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected guanosine phenylphosphonate compound. This solution was stored to calculate the condensation yield; and
(4) Condensation reaction was conducted for 5 minutes using the 0.2 M guanosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. After condensation of thymidylphosphonic acid as the 18th nucleotide, an N-benzoylgalactosamine compound was bound to the 5'-terminus. In this case, an N-benzoylgalactosamineamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected thymidylphosphonic acid compound; and
(4) Condensation reaction was conducted for 5 minutes using the 0.2 M N-benzoylgalactosaminephosphoramidite unit and 0.4 M tetrazole.

The N-benzoylgalactosamine compound could be introduced into the 5'-terminus of the oligonucleotide by the above-mentioned operations. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/ pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenylphosphonate bonds were introduced into the 5'- and 3'-terminal mononucleotides and N-benzoylgalactosamine was bound to the 5'-terminus: (N-BzGal)-$T_{Ph}$GGGAGCCATAGCGAGG$_{Ph}$C was synthesized.

Yield: 25.8 OD
λmax: 256.1 nm
λmin: 228.6 nm

[Example 44]

Synthesis of Phosphate Reverse Sequence Oligonucleotide into Which Phenylphosphonate Bonds Were Partially Introduced: $C_{Ph}G_{Ph}GAGCGATACCGAGG_{Ph}G_{Ph}T$ (SEQ ID NO: 37)

50 mg (1 micromol) of CPG into which 20 micromols/g of a thymidine compound was introduced, the 5'-hydroxyl group of said thymidine compound being protected by a DMTr group, was packed into a reaction column mounted on an automatic synthesizer (DNA Synthesizer Model 381 A, ABI). Then, a guanosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% $TCA/CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M guanosinephenylphosphonamidite unit and 0.4 M tetrazole;
(5) An oxidation reaction was conducted for 30 seconds using 0.1 M iodine/water/pyridine/THF;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected guanosine phenylphosphonate compound could be condensed with the thymidine compound introduced into CPG. A subsequent nucleotide sequence of a guanosine phenylphosphonate compound was bound thereto by repetition of operations similar to those described above. A further subsequent nucleotide sequence of guanosine phosphate was bound thereto. In this case, a guanosine-phosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% $TCA/CH_2Cl_2$ was conducted for 60 seconds to remove the 5'-Pix group of the protected guanosine phenylphosphonate compound. This solution was stored to calculate the condensation yield; and
(4) Condensation reaction was conducted for 5 minutes using the 0.2 M guanosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate reverse sequence oligonucleotide in which phenylphosphonate bonds were introduced into the 5'- and 3'-terminal dinucleotides: $C_{Ph}G_{Ph}GAGCGATACCGAGG_{Ph}G_{Ph}T$ was synthesized.

Yield: 14.8 OD
λmax: 257.3 nm
λmin: 229.9 nm

[Example 45]

Synthesis of Phosphate Reverse Sequence Oligonucleotide into Which Phenylphosphonate Bonds Were Partially Introduced and to the 5'-Terminus of Which Gentiobiose Was Bound: (Gen)-$C_{Ph}G_{Ph}GAGCGATACC$-$GAGG_{Ph}G_{Ph}T$ (SEQ ID NO: 37)

50 mg (1 micromol) of CPG into which 20 micromols/g of a thymidine compound was introduced, the 5'-hydroxyl group of said thymidine compound being protected by a DMTr group, was packed into a reaction column mounted on an automatic synthesizer (DNA Synthesizer Model 381 A, ABI). Then, a guanosinephenylphosphonamidite unit was dissolved in anhydrous acetonitrile to give a concentration of 0.2

M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephenylphosphonamidite unit and 0.4 M tetrazole;

(5) An oxidation reaction was conducted for 30 seconds using 0.1 M iodine/water/pyridine/THF;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine phenylphosphonate compound could be condensed with the thymidine compound introduced into CPG. A subsequent nucleotide sequence of a guanosine phenylphosphonate compound was bound thereto by repetition of operations similar to those described above. A further subsequent nucleotide sequence of guanosine phosphate was bound thereto. In this case, a guanosine-phosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected guanosine phenylphosphonate compound. This solution was stored to calculate the condensation yield; and

(4) Condensation reaction was conducted for 5 minutes using the 0.2 M guanosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. After condensation of cytidylphosphonic acid as the 18th nucleotide, a gentiobiose compound was bound to the 5'-terminus. In this case, a gentiobioseamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected thymidylphosphonic acid compound; and

(4) Condensation reaction was conducted for 5 minutes using the 0.2 M gentiobiosephosphoramidite unit and 0.4 M tetrazole.

The gentiobiose compound could be introduced into the 5'-terminus of the oligonucleotide by the above-mentioned operations. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/ pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenylphosphonate bonds were introduced into the 5'- and 3'-terminal mononucleotides and gentiobiose was bound to the 5'-terminus: (Gen)-$C_{Ph}G_{Ph}$GAGCGATACCGAGG$G_{Ph}G_{Ph}$T was synthesized.

Yield: 12.2 OD
λmax: 257.2 nm
λmin: 230.0 nm

[Example 46]

Synthesis of Phosphate Antisense Reverse Sequence Oligonucleotide into Which Phenylphosphonate Bonds Were Partially Introduced and to the 5'-Terminus of Which Melibiose Was Bound: (Mel)-$C_{Ph}G_{Ph}$GAGCGATACCGAGG$G_{Ph}G_{Ph}$T (SEQ ID NO: 37)

50 mg (1 micromol) of CPG into which 20 micromols/g of a thymidine compound was introduced, the 5'-hydroxyl group of said thymidine compound being protected by a DMTr group, was packed into a

reaction column mounted on an automatic synthesizer (DNA Synthesizer Model 381 A, ABI). Then, a guanosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephenylphosphonamidite unit and 0.4 M tetrazole;

(5) An oxidation reaction was conducted for 30 seconds using 0.1 M iodine/water/pyridine/THF;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine phenylphosphonate compound could be condensed with the thymidine compound introduced into CPG. A subsequent nucleotide sequence of a guanosine phenylphosphonate compound was bound thereto by repetition of operations similar to those described above. A further subsequent nucleotide sequence of guanosine phosphate was bound thereto. In this case, a guanosine-phosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected guanosine phenylphosphonate compound. This solution was stored to calculate the condensation yield; and

(4) Condensation reaction was conducted for 5 minutes using the 0.2 M guanosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. After condensation of cytidylphosphonic acid as the 18th nucleotide, a melibiose compound was bound to the 5'-terminus. In this case, a melibioseamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected thymidylphosphonic acid compound; and

(4) Condensation reaction was conducted for 5 minutes using the 0.2 M melibiosephosphoramidite unit and 0.4 M tetrazole.

The gentiobiose compound could be introduced into the 5'-terminus of the oligonucleotide by the above-mentioned operations. Deprotecting operations after completion of the synthesis reaction were conducted according to methods known in the art [Oligonucleotide Synthesis: A Practical Approach, edited by M. J. Gait, IRL PRESS, (1984)]. CPG was taken out of the reaction column, and reacted with an ammonia/ pyridine (5:1) solution at 60°C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenylphosphonate bonds were introduced into the 5'- and 3'-terminal mononucleotides and gentiobiose was bound to the 5'-terminus: (Mel)-$C_{Ph}G_{Ph}$GAGCGATACCGAGG$G_{Ph}$... 

Yield: 15.3 OD

λmax: 257.2 nm

λmin: 230.3 nm

[Example 47]

Synthesis of Phosphate Antisense Oligonucleotide into Which Phenylphosphonate Bonds Were Partially Introduced: $T_{Ph}G_{Ph}GGAGC_{Ph}C_{Ph}AT_{Ph}AGC_{Ph}GAG_{Ph}G_{Ph}C$ (SEQ ID NO: 39)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% $TCA/CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M guanosinephenylphosphonamidite unit and 0.4 M tetrazole;
(5) An oxidation reaction was conducted for 30 seconds using 0.1 M iodine/water/pyridine/THF;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected guanosine phenylphosphonate compound could be condensed with the cytidine compound introduced into CPG. A subsequent nucleotide sequence of a guanosine phenylphosphonate compound was bound thereto by repetition of operations similar to those described above. A further subsequent nucleotide sequence of adenosine phosphate was bound thereto. In this case, an adenosinephosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% $TCA/CH_2Cl_2$ was conducted for 60 seconds to remove the 5'-Pix group of the protected guanosine phenylphosphonate compound. This solution was stored to calculate the condensation yield; and
(4) Condensation reaction was conducted for 5 minutes using the 0.2 M adenosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. Deprotecting operations after completion of the synthesis reaction were conducted according to methods known in the art [Oligonucleotide Synthesis: A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC ($\mu$Bondasphere $C_{18}$). By the above-mentioned operations, a desired phosphate antisense oligonucleotide in which phenylphosphonate bonds were introduced into the 5'- and 3'-each two terminal mononucleotides: $T_{Ph}G_{Ph}GGAGC_{Ph}C_{Ph}AT_{Ph}AGC_{Ph}GAG_{Ph}G_{Ph}C$was synthesized.

Yield: 43.1 OD
$\lambda$max: 257.6 nm
$\lambda$min: 229.4 nm

[Example 48]

Synthesis of Phosphate Antisense Oligonucleotide into Which Phenylphosphonate Bonds Were Partially Introduced and to the 5'-Terminus of Which Gentiobiose Was Bound: (Gen)-$T_{Ph}G_{Ph}GGAGC_{Ph}C_{Ph}AT_{Ph}AGC_{Ph}GAG_{Ph}G_{Ph}C$ (SEQ ID NO: 39)

50 mg (1 micromol) of CPG into which 20 micromols/g of a thymidine compound was introduced, the 5'-hydroxyl group of said thymidine compound being protected by a DMTr group, was packed into a reaction column mounted on an automatic synthesizer (DNA Synthesizer Model 381 A, ABI). Then, a guanosinephenylphosphonamidite unit was dissolved in anhydrous acetonitrile to give a concentration of 0.2

M, and the resulting solution was filled into a unit storage bottle, which was mounted on the automatic synthesizer. Then, the following operations were carried out in the reaction column by the use of the automatic synthesizer:

(1) Treatment with 3.0% $TCA/CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephenylphosphonamidite unit and 0.4 M tetrazole;

(5) An oxidation reaction was conducted for 30 seconds using 0.1 M iodine/water/pyridine/THF;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine phenylphosphonate compound could be condensed with the thymidine compound introduced into CPG. A subsequent nucleotide sequence of a guanosine phenylphosphonate compound was bound thereto by repetition of operations similar to those described above. A further subsequent nucleotide sequence of adenosine phosphate was bound thereto. In this case, an adenosine-phosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% $TCA/CH_2Cl_2$ was conducted for 60 seconds to remove the 5'-Pix group of the protected guanosine phenylphosphonate compound. This solution was stored to calculate the condensation yield; and

(4) Condensation reaction was conducted for 5 minutes using the 0.2 M adenosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. After condensation of thymidylphosphonic acid as the 18th nucleotide, a gentiobiose compound was bound to the 5'-terminus. In this case, a gentiobioseamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% $TCA/CH_2Cl_2$ was conducted for 60 seconds to remove the 5'-Pix group of the protected thymidylphosphonic acid compound; and

(4) Condensation reaction was conducted for 5 minutes using the 0.2 M gentiobiosephosphoramidite unit and 0.4 M tetrazole.

The gentiobiose compound could be introduced into the 5'-terminus of the oligonucleotide by the above-mentioned operations. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/ pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenylphosphonate bonds were introduced into the 5'- and 3'-terminal mononucleotides and gentiobiose was bound to the 5'-terminus: (Gen)-$T_{Ph}G_{Ph}GGAGC_{Ph}C_{Ph}AT_{Ph}AGC_{Ph}GAG_{Ph}G_{Ph}C$ was synthesized.

Yield: 43.3 OD

λmax: 257.9 nm

λmin: 229.5 nm

[Example 49]

Synthesis of Phosphate Antisense Oligonucleotide into Which Phenylphosphonate Bonds Were Partially Introduced: $C_{Ph}G_{Ph}GAGC_{Ph}GAT_{Ph}AC_{Ph}C_{Ph}GAGG_{Ph}G_{Ph}T$ (SEQ ID NO: 40)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA

Synthesizer Model 381 A, ABI). Then, a guanosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% $TCA/CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M guanosinephenylphosphonamidite unit and 0.4 M tetrazole;
(5) An oxidation reaction was conducted for 30 seconds using 0.1 M iodine/water/pyridine/THF;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected guanosine phenylphosphonate compound could be condensed with the cytidine compound introduced into CPG. A subsequent nucleotide sequence of a guanosine phenylphosphonate compound was bound thereto by repetition of operations similar to those described above. A further subsequent nucleotide sequence of guanosine phosphate was bound thereto. In this case, a guanosinephosphonamidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% $TCA/CH_2Cl_2$ was conducted for 60 seconds to remove the 5'-Pix group of the protected guanosine phenylphosphonate compound. This solution was stored to calculate the condensation yield; and
(4) Condensation reaction was conducted for 5 minutes using the 0.2 M guanosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60°C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenylphosphonate bonds were introduced into each of the 5'- and 3'- two terminal mononucleotides: $C_{Ph}G_{Ph}GAGC_{Ph}GAT_{Ph}AC_{Ph}C_{Ph}GAGG_{Ph}G_{Ph}T$ was synthesized.

Yield: 38.0 OD
λmax: 256.7 nm
λmin: 229.0 nm

[Example 50]

Synthesis of Phosphate Antisense Oligonucleotide into Which Phenylphosphonate Bonds Were Partially Introduced: $C_{Ph}C_{Ph}CCCACCACTTCCC_{Ph}C_{Ph}T$ (SEQ ID NO: 41)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% $TCA/CH_2Cl_2$ was conducted for 60 seconds to remove the 5'-DMTr group. This solution was stored to calculate the condensation yield;
(2) CPG was washed with acetonitrile;
(3) CPG was dried with an argon gas;

(4) Condensation reaction was conducted for 5 minutes using the 0.2 M guanosinephenylphosphonamidite unit and 0.4 M tetrazole;

(5) An oxidation reaction was conducted for 30 seconds using 0.1 M iodine/water/pyridine/THF;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected cytidine phenylphosphonate compound could be condensed with the thymidine compound introduced into CPG. A subsequent nucleotide sequence of cytidine phosphate was bound thereto. In this case, a cytidinephosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected guanosine phenylphosphonate compound. This solution was stored to calculate the condensation yield; and

(4) Condensation reaction was conducted for 5 minutes using the 0.2 M cytidineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. After condensation of cytidylphosphonic acid as the 18th nucleotide, a gentiobiose compound was bound to the 5'-terminus. In this case, a gentiobioseamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected thymidylphosphonic acid compound; and

(4) Condensation reaction was conducted for 5 minutes using the 0.2 M gentiobiosephosphoramidite unit and 0.4 M tetrazole.

The gentiobiose compound could be introduced into the 5'-terminus of the oligonucleotide by the above-mentioned operations. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/ pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenyl-phosphonate bonds were introduced into each of the 5'- and 3'- two terminal mononucleotides: $C_{Ph}C_{Ph}$CCCACCACTTCCC$C_{Ph}C_{Ph}$T was synthesized.

Yield: 37.6 OD

λmax: 256.1 nm

λmin: 229.1 nm

[Example 51]

Synthesis of Phosphate Reverse Sequence Oligonucleotide into Which Phenylphosphonate Bonds Were Partially Introduced and to the 5'-Terminus of Which Gentiobiose Was Bound: (Gen)-$C_{Ph}C_{Ph}$CCCACCACTTCCC$C_{Ph}C_{Ph}$T (SEQ ID NO: 41)

50 mg (1 micromol) of CPG into which 20 micromols/g of a thymidine compound was introduced, the 5'-hydroxyl group of said thymidine compound being protected by a DMTr group, was packed into a reaction column mounted on an automatic synthesizer (DNA Synthesizer Model 381 A, ABI). Then, a cytidinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M cytidinephenylphosphonamidite unit and 0.4 M tetrazole;

(5) An oxidation reaction was conducted for 30 seconds using 0.1 M iodine/water/pyridine/THF;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected cytidine phenylphosphonate compound could be condensed with the thymidine compound introduced into CPG. A subsequent nucleotide sequence of a cytidine phenylphosphonate compound was bound thereto by repetition of operations similar to those described above. A further subsequent nucleotide sequence of cytidine phosphate was bound thereto. In this case, a cytidine-phosphoramidite unit was dissolved to give a concentration of 0.2 M, and the solution was loaded on the automatic synthesizer, followed by repetition of the above-mentioned operations (1) to (7) With the exception that the operations (1) and (4) were conducted as follows:

(1) Treatment with 3.0% TCA/$CH_2Cl_2$ was conducted for 60 seconds to remove the 5'-Pix group of the protected guanosine phenylphosphonate compound. This solution was stored to calculate the condensation yield; and

(4) Condensation reaction was conducted for 5 minutes using the 0.2 M cytidineamidite unit and 0.4 M tetrazole.

The gentiobiose compound could be introduced into the 5'-terminus of the oligonucleotide by the above-mentioned operations. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/ pyridine (5:1) solution at 60 °C for 6 hours, thereby excising the nucleotide chain from the solid phase carrier and removing the protective group. Then, the product was purified by the use of reverse phase HPLC (µBondasphere $C_{18}$). By the above-mentioned operations, a desired phosphate antisense oligonucleotide in which phenylphosphonate bonds were introduced into the 5'- and 3'-terminal dinucleotides and gentiobiose was bound to the 5'-terminus: (Gen)-$C_{Ph}C_{Ph}$CCCACCACTTCCC$C_{Ph}C_{Ph}$T was synthesized.

Yield: 55.7 OD

λmax: 257.9 nm

λmin: 234.5 nm

[Example 52]

Synthesis of Phosphate Antisense Oligonucleotide into Which Phenylphosphonate Bonds Were Partially Introduced: $C_{Ph}C_{Ph}$CTGTCCCGGGATA$G_{Ph}G_{Ph}$T (SEQ ID NO: 42)

50 mg (1 micromol) of CPG into which 20 micromols/g of a thymidine compound was introduced, the 5'-hydroxyl group of said thymidine compound being protected by a DMTr group, was packed into a reaction column mounted on an automatic synthesizer (DNA Synthesizer Model 381 A, ABI). Then, a guanosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephenylphosphonamidite unit and 0.4 M tetrazole;

(5) An oxidation reaction was conducted for 30 seconds using 0.1 M iodine/water/pyridine/THF;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine phenylphosphonate compound could be condensed with the thymidine compound introduced into CPG. A subsequent nucleotide sequence of a guanosine phenylphosphonate compound was bound thereto by repetition of operations similar to those described above. A further subsequent nucleotide sequence of adenosine phosphate was bound thereto. In

this case, a adenosinephosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected guanosine phenylphosphonate compound. This solution was stored to calculate the condensation yield; and

(4) Condensation reaction was conducted for 5 minutes using the 0.2 M adenosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenylphosphonate bonds were introduced into the 5'- and 3'-each two terminal mononucleotides: $C_{Ph}C_{Ph}$CTGTCCCGGGATAG$_{Ph}$G$_{Ph}$T was synthesized.

Yield: 57.1 OD
λmax: 259.1 nm
λmin: 230.9 nm

[Example 53]

Synthesis of Phosphate Antisense Oligonucleotide into Which Phenylphosphonate Bonds Were Partially Introduced and to the 5'-Terminus of Which Gentiobiose Was Bound: (Gen)-$C_{Ph}C_{Ph}$CTGTCCCGGGATAG$_{Ph}$G$_{Ph}$T (SEQ ID NO: 42)

50 mg (1 micromol) of CPG into which 20 micromols/g of a thymidine compound was introduced, the 5'-hydroxyl group of said thymidine compound being protected by a DMTr group, was packed into a reaction column mounted on an automatic synthesizer (DNA Synthesizer Model 381 A, ABI). Then, a guanosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephenylphosphonamidite unit and 0.4 M tetrazole;

(5) An oxidation reaction was conducted for 30 seconds using 0.1 M iodine/water/pyridine/THF;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine phenylphosphonate compound could be condensed with the thymidine compound introduced into CPG. A subsequent nucleotide sequence of a guanosine phenylphosphonate compound was bound thereto by repetition of operations similar to those described above. A further subsequent nucleotide sequence of adenosine phosphate was bound thereto. In this case, an adenosine-phosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected guanosine phenylphosphonate compound. This solution was stored to calculate the condensation yield; and

(4) Condensation reaction was conducted for 5 minutes using the 0.2 M adenosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. After condensation of cytidyl-phosphonic acid as the 18th nucleotide, a gentiobiose compound was bound to the 5'-terminus. In this case, a gentiobioseamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% $TCA/CH_2Cl_2$ was conducted for 60 seconds to remove the 5'-Pix group of the protected thymidylphosphonic acid compound; and
(4) Condensation reaction was conducted for 5 minutes using the 0.2 M gentiobiosephosphoramidite unit and 0.4 M tetrazole.

The gentiobiose compound could be introduced into the 5'-terminus of the oligonucleotide by the above-mentioned operations. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/ pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenylphosphonate bonds were introduced into the 5'- and 3'-terminal mononucleotides and gentiobiose was bound to the 5'-terminus: (Gen)-$C_{Ph}C_{Ph}$CTGTCCCGGGATAG$_{Ph}$G$_{Ph}$T was synthesized.

Yield: 52.4 OD
λmax: 258.4 nm
λmin: 230.7 nm

[Example 54]

Synthesis of Phenylphosphonate Antisense Oligonucleotide $T_{Ph}G_{Ph}G_{Ph}G_{Ph}A_{Ph}G_{Ph}C_{Ph}C_{Ph}A_{Ph}T_{Ph}A_{Ph}G_{Ph}C_{Ph}G_{Ph}A_{Ph}G_{Ph}G_{Ph}C$ (SEQ ID NO: 43)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% $TCA/CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M guanosinephenylphosphonamidite unit and 0.4 M tetrazole;
(5) An oxidation reaction was conducted for 30 seconds using 0.1 M iodine/water/pyridine/THF;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected guanosine phenylphosphonate compound could be condensed with the cytidine compound introduced into CPG. A subsequent nucleotide sequence of guanosine phosphate was bound thereto.

The nucleotide chain was successively extended by repetition of the above-mentioned operations. Trityl alcohol released by the operation (1) was colorimetrically determined, and the average yield of each condensation reaction calculated from the results was 99% or more. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a

desired phosphate antisense oligonucleotide in which phenylphosphonate bonds were introduced into the 5'- and 3'-each two terminal mononucleotides: $T_{Ph}G_{Ph}G_{Ph}G_{Ph}A_{Ph}G_{Ph}C_{Ph}C_{Ph}A_{Ph}T_{Ph}A_{Ph}G_{Ph}C_{Ph}G_{Ph}A_{Ph}G_{Ph}G_{Ph}C$ was synthesized.

Yield: 35.6 OD
λmax: 254.7 nm
λmin: 227.5 nm

[Example 55]

Synthesis of Phenylphosphonate Antisense Oligonucletode to the 5'-Terminus of Which Gentiobiose Was Bound: (Gen)-$T_{Ph}G_{Ph}G_{Ph}G_{Ph}A_{Ph}G_{Ph}C_{Ph}C_{Ph}A_{Ph}T_{Ph}A_{Ph}G_{Ph}C_{Ph}G_{Ph}A_{Ph}G_{Ph}G_{Ph}C$ (SEQ ID NO: 43)

50 mg (1 micromol) of CPG into which 20 micromols/g of a thymidine compound was introduced, the N-4 amino group and the 5'-hydroxyl group of said thymidine compound being protected by a benzoyl group and a DMTr group, repsectively, was packed into a reaction column mounted on an automatic synthesizer (DNA Synthesizer Model 381 A, ABI). Then, a guanosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% $TCA/CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M guanosinephenylphosphonamidite unit and 0.4 M tetrazole;
(5) An oxidation reaction was conducted for 30 seconds using 0.1 M iodine/water/pyridine/THF;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected guanosine phenylphosphonate compound could be condensed with the cytidine compound introduced into CPG. A subsequent nucleotide sequence of a guanosine phenylphosphonate compound was bound thereto by repetition of operations similar to those described above. The nucleotide chain was successively extended by repetition of the above-mentioned operations. Trityl alcohol released by the operation (1) was colorimetrically determined, and the average yield of each condensation reaction calculated from the results was 99% or more. After condensation of thymidylphosphonic acid as the 18th nucleotide, a gentiobiose compound was bound to the 5'-terminus. In this case, a gentiobioseamidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% $TCA/CH_2Cl_2$ was conducted for 60 seconds to remove the 5'-Pix group of the protected thymidylphosphonic acid compound; and
(4) Condensation reaction was conducted for 5 minutes using the 0.2 M gentiobiosephosphoramidite unit and 0.4 M tetrazole.

The gentiobiose compound could be introduced into the 5'-terminus of the oligonucleotide by the above-mentioned operations. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/ pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenylphosphonate bonds were introduced into all the internucleotides and gentiobiose was bound to the 5'-terminus: (Gen)-$T_{Ph}G_{Ph}G_{Ph}G_{Ph}A_{Ph}G_{Ph}C_{Ph}C_{Ph}A_{Ph}T_{Ph}A_{Ph}G_{Ph}C_{Ph}G_{Ph}A_{Ph}G_{Ph}G_{Ph}C$ was synthesized.

Yield: 23.1 OD
λmax: 253.4 nm
λmin: 226.7 nm

[Example 56]

Synthesis of Phenylphosphonate Antisense Oligonucleotide into which Phenylphosphonate Bonds are introduced $G_{Ph}G_{Ph}AGCCATAGCGAG_{Ph}G_{Ph}C$ (SEQ ID NO: 44)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;
(2) CPG was washed with acetonitrile;
(3) CPG was dried with an argon gas;
(4) Condensation reaction was conducted for 5 minutes using the 0.2 M guanosinephenylphosphonamidite unit and 0.4 M tetrazole;
(5) An oxidation reaction was conducted for 30 seconds using 0.1 M iodine/water/pyridine/THF;
(6) A 0.25 M acetic anhydride/1-methylimidazole/lutidine/tetrahydrofuran solution was reacted for 30 seconds to block the unreacted 5'-hydroxyl group; and
(7) Washing with acetonitrile and drying with an argon gas were carried out.

According to the above-mentioned operations, the protected guanosine phenylphosphonate compound could be condensed with the cytidine compound introduced into CPG. A subsequent nucleotide sequence of a guanosine phenylphosphonate compound was bound thereto by repetition of operations similar to those described above. A further subsequent nucleotide sequence of adenosine phosphate was bound thereto. In this case, an adenosine-phosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected guanosine phenylphosphonate compound. This solution was stored to calculate the condensation yield; and
(4) Condensation reaction was conducted for 5 minutes using the 0.2 M adenosineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenylphosphonate bonds were introduced into each of the 5'- and 3'- two terminal dinucleotides: $G_{Ph}G_{Ph}AGCCATAGCGAG_{Ph}G_{Ph}C$ was synthesized.

Yield: 38.3 OD
λmax: 256.3 nm
λmin: 231.9 nm

[Example 57]

Synthesis of Phosphonate Antisense Oligonucleotide into which Phenylphosphonate Bonds are introduced $A_{Ph}G_{Ph}CCATAGCG_{Ph}A_{Ph}G$ (SEQ ID NO: 45)

50 mg (1 micromol) of CPG into which 20 micromols/g of a cytidine compound was introduced, the N-2 amino group and the 5'-hydroxyl group of said cytidine compound being protected by an isobutiryl group and a DMTr group, respectively, was packed into a reaction column mounted on an automatic synthesizer (DNA Synthesizer Model 381 A, ABI). Then, an adenosinephenylphosphonamidite unit was dissolved in anhydrous acetonitrile to give a concentration of 0.2 M, and the resulting solution was filled into a unit

storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M adenosinephenylphosphonamidite unit and 0.4 M tetrazole;

(5) An oxidation reaction was conducted for 30 seconds using 0.1 M iodine/water/pyridine/THF;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected adenosine phenylphosphonate compound could be condensed with the guanosine compound introduced into CPG. A subsequent nucleotide sequence of a guanosine phenylphosphonate compound was bound thereto by repetition of operations similar to those described above. A further subsequent nucleotide sequence of cytidine phosphate was bound thereto. In this case, a cytidine-phosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected guanosine phenylphosphonate compound. This solution was stored to calculate the condensation yield; and

(4) Condensation reaction was conducted for 5 minutes using the 0.2 M cytidineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (µBondasphere $C_{18}$). By the above-mentioned operations, a desired phosphate antisense oligonucleotide in which phenylphosphonate bonds were introduced into each of the 5'- and 3'- two terminal dinucleotides: $A_{Ph}G_{Ph}CCATAGCG_{Ph}A_{Ph}G$ was synthesized.

Yield: 34.6 OD
λmax: 258.1 nm
λmin: 230.4 nm

[Example 58]

Synthesis of Phenylphosphonate Antisense Oligonucleotide into which Phenylphosphonate Bonds are introduced $A_{Ph}G_{Ph}CCATA_{Ph}G_{Ph}C$ (SEQ ID NO: 46)

50 mg (1 micromol) of CPG into which 20 micromols/g of a cytidine compound was introduced, the N-4 amino group and the 5'-hydroxyl group of said cytidine compound being protected by a benzoyl group and a DMTr group, respectively, was packed into a reaction column mounted on an automatic synthesizer (DNA Synthesizer Model 381 A, ABI). Then, a guanosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephenylphosphonamidite unit and 0.4 M tetrazole;

(5) An oxidation reaction was conducted for 30 seconds using 0.1 M iodine/water/pyridine/THF;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine phenylphosphonate compound could be condensed with the cytidine compound introduced into CPG. A subsequent nucleotide sequence of an adenosine phenylphosphonate compound was bound thereto by repetition of operations similar to those described above. A further subsequent nucleotide sequence of thymidine phosphate was bound thereto. In this case, a thymidine-phosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% $TCA/CH_2Cl_2$ was conducted for 60 seconds to remove the 5'-Pix group of the protected guanosine phenylphosphonate compound. This solution was stored to calculate the condensation yield; and

(4) Condensation reaction was conducted for 5 minutes using the 0.2 M thymidineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenylphosphonate bonds were introduced into each of the 5'- and 3'- two terminal dinucleotides : $A_{Ph}G_{Ph}CCATA_{Ph}G_{Ph}C$ was synthesized.

Yield: 33.0 OD
λmax: 258.6 nm
λmin: 229.8 nm

[Example 59]

Synthesis of Phenylphosphonate Antisense Oligonucleotide into which Phenylphosphonate Bonds are introduced $G_{Ph}G_{Ph}TTTTTTTTTTTTTG_{Ph}G_{Ph}G$ (SEQ ID NO: 47)

50 mg (1 micromol) of CPG into which 20 micromols/g of a guanosine compound was introduced, the N-2 amino group and the 5'-hydroxyl group of said guanosine compound being protected by an isobutiryl group and a DMTr group, respectively, was packed into a reaction column mounted on an automatic synthesizer (DNA Synthesizer Model 381 A, ABI). Then, a guanosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% $TCA/CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephenylphosphonamidite unit and 0.4 M tetrazole;

(5) An oxidation reaction was conducted for 30 seconds using 0.1 M iodine/water/pyridine/THF;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine phenylphosphonate compound could be condensed with the cytidine compound introduced into CPG. A subsequent nucleotide sequence of a guanosine phenylphosphonate compound was bound thereto by repetition of operations similar to those described above. A further subsequent nucleotide sequence of thymidine phosphate was bound thereto. In this case, a thymidine-phosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected guanosine phenylphosphonate compound. This solution was stored to calculate the condensation yield; and

(4) Condensation reaction was conducted for 5 minutes using the 0.2 M thymidineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the reaction column, and reacted with an ammonia/pyridine (5:1) solution at 60 °C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphate antisense oligonucleotide in which phenylphosphonate bonds were introduced into each of the 5'- and 3'- two terminal mononucleotides: $G_{Ph}G_{Ph}$TTTTTTTTTTTTT$G_{Ph}G_{Ph}$G was synthesized.

Yield: 37.0 OD

λmax: 263.7 nm

λmin: 232.5 nm

[Example 60]

Synthesis of Phosphonate Antisense Oligonucleotide into which Phenylphosphonate Bonds are introduced $A_{Ph}A_{Ph}$TTTTTTTTTTTTT$A_{Ph}A_{Ph}$A (SEQ ID NO: 48)

50 mg (1 micromol) of CPG into which 20 micromols/g of an adenosine compound was introduced, the N-6 amino group and the 5'-hydroxyl group of said adenosine compound being protected by a benzoyl group and a DMTr group, respectively, was packed into a reaction column mounted on an automatic synthesizer (DNA Synthesizer Model 381 A, ABI). Then, an adenosinephenylphosphonamidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile;

(3) CPG was dried with an argon gas;

(4) Condensation reaction was conducted for 5 minutes using the 0.2 M guanosinephenylphosphonamidite unit and 0.4 M tetrazole;

(5) An oxidation reaction was conducted for 30 seconds using 0.1 M iodine/water/pyridine/THF;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected adenosine phenylphosphonate compound could be condensed with the adenosine compound introduced into CPG. A subsequent nucleotide sequence of an adenosine phenylphosphonate compound was bound thereto by repetition of operations similar to those described above. A further subsequent nucleotide sequence of thymidine phosphate was bound thereto. In this case, a thymidine-phosphoramidite unit was dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-Pix group of the protected guanosine phenylphosphonate compound. This solution was stored to calculate the condensation yield; and

(4) Condensation reaction was conducted for 5 minutes using the 0.2 M thymidineamidite unit and 0.4 M tetrazole.

The nucleotide chain was successively extended by repetition of the above-mentioned operations. Trityl alcohol released by the operation (1) was colorimetrically determined, and the average yield of each condensation reaction calculated from the results was 99% or more. Deprotecting operations after completion of the synthesis reaction were conducted according to methods known in the art [Oligonucleotide Synthesis; A Practical Approach, edited by M. J. Gait, IRL PRESS, (1984)]. CPG was taken out of the

reaction column, and reacted with an ammonia/pyridine (5:1) solution at 60 ° C for 6 hours, thereby excising the nucleotide chain from the solid phase carrier and removing the protective group. Then, the product was purified by the use of reverse phase HPLC (µBondasphere $C_{18}$). By the above-mentioned operations, a desired phosphate antisense oligonucleotide in which phenylphosphonate bonds were introduced into each of the 5'- and 3'- two terminal mononucleotides: $A_{Ph}A_{Ph}$TTTTTTTTTTTTT$A_{Ph}A_{Ph}$A was synthesized.

Yield: 77.5 OD
λmax: 263.6 nm
λmin: 233.8 nm

[Example 61]

Synthesis of 1,3-O-Bis(2,3,4,6-tetraacetylgalactosyl)-2-O-benzylglycerol

2-O-benzylglycerol (182.2 mg, 1.00 mmol) and 1-O-fluoro-2,3,4,6-O-tetraacetylgalactose (770.6 mg, 2.20 mmols) were dissolved in anhydrous methylene chloride (5 ml), and trifluoroborane etherate ($BF_3$-$Et_2O$)-(425.8 mg, 3.00 mmols) and a molecular sieve (1.0 g) were added to this solution. The resulting reaction solution was stirred at room temperature for 3 hours, followed by filtration using Celite. The filtrate was diluted by adding 50 ml of methylene chloride, and the resulting solution was washed with three 50 ml portions of a saturated aqueous solution of sodium hydrogencarbonate, followed by removal of the solvent by distillation under reduced pressure. Then, the resulting reaction product was purified by silica gel column chromatography (hexane/ethyl acetate = 8:2) to obtain a desired product, 1,3-O-bis(2,3,4,6-tetraacetylgalactosyl)-2-O-benzylglycerol.

[Example 62]

Synthesis of 3-O-Bis(2.3,4,6-tetraacetylgalactosyl)glycerol

1,3-O-Bis(2,3,4,6-tetraacetylgalactosyl)-2-O-benzylglycerol (842.8 mg, 1.00 mmol) was dissolved in methanolethyl acetate (1:1) (10 ml), and 10% $Pd(OH)_2$ (30 mg) was added thereto. The resulting solution was subjected to medium-pressure hydrogenation (3.5 $kg/cm^2$) at room temperature, followed by reaction for 10 hours. After completion of reaction, filtration was conducted using Celite. The filtrate was diluted by adding 50 ml of methylene chloride, and the resulting solution was washed with three 50 ml portions of a saturated aqueous solution of sodium hydrogencarbonate, followed by removal of the solvent by distillation under reduced pressure. Then, the resulting reaction product was purified by silica gel column chromatography (hexane/ethyl acetate = 8:2) to obtain a desired product, 1,3-O-bis(2,3,4,6-tetraacetylgalactosyl)-glycerol.

[Example 63]

Synthesis of 1,3-O-Bis(2,3,4,6-tetraacetylgalactosyl)-2-O-(N,N-diisopropylamino-O-cyanoethyl-phosphoramidite)glycerol

1,3-O-Bis(2,3,4,6-tetraacetylgalactosyl)glycerol (752.7 mg, 1.00 mmol) was dehydrated with three 2 ml portions of anhydrous pyridine by azeotropic distillation, and further dehydrated with three 2 ml portions of anhydrous toluene and with three 2 ml portions of anhydrous methylene chloride by azeotropic distillation. Then, the product thus dehydrated was dissolved in 5 ml of methylene chloride. Diisopropylethylamine (0.87 ml, 5.00 mmols) and N,N-diisopropylamino-O-cyanoethylphosphorochloride (0.45 ml, 2.00 mmols) were added to this solution, followed by reaction at room temperature for 1 hour. Then, 50 ml of methylene chloride was added thereto for dilution. The resulting solution was washed with three 50 ml portions of a saturated aqueous solution of sodium hydrogencarbonate, and thereafter dried on anhydrous sodium sulfate. After removal of the solvent by distillation under reduced pressure, the resulting product was purified by silica gel column chromatography (hexane/methylene chloride = 8:2, 2% triethylamine) to obtain a desired product, 1,3-O-bis(2,3,4,6-tetraacetylgalactosyl)-2-O-(N,N-diisopropyl amino-O-cyanoethyl-phosphoramidite)glycerol.

[Example 64]

Synthesis of Phosphorothioate Antisense Oigonucleotide to Which 1,3-O-Bisgalactosylglycerol Was Bound: $((Gal)_2$Glyc)-TsGsGsGsAsGsCsCsAsTsAsGsCsGsAsGsGsC (SEQ ID NO: 31)

50 mg (1 micromol) of CPG into which 20 micromols/g of cytidine derivative was introduced, the N-4 amino group and the 5'-hydroxyl group of said cytidine derivative being protected by a benzoyl group and a DMTr group, respectively, was packed into a reaction column mounted on an automatic synthesizer (DNA Synthesizer Model 381 A, ABI). Then, a guanosinephosphoramidite unit was dissolved in anhydrous acetonitrile to give a concentration of 0.2 M, and the resulting solution was filled into a unit storage bottle, which was mounted on the automatic synthesizer. Then, the following operations were carried out in the reaction column by the use of the automatic synthesizer:

(1) Treatment with 3.0% TCA/$CH_2Cl_2$ was conducted for 60 seconds to remove the 5'-DMTr group. This solution was stored to calculate the condensation yield;

(2) CPG was washed with acetonitrile; (3) CPG was dried with an argon gas;

(4) Condensation reaction was conducted for 5 minutes using the 0.2 M guanosinephosphoramidite unit and 0.4 M tetrazole;

(5) Sulfidizing reaction was conducted for 15 minutes using 0.5 M tetraethylthiuram disulfide/acetonitrile;

(6) A 0.25 M acetic anhydride/1-methylimidazole/lutidine/tetrahydrofuran solution was reacted for 30 seconds to block the unreacted 5'-hydroxyl group; and

(7) Washing with acetonitrile and drying with an argon gas were carried out.

According to the above-mentioned operations, the protected guanosine derivative could be condensed with the cytidine derivative introduced into CPG, by a phosphorothioate bond. A subsequent nucleotide sequence of guanosine derivative was bound thereto. In this case, a guanosinephosphoramidite unit was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-DMTr group of the protected guanosine derivative. This solution was stored to calculate the condensation yield; and

(4) Condensation reaction was conducted for 5 minutes using the 0.2 M guanosinephosphoramidite unit and 0.4 M tetrazole.

The nucleotide chain was successively extended by repetition of the above-mentioned operations. After condensation of thymidylic acid as the 18th nucleotide, the galactosylglycerol derivative synthesized in Example 63 was bound to the 5'-terminus. In this case, the galactosylglycerol derivative was similarly dissolved to give a concentration of 0.2 M, and the solution was loaded on the automatic synthesizer, followed by repetition of the above-mentioned operations (1) to (7) with the exception that the operations (1) and (4) were conducted as follows:

(1) Treatment with 3.0% TCA/$CH_2Cl_2$ was conducted for 60 seconds to remove the 5'-DMTr group of the protected thymidine derivative; and

(4) Condensation reaction was conducted for 5 minutes using the 0.2 M galactosylglycerol-phosphoramidite unit synthesized in Example 63 and 0.4 M tetrazole.

By the above-mentioned operations, the galactosylglycerol derivative could be introduced into the 5'-terminus of the antisense oligonucleotide. Deprotecting operations after completion of synthesis reaction were conducted according to methods known in the art [Oligonucleotide Synthesis; A Practical Approach, edited by M. J. Gait, IRL PRESS, (1984)]. CPG was taken out of the reaction column, and reacted in an ammonia/pyridine (5:1) solution at 60°C for 6 hours, thereby excising the nucleotide chain from the solid phase carrier and removing the protective group. Then, the product was purified by the use of reverse phase HPLC (μBondasphere $C_{18}$). By the above-mentioned operations, a desired phosphorothioate antisense oligonucleotide to which galactosylglycerol was bound: $((Gal)_2$Glyc)-TsGsGsGsAsGsCsCsAsTsAsG-sCsGsAsGsGsC was synthesized.

The saccharide compounds and the oligonucleotide compounds synthesized by the above Reference Examples and the working Examples are summarized as follows:

[Reference Example 1]

$T_sG_sG_sG_sA_sG_sC_sC_sA_sT_sA_sG_sC_sG_sA_sG_sG_sC$ (SEQ ID NO: 31)

[Reference Example 2]

$C_sG_sG_sA_sG_sC_sG_sA_sT_sA_sC_sC_sG_sA_sG_sG_sG_sT$ (SEQ ID NO:36)

[Reference Example 3]

(N-BzGalPO)-$C_sG_sG_sA_sG_sC_sG_sA_sT_sA_sC_sC_sG_sA_sG_sG_sG_sT$ (N-BzGalPO)-SEQ ID NO: 36)

[Reference Example 4]
$C_{Me}G_{Me}GAGCGATACCGAGG_{Me}G_{Me}T$ (SEQ ID NO: 38)
[Reference Example 5]
$T_{Me}G_{Me}GGAGCCATAGCGAG_{Me}G_{Me}C$ (SEQ ID NO: 34)
[Example 1]
1,2,3,4-O-Tetraacetylgalactose-6-O-N,N-diisopropylamino-O-cyanoethylphosphoramidite
[Example 2]
1,2,3,4-O-Tetraacetylmannose-6-O-N,N-diisopropylamino-O-cyanoethylphosphoramidite
[Example 3]
(GAL)-$T_sG_sG_sG_sA_sG_sC_sC_sA_sT_sA_sG_sC_sG_sA_sG_sG_sC$ ((GAL)-SEQ ID NO: 31)
[Example 4]
(MAN)-$T_sG_sG_sG_sA_sG_sC_sC_sA_sT_sA_sG_sC_sG_sA_sG_sG_sC$ ((MAN)-SEQ ID NO: 31)
[Example 5]
1-O-n-Octyl-2,3,4-O-tribenzoylglucoside-6-O-N,N-diisopropylamino-O-cyanoethylphosphoramidite
[Example 6]
(n-oct-Glc)-$T_sG_sG_sG_sA_sG_sC_sC_sA_sT_sA_sG_sC_sG_sA_sG_sG_sC$ ((n-oct-Glc)-SEQ ID NO: 31)
[Example 7]
1-O-n-Octyl-2,3,4-O-tribenzoylthioglucoside-6-O-N,N-diisopropylamino-O-cyanoethylphosphoramidite
[Example 8]
(n-oct-SGlc)-$T_sG_sG_sG_sA_sG_sC_sC_sA_sT_sA_sG_sC_sG_sA_sG_sG_sC$ ((n-oct-SGlc)-SEQ ID NO: 31)
[Example 9]
1-O-n-Octyl-2,3,4-O-tribenzoylglucoside-6-O-(N,N-diisopropylamino)phenylphosphonoamidite
[Example 10]
(n-oct-GlcPh)-$T_sG_sG_sG_sA_sG_sC_sC_sA_sT_sA_sG_sC_sG_sA_sG_sG_sC$ ((n-oct-GlcPh)-SEQ ID NO: 31)
[Example 11]
1-O-n-Octyl-2,3,4-O-tribenzoylthioglucoside-6-O-(N,N-diisopropylamino)phenylphosphonoamidite
[Example 12]
(n-oct-SGlcPh)-$T_sG_sG_sG_sA_sG_sC_sC_sA_sT_sA_sG_sC_sG_sA_sG_sG_sC$ ((n-oct-SGlcPh)-SEQ ID NO: 31)
[Example 13]
1-O-phenyl-2,3,4-O-tribenzoylglucoside-6-O-N,N-diisopropylamino-O-cyanoethylphosphoramidite
[Example 14]
(PhGlc)-$T_sG_sG_sG_sA_sG_sC_sC_sA_sT_sA_sG_sC_sG_sA_sG_sG_sC$ ((PhGlc)-SEQ ID NO: 31)
[Example 15]
1-O-phenyl-2,3,4-O-tribenzoylgalactoside-6-O-N,N-diisopropylamino-O-cyanoethylphosphoramidite
[Example 16]
(PhGal)-$T_sG_sG_sG_sA_sG_sC_sC_sA_sT_sA_sG_sC_sG_sA_sG_sG_sC$ ((PhGal)-SEQ ID NO: 31)
[Example 17]
1,3,4-O-Tribenzoyl,2-N-benzoyl-galactosamine-6-O-N,N-diisopropylamino-O-cyanoethyl-phosphoramidite
[Example 18]
(N-BzGalPO)-$T_sG_sG_sG_sA_sG_sC_sC_sA_sT_sA_sG_sC_sG_sA_sG_sG_sC$ ((N-BzGalPO)-SEQ ID NO: 31)
[Example 19]
1,3,4-O-Triacetyl,2-N-acetyl-galactosamine-6-O-N,N-diisopropylamino-O-cyanoethyl-phosphoramidite
[Example 20]
(N-AcGal)-$T_sG_sG_sG_sA_sG_sC_sC_sA_sT_sA_sG_sC_sG_sA_sG_sG_sC$ ((N-AcGal)-SEQ ID NO: 31)
[Example 21]
1,3,4-O-Triacetyl,2-N-acetyl-glucosamine-6-O-N,N-diisopropylamino-O-cyanoethyl-phosphoramidite
[Example 22]
(N-AcGul)-$T_sG_sG_sG_sA_sG_sC_sC_sA_sT_sA_sG_sC_sG_sA_sG_sG_sC$ ((N-AcGul)-SEQ ID NO: 31)
[Example 23]
1,3,4-O-Tribenzoyl,2-N-trifluoroacetyl-galactosamine-6-O-N,N-diisopropylamino-O-cyanoethylphosphoramidite
[Example 24]
(GalN)-$T_sG_sG_sG_sA_sG_sC_sC_sA_sT_sA_sG_sC_sG_sA_sG_sG_sC$ ((GalN)-SEQ ID NO: 31)
[Example 25]
1,3,4-O-Tribenzoyl,2-N-trifluoroacetyl-glucosamine-6-O-N,N-diisopropylamino-O-cyanoethyl-phosphoramidite
[Example 26]
(GulN)-$T_sG_sG_sG_sA_sG_sC_sC_sA_sT_sA_sG_sC_sG_sA_sG_sG_sC$ ((GulN)-SEQ ID NO: 31)

[Example 27]
Heptabenzoylmelibiose-N,N-diisopropylamino-O-cyanoethylphosphoramidite
[Example 28]
(Mel)-$T_sG_sG_sG_sA_sG_sC_sC_sA_sT_sA_sG_sC_sG_sA_sG_sG_sC$ ((Mel)-SEQ ID NO: 31)
[Example 29]
Heptabenzoylgentiobiose-N,N-diisopropylamino-O-cyanoethylphosphoramidite
[Example 30]
(Gen)-$T_sG_sG_sG_sA_sG_sC_sC_sA_sT_sA_sG_sC_sG_sA_sG_sG_sC$ ((Gen)-SEQ ID NO: 31)
[Example 31]
Octabenzoylisomaltotriose-N,N-diisopropylamino-O-cyanoethylphosphoramidite
[Example 32]
(Iso)-$T_sG_sG_sG_sA_sG_sC_sC_sA_sT_sA_sG_sC_sG_sA_sG_sG_sC$ ((Iso)-SEQ ID NO: 31)
[Example 33]
$T_{Ph}G_{Ph}$GGAGCCATAGCGA$G_{Ph}G_{Ph}$C (SEQ ID NO: 32)
[Example 34]
(Gen)-$T_{Ph}G_{Ph}$GGAGCCATAGCGA$G_{Ph}G_{Ph}$C ((Gen)-SEQ ID NO: 32)
[Example 35]
(Mel)-$T_{Ph}G_{Ph}$GGAGCCATAGCGA$G_{Ph}G_{Ph}$C ((Mel)-SEQ ID NO: 32)
[Example 36]
$T_{Ph}$GGGAG$C_{Ph}C_{Ph}$A$T_{Ph}$AG$C_{Ph}$GAGG$C_{Ph}$T (SEQ ID NO: 33)
[Example 37]
(Gen)-$T_{Ph}$GGGAG$C_{Ph}C_{Ph}$A$T_{Ph}$AG$C_{Ph}$GAGG$C_{Ph}$T ((Gen)-SEQ ID NO: 33)
[Example 38]
(Gen)-$T_{Me}G_{Me}$GGAGCCATAGCGA$G_{Me}G_{Me}$C ((Gen)-SEQ ID NO: 34)
[Example 39]
(Mel)-$T_{Me}G_{Me}$GGAGCCATAGCGA$G_{Me}G_{Me}$C ((Mel)-SEQ ID NO: 34)
[Example 40]
(N-BzGal)-$T_{Ph}G_{Ph}$GGAGCCATAGCGA$G_{Ph}G_{Ph}$C ((N-BzGal)-SEQ ID NO: 32)
[Example 41]
$T_{Ph}$GGGAGCCATAGCGAG$G_{Ph}$C (SEQ ID NO: 35)
[Example 42]
(Gen)-$T_{Ph}$GGGAGCCATAGCGAG$G_{Ph}$C ((Gen)-SEQ ID NO: 35)
[Example 43]
(N-BzGal)-$T_{Ph}$GGGAGCCATAGCGAG$G_{Ph}$C ((N-BzGal)-SEQ ID NO: 35)
[Example 44]
$C_{Ph}G_{Ph}$GAGCGATACCGAG$G_{Ph}G_{Ph}$T (SEQ ID NO: 37)
[Example 45]
(Gen)-$C_{Ph}G_{Ph}$GAGCGATACCGAG$G_{Ph}G_{Ph}$T ((Gen)-SEQ ID NO: 37)
[Example 46]
(Mel)-$C_{Ph}G_{Ph}$GAGCGATACCGAG$G_{Ph}G_{Ph}$T ((Mel)-SEQ ID NO: 37)
[Example 47]
$T_{Ph}G_{Ph}$GGAG$C_{Ph}C_{Ph}$A$T_{Ph}$AG$C_{Ph}$GA$G_{Ph}G_{Ph}$C (SEQ ID NO: 39)
[Example 48]
(Gen)-$T_{Ph}G_{Ph}$GGAG$C_{Ph}C_{Ph}$A$T_{Ph}$AG$C_{Ph}$GA$G_{Ph}G_{Ph}$C ((Gen)-SEQ ID NO: 39)
[Example 49]
$C_{Ph}G_{Ph}$GAG$C_{Ph}$GA$T_{Ph}$A$C_{Ph}C_{Ph}$GAG$G_{Ph}G_{Ph}$T (SEQ ID NO: 40)
[Example 50]
$C_{Ph}C_{Ph}$CCCACCACTTCC$C_{Ph}C_{Ph}$T (SEQ ID NO: 41)
[Example 51]
(Gen)-$C_{Ph}C_{Ph}$CCCACCACTTCC$C_{Ph}C_{Ph}$T ((Gen)-SEQ ID NO: 41)
[Example 52]
$C_{Ph}C_{Ph}$CTGTCCCGGGATA$G_{Ph}G_{Ph}$T (SEQ ID NO: 42)
[Example 53]
(Gen)-$C_{Ph}C_{Ph}$CTGTCCCGGGATA$G_{Ph}G_{Ph}$T ((Gen)-SEQ ID NO: 42)
[Example 54]
$T_{Ph}G_{Ph}G_{Ph}G_{Ph}A_{Ph}G_{Ph}C_{Ph}C_{Ph}A_{Ph}T_{Ph}A_{Ph}G_{Ph}C_{Ph}G_{Ph}A_{Ph}G_{Ph}G_{Ph}C$ (SEQ ID NO: 43)
[Example 55]
(Gen)-$T_{Ph}G_{Ph}G_{Ph}G_{Ph}A_{Ph}G_{Ph}C_{Ph}C_{Ph}A_{Ph}T_{Ph}A_{Ph}G_{Ph}C_{Ph}G_{Ph}A_{Ph}G_{Ph}G_{Ph}C$ ((Gen)-SEQ ID NO: 43)

[Example 56]
$G_{Ph}G_{Ph}$AGCCATAGCGAG$_{Ph}$G$_{Ph}$C (SEQ ID NO: 44)
[Example 57]
$A_{Ph}G_{Ph}$CCATAGCG$_{Ph}$A$_{Ph}$G (SEQ ID NO: 45)
[Example 58]
$A_{Ph}G_{Ph}$CCATA$_{Ph}$G$_{Ph}$C (SEQ ID NO: 46)
[Example 59]
$G_{Ph}G_{Ph}$TTTTTTTTTTTTTG$_{Ph}$G$_{Ph}$G (SEQ ID NO: 47)
[Example 60]
$A_{Ph}A_{Ph}$TTTTTTTTTTTTTA$_{Ph}$A$_{Ph}$A (SEQ ID NO: 48)
[Example 61]
1,3-O-Bis(2,3,4,6-tetraacetylgalactosyl)-2-O-benzylglycerol
[Example 62]
3-O-Bis(2.3,4,6-tetraacetylgalactosyl)-glycerol
[Example 63]
1,3-O-Bis(2,3,4,6-tetraacetylgalactosyl)-2-O-(N,N-diisopropylamino-O-cyanoethylphosphoramidite)-glycerol
[Example 64]
$((Gal)_2$Glyc)-TsGsGsGsAsGsCsCsAsTsAsGsCsGsAsGsGsC [$((Gal)_2$-Glyc)-SEQ ID NO:31]

[Test Example 1]

The ICAM-1 (intercellular adhesion molecule 1) expression inhibition activity of the phosphorothioate antisense compounds of the present invention listed below in Table 1 encapsulated in cationic lipofectin (DOTMA), the unmodified phosphorothioate antisense type oligonucleotides having homologous nucleotide sequences similarly encapsulated in DOTMA, and the corresponding reverse type nucleotide sequence oligonucleotides, targeted to a nucleotide sequence in the vicinity of the translation initiation site of pre-mRNA of the cell adhesive protein ICAM-1 expression gene was assayed.

The ICAM-1 expression inhibition activity was assayed according to conventional methods [M. Y. Chiang et al, J. Biol. Chem., 266, 18162 (1991) incorporated herein by reference]. Namely, A549 cells (human lung cancer-derived cells) were washed with a serum-free medium (High Glucose D-MEM) three times, and then added in an amount of 100 µl/well to a serum-free medium supplemented with an appropriate amount (1.00 µM or 0.33 µM) of the phosphorothioate antisense compound of the present invention and 10 µg/ml of lipofectin (DOTMA), followed by cultivation at 37°C for 4 hours. Subsequently, the medium was replaced with a 10% serum-containing medium supplemented with the same amount of the antisense compound, and cultivation was further continued at 37°C for 4 hours. Then, hILI-$\beta$ (to a final concentration of 0.1 ng/ml) or hTNF-$\alpha$ (to a final concentration of 1 ng/ml) was added to induce expression of ICAM-1. After cultivation for 15 hours, the amount of ICAM-1 expressed was measured by the ICAM-1 Cell EIA to assay the ICAM-1 expression inhibition activity of the phosphorothioate antisense compound of the present invention encapsulated in lipofectin. At the same time, evaluation of cell inhibition was carried out by crystal violet staining. The ICAM relative inhibition of the phosphorothioate antisense compounds of the present invention encapsulated in lipofectin when compared to the activity of the unmodified phosphorothioate antisense compounds encapsulated in lipofectin is taken as 100 and the cell inhibition are shown in Table 1 as the ICAM-1 inhibition activity.

Table 1

| ICAM-1 Inhibition Activity (DOTMA Concentration: 10 µg/ml) | | | |
|---|---|---|---|
| Example No. | DNA Conc. (µM) | ICAM-1 Relative Inhibition (%) | Cell Inhibition (%) |
| 10 | 1.00 | 104.2 | 12.7 |
| 12 | 0.33 | 108.9 | 12.3 |
| 14 | 0.33 | 122.9 | 15.6 |
| 16 | 1.00 | 106.3 | 14.9 |
| | 0.33 | 140.9 | 15.3 |
| 18 | 0.33 | 112.6 | -5.1 |
| 20 | 1.00 | 112.1 | 9.6 |
| 22 | 1.00 | 123.0 | 20.6 |
| | 0.33 | 158.0 | 13.2 |
| 24 | 1.00 | 104.7 | 8.7 |
| | 0.33 | 162.2 | 7.4 |
| 26 | 1.00 | 106.7 | 10.9 |
| | 0.33 | 146.9 | 1.1 |
| 32 | 1.00 | 102.9 | 41.1 |
| | 0.33 | 108.2 | 26.7 |
| Reference Example No. | | | |
| 2 | 1.00 | -8.2 | 13.5 |
| | 0.33 | -0.9 | 19.7 |

It was observed that all the phosphorothioate antisense oligonucleotides of the present invention, when encapsulated in lipofectin, had an ICAM-1 expression inhibition effect nearly equal to or higher than the unmodified phosphorothioate antisense compounds having the same nucleotide sequences encapsulated in lipofectin. The results proved that the antisense compounds wherein the anomeric saccharide hydroxyl group was substituted by the phenyl group was bound as shown in Example 16 and the antisense compounds into which the 2-amino saccharide compounds were introduced as shown in Examples 18, 20, 22, 24 and 26 were enhanced in inhibition effect. These antisense compounds were similar to the unmodified ones in cell toxicity. An inhibition effect was scarcely observed in the phosphorothioate oligonucleotide having the reverse sequence shown in Reference Example 2. These facts revealed that the antisense effect was enhanced by modifying the 5'-terminuses of the antisense compounds with the saccharide compounds.

[Test Example 2]

The ICAM-1 expression inhibition activity of the phosphorothioate antisense compounds of the present invention, the unmodified phosphorothioate antisense compounds and the corresponding reverse type nucleotide sequence oligonucleotides, targeted to the vicinity of a translation initiation site of pre-mRNA of a cell adhesive protein ICAM-1 (intercellular adhesion molecule 1) expression gene was assayed.

The ICAM-1 expression inhibition activity was assayed according to a conventional method [M. Y. Chiang et al, J. Biol. Chem., 266, 18162 (1991)]. Namely, A549 cells (human lung cancer-derived cells) were washed with a serum-free medium (High Glucose D-MEM) three times, and then added in an amount of 100 µl/well to a serum-free medium supplemented with an appropriate amount (20.0 µM or 30.0 µM) of the phosphorothioate antisense compound of the present invention, followed by cultivation at 37°C for 4 hours. Subsequently, the medium was replaced with a 10% serum-containing medium supplemented with the same amount of the antisense compound, and cultivation was further continued at 37°C for 4 hours. Then, hILI-$\beta$ (to a final concentration of 0.1 ng/ml) or hTNF-$\alpha$ (to a final concentration of 1 ng/ml) was added to induce expression of ICAM-1. After cultivation for 15 hours, the amount of ICAM-1 expressed was measured by the ICAM-1 Cell EIA to assay the ICAM-1 expression inhibition activity of the phosphorothioate antisense compound of the present invention. At the same time, evaluation of cell inhibition was carried out by crystal violet staining. The ICAM-1 inhibition of the phosphorothioate antisense compounds of the present invention not encapsulated in lipofectin and their cell inhibition are shown in Table 2.

Table 2

| ICAM-1 Inhibition Activity | | | |
|---|---|---|---|
| Example No. | DNA Conc. (μM) | ICAM-1 Relative Inhibition (%) | Cell Inhibition (%) |
| 18 | 30.0 | 33.5 | 4.9 |
| Reference Example No. | | | |
| 1 | 30.0 | -5.1 | 5.5 |
| 3 | 30.0 | -9.6 | 5.9 |

When not encapsulated in lipofectin, the unmodified phosphorothioate antisense compounds exhibited little ICAM expression inhibition effect. However, the ICAM-1 expression inhibition effect was significantly observed in the phosphorothioate antisense compound to which N-benzoylgalactosamine was bound as shown in Example 18. This antisense compound was similar to the unmodified one in cell toxicity. An inhibition effect was scarcely observed in the phosphorothioate oligonucleotide having the reverse sequence, to which N-benzoylgalactosamine was bound, shown in Reference Example 3. These facts revealed that the antisense effect was enhanced by modifying the 5'-terminus of the antisense compound with N-benzoylgalactosamine.

[Test Example 3]

The ICAM-1 expression inhibition activity of the phosphate antisense oligonucleotides of the present invention partially having phosphonate bonds, the unmodified phosphorothioate antisense oligonucleotides having homologous nucleotide sequences and the corresponding reverse type nucleotide sequence oligonucleotides, targeted to the vicinity of a translation initiation site of pre-mRNA of a cell adhesive protein ICAM-1 (intercellular adhesion molecule 1) expression gene was assayed.

The ICAM-1 expression inhibition activity was assayed according to a conventional method [M. Y. Chiang et al, J. Biol. Chem., 266, 18162 (1991)]. Namely, A549 cells (human lung cancer-derived cells) were washed with a serum-free medium (High Glucose D-MEM) three times, and then added in an amount of 100 μl/well to a serum-free medium supplemented with 30.0 μM of the antisense compound of the present invention, followed by cultivation at 37°C for 4 hours. Subsequently, the medium was replaced with a 10% serum-containing medium supplemented with the same amount of the antisense compound, and cultivation was further continued at 37°C for 4 hours. Then, hILI-β (to a final concentration of 0.1 ng/ml) or hTNF-α (to a final concentration of 1 ng/ml) was added to induce expression of ICAM-1. After cultivation for 15 hours, the amount of ICAM-1 expressed was measured by the ICAM-1 Cell EIA to assay the ICAM-1 expression inhibition activity of the phosphate antisense compound of the present invention partially having phosphonate bonds. At the same time, evaluation of cell inhibition was carried out by crystal violet staining. The ICAM-1 inhibition of the antisense compounds of the present invention not encapsulated in lipofectin and the cell inhibition are shown in Table 3.

Table 3

| ICAM-1 Inhibition Activity | | | |
|---|---|---|---|
| Example No. | DNA Conc. (μM) | ICAM-1 Relative Inhibition (%) | Cell Inhibition (%) |
| 33 | 30.0 | 36.3 | 0.1 |
| 34 | 30.0 | 57.1 | 9.9 |
| 35 | 30.0 | 39.2 | 3.3 |
| 44 | 30.0 | 43.5 | 4.3 |
| 45 | 30.0 | 48.5 | 3.5 |
| 46 | 30.0 | 41.3 | 3.6 |
| 54 | 30.0 | 24.3 | 17.3 |
| 55 | 30.0 | 21.1 | 10.8 |
| 56 | 30.0 | 18.1 | -1.4 |
| 59 | 30.0 | 28.2 | -20.7 |
| Reference Example No. | | | |
| 1 | 30.0 | 8.8 | 9.3 |

When not encapsulated in lipofectin, the unmodified phosphorothioate antisense compounds exhibited little ICAM expression inhibition effect. However, an ICAM-1 expression inhibition effect was significantly observed in the phosphate antisense compounds partially having phenylphosphonate bonds as shown in Examples 33 to 35, 44 to 46, 54 and 55 of the present invention. In this case, little cell toxicity was observed. This revealed that the phenylphosphonate bonds partially introduced contributed to the inhibition effect against ICAM-1 expression.

The oligonucleotides (I) of the present invention or the salts thereof can (1) inhibit expression of genes associated with the development of malignant tumors in malignant tumor cells, (2) inhibit expression of viral genes derived from viruses, (3) inhibit expression of genes producing proteins which contribute to the development of inflammations, (4) control expression of drug-resistant genes which can cause complications in chemotherapy of malignant tumors, and (5) inhibit production of cell growth factors related to reangiostenosis after PTCA, by complementation to DNAs or mRNAs of genes inducing various diseases. They can therefore be used as antitumor agents, antiviral agents, antiinflammatory agents and drugs for controlling expression of specific genes such as resistance genes. In addition, because they bind to a target DNA or mRNA with greater efficiency than standard DNA segments, they effectively can be used as probes. These compounds can also be used in drug assay screens.

SEQUENCE LISTING

(1) GENERAL INFORMATION:

(i) APPLICANT:
(A) NAME: Takeda Chemical Industries, Ltd.
(B) STREET: 1-1 Doshomachi 4-chome
(C) CITY: Chuo-ku, Osaka
(E) COUNTRY: Japan
(F) POSTAL CODE (ZIP): 541

(ii) TITLE OF INVENTION: OLIGONUCLEOTIDE COMPOUNDS, THEIR PRODUCTION AND USE

(iii) NUMBER OF SEQUENCES: 55

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO:1:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:1:

TGGGAGCCAT AGCGAGGC 18

(2) INFORMATION FOR SEQ ID NO:2:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:2:

TGGGAGCCAT AGCGAGGCT 19

(2) INFORMATION FOR SEQ ID NO:3:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:3:

GGCTGCTGGG AGCCATAGCG AGGCTGAGGT 30

(2) INFORMATION FOR SEQ ID NO:4:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:4:

GGCTGCTGGG AGCCATAGCG AGGCTGAGG 29

(2) INFORMATION FOR SEQ ID NO:5:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:5:

GGCTGCTGGG AGCCATAGCG AGGCTGAG 28

(2) INFORMATION FOR SEQ ID NO:6:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:6:

GGCTGCTGGG AGCCATAGCG AGGCTGA 27

(2) INFORMATION FOR SEQ ID NO:7:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:7:

GGCTGCTGGG AGCCATAGCG AGGCTG 26

(2) INFORMATION FOR SEQ ID NO:8:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:8:

GGCTGCTGGG AGCCATAGCG AGGCT 25

(2) INFORMATION FOR SEQ ID NO:9:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:9:

GGCTGCTGGG AGCCATAGCG AGGC 24

(2) INFORMATION FOR SEQ ID NO:10:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:10:

GGCTGCTGGG AGCCATAGCG AGG 23

(2) INFORMATION FOR SEQ ID NO:11:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:11:

GGCTGCTGGG AGCCATAGCG AG 22

(2) INFORMATION FOR SEQ ID NO:12:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:12:

GGCTGCTGGG AGCCATAGCG A 21

(2) INFORMATION FOR SEQ ID NO:13:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:13:

GGCTGCTGGG AGCCATAGCG 20

(2) INFORMATION FOR SEQ ID NO:14:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:14:

GGCTGCTGGG AGCCATAGC 19

(2) INFORMATION FOR SEQ ID NO:15:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:15:

GGCTGCTGGG AGCCATAG 18

(2) INFORMATION FOR SEQ ID NO:16:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:16:

GGCTGCTGGG AGCCATA 17

(2) INFORMATION FOR SEQ ID NO:17:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:17:

GGCTGCTGGG AGCCAT 16

(2) INFORMATION FOR SEQ ID NO:18:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:18:

GCTGCTGGGA GCCATAGCGA GGCTGAGGT 29

(2) INFORMATION FOR SEQ ID NO:19:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:19:

CTGCTGGGAG CCATAGCGAG GCTGAGGT 28

(2) INFORMATION FOR SEQ ID NO:20:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:20:

TGCTGGGAGC CATAGCGAGG CTGAGGT 27

(2) INFORMATION FOR SEQ ID NO:21:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:21:

GCTGGGAGCC ATAGCGAGGC TGAGGT 26

(2) INFORMATION FOR SEQ ID NO:22:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:22:

CTGGGAGCCA TAGCGAGGCT GAGGT 25

(2) INFORMATION FOR SEQ ID NO:23:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:23:

TGGGAGCCAT AGCGAGGCTG AGGT 24

(2) INFORMATION FOR SEQ ID NO:24:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:24:

GGGAGCCATA GCGAGGCTGA GGT 23

(2) INFORMATION FOR SEQ ID NO:25:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:25:

GGAGCCATAG CGAGGCTGAG GT 22

(2) INFORMATION FOR SEQ ID NO:26:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:26:

GAGCCATAGC GAGGCTGAGG T 21

(2) INFORMATION FOR SEQ ID NO:27:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:27:

AGCCATAGCG AGGCTGAGGT 20

(2) INFORMATION FOR SEQ ID NO:28:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:28:

GCCATAGCGA GGCTGAGGT 19

(2) INFORMATION FOR SEQ ID NO:29:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:29:

CCATAGCGAG GCTGAGGT 18

(2) INFORMATION FOR SEQ ID NO:30:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:30:

CATAGCGAGG CTGAGGT 17

(2) INFORMATION FOR SEQ ID NO:31:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:31:

NNNNNNNNNN NNNNNNNC 18

(2) INFORMATION FOR SEQ ID NO:32:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:32:

NNGGAGCCAT AGCGANNC 18

(2) INFORMATION FOR SEQ ID NO:33:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:33:

NGGGAGNNAN AGNGAGGNT 19

(2) INFORMATION FOR SEQ ID NO:34:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:34:

NNGGAGCCAT AGCGANNC 18

(2) INFORMATION FOR SEQ ID NO:35:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:35:

NGGGAGCCAT AGCGAGNC 18

(2) INFORMATION FOR SEQ ID NO:36:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:36:

NNNNNNNNNN NNNNNNNT 18

(2) INFORMATION FOR SEQ ID NO:37:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:37:

NNGAGCGATA CCGAGNNT 18

(2) INFORMATION FOR SEQ ID NO:38:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:38:

NNGAGCGATA CCGAGNNT 18

(2) INFORMATION FOR SEQ ID NO:39:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:39:

NNGGAGNNAN AGNGANNC 18

(2) INFORMATION FOR SEQ ID NO:40:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:40:

NNGAGNGANA NNGAGNNT 18

(2) INFORMATION FOR SEQ ID NO:41:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:41:

NNCCCACCAC TTCCNNT 17

(2) INFORMATION FOR SEQ ID NO:42:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:42:

NNCTGTCCCG GGATANNT 18

(2) INFORMATION FOR SEQ ID NO:43:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:43:

NNNNNNNNNN NNNNNNNC 18

(2) INFORMATION FOR SEQ ID NO:44:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:44:

NNAGCCATAG CGANNC 16

(2) INFORMATION FOR SEQ ID NO:45:

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:45:

NNCCATAGCN NG 12

(2) INFORMATION FOR SEQ ID NO:46:

(i) SEQUENCE CHARACTERISTICS:
(A) LENGTH: 9 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:46:

NNCCATNNC 9

(2) INFORMATION FOR SEQ ID NO:47:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:47:

NNTTTTTTTT TTTTTNNG 18

(2) INFORMATION FOR SEQ ID NO:48:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:48:

NNTTTTTTTT TTTTTNNA 18

(2) INFORMATION FOR SEQ ID NO:49:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:49:

CCCCCACCAC TTCCCCT 17

(2) INFORMATION FOR SEQ ID NO:50:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:50:

CCCTGTCCCG GGATAGGT 18

(2) INFORMATION FOR SEQ ID NO:51:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:51:

GGAGCCATAG CGAGGC 16

(2) INFORMATION FOR SEQ ID NO:52:

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:52:

AGCCATAGCG AG 12

(2) INFORMATION FOR SEQ ID NO:53:

(i) SEQUENCE CHARACTERISTICS:
(A) LENGTH: 9 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:53:

AGCCATAGC 9

(2) INFORMATION FOR SEQ ID NO:54:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:54:

GGTTTTTTTT TTTTTGGG 18

(2) INFORMATION FOR SEQ ID NO:55:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other nucleic acid, synthetic DNA

(xi) SEQUENCE DESCRIPTION: SEQ ID NO:55:

AATTTTTTTT TTTTTAAA 18

## Claims

1. An oligonucleotide compound or a salt thereof, which has a nucleotide sequence represented by formula (I):

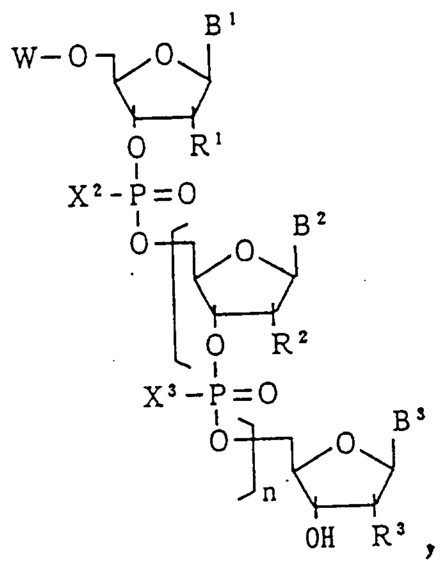

(I)

wherein W represents hydrogen or a protective group; $B^1$, $B^2$ and $B^3$ each represent nucleic acid residues, wherein each $B^2$ may be the same or different when n is 2 or more; $X^1$, $X^2$ and $X^3$ each represent OH, SH, $C_{1-5}$ alkyl, $C_{1-5}$ alkoxy, $C_{1-5}$ monoalkylamino or an aromatic ring group which may be substituted or unsubstituted, wherein each $X^3$ may be the same or different when n is 2 or more; $R^1$, $R^2$ and $R^3$ each represent hydrogen, OH, halogen, $C_{1-5}$ alkoxy or $C_{1-5}$ alkoxyalkyloxy, wherein each $R^2$ may be the same or different when n is 2 or more; and n represents an integer from 1 to 98;
wherein (1) W represents hydrogen or a protective group when $X^2$ and $X^3$ each represent OH, SH, $C_{1-5}$ alkyl, $C_{1-5}$ alkoxy, $C_{1-5}$ monoalkylamino or an aromatic ring group which may be substituted or unsubstituted, and at least one of $X^2$ and $X^3$ is an aromatic ring group which may be substituted or unsubstituted; or
(2) W is represented by formula

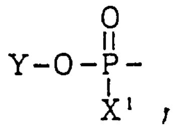

or

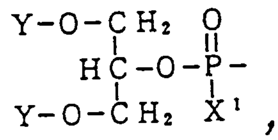

in which Y represents a saccharide residue; and $X^1$ represents OH, SH, $C_{1-5}$ alkyl, $C_{1-5}$ alkoxy, $C_{1-5}$ monoalkylamino or an aromatic ring group which may be substituted or unsubstituted when $X^2$ and $X^3$ each represent OH, SH, $C_{1-5}$ alkyl, $C_{1-5}$ alkoxy or $C_{1-5}$ monoalkylamino.

**2.** The oligonucleotide compound or a salt thereof as claimed in claim 1, which has a nucleotide sequence represented by formula (Ia):

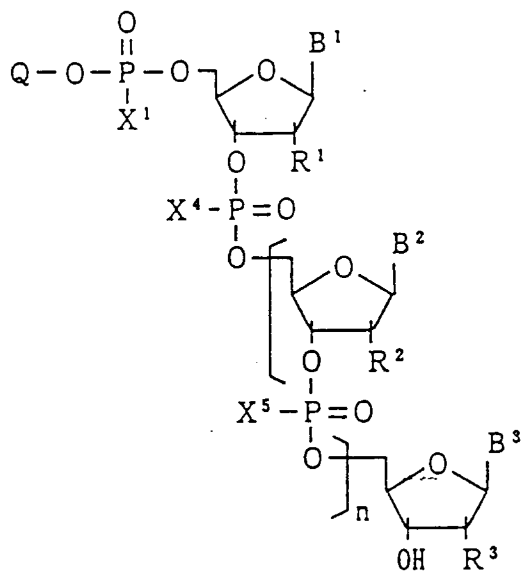

(I a)

in which Q represents Y or a formula

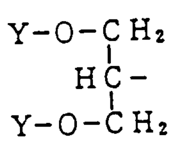

Y represents a saccharide residue; $X^1$ represents OH, SH, $C_{1-5}$ alkyl, $C_{1-5}$ alkoxy, $C_{1-5}$ monoalkylamino or an aromatic ring group which may be substituted or unsubstituted, $B^1$, $B^2$ and $B^3$ each represent nucleic acid residues, wherein each $B^2$ may be the same or different when n is 2 or more; $X^4$ and $X^5$ each represent OH, SH, $C_{1-5}$ alkyl, $C_{1-5}$ alkoxy or $C_{1-5}$ monoalkylamino, wherein each $X^5$ may be the same or different when n is 2 or more; $R^1$, $R^2$ and $R^3$ each represent hydrogen, OH, halogen, $C_{1-5}$ alkoxy or $C_{1-5}$ alkoxyalkyloxy, wherein each $R^2$ may be the same or different when n is 2 or more; and n represents an integer from 1 to 98.

**3.** The oligonucleotide compound as claimed in claim 2, in which a saccharide of the saccharide residue represented by Y is selected from the group consisting of (1) monosaccharides which may be substituted or unsubstituted, (2) oligosaccharides which may be substituted or unsubstituted, and (3) glycoside compounds of the above-mentioned monosaccharides or the above-mentioned oligosaccharides.

**4.** The oligonucleotide compound as claimed in claim 2, in which a saccharide of the saccharide residue represented by Y is selected from the group consisting of:

(i) a monosaccharide of which a hydroxyl group which is a hydroxyl group may be substituted by about 1 to about 4 substituent groups selected from the group consisting of (a) acyl groups which may be substituted by about 1 to about 5 substituent groups selected from the group consisting of halogen, $C_{1-10}$ alkylcarbonyl, nitro, $C_{1-10}$ alkyl, $C_{1-10}$ alkoxy, phenyl, and naphthyl, (b) alkyl groups which may be substituted by about 1 to about 5 substituent groups selected from the group consisting of halogen, $C_{1-10}$ alkylcarbonyl, nitro, $C_{1-10}$ alkyl, $C_{1-10}$ alkoxy, phenyl and naphthyl, and (c) amino groups which may be substituted or unsubstituted,

(ii) an oligosaccharide of which a hydroxyl group may be substituted by about 1 to about 4 substituent groups selected from the group consisting of (a) acyl groups which may be substituted by about 1 to about 5 substituent groups selected from the group consisting of halogen, $C_{1-10}$ alkylcarbonyl, nitro, $C_{1-10}$ alkyl, $C_{1-10}$ alkoxy, phenyl and naphthyl, (b) alkyl groups which may be substituted by about 1 to about 5 substituent groups selected from the group consisting of halogen, $C_{1-10}$ alkylcarbonyl, nitro, $C_{1-10}$ alkyl, $C_{1-10}$ alkoxy, phenyl and naphthyl, and (c) amino groups which may be substituted or unsubstituted, or

(iii) a glycoside compound in which the hydroxyl group of the above-mentioned monosaccharide or oligosaccharide is substituted by a non-saccharide component of the formula -O-$R^7$, -S-$R^8$ or -N-$R^9$, wherein $R^7$, $R^8$ and $R^9$ each represent hydrocarbon groups which may be substituted or unsubstituted.

**5.** The oligonucleotide compound as claimed in claim 3, in which the monosaccharide is selected from the group consisting of an aldopentose, a ketopentose, an aldohexose and a ketohexose.

**6.** The oligonucleotide compound as claimed in claim 5, in which the aldopentose is ribose, arabinose, xylose or lyxose, the ketopentose is ribulose or xylulose, the aldohexose is allose, altrose, glucose, mannose, gulose, idose, galactose or talose, and the ketohexose is psicose, fructose, sorbose or tagatose.

**7.** The oligonucleotide compound as claimed in claim 3, in which the oligosaccharide is selected from the group consisting of lactose, $\alpha,\alpha$-trehalose, N-acetylneuraminyllactose, difucosyllactose, fucosyllactose, lactulose, gentianose, isolychnoses, lychnoses, planteose, sucrose, bifrucose, 1,6-diglucosylmannitol, galactosylsucrose, 6-$\beta$-glucosylmannitol, laminaribiose, inulobioses, lycopose, maltose, isomaltotriose, melibiose, neobifucose, neokestose, eruloses, 6-$\alpha$-galactosylgalactose, kojibiose, maltlose, melezitose, turanose, sophorose, gentiooligosaccharides, vicianose, primeverose, sambubiose, lycobiose, lycotriose, solabiose, strophantobiose, digilanidobiose, odorotriose, fungitetraose and $\beta$-1,3-xylooligo.

**8.** The oligonucleotide compound as claimed in claim 3, in which the glycoside compound is one in which the hydroxyl group of the monosaccharide or the oligosaccharide is substituted by a non-saccharide component of the formula -O-$R^7$, -S-$R^8$ or -N-$R^9$, wherein $R^7$, $R^8$ and $R^9$ each represent hydrocarbon groups which may be substituted or unsubstituted.

**9.** The oligonucleotide compound as claimed in claim 8, in which each of $R^7$, $R^8$ and $R^9$ is selected from the group consisting of:

(i) a $C_{1-30}$ alkyl group which may be substituted by about 1 to about 5 substituent groups selected from the group consisting of halogen, $C_{1-10}$ alkylcarbonyl, nitro and $C_{1-10}$ alkoxy,

(ii) a $C_{2-4}$ alkenyl group which may be substituted by about 1 to about 5 substituent groups selected from the group consisting of halogen, $C_{1-10}$ alkylcarbonyl, nitro and a $C_{1-10}$ alkoxy,

(iii) a $C_{2-4}$ alkynyl group which may be substituted by about 1 to about 5 substituent groups selected from the group consisting of halogen, $C_{1-10}$ alkylcarbonyl, nitro and $C_{1-10}$ alkoxy,

(iv) a $C_{6-12}$ aryl group which may be substituted by about 1 to about 5 substituent groups selected from the group consisting of halogen, $C_{1-10}$ alkylcarbonyl, nitro, $C_{1-10}$ alkyl and $C_{1-10}$ alkoxy, and

(v) an $C_{7-14}$ aralkyl group which may be substituted by about 1 to about 5 substituent groups selected from the group consisting of halogen, $C_{1-10}$ alkylcarbonyl, nitro, $C_{1-10}$ alkyl and a $C_{1-10}$ alkoxy.

**10.** The oligonucleotide compound as claimed in claim 3, in which the glycoside compound is selected from the group consisting of n-octylglucoside, n-octyl-galactoside, n-octylmannoside, n-octylthioglucoside, n-octylthiogalactoside, n-octylthiomannoside, stearylglycoside, stearylgalactoside, stearylmannoside, stearylthioglycoside, stearylthiogalactoside, stearylthiomannoside, phenylglucoside, phenylgalactoside and phenylmannoside.

**11.** The oligonucleotide compound as claimed in claim 2, in which Q is Y, and a saccharide of the saccharide residue represented by Y is a monosaccharide, oligosaccharide or a glycoside compound thereof of which a hydroxyl group may be substituted by $C_{1-10}$ alkylcarbonyl, phenylcarbonyl or $C_{1-20}$ alkyl group.

**12.** The oligonucleotide compound as claimed in claim 11, in which the monosaccharide is selected from the group consisting of galactose, mannose, galactosamine and glucosamine.

**13.** The oligonucleotide compound as claimed in claim 11, in which the oligosaccharide is melibiose, gentiobiose or isomaltotriose.

**14.** The oligonucleotide compound as claimed in claim 11, in which the glycoside compound is selected from the group consisting of n-octylglucoside, n-octylthioglucoside or phenylglucoside.

**15.** The oligonucleotide compound as claimed in claim 4, in which the monosaccharide of which a hydroxyl group is substituted by amino groups which may be substituted or unsubstituted is glucosamine or galactosamine in which the 2-amino groups may be substituted by a $C_{1-6}$ alkylcarbonyl group or a phenylcarbonyl group.

**16.** The oligonucleotide compound as claimed in claim 2, in which Q is Y, and a saccharide of the saccharide residue represented by Y is (1) galactose or mannose of which a hydroxyl group may be substituted by a $C_{1-6}$ alkylcarbonyl group, (2) melibiose, gentiobiose or isomaltotriose of which an alcoholic hydroxyl group may be substituted by a phenylcarbonyl group, (3) n-octylglucoside, n-octylthioglucoside or phenylglucoside, or (4) glucosamine or galactosamine in which 2-amino groups may be substituted by a $C_{1-10}$ alkylcarbonyl group or a phenylcarbonyl group.

**17.** The oligonucleotide compound as claimed in claim 2, in which Q is Y, and a saccharide of the saccharide residue represented by Y is selected from the group consisting of galactose, mannose, melibiose, gentiobiose, isomaltotriose, n-octylglucoside, n-octylthioglucoside, phenylglucoside, galactosamine, N-benzoylgalactosamine, N-acetylgalactosamine, glucosamine, N-benzoylglucosamine and N-acetylglucosamine.

**18.** The oligonucleotide compound as claimed in claim 2, in which Q is represented by the formula,

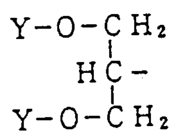

and a saccharide of the saccharide residue represented by Y is a monosaccharide.

**19.** The oligonucleotide compound as claimed in claim 18, in which the monosaccharide is galactose, mannose or glucose.

**20.** The oligonucleotide compound as claimed in claim 2, in which the nucleic acid residue is selected from the group consisting of hypoxanthine-9-yl, guanine-9-yl, adenine-9-yl, uracil-1-yl, thymine-1-yl and cytosine-1-yl.

**21.** The oligonucleotide compound as claimed in claim 2, in which $X^1$ is (1) OH, (2) SH, (3) $C_{1-5}$ alkyl, (4) $C_{1-5}$ alkoxy, (5) $C_{1-5}$ monoalkylamino, (6) $C_{6-11}$ aryl which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl, or (7) a 5 to 13-membered aromatic heterocyclic group which comprises 1 to 12 carbon atoms and 1 to about 4 hetero atomos which are selected from the group consisting of nitrogen, oxygen and sulfur atoms wherein the aromatic heterocyclic group may be substituted by about 1 to about 5 substituents which are selected from the group consisting of $C_{1-6}$

alkyl, $C_{2-6}$ alkenyl, $C_{2-6}$ alkynyl, $C_{3-6}$ cycloalkyl, $C_{5-7}$ cycloalkenyl, $C_{7-11}$ aralkyl, $C_{6-14}$ aryl, $C_{1-6}$ alkoxy, $C_{6-14}$ aryloxy, $C_{1-6}$ alkylcarbonyl, $C_{6-14}$ arylcarbonyl, $C_{1-6}$ alkanoyloxy, $C_{6-14}$ arylcarbonyloxy, carboxyl, $C_{1-6}$ alkoxycarbonyl, carbamoyl, N-mono-$C_{1-4}$ alkylcarbamoyl, N,N-di-$C_{1-4}$ alkylcarbamoyl, halogen, mono-, di- or tri-halogeno-$C_{1-4}$ alkyl, oxo, amidino, imino, amino, mono- or di-$C_{1-4}$ alkylamino, OH and nitro; and $X^4$ and $X^5$ are each OH, SH, $C_{1-5}$ alkyl, $C_{1-5}$ alkoxy or $C_{1-5}$ monoalkylamino.

**22.** The oligonucleotide compound as claimed in claim 21, in which the $C_{6-11}$ aryl group is phenyl or naphthyl, and the 5 to 13-membered aromatic heterocyclic group which comprises 1 to 12 carbon atoms and about 1 to about 4 hetero atomos which are selected from the group consisting of nitrogen, oxygen and sulfur atoms is selected from the group consisting of 2- or 3-thienyl, 2- or 3-furyl, 2- or 3-pyrrolyl, 2-, 3- or 4-pyridyl, 2-, 4- or 5-oxazolyl, 2-, 4- or 5-thiazolyl, 3-, 4- or 5-pyrazolyl, 2-, 4- or 5-imidazolyl, 3-, 4- or 5-isoxazolyl, 3-, 4- or 5-isothiazolyl, 3- or 5-(1,2,4-oxadiazolyl), 1,3,4-oxadiazolyl, 3- or 5-(1,2,4-thiadiazolyl), 1,3,4-thiadiazolyl, 4- or 5-(1,2,3-thiadiazolyl), 1,2,5-thiadiazolyl, 1,2,3-triazolyl, 1,2,4-triazolyl, 1H- or 2H-tetrazolyl, N-oxide-2-, 3- or 4-pyridyl, 2-, 4- or 5-pyrimidinyl, N-oxide-2-, 4- or 5-pyrimidinyl, thiomorpholinyl, morpholinyl, oxoimidadinyl, dioxotriazinyl, pyrrolidinyl, piperidinyl, pyranyl, thiopyranyl, 1,4-oxazinyl, 1,4-thiazinyl, 1,3-thiazinyl, piperadinyl, triazinyl, oxotriazinyl, 3- or 4-pyridazinyl, pyrazinyl, N-oxide-3- or 4-pyridazinyl, benzofuryl, benzothiazolyl, benzooxazolyl, tetrazolo-[1,5-b]pyridazinyl, triazolo[4,5-b]pyridazinyl, benzoimidazolyl, quinolyl, isoquinolyl, cynnorynyl, phthalazinyl, quinazolinyl, quinoxalinyl, indolizinyl, quinolizinyl, 1,8-naphthylizinyl, purinyl, pteridinyl, dibenzofuranyl, carbazolyl, acridinyl, phenanthridinyl, chromanyl, benzoxazinyl, phenazinyl, phenothiazinyl and phenoxazinyl.

**23.** The oligonucleotide compound as claimed in claim 2, in which $X^1$ is OH; SH; $C_{1-5}$ alkyl; $C_{1-5}$ alkoxy; $C_{1-5}$ monoalkylamino; or phenyl which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl; and $X^4$ and $X^5$ are each OH, SH, $C_{1-5}$ alkyl, $C_{1-5}$ alkoxy or $C_{1-5}$ monoalkylamino.

**24.** The oligonucleotide compound as claimed in claim 2, in which $X^1$ is selected from the group consisting of OH, SH, methyl, ethyl, propyl, phenyl, (o-, m-, p-)alkoxyphenyl, (o-, m-, p-)alkylphenyl, methoxy and ethoxy; and $X^4$ and $X^5$ are each selected from the group consisting of OH, SH, methyl, ethyl, propyl, methoxy and ethoxy.

**25.** The oligonucleotide compound as claimed in claim 2, in which $X^1$ is selected from the group consisting of OH, SH, methyl, ethyl, propyl, phenyl, (o-, m-, p-) $C_{1-10}$ alkoxyphenyl, (o-, m-, p-) $C_{1-10}$ alkylphenyl, methoxy and ethoxy; and $X^4$ and $X^5$ are each selected from the group consisting of OH, SH, methyl, ethyl, propyl, methoxy and ethoxy.

**26.** The oligonucleotide compound as claimed in claim 2, in which $X^1$ is OH, SH, methyl or phenyl, and $X^4$ and $X^5$ are each OH, SH or methyl.

**27.** The oligonucleotide compound as claimed in claim 2, in which $R^1$, $R^2$ and $R^3$ are each selected from the group consisting of hydrogen, OH, fluoro, chloro, bromo, methoxy, ethoxy and methoxymethyloxy.

**28.** The oligonucleotide compound as claimed in claim 2, in which $R^1$, $R^2$ and $R^3$ are each hydrogen.

**29.** The oligonucleotide compound as claimed in claim 2, in which n is an integer from 1 to 38.

**30.** The oligonucleotide compound as claimed in claim 2, which contains a nucleotide sequence represented by the nucleotides CAT.

**31.** The oligonucleotide compound as claimed in claim 2, which is represented by a formula:

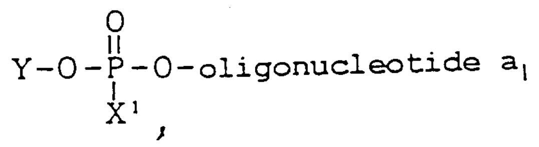

wherein Y represents a saccharide residue, and $X^1$ represents OH, SH, $C_{1-5}$ alkyl, $C_{1-5}$ alkoxy, $C_{1-5}$ monoalkylamino or an aromatic ring group which may be substituted or unsubstituted; "oligonucleotide $a_1$" represents a nucleotide sequence selected from the group consisting of SEQ ID NOs.: 1 to 31 and SEQ ID NO:34.

**32.** The oligonucleotide compound as claimed in claim 31, in which a saccharide of the saccharide residue represented by Y is selected from the group consisting of galactose, mannose, melibiose, gentiobiose, isomaltotriose, n-octylglucoside, n-octylthioglucoside, phenylglucoside, galactosamine, N-benzoylgalactosamine, N-acetylgalactosamine, glucosamine, N-benzoylglucosamine and N-acetylglucosamine; $X^1$ is OH, SH, methyl or phenyl.

**33.** The oligonucleotide compound as claimed in claim 2, which is represented by a formula:

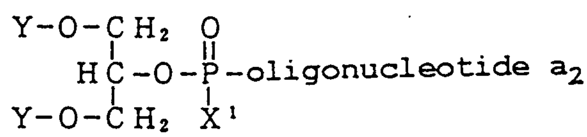

wherein Y represents a saccharide residue, and $X^1$ represents OH, SH, $C_{1-5}$ alkyl, $C_{1-5}$ alkoxy, $C_{1-5}$ monoalkylamino or an aromatic ring group which may be substituted or unsubstituted; "oligonucleotide $a_2$" represents a nucleotide sequence selected from the group consisting of SEQ ID NOs.: 1 to 31 and SEQ ID NO:34.

**34.** The oligonucleotide compound as claimed in claim 33, in which a saccharide of the saccharide residue represented by Y is selected from the group consisting of galactose, mannose and glucose; $X^1$ is OH, SH, methyl or phenyl.

**35.** The oligonucleotide compound as claimed in claim 31, in which each of "oligonucleotide $a_1$" and "oligonucleotide $a_2$" is represented by SEQ ID NO: 1, SEQ ID NO:31 or SEQ ID NO:34.

**36.** The oligonucleotide compound as claimed in claim 1, in which the oligonucleotide compound is an antisense oligonucleotide compound.

**37.** The oligonucleotide compound as claimed in claim 1, which is encapsulated in a liposome.

**38.** The oligonucleotide compound or a salt thereof as claimed in claim 1, which has a nucleotide sequence represented by formula (Ib):

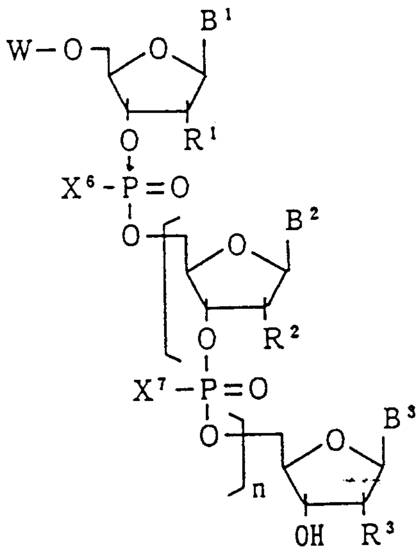

(I b)

wherein W represents hydrogen or a protective group; $B^1$, $B^2$ and $B^3$ each represent nucleic acid residues, wherein each $B^2$ may be the same or different when n is 2 or more; $X^6$ and $X^7$ each represent OH, SH, $C_{1-5}$ alkyl, $C_{1-5}$ alkoxy, $C_{1-5}$ monoalkylamino or an aromatic ring group which may be substituted or unsubstituted, wherein each $X^7$ may be the same or different when n is 2 or more, at least one of $X^6$ and $X^7$ represents an aromatic, ring group which may be substituted or unsubstituted; $R^1$, $R^2$ and $R^3$ each represent hydrogen, OH, halogen, $C_{1-5}$ alkoxy or $C_{1-5}$ alkoxyalkyloxy, wherein each $R^2$ may be the same or different when n is 2 or more; and n represents an integer from 1 to 98.

**39.** The oligonucleotide compound or the salt thereof as claimed in claim 38, wherein a protective group represented by W is selected from the group consisting of
(i) formula

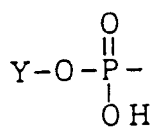

wherein Y is a saccharide residue;
(ii) formula

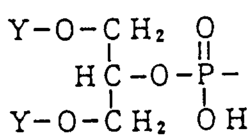

wherein Y is a saccharide residue;
(iii) $C_{1-6}$ alkyl which may be substituted with halogen atom(s);
(iv) $C_{6-11}$ aryl which may be substituted with halogen atom(s) or $C_{1-6}$ alkyl;
(v) $C_{7-12}$ aralkyl which may be substituted with halogen atom(s) or $C_{1-6}$ alkyl;
(vi) $C_{1-6}$ alkylcarbonyl which may be substituted with halogen atom(s);
(vii) phenyloxycarbonyl;
(viii) $C_{7-12}$ aralkyl-carbonyl;
(ix) pyranyl;
(x) furanyl;
(xi) silyl;
(xii) trityl which may be substituted with 1 to 3 methoxy groups; and
(xiii) phenylxanthenyl which may be substituted with methoxy groups.

**40.** The oligonucleotide compound or a salt thereof as claimed in claim 38, wherein W is hydrogen or represented by
formula

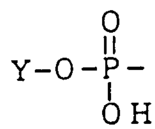

or formula

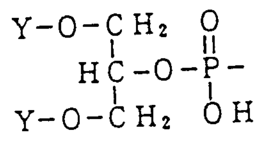

wherein Y is a saccharide residue.

**41.** The oligonucleotide compound or a salt thereof as claimed in claim 39, in which a saccharide of the saccharide residue represented by Y is selected from the group consisting of (1) monosaccharides which may be substituted or unsubstituted, (2) oligosaccharides which may be substituted or unsubstituted, and (3) glycoside compounds of the above-mentioned monosaccharides or the above-mentioned oligosaccharides.

**42.** The oligonucleotide compound as claimed in claim 39, in which a saccharide of the saccharide residue represented by Y is selected from the group consisting of:
(i) a monosaccharide of which a hydroxyl group may be substituted by about 1 to about 4 substituent groups selected from the group consisting of (a) acyl groups which may be substituted by about 1 to about 5 substituent groups selected from the group consisting of halogen, $C_{1-10}$ alkylcarbonyl, nitro, $C_{1-10}$ alkyl, $C_{1-10}$ alkoxy, phenyl and naphthyl, (b) alkyl groups which may be substituted by about 1 to about 5 substituent groups selected from the group consisting of halogen, $C_{1-10}$ alkylcarbonyl, nitro, $C_{1-10}$ alkyl, $C_{1-10}$ alkoxy, phenyl and naphthyl, and (c) amino groups which may be substituted or unsubstituted,
(ii) an oligosaccharide of which a hydroxyl group may be substituted by about 1 to about 4 substituent groups selected from the group consisting of (a) acyl groups which may be substituted by about 1 to about 5 substituent groups selected from the group consisting of halogen, $C_{1-10}$ alkylcarbonyl, nitro, $C_{1-10}$ alkyl, $C_{1-10}$ alkoxy, phenyl and naphthyl, (b) alkyl groups which may be substituted by about 1 to about 5 substituent groups selected from the group consisting of halogen, $C_{1-10}$ alkylcarbonyl, nitro, $C_{1-10}$ alkyl, $C_{1-10}$ alkoxy, phenyl and naphthyl, and (c) amino groups which may be substituted or unsubstituted, and
(iii) a glycoside compound in which the hydroxyl group of the above-mentioned monosaccharide or oligosaccharide is substituted by a non-saccharide component of the formula $-O-R^7$, $-S-R^8$ or $-N-R^9$, wherein $R^7$, $R^8$ and $R^9$ each represent hydrocarbon groups which may be substituted or unsubstituted.

**43.** The oligonucleotide compound as claimed in claim 41, in which the monosaccharide is an aldopentose, a ketopentose, an aldohexose or a ketohexose.

**44.** The oligonucleotide compound as claimed in claim 43, in which the aldopentose is ribose, arabinose, xylose or lyxose, the ketopentose is ribulose or xylulose, the aldohexose is allose, altrose, glucose, mannose, gulose, idose, galactose or talose, and the ketohexose is psicose, fructose, sorbose or tagatose.

**45.** The oligonucleotide compound as claimed in claim 41, in which the oligosaccharide is selected from the group consisting of lactose, α,α-trehalose, N-acetylneuraminyllactose, difucosyllactose, fucosyllactose, lactulose, gentianose, isolychnoses, lychnoses, planteose, sucrose, bifrucose, 1,6-diglucosylmannitol, galactosylsucrose, 6-$\beta$-glucosylmannitol, laminaribiose, inulobioses, lycopose, maltose, isomaltotriose, melibiose, neobifucose, neokestose, eruloses, 6-$\alpha$-galactosylgalactose, kojibiose, maltlose, melezitose, turanose, sophorose, gentiooligosaccharides, vicianose, primeverose, sambubiose, lycobiose, lycotriose, solabiose, strophantobiose, digilanidobiose, odorotriose, fungitetraose and $\beta$-1,3-xylooligo.

**46.** The oligonucleotide compound as claimed in claim 41, in which the glycoside compound is one in which the hydroxyl group of the monosaccharide or the oligosaccharide is substituted by a non-saccharide component of the formula -O-$R^7$, -S-$R^8$ or -N-$R^9$, wherein $R^7$, $R^8$ and $R^9$ each represent hydrocarbon groups which may be substituted or unsubstituted.

**47.** The oligonucleotide compound as claimed in claim 46, in which each of $R^7$, $R^8$ and $R^9$ is:
(i) a $C_{1-30}$ alkyl group which may be substituted by about 1 to about 5 substituent groups selected from the group consisting of halogen, $C_{1-10}$ alkylcarbonyl, nitro and $C_{1-10}$ alkoxy,
(ii) a $C_{2-4}$ alkenyl group which may be substituted by about 1 to about 5 substituent groups selected from the group consisting of halogen, $C_{1-10}$ alkylcarbonyl, nitro and a $C_{1-10}$ alkoxy,
(iii) a $C_{2-4}$ alkynyl group which may be substituted by about 1 to about 5 substituent groups selected from the group consisting of halogen, $C_{1-10}$ alkylcarbonyl, nitro and $C_{1-10}$ alkoxy,
(iv) a $C_{6-12}$ aryl group which may be substituted by about 1 to about 5 substituent groups selected from the group consisting of halogen, $C_{1-10}$ alkylcarbonyl, nitro, $C_{1-10}$ alkyl and $C_{1-10}$ alkoxy, or
(v) an $C_{7-14}$ aralkyl group which may be substituted by about 1 to about 5 substituent groups selected from the group consisting of halogen, $C_{1-10}$ alkylcarbonyl, nitro, $C_{1-10}$ alkyl and a $C_{1-10}$ alkoxy.

**48.** The oligonucleotide compound as claimed in claim 41, in which the glycoside compound is selected from the group consisting of n-octylglucoside, n-octyl-galactoside, n-octylmannoside, n-octylthioglucoside, n-octylthiogalactoside, n-octylthiomannoside, stearylglycoside, stearylgalactoside, stearylmannoside, stearylthioglycoside, stearylthiogalactoside, stearylthiomannoside, phenylglucoside, phenylgalactoside and phenylmannoside.

**49.** The oligonucleotide compound as claimed in claim 42, in which the monosaccharide of which a hydroxyl group is substituted by amino groups which may be substituted is glucosamine or galactosamine wherein 2-amino groups may be substituted by a $C_{1-6}$ alkylcarbonyl group or a phenylcarbonyl group.

**50.** The oligonucleotide compound as claimed in claim 38, in which W is hydrogen or reoresented by the formula

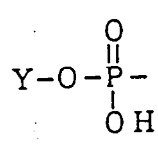

and a saccharide of the saccharide residue represented by Y is a monosaccharide, oligosaccharide or a glycoside compound thereof of which a hydroxyl group may be substituted by $C_{1-10}$ alkylcarbonyl, phenylcarbonyl or $C_{1-20}$ alkyl group.

**51.** The oligonucleotide compound as claimed in claim 50, in which the monosaccharide is galactose, mannose, galactosamine or glucosamine.

**52.** The oligonucleotide compound as claimed in claim 50, in which the oligosaccharide is melibiose, gentiobiose or isomaltotriose.

**53.** The oligonucleotide compound as claimed in claim 50, in which the glycoside compound is n-octylglucoside, n-octylthioglucoside or phenylglucoside.

**54.** The oligonucleotide compound as claimed in claim 38, in which W is hydrogen or represented by the formula

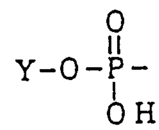

and a saccharide of the saccharide residue represented by Y is (1) galactose or mannose of which an alcoholic hydroxyl group may be substituted by a $C_{1-6}$ alkylcarbonyl group, (2) melibiose, gentiobiose or isomaltotriose of which a hydroxyl group may be substituted by a phenylcarbonyl group, (3) n-octylglucoside, n-octylthioglucoside or phenylglucoside, or (4) glucosamine or galactosamine wherein 2-amino groups may be substituted by a $C_{1-10}$ alkylcarbonyl group or a phenylcarbonyl group.

**55.** The oligonucleotide compound as claimed in claim 38, in which W is hydrogen or represented by the formula

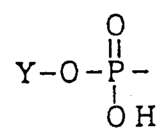

and a saccharide of the saccharide residue represented by Y is selected from the group consisting of galactose, mannose, melibiose, gentiobiose, isomaltotriose, n-octylglucoside, n-octylthioglucoside, phenylglucoside, galactosamine, N-benzoylgalactosamine, N-acetylgalactosamine, glucosamine, N-benzoylglucosamine and N-acetylglucosamine.

**56.** The oligonucleotide compound as claimed in claim 38, in which W is hydrogen or represented by the formula,

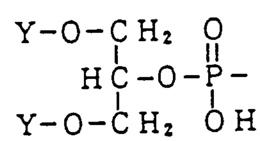

and a saccharide of the saccharide residue represented by Y is a monosaccharide.

**57.** The oligonucleotide compound as claimed in claim 56, in which the monosaccharide is galactose, mannose or glucose.

**58.** The oligonucleotide compound as claimed in claim 38, in which the nucleic acid residue is selected from the group consisting of hypoxanthine-9-yl, guanine-9-yl, adenine-9-yl, uracil-1-yl, thymine-1-yl and cytosine-1-yl.

**59.** The oligonucleotide compound as claimed in claim 38, in which $X^6$ and $X^7$ are each (1) OH, (2) SH, (3) $C_{1-5}$ alkyl, (4) $C_{1-5}$ alkoxy, (5) $C_{1-5}$ monoalkylamino, (6) $C_{6-11}$ aryl which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl, or (7) a 5 to 13-membered aromatic heterocyclic ring which comprises 1 to 12 carbon atoms and 1 to about 4 hetero atomos which are selected from the group consisting of nitrogen, oxygen and sulfur atoms, wherein the 13-memebered aromatic heterocyclic ring which may be substituted by about 1 to about 5 substituents which are selected from the group consisting of $C_{1-6}$ alkyl, $C_{2-6}$ alkenyl, $C_{2-6}$ alkynyl, $C_{3-6}$ cycloalkyl, $C_{5-7}$ cycloalkenyl, $C_{7-11}$ aralkyl, $C_{6-14}$ aryl, $C_{1-6}$ alkoxy, $C_{6-14}$ aryloxy, $C_{1-6}$ alkylcarbonyl, $C_{6-14}$ arylcarbonyl, $C_{1-6}$ alkanoyloxy, $C_{6-14}$ arylcarbonyloxy, carboxyl, $C_{1-6}$ alkoxycarbonyl,

carbamoyl, N-mono-$C_{1-4}$ alkylcarbamoyl, N,N-di-$C_{1-4}$ alkylcarbamoyl, halogen, mono-, di- or tri-halogeno-$C_{1-4}$ alkyl, oxo, amidino, imino, amino, mono- or di-$C_{1-4}$ alkylamino, OH and nitro.

**60.** The oligonucleotide compound as claimed in claim 59, in which the $C_{6-11}$ aryl group is phenyl or naphthyl, and the 5 to 13-membered aromatic heterocyclic ring group is selected from the group consisting of 2- or 3-thienyl, 2-or 3-furyl, 2- or 3-pyrrolyl, 2-, 3- or 4-pyridyl, 2-, 4-or 5-oxazolyl, 2-, 4- or 5-thiazolyl, 3-, 4- or 5-pyrazolyl, 2-, 4- or 5-imidazolyl, 3-, 4- or 5-isoxazolyl, 3-, 4- or 5-isothiazolyl, 3- or 5-(1,2,4-oxadiazolyl), 1,3,4-oxadiazolyl, 3- or 5-(1,2,4-thiadiazolyl), 1,3,4-thiadiazolyl, 4- or 5-(1,2,3-thiadiazolyl), 1,2,5-thiadiazolyl, 1,2,3-triazolyl, 1,2,4-triazolyl, 1H- or 2H-tetrazolyl, N-oxide-2-, 3- or 4-pyridyl, 2-, 4- or 5-pyrimidinyl, N-oxide-2-, 4- or 5-pyrimidinyl, thiomorpholinyl, morpholinyl, oxoimidadinyl, dioxotriazinyl, pyrrolidinyl, piperidinyl, pyranyl, thiopyranyl, 1,4-oxazinyl, 1,4-thiazinyl, 1,3-thiazinyl, piperadinyl, triazinyl, oxotriazinyl, 3- or 4-pyridazinyl, pyrazinyl, N-oxide-3- or 4-pyridazinyl, benzofuryl, benzothiazolyl, benzooxazolyl, tetrazolo[1,5-b]pyridazinyl, triazolo[4,5-b]pyridazinyl, benzoimidazolyl, quinolyl, isoquinolyl, cynnorynyl, phthalazinyl, quinazolinyl, quinoxalinyl, indolizinyl, quinolizinyl, 1,8-naphthylizinyl, purinyl, pteridinyl, dibenzofuranyl, carbazolyl, acridinyl, phenanthridinyl, chromanyl, benzoxazinyl, phenazinyl, phenothiazinyl and phenoxazinyl.

**61.** The oligonucleotide compound as claimed in claim 38, in which $X^6$ and $X^7$ are each OH; SH; $C_{1-5}$ alkyl; $C_{1-5}$ alkoxy; $C_{1-5}$ monoalkylamino; or phenyl which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl.

**62.** The oligonucleotide compound as claimed in claim 38, in which $X^6$ and $X^7$ are each selected from the group consisting of OH, SH, methyl, ethyl, propyl, phenyl, (o-, m-, p-)alkoxyphenyl, (o-, m-, p-)alkylphenyl, methoxy and ethoxy.

**63.** The oligonucleotide compound as claimed in claim 38, in which $X^6$ and $X^7$ are each selected from the group consisting of OH, SH, methyl, ethyl, propyl, phenyl, (o-, m-, p-) $C_{1-10}$ alkoxyphenyl, (o-, m-, p-) $C_{1-10}$ alkylphenyl, methoxy and ethoxy.

**64.** The oligonucleotide compound as claimed in claim 38, in which $X^6$ and $X^7$ are each OH, SH, methyl or phenyl.

**65.** The oligonucleotide compound as claimed in claim 38, in which $R^1$, $R^2$ and $R^3$ are each selected from the group consisting of hydrogen, OH, fluoro, chloro, bromo, methoxy, ethoxy and methoxymethyloxy.

**66.** The oligonucleotide compound as claimed in claim 38, in which $R^1$, $R^2$ and $R^3$ are each hydrogen.

**67.** The oligonucleotide compound as claimed in claim 38, in which n is an integer from 1 to 38.

**68.** The oligonucleotide compound or a salt thereof as claimed in claim 38, which has a nucleotide sequence represented by formula:

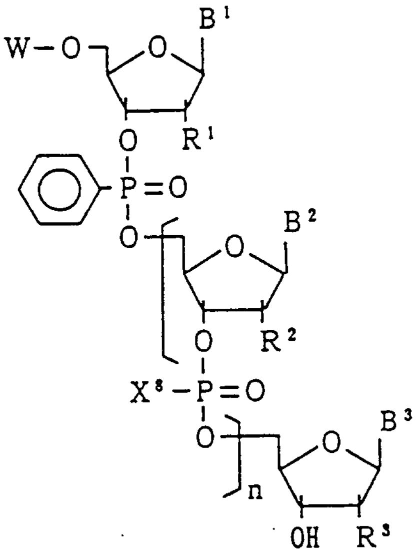

wherein W represents hydrogen or a protective group; $B^1$, $B^2$ and $B^3$ each represent nucleic acid residues, wherein each $B^2$ may be the same or different when n is 2 or more; $X^8$ represents OH, SH, $C_{1-5}$ alkyl, $C_{1-5}$ alkoxy, $C_{1-5}$ monoalkylamino or an aromatic ring group which may be substituted or unsubstituted; $R^1$, $R^2$ and $R^3$ each represent hydrogen, OH, halogen, $C_{1-5}$ alkoxy or $C_{1-5}$ alkoxyalkyloxy, wherein each $R^2$ may be the same or different when n is 2 or more; and n represents an integer from 1 to 98.

**69.** The oligonucleotide compound or a salt thereof as claimed in claim 38, which has a nucleotide sequence represented by formula:

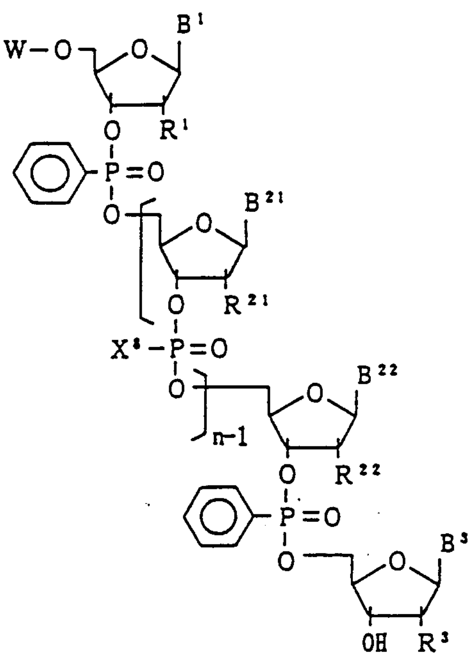

wherein W represents hydrogen or a protective group; $B^1$, $B^{21}$, $B^{22}$ and $B^3$ each represent nucleic acid residues, wherein each $B^{21}$ may be the same or different when n is 2 or more; $X^8$ represents OH, SH, $C_{1-5}$ alkyl, $C_{1-5}$ alkoxy, $C_{1-5}$ monoalkylamino or an aromatic ring group which may be substituted or unsubstituted; $R^1$, $R^2$ and $R^3$ each represent hydrogen, OH, halogen, $C_{1-5}$ alkoxy or $C_{1-5}$ alkoxyalkyloxy, wherein each $R^2$ may be the same or different when n is 2 or more; and n represents

an integer from 1 to 98.

**70.** The oligonucleotide compound as claimed in claim 38, which is represented by a formula:

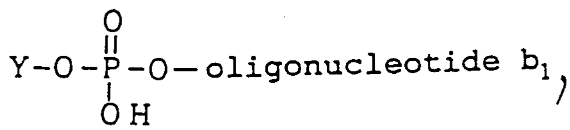

wherein Y represents a saccharide residue, and "oligonucleotide $b_1$" represents a nucleotide sequence selected from the group consisting of SEQ ID NO: 32, SEQ ID NO:33, SEQ ID NO:35, SEQ ID NO:37, SEQ ID NO:39, SEQ ID NO: 40, SEQ ID NO:41, SEQ ID NO:42, SEQ ID NO:43, SEQ ID NO:44, SEQ ID NO:45, SEQ ID NO:46, SEQ ID NO:47 and SEQ ID NO:48.

**71.** The oligonucleotide compound as claimed in claim 70, in which a saccharide of the saccharide residue represented by Y is selected from the group consisting of galactose, mannose, melibiose, gentiobiose, isomaltotriose, n-octylglucoside, n-octylthioglucoside, phenylglucoside, galactosamine, N-benzoylgalactosamine, N-acetylgalactosamine, glucosamine, N-benzoylglucosamine and N-acetylglucosamine.

**72.** The oligonucleotide compound as claimed in claim 70, in which a saccharide of the saccharide residue represented by Y is melibiose, gentiobiose or N-benzoylgalactosamine.

**73.** The oligonucleotide compound as claimed in claim 38, which is represented by a formula:

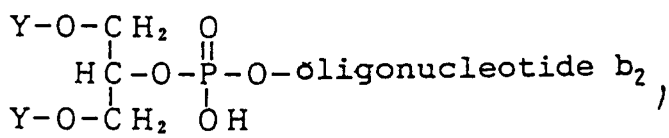

wherein Y represents a saccharide residue, and "oligonucleotide $b_2$" represents a nucleotide sequence selected from the group consisting of SEQ ID NO: 32, SEQ ID NO:33, SEQ ID NO:35, SEQ ID NO:37, SEQ ID NO:39, SEQ ID NO: 40, SEQ ID NO:41, SEQ ID NO:42, SEQ ID NO:43, SEQ ID NO:44, SEQ ID NO:45, SEQ ID NO:46, SEQ ID NO:47 and SEQ ID NO:48.

**74.** The oligonucleotide compound as claimed in claim 73, in which a saccharide of the saccharide residue represented by Y is galactose, mannose or glucose.

**75.** The oligonucleotide compound as claimed in claim 73, in which a saccharide of the saccharide residue represented by Y is galactose.

**76.** A method for producing the oligonucleotide compound as claimed in claim 1, which comprises removing a protective group of (a) a compound represented by formula:

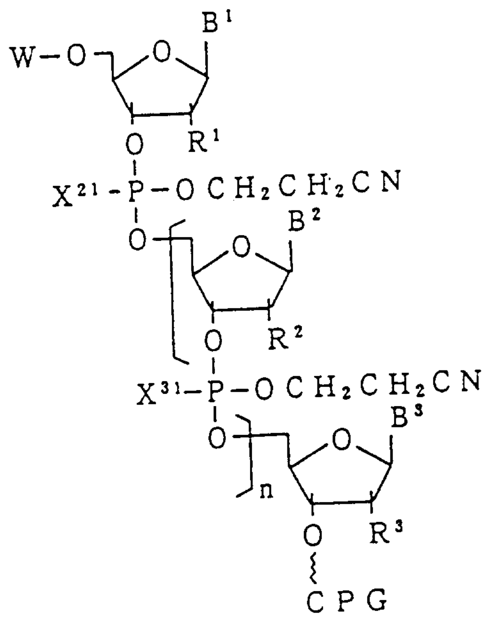

wherein W, $B^1$, $B^2$, $B^3$, $R^1$, $R^2$, $R^3$ and n have the same meanings as given in claim 1; $X^{21}$ and $X^{31}$ each represent O or S, wherein each $X^{31}$ may be the same or different when n is 2 or more; and CPG represents a solid phase carrier; or (b) a compound represented by formula:

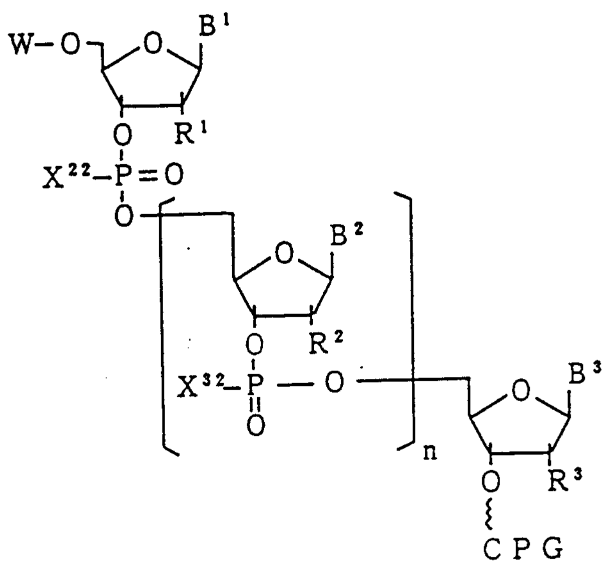

wherein W, $B^1$, $B^2$, $B^3$, $R^1$, $R^2$, $R^3$ and n have the same meanings as given in claim 1; $X^{22}$ and $X^{32}$ each represent $C_{1-5}$ alkyl, $C_{1-5}$ alkoxy, $C_{1-5}$ monoalkylamino or aromatic ring group which may be substituted or unsubstituted, wherein each $X^{32}$ may be the same or different when n is 2 or more; and CPG represents a solid phase carrier.

**77.** A method for producing the oligonucleotide compound as claimed in claim 2, which comprises removing a protective group of (a) a compound represented by formula:

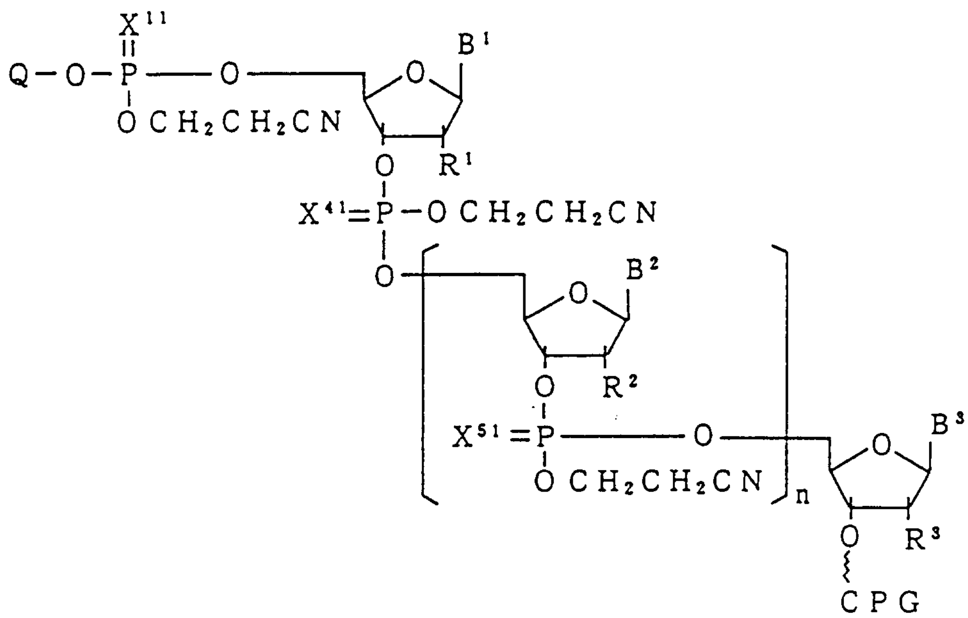

wherein Q, $B^1$, $B^2$, $B^3$, $R^1$, $R^2$, $R^3$ and n have the same meanings as given in claim 2; $X^{11}$, $X^{41}$ and $X^{51}$ each represent O or S, wherein each $X^{51}$ may be the same or different when n is 2 or more; and CPG represents a solid phase carrier; or (b) a compound represented by formula:

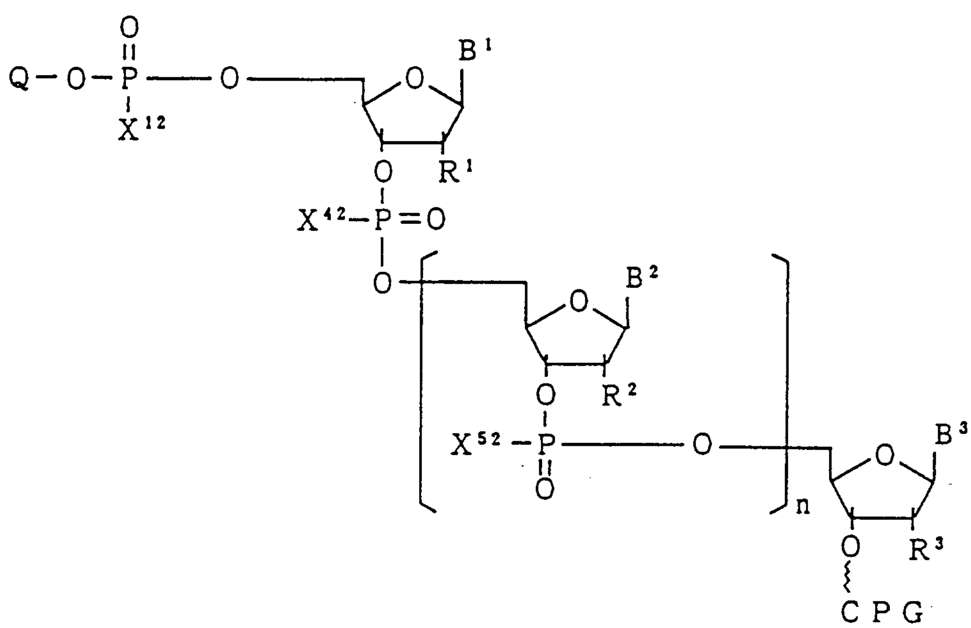

wherein Q, $B^1$, $B^2$, $B^3$, $R^1$, $R^2$, $R^3$ and n have the same meanings as given in claim 2; $X^{12}$, $X^{42}$ and $X^{52}$ each represent $C_{1-5}$ alkyl, $C_{1-5}$ alkoxy, $C_{1-5}$ monoalkylamino or an aromatic ring group which may be substituted or unsubstituted, wherein each $X^{52}$ may be the same or different when n is 2 or more; and CPG represents a solid phase carrier.

**78.** A method for producing the oligonucleotide compound as claimed in claim 38, which comprises removing a protective group of (a) a compound represented by formula:

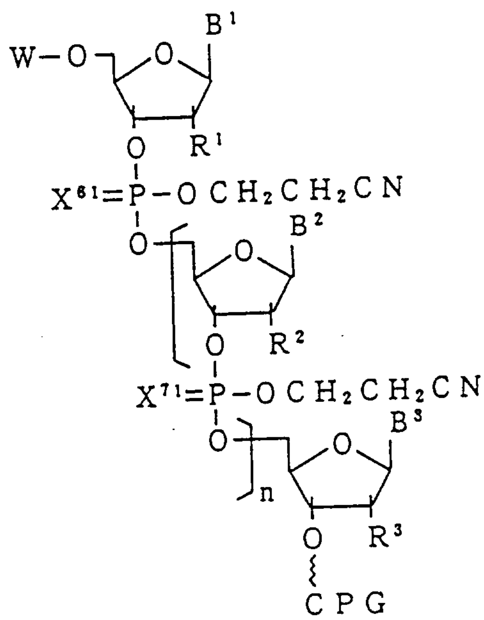

wherein W, $B^1$, $B^2$, $B^3$, $R^1$, $R^2$, $R^3$ and n have the same meanings as given in claim 38; $X^{61}$ and $X^{71}$ each represent O or S, wherein each $X^{71}$ may be the same or different when n is 2 or more; and CPG represents a solid phase carrier; or (b) a compound represented by formula:

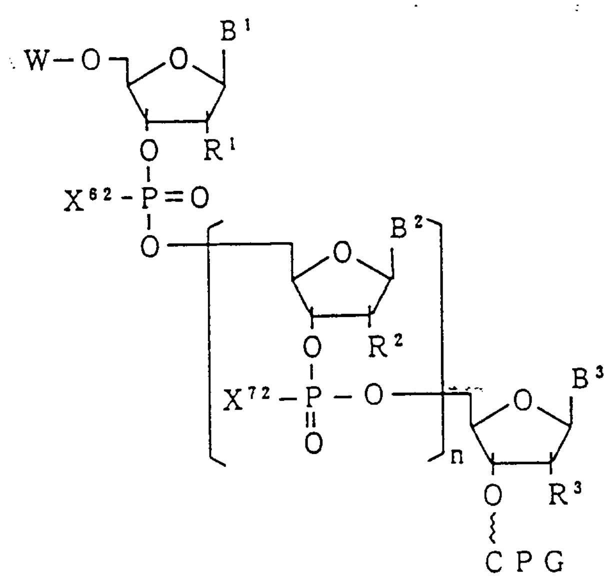

wherein W, $B^1$, $B^2$, $B^3$, $R^1$, $R^2$, $R^3$ and n have the same meanings as given in claim 38; $X^{62}$ and $X^{72}$ each represent $C_{1-5}$ alkyl, $C_{1-5}$ alkoxy, $C_{1-5}$ monoalkylamino or aromatic ring group which may be substituted or unsubstituted, wherein each $X^{72}$ may be the same or different when n is 2 or more; and CPG represents a solid phase carrier.

**79.** A composition for inhibiting a gene expression comprising the oligonucleotide compound or the salt thereof described in claim 1.

**80.** The composition for inhibiting a gene expression as claimed in claim 79, in which the gene is ICAM-1.

**81.** A composition for inhibiting a reangiogenesis after PTCA (percutaneous transluminal coronary angioplasty) which comprises a nucleotide compound or a salt thereof of claim 1.

**82.** A saccharide derivative represented by the following formula:

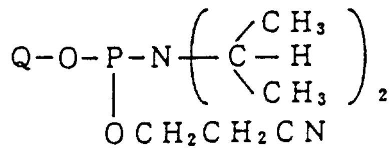

wherein Q represents Y or formula:

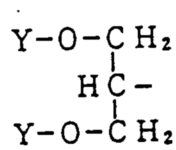
,

wherein Y represents a saccharide residue.

**83.** The saccharide derivative as claimed in claim 82, in which Q is Y, and a saccharide of the saccharide residue represented by Y is selected from the group consisting of (1) galactose, mannose, melibiose, gentiobiose or isomaltotriose wherein a hydroxyl group thereof may be substituted by a $C_{1-5}$ alkylcarbonyl or a benzoyl group, (2) glucoside or thioglucoside wherein a hydroxyl group other than a hemiacetal hydroxyl group thereof may be substituted by a $C_{1-5}$ alkylcarbonyl, a benzoyl or a $C_{1-20}$ alkyl, and (3) glucosamine or galactosamine wherein a hydroxyl group thereof may be substituted by a $C_{1-5}$ alkylcarbonyl or a benzoyl which may be substituted by halogens.

**84.** The saccharide derivative as claimed in claim 82, in which Q is Y, and a saccharide of the saccharide residue represented by Y is selected from the group consisting of 1,2,3,4-O-tetraacetylgalactose, 1,2,3,4-O-tetraacetylmannose, 1-O-n-octyl-2,3,4-O-tribenzoylglucoside, 1-O-n-octyl-2,3,4-O-trithiobenzoylglucoside, 1-O-phenyl-2,3,4-O-tribenzoylglucoside, 1,3,4-O-tribenzoyl,2-N-benzoylgalactosamine, 1,3,4-O-triacetyl,2-N-acetylgalactosamine, 1,3,4-O-triacetyl,2-N-acetylglucosamine, 1,3,4-O-tribenzoyl,2-N-trifluoroacetylgalactosamine, 1,3,4-O-tribenzoyl,2-N-trifluoroacetylglucosamine, heptabenzoylmelibiose, heptabenzoylgentiobiose and octabenzoylmaltotriose.

**85.** The saccharide derivative as claimed in claim 82, in which Q is represented by formula:

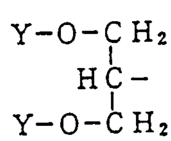
,

wherein Y represents a saccharide residue, and a saccharide of the saccharide residue represented by Y is a monosaccharide of which a hydroxyl group may be substituted by $C_{1-6}$ alkyl carbonyl.

**86.** The saccharide compound as claimed in claim 82, in which Q is represented by formula:

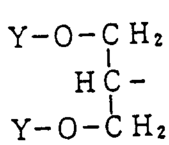
,

wherein Y represents a saccharide residue, and a saccharide of the saccharide residue represented by Y is galactose, mannose or glucose of which a hydroxyl group may be substituted by $C_{1-6}$ alkyl

carbonyl.

**87.** A saccharide derivative which is represented by formula:

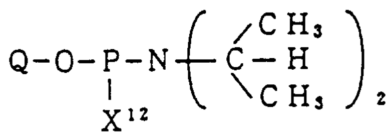

wherein Q is Y or represented by formula

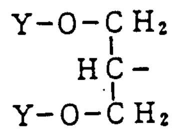

in which Y represents a saccharide residue; and $X^{12}$ represents $C_{1-5}$ alkyl, $C_{1-5}$ alkoxy, $C_{1-5}$ monoalkylamino or an aromatic ring group which may be substituted or unsubstituted.

**88.** The saccharide derivative as claimed in claim 87, in which Q is Y, and a saccharide of the saccharide residue represented by Y is (1) galactose, mannose, melibiose, gentiobiose or isomaltotriose wherein a hydroxyl group thereof may be substituted by a $C_{1-5}$ alkylcarbonyl or benzoyl group, (2) glucoside or thioglucoside wherein a hydroxyl group thereof is substituted by a $C_{1-5}$ alkylcarbonyl, benzoyl or a $C_{1-20}$ alkyl, or (3) glucosamine or galactosamine, or a hydroxyl group thereof is substituted by $C_{1-5}$ alkylcarbonyl or benzoyl which may be substituted by halogens.

**89.** The saccharide compound as claimed in claim 87, in which Q is Y, and a saccharide of the saccharide residue represented by Y is selected from the group consisting of 1,2,3,4-O-tetraacetylgalactose, 1,2,3,4-O-tetraacetylmannose, 1-O-n-octyl-2,3,4-O-tribenzoylglucoside, 1-O-n-octyl-2,3,4-O-trithiobenzoylglucoside, 1-O-phenyl-2,3,4-O-tribenzoylglucoside, 1,3,4-O-tribenzoyl,2-N-benzoylgalactosamine, 1,3,4-O-triacetyl,2-N-acetylgalactosamine, 1,3,4-O-triacetyl,2-N-acetylglucosamine, 1,3,4-O-tribenzoyl,2-N-trifluoroacetylgalactosamine, 1,3,4-O-tribenzoyl,2-N-trifluoroacetylglucosamine, heptabenzoylmelibiose, heptabenzoylgentiobiose and octabenzoylmaltotriose.

**90.** The saccharide derivative as claimed in claim 87, in which Q is represented by formula:

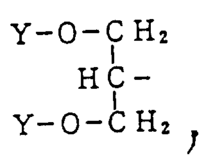

wherein Y represents a saccharide residue, and a saccharide of the saccharide residue represented by Y is a monosaccharide of which a hydroxyl group may be substituted by $C_{1-6}$ alkylcarbonyl.

**91.** The saccharide derivative as claimed in claim 87, in which
Q is represented by formula:

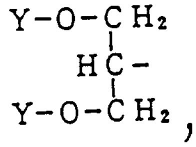

wherein Y represents a saccharide residue, and a saccharide of the saccharide residue represented by Y is galactose, mannose or glucose of which a hydroxyl group may be substituted by $C_{1-6}$ alkylcarbonyl.

**92.** The saccharide derivative as claimed in claim 87, in which $X^{12}$ is $C_{1-5}$ alkyl; $C_{1-5}$ alkoxy; $C_{1-5}$ monoalkylamino; or phenyl which may be substituted by about 1 to about 5 substituent groups selected from the group consisting of halogen, $C_{1-10}$ alkylcarbonyl, OH, nitro, $C_{1-10}$ alkyl, $C_{1-10}$ alkoxy, phenyl and naphthyl.

**93.** A nucleotide compound which is represented by formula:

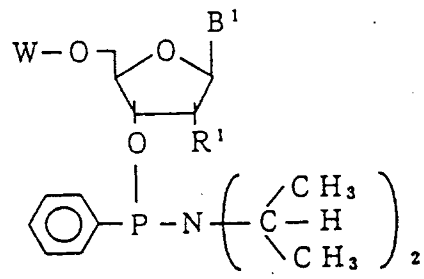

wherein W represents hydrogen or a protective group; $B^1$ represents a nucleic acid residue, $R^1$ represents hydrogen, OH, halogen, $C_{1-5}$ alkoxy or $C_{1-5}$ alkoxyalkyloxy.

**94.** The nucleotide compound as claimed in claim 93, in which W is hydrogen or represented by formula

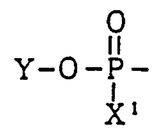

or

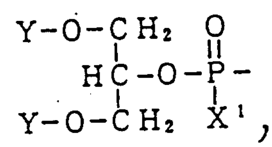

wherein Y represents a saccharide residue; $X^1$ represents OH, SH, $C_{1-5}$ alkyl, $C_{1-5}$ alkoxy, $C_{1-5}$ monoalkylamino or an aromatic ring group which may be substituted or unsubstituted.

**95.** The nucleotide compound as claimed in claim 93, in which W is hydrogen or represented by formula

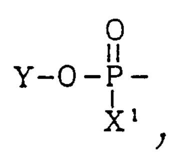

and a saccharide of the saccharide residue represented by Y is (1) galactose, mannose, melibiose, gentiobiose or isomaltotriose wherein a hydroxyl group thereof may be substituted by a $C_{1-5}$ alkylcarbonyl or a benzoyl group, (2) glucoside or thioglucoside wherein a hydroxyl group other than a hemiacetal hydroxyl group thereof may be substituted by $C_{1-5}$ alkylcarbonyl, benzoyl or $C_{1-20}$ alkyl, or (3) glucosamine or galactosamine wherein a hydroxyl group thereof may be substituted by a $C_{1-5}$ alkylcarbonyl or a benzoyl which may be substituted by halogens.

**96.** The nucleotlde compound as claimed in claim 93, in which W is hydrogen or represented by formula

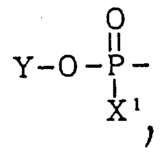

and a saccharide of the saccharide residue represented by Y is selected from the group consisting of 1,2,3,4-O-tetraacetylgalactose, 1,2,3,4-O-tetraacetylmannose, 1-O-n-octyl-2,3,4-O-tribenzoylglucoside, 1-O-n-octyl-2,3,4-O-trithiobenzoylglucoside, 1-O-phenyl-2,3,4-O-tribenzoylglucoside, 1,3,4-O-tribenzoyl,2-N-benzoylgalactosamine, 1,3,4-O-triacetyl,2-N-acetylgalactosamine, 1,3,4-O-triacetyl,2-N-acetylglucosamine, 1,3,4-O-tribenzoyl,2-N-trifluoroacetylgalactosamine, 1,3,4-O-tribenzoyl,2-N-trifluoroacetylglucosamine, heptabenzoylmelibiose, heptabenzoylgentiobiose and octabenzoylmaltotriose.

**97.** The oligonucleotide compound as claimed in claim 93, in which W is hydrogen or represented by formula:

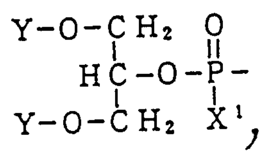

wherein a saccharide of the saccharide residue represented by Y is a monosaccharide of which an alcoholic hydroxyl group may be substituted by $C_{1-5}$ alkylcarbonyl.

**98.** The saccharide derivative as claimed in 93, in which W is hydrogen or represented by formula:

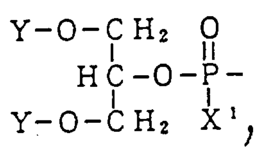

wherein a saccharide of the saccharide residue represented by Y is galactose, mannose or glucose of which an alcoholic hydroxyl group may be substituted by $C_{1-5}$ alkylcarbonyl.

**99.** A saccharide derivative represented by the following formula:

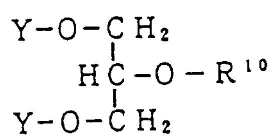

wherein Y represents a saccharide residue and $R^{10}$ represents hydrogen or a benzyl group.

**100.** A use of the oligonucleotide compound or the salt thereof claimed in claim 1 for manufacturing a composition for inhibiting a gene expression.

**101.** The use of the oligonucleotide compound or the salt thereof as claimed in claim 100, which is a composition for inhibiting expression of a gene contributing to the development of a malignant tumor, a virus disease or an inflammation.

**102.** The use of the oligonucleotide compound or the salt thereof as claimed in claim 100, in which inhibition of the gene expression is (1) inhibition of transcription from DNA to pre mRNA, (2) inhibition of splicing from pre mRNA to mature mRNA, or (3) inhibition of translation from mature mRNA to a protein.

**103.** The use of the oligonucleotide compound or the salt thereof as claimed in claim 101, in which the gene contributing to the development of the malignant tumor is a gene selected from the group consisting of src, fps, yes, ros, myb, myc, erb, rel, mos, abl, ras, fos, fes, fms, sis, raf, neu and p53, the gene contributing to the development of the virus disease is a gene selected from the group consisting of HIV, an influenza virus, a herpes virus, a papilloma virus, a poliovirus, a picornavirus and an adenovirus, and the gene contributing to the development of the inflammation is a gene selected from the group consisting of ICAM-1, ELAM-1 and VLAM.

**104.** The use of the oligonucleotide compound or the salt thereof as claimed in claim 100, in which the gene is ICAM-1.

**105.** A use of the oligonucleotide compound or the salt thereof as claimed in any one of claim 1 to 75 for manufacturing a composition for inhibiting a reangiogenesis after PTCA (percutaneous transluminal coronary angioplasty).

**106.** A use of the saccharide derivative as claimed in claim 85 for manufacturing the oligonucleotide compounds or the salts thereof as claimed in claim 2.

**107.** A use of the saccharide derivative as claimed in claim 87 for manufacturing the oligonucleotide compounds or the salts thereof as claimed in claim 2.

**108.** A use of the saccharide derivative as claimed in claim 93 for manufacturing the oligonucleotide compounds or the salts thereof as claimed in claim 38.

**109.** A use of the saccharide derivative as claimed in claim 99 for manufacturing the saccharide derivative or the salts thereof wherein Q represents a formula

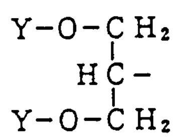

as claimed in claim 82.